# EUROPEAN PATENT APPLICATION

(11) **EP 4 234 544 A1**
(43) Date of publication of application: **30.08.2023**
(21) Application number: 21882143.7
(22) Date of filing: 22.10.2021
(51) Int. Cl.: C07D 401/02, C07D 401/14, A61K 31/445, A61K 31/454, A61P 19/04, A61P 9/10

(54) **FUSED RING-CONTAINING COMPOUND, APPLICATION THEREOF, AND COMPOSITION CONTAINING SAME**

(30) Priority: 23.10.2020 CN 202011145113
(71) Applicant: Generos Biopharma Ltd., George Town, Grand Cayman KY1-1206 (KY)
(72) Inventor: LU, Cenbin, Hangzhou, Zhejiang 310018 (CN); XU, Xiao, Hangzhou, Zhejiang 310018 (CN); CAI, Zehong, Hangzhou, Zhejiang 310018 (CN); FU, Xinyuan, Hangzhou, Zhejiang 310018 (CN); LUFEI, Chengchen, Hangzhou, Zhejiang 310018 (CN); LIU, Xinyu, Hangzhou, Zhejiang 310018 (CN); MA, Zhongnan, Hangzhou, Zhejiang 310018 (CN); RONG, Frank, Hangzhou, Zhejiang 310018 (CN); ZHOU, Yi, Hangzhou, Zhejiang 310018 (CN)
(74) Representative: Impact Intellectual Property LLP
(86) International application number: PCT/CN2021/125660
(87) International publication number: WO 2022/083731

(57) **Abstract**

A fused ring-containing compound, an application thereof, and a composition containing same, and specifically, a fused ring-containing compound represented by formula II, a pharmaceutically acceptable salt or solvate thereof, or a solvate of said pharmaceutically acceptable salt. The compound has good STATS inhibitory activity.

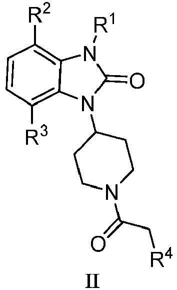

## Description

The present application claims priority to the Chinese Patent Application 202011145113.2 filed on Oct. 23, 2020, which is incorporated herein by reference in its entirety.

### TECHNICAL FIELD

The present invention provides a fused ring-containing compound, use thereof and a composition comprising same.

### BACKGROUND

Rheumatoid arthritis is a chronic, systemic autoimmune disease characterized by inflammation of the synovial membrane in the joints. The incidence rate is about 1% in total adults. It mainly manifests itself in symmetric, multiple, recurrent arthritis, to which the small joints of the hands and feet are most susceptible. During an early stage or acute episode, the involved joints are mostly red, swollen, warm and painful and have a limited range of motion. In an advanced stage, the involved joints may be damaged, stiff, deformed and dysfunctional, and osteoporosis and skeletal muscle atrophy develop. In the whole course, the disease may be accompanied by fever, anemia, weight loss and lesions such as vasculitis and subcutaneous nodules, and various organs of the body may also be involved. It is very likely to cause disability and it significantly increases mortality among patients. The pathogenesis of rheumatoid arthritis is not yet completely known in modern medicine, and there is no cure for it as yet. Patients around the world are greatly suffering from it, physically and financially.

Over the last two decades, there has been a deeper understanding of the pathogenesis of arthritis: over-activation of immune cells and elevated levels of inflammatory cytokines are considered to have caused the conditions in the joints and other parts of the body. Some major advances have been made in the treatment, and a range of drugs against rheumatic factors have been developed, including methotrexate, anti-tumor necrosis factor (TNFα) antibodies, interleukin (IL) 1, interleukin-6 antibodies and JAK kinase inhibitors. These antirheumatic drugs can control the progression of the disease to some extent and improve patients' quality of life. However, the effect does not last long, and these drugs are effective only in a small fraction of the patients and come with side effects with varying degrees of severity. Methotrexate, for example, as an anticancer drug, is significantly non-specifically cytotoxic per se and hence very harmful to the gastrointestinal tract, the liver and the kidney, and due to insufficient efficacy or severe adverse reactions, about half of the patients stop taking the drug within one year of beginning the treatment. Anti-TNFα antibodies are effective only in 30-40% of the patients and also come with risks of random infection and cancer. Moreover, they have been reported to increase the likelihood of developing multiple sclerosis and, after long-term use, to produce neutralizing antibodies that result in loss of efficacy (Ann Rheum Dis. 2014 Mar; 73(3): 529-35). There is an urgent need to discover a new pathogenesis and develop better products that target it.

The pathogenesis of rheumatic arthritis shows severely dysregulated innate and adaptive immune responses leading to chronic inflammation. Helper T cells play a crucial role in both the development and progression of arthritis. When they fail to function properly, T lymphocytes are constantly activated and abnormally proliferate, and at the same time, fibroblasts in joint tissues also proliferate correspondingly, causing the apparent condition of arthritis. The JAK-STAT pathway plays an important role in regulating T cells and other immune cells, and the STATS factor in it plays a key role in both the development and the maturation of various T cell subtypes.

Recently, our team has discovered a new helper T cell named Th-GM. The cell plays a driving role in many inflammatory diseases and is a new pathogenesis of inflammation. The Th-GM cell is characterized by having a STATS signaling pathway activated by interleukin 7 and causing high expression of the downstream colony-stimulating factor (GM-CSF) and interleukin 3. Furthermore, RNA sequencing revealed that the Th-GM cell shows a distinctive gene expression profile-it is completely different from the known Th-1 and Th-17 helper T cells. Genetic experiments also show that knocking out the STATS gene can significantly reduce inflammation of the nervous system and arthritis in mice with multiple sclerosis (references: CN107002037A and PCT/US2018/031217). Due to the key role of STATS in regulating Th-GM cells, its role in other inflammatory cells cannot be ruled out. We believe that STATS inhibitors will be a very promising candidate for the treatment of arthritis.

The design and synthesis of a range of novel STATS inhibitors are illustrated herein.

### SUMMARY

The present invention aims to address the technical problem that the existing STATS inhibitors are somewhat similar in structure and to this end provides a fused ring-containing compound, use thereof and a composition comprising same. The compound has a novel structure and has good inhibitory activity against STATS.

The present invention provides a fused ring-containing compound represented by formula **II,** a pharmaceutically acceptable salt thereof, a solvate thereof or a solvate of the pharmaceutically acceptable salt thereof, wherein 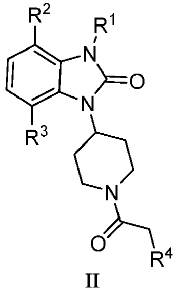
R¹ is hydrogen or 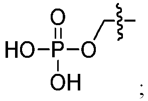
R² is hydrogen or chlorine;
R³ is hydrogen, cyano, nitro, acetyl, trifluoromethanesulfonyl, or C₁-C₃ alkyl substituted with one or more halogens;
R⁴ is 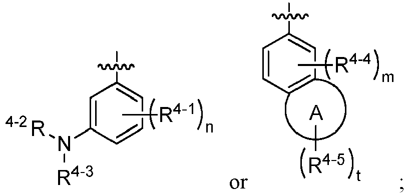
n is 0, 1, 2, 3 or 4;
R⁴⁻¹ is independently cyano, a halogen, C₁-C₃ alkyl, or C₁-C₃ alkyl substituted with one or more halogens;
R⁴⁻² and R⁴⁻³ are independently C₁-C₃ alkyl;
m is 0, 1, 2, 3 or 4 (m represents the number of substituents on the benzene ring);
R⁴⁻⁴ is independently cyano, hydroxy, a halogen, C₁-C₃ alkyl, C₁-C₃ alkoxy, or C₁-C₃ alkyl substituted with one or more halogens;
ring A is 5- or 6-membered heteroaryl having 1, 2 or 3 heteroatoms selected from one or more of N, O and S;
t is 0, 1, 2, 3 or 4 (t represents the number of substituents on ring A);
R⁴⁻⁵ is independently cyano, hydroxy, a halogen, C₁-C₃ alkyl, C₁-C₃ alkoxy, or C₁-C₃ alkyl substituted with one or more halogens;
however, the fused ring-containing compound represented by formula **II** is not the following compounds: 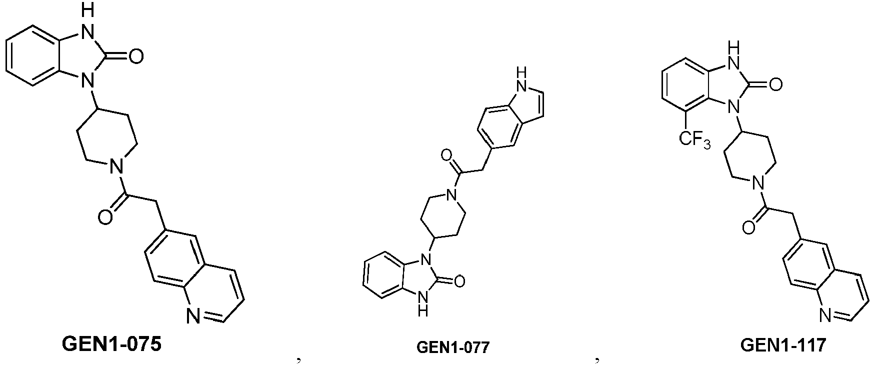 and

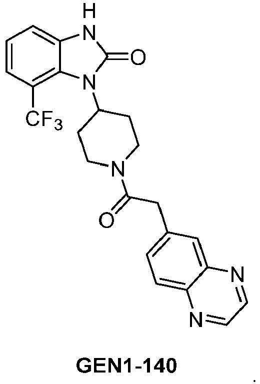

In one embodiment, in the fused ring-containing compound represented by formula **II,** the pharmaceutically acceptable salt thereof, the solvate thereof, or the solvate of the pharmaceutically acceptable salt thereof, certain groups may be defined as follows, and groups not involved in the embodiment may be defined as described in any one of the above embodiments (hereinafter referred to as "in one embodiment" for the sake of simplicity): R² is hydrogen.

In one embodiment, R¹ is

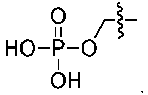

In one embodiment, R³ is C₁-C₃ alkyl substituted with one or more halogens.

In one embodiment, n is 0.

In one embodiment, R⁴ is

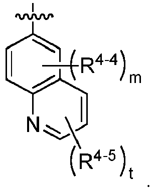

In one embodiment, m is 0 or 1.

In one embodiment, R⁴⁻⁴ is independently a halogen or C₁-C₃ alkyl.

In one embodiment, t is 0 or 1.

In one embodiment, R⁴⁻⁵ is independently cyano, a halogen, C₁-C₃ alkyl, C₁-C₃ alkoxy, or C₁-C₃ alkyl substituted with one or more halogens.

In one embodiment, R⁴⁻⁵ is independently a halogen.

In one embodiment, when the R¹ is hydrogen, m + t is greater than or equal to 1.

In one embodiment, when the R¹ is hydrogen, m + t = 1.

In one embodiment, R¹ is hydrogen or 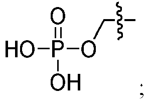
R² is hydrogen;
R³ is C₁-C₃ alkyl substituted with one or more halogens;
R⁴ is 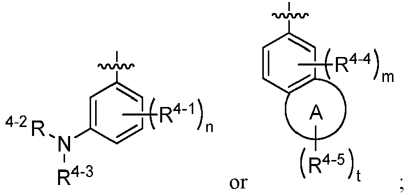
n is 0;
R⁴⁻² and R⁴⁻³ are independently C₁-C₃ alkyl;
m is 0 or 1;
R⁴⁻⁴ is a halogen or C₁-C₃ alkyl;
ring A is 5- or 6-membered heteroaryl having 1, 2 or 3 heteroatoms selected from one or more of N, O and S; t is 0 or 1;
R⁴⁻⁵ is cyano, a halogen, C₁-C₃ alkyl, C₁-C₃ alkoxy, or C₁-C₃ alkyl substituted with one or more halogens; however, the fused ring-containing compound represented by formula II is not the following compounds:

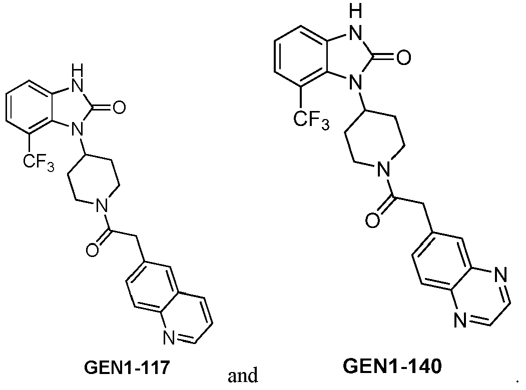

In one embodiment, R¹ is hydrogen or 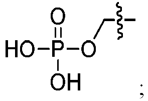
R² is hydrogen;
R³ is C₁-C₃ alkyl substituted with one or more halogens;
R⁴ is 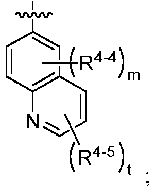
m is 0 or 1;
R⁴⁻⁴ is a halogen or C₁-C₃ alkyl;
t is 0 or 1; R⁴⁻⁵ is a halogen; however, the fused ring-containing compound represented by formula II is not

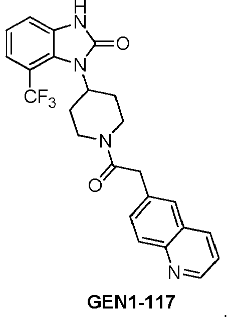

In one embodiment, R¹ is 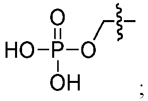
R² is hydrogen;
R³ is C₁-C₃ alkyl substituted with one or more halogens;
R⁴ is 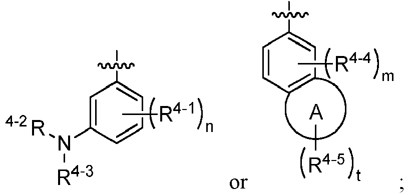
n is 0;
R⁴⁻² and R⁴⁻³ are independently C₁-C₃ alkyl;
m is 0 or 1;
R⁴⁻⁴ is a halogen or C₁-C₃ alkyl;
ring A is 5- or 6-membered heteroaryl having 1, 2 or 3 heteroatoms selected from one or more of N, O and S; t is 0 or 1;
R⁴⁻⁵ is cyano, a halogen, C₁-C₃ alkyl, C₁-C₃ alkoxy, or C₁-C₃ alkyl substituted with one or more halogens.

In one embodiment, R¹ is 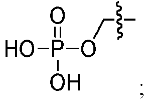
R² is hydrogen;
R³ is C₁-C₃ alkyl substituted with one or more halogens;
R⁴ is 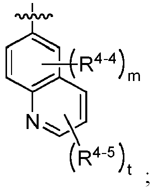
m is 0 or 1;
R⁴⁻⁴ is a halogen or C₁-C₃ alkyl;
t is 0 or 1;
R⁴⁻⁵ is a halogen.

In one embodiment, when the R³ is C₁-C₃ alkyl substituted with one or more halogens, the "more" may mean 2 or 3.

In one embodiment, when the R³ is C₁-C₃ alkyl substituted with one or more halogens, the halogen may be fluorine or chlorine, and may also be fluorine.

In one embodiment, when the R³ is C₁-C₃ alkyl substituted with one or more halogens, the C₁-C₃ alkyl may be methyl, ethyl, n-propyl or isopropyl, and may also be methyl.

In one embodiment, when the R³ is C₁-C₃ alkyl substituted with multiple halogens, the "C₁-C₃ alkyl substituted with multiple halogens" may be trifluoromethyl.

In one embodiment, when the R⁴⁻² is C₁-C₃ alkyl, the C₁-C₃ alkyl may be methyl, ethyl, n-propyl or isopropyl, and may also be methyl.

In one embodiment, when the R⁴⁻³ is C₁-C₃ alkyl, the C₁-C₃ alkyl may be methyl, ethyl, n-propyl or isopropyl, and may also be methyl.

In one embodiment, when the R⁴ is 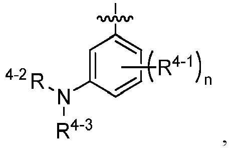 the 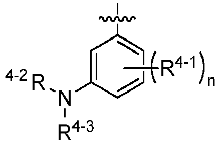 may be

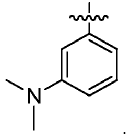

In one embodiment, when the R⁴⁻¹ is a halogen, the halogen may be fluorine or chlorine, and may also be chlorine.

In one embodiment, when the R⁴⁻¹ is C₁-C₃ alkyl, the C₁-C₃ alkyl may be methyl, ethyl, n-propyl or isopropyl, and may also be methyl.

In one embodiment, when the R⁴⁻¹ is C₁-C₃ alkyl substituted with one or more halogens, the "more" may mean 2 or 3.

In one embodiment, when the R⁴⁻¹ is C₁-C₃ alkyl substituted with one or more halogens, the halogen may be fluorine or chlorine, and may also be fluorine.

In one embodiment, when the R⁴⁻¹ is C₁-C₃ alkyl substituted with one or more halogens, the C₁-C₃ alkyl may be methyl, ethyl, n-propyl or isopropyl, and may also be methyl.

In one embodiment, when the R⁴⁻¹ is C₁-C₃ alkyl substituted with multiple halogens, the "C₁-C₃ alkyl substituted with multiple halogens" may be trifluoromethyl.

In one embodiment, when the R⁴⁻⁴ is a halogen, the halogen may be fluorine or chlorine, and may also be chlorine.

In one embodiment, when the R⁴⁻⁴ is C₁-C₃ alkyl, the C₁-C₃ alkyl may be methyl, ethyl, n-propyl or isopropyl, and may also be methyl.

In one embodiment, when the R⁴⁻⁴ is C₁-C₃ alkyl substituted with one or more halogens, the "more" may mean 2 or 3.

In one embodiment, when the R⁴⁻⁴ is C₁-C₃ alkyl substituted with one or more halogens, the halogen may be fluorine or chlorine, and may also be fluorine.

In one embodiment, when the R⁴⁻⁴ is C₁-C₃ alkyl substituted with one or more halogens, the C₁-C₃ alkyl may be methyl, ethyl, n-propyl or isopropyl, and may also be methyl.

In one embodiment, when the R⁴⁻⁴ is C₁-C₃ alkyl substituted with multiple halogens, the "C₁-C₃ alkyl substituted with multiple halogens" may be trifluoromethyl.

In one embodiment, when the ring A is 5- or 6-membered heteroaryl having 1, 2 or 3 heteroatoms selected from N, O and S, the 5- or 6-membered heteroaryl having 1, 2 or 3 heteroatoms selected from N, O and S may be a thiophene ring, a pyrrole ring, a pyridine ring or a pyrazine ring.

In one embodiment, when the R⁴ is 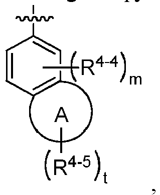 the 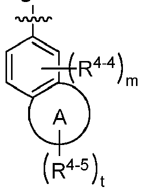 may be

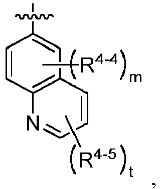

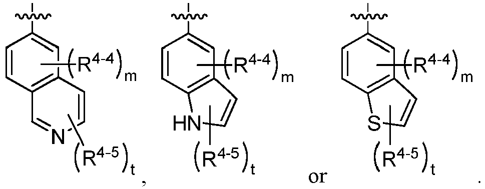

In one embodiment, when the R⁴⁻⁵ is a halogen, the halogen may be fluorine or chlorine, and may also be chlorine.

In one embodiment, when the R⁴⁻⁵ is C₁-C₃ alkyl, the C₁-C₃ alkyl may be methyl, ethyl, n-propyl or isopropyl, and may also be methyl.

In one embodiment, when the R⁴⁻⁵ is C₁-C₃ alkoxy, the C₁-C₃ alkoxy may be methoxy, ethoxy, n-propoxy or isopropoxy, and may also be methoxy.

In one embodiment, when the R⁴⁻⁵ is C₁-C₃ alkyl substituted with one or more halogens, the "more" may mean 2 or 3.

In one embodiment, when the R⁴⁻⁵ is C₁-C₃ alkyl substituted with one or more halogens, the halogen may be fluorine or chlorine, and may also be fluorine.

In one embodiment, when the R⁴⁻⁵ is C₁-C₃ alkyl substituted with one or more halogens, the C₁-C₃ alkyl may be methyl, ethyl, n-propyl or isopropyl, and may also be methyl.

In one embodiment, when the R⁴⁻⁵ is C₁-C₃ alkyl substituted with multiple halogens, the "C₁-C₃ alkyl substituted with multiple halogens" may be trifluoromethyl.

In one embodiment, when the R¹ is 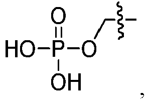 the pharmaceutically acceptable salt may be a sodium salt; that is, one or two hydrogens of 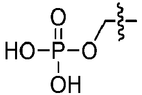 are replaced with sodium ions.

In one embodiment, the fused ring-containing compound represented by formula II is any one of the following compounds:

| **Compound No.** | **Structural formula** |
|---|---|
| GEN1-284 | 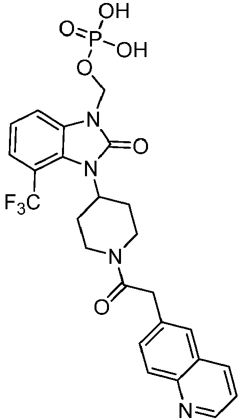 |
| GEN1-284 salt | 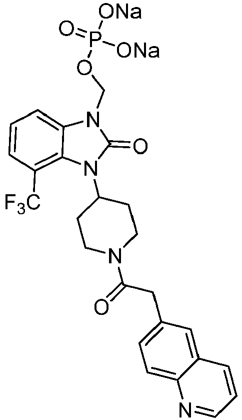 |
| GEN1-295 | 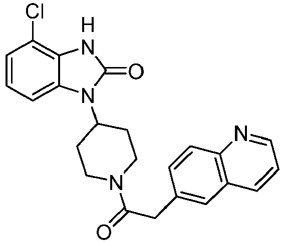 |
| GEN1-296 | 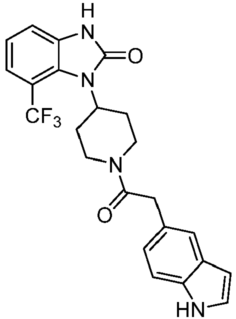 |
| GEN1-297 | 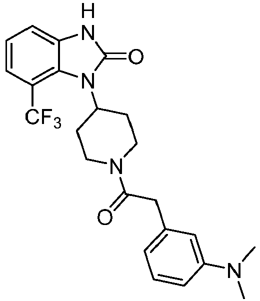 |
| GEN1-298 | 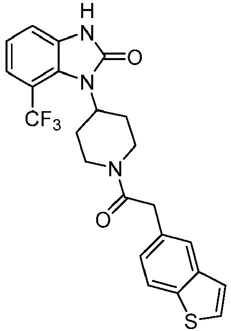 |
| GEN1-299 | 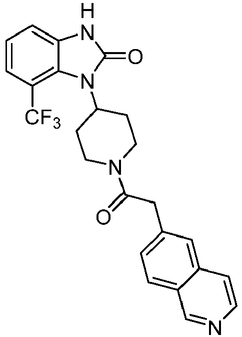 |
| GEN1-300 | 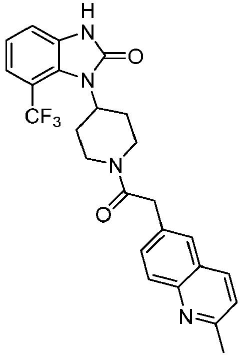 |
| GEN1-301 | 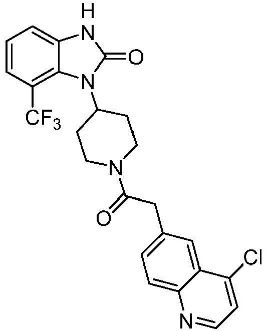 |
| GEN1-302 | 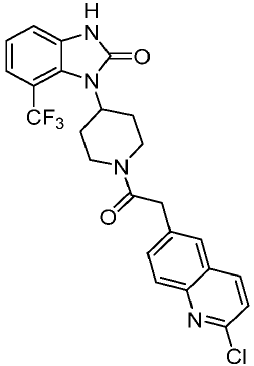 |
| GEN1-303 | 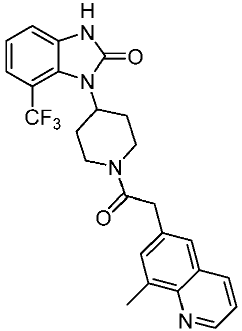 |
| GEN1-304 | 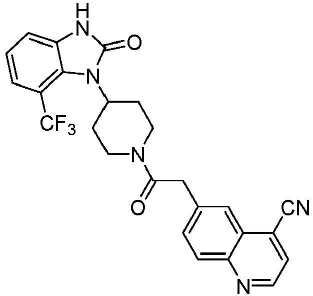 |
| GEN1-305 | 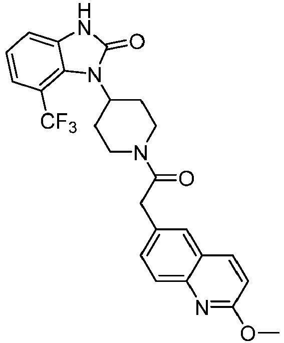 |
| GEN1-306 | 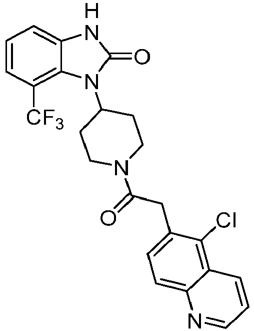 |
| GEN1-307 | 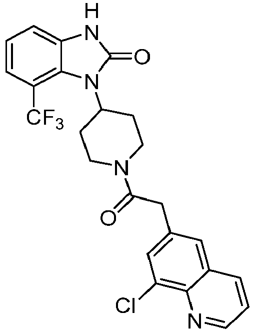 |
| GEN1-308 | 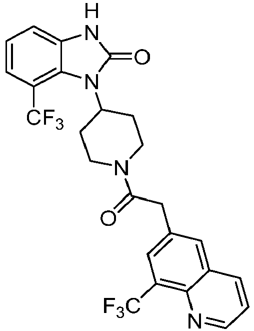 |
| GEN1-309 | 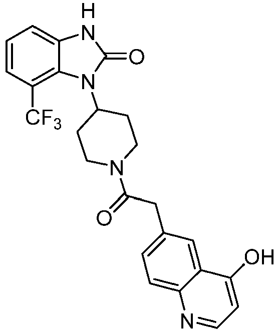 |
| GEN1-310 | 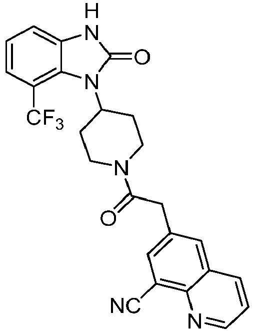 |
| GEN1-311 | 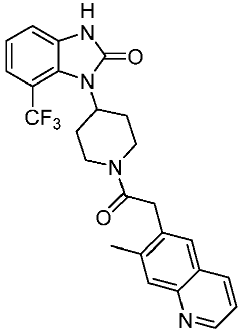 |
| GEN1-312 | 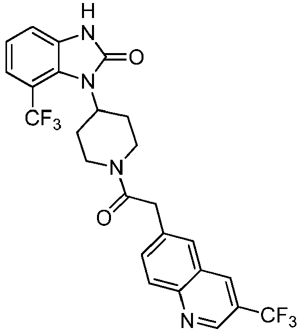 |
| GEN1-313 | 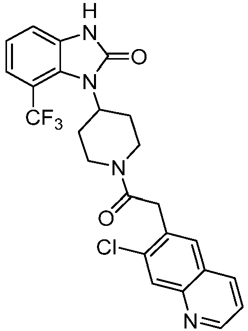 |

In one embodiment, the pharmaceutically acceptable salt of the fused ring-containing compound represented by formula **II** is the following compound:

| **Compound No.** | **Structural formula** |
|---|---|
| GEN1-284 salt | 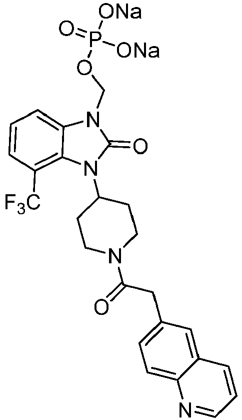 |

The present invention also provides a pharmaceutical composition comprising a substance **Y** and a pharmaceutical adjuvant material;
the substance **Y** is the fused ring-containing compound represented by formula **II,** the pharmaceutically acceptable salt thereof, the solvate thereof or the solvate of the pharmaceutically acceptable salt thereof described above.

The present invention further provides use of the substance **Y** in the preparation of a STATS inhibitor or a TNF-α generation inhibitor; the STATS inhibitor is for use *in vitro,* and the TNF-α generation inhibitor is for use *in vitro;*
the substance **Y** is the fused ring-containing compound represented by formula **II,** the pharmaceutically acceptable salt thereof, the solvate thereof or the solvate of the pharmaceutically acceptable salt thereof described above.

The present invention further provides use of the substance **Y** in the preparation of a medicament;
the substance **Y** is the fused ring-containing compound represented by formula **II**, the pharmaceutically acceptable salt thereof, the solvate thereof or the solvate of the pharmaceutically acceptable salt thereof described above.

In the use, the medicament may be:
(1) a medicament for treating and/or preventing a STAT5-associated disease; or,
(2) a medicament for treating and/or preventing any one of the following diseases: arthritis, atherosclerosis or psoriasis.

In the use, the STAT5-associated disease may be arthritis, atherosclerosis or psoriasis.

In the use, the arthritis may be rheumatoid arthritis, adjuvant arthritis or collagen-induced arthritis.

In the use, the psoriasis may be imiquimod-induced psoriasis.

The present invention further provides a method for treating and/or preventing a STAT5-associated disease, the method comprising administering to a patient a therapeutically effective amount of the substance **Y;**
the substance **Y** is the fused ring-containing compound represented by formula **II,** the pharmaceutically acceptable salt thereof, the solvate thereof or the solvate of the pharmaceutically acceptable salt thereof described above.

In the use, the STAT5-associated disease may be arthritis, atherosclerosis or psoriasis.

In the method, the arthritis may be rheumatoid arthritis, adjuvant arthritis or collagen-induced arthritis.

In the method, the psoriasis may be imiquimod-induced psoriasis.

The present invention further provides a method for treating and/or preventing arthritis, atherosclerosis or psoriasis, the method comprising administering to a patient a therapeutically effective amount of the substance **Y;**
the substance **Y** is the fused ring-containing compound represented by formula **II,** the pharmaceutically acceptable salt thereof, the solvate thereof or the solvate of the pharmaceutically acceptable salt thereof described above.

In the method, the arthritis may be rheumatoid arthritis, adjuvant arthritis or collagen-induced arthritis.

In the method, the psoriasis may be imiquimod-induced psoriasis.

Unless otherwise specified, the terms used in the present invention have the following meanings: The term "more" or "multiple" means 2, 3, 4 or 5.

The term "pharmaceutically acceptable" means that the salt, solvent, adjuvant material, etc., are generally non-toxic, safe, and suitable for use in patients.

The term "pharmaceutically acceptable salt" refers to a salt prepared with the compound of the present invention and a relatively non-toxic, pharmaceutically acceptable acid or base. When the compound of the present invention contains a relatively acidic functional group, a base addition salt can be obtained in a pure solution or a suitable inert solvent by contacting the free form of such a compound with a sufficient amount of a pharmaceutically acceptable base. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compound of the present invention contains a relatively basic functional group, an acid addition salt can be obtained in a pure solution or a suitable inert solvent by contacting the free form of such a compound with a sufficient amount of a pharmaceutically acceptable acid. The pharmaceutically acceptable acid includes inorganic acids including, but not limited to: hydrochloric acid, hydrobromic acid, hydroiodic acid, nitric acid, carbonic acid, phosphoric acid, phosphorous acid, sulfuric acid, and the like. The pharmaceutically acceptable acid includes organic acids including, but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, sugar acid, formic acid, ethanesulfonic acid, pamoic acid (i.e., 4,4'-methylene-bis(3-hydroxy-2-naphthoic acid)), amino acids (e.g., glutamic acid and arginine), and the like. When the compound of the present invention contains a relatively acidic functional group and a relatively basic functional group, it may be converted to a base addition salt or an acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

When any variable (e.g., R⁴⁻¹) occurs multiple times in the definition of a compound, the definition of the variable at each occurrence is independent of the definition at other occurrences, and the meanings are independent of each other. Thus, if a group is substituted with 1, 2 or 3 R⁴⁻¹ groups, it means that the group may be substituted with at most 3 R⁴⁻¹, and the definition of R⁴⁻¹ at that occurrence is independent of the definition of R⁴⁻¹ at other occurrences. In addition, a combination of substituents and/or variables is permissible only if the combination results in a stable compound.

The term "solvate" refers to a substance formed upon crystallization of a compound with a solvent (including, but not limited to: water, methanol, ethanol, and the like). Solvates are divided into stoichiometric and non-stoichiometric solvates.

The term "solvate of the pharmaceutically acceptable salt" refers to a substance formed by combining a compound and a pharmaceutically acceptable (relatively non-toxic, safe, suitable for use in patients) acid or base with a solvent (including, but not limited to: water, methanol, ethanol, and the like), wherein the pharmaceutically acceptable salt has the same meaning as the term "pharmaceutically acceptable salt" described above; the solvent is stoichiometric or non-stoichiometric. Solvates of the pharmaceutically acceptable salt include, but are not limited to, hydrochloride monohydrate.

The term "halogen" refers to fluorine, chlorine, bromine or iodine.

The term "alkyl" refers to a linear or branched alkyl group having a specified number of carbon atoms. Examples of alkyl include methyl, ethyl, n-propyl, isopropyl, and the like.

The term "alkoxy" refers to the group -O-R^{X}, wherein R^{X} is alkyl as defined above.

The term "heteroaryl" refers to an aromatic group containing a heteroatom, preferably an aromatic 5-6 membered monocyclic ring containing 1, 2 or 3 heteroatoms independently selected from nitrogen, oxygen and sulfur, e.g., furanyl, pyridinyl, pyridazinyl, pyrimidinyl, pyrazinyl, thienyl, and the like.

The term "pharmaceutical adjuvant material" refers to excipients and additives used in the manufacture of a pharmaceutical product and in the preparation of a pharmaceutical formulation, and refers to all the substances, other than the active ingredient, included in a pharmaceutical preparation. See Pharmacopoeia of the People's Republic of China (2015 Edition), Volume IV; or Handbook of Pharmaceutical Excipients (Raymond C Rowe, 2009 Sixth Edition).

The term "treating" refers to therapeutic therapy. In reference to a particular condition, treating means: (1) to ameliorate the disease or one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition, or one or more of the symptoms, effects or side effects associated with the condition or treatment thereof, or (4) to slow the progression of the condition or one or more of the biological manifestations of the condition.

The term "preventing" refers to a reduction in risk of acquiring or developing a disease or disorder.

The term "STATS inhibitor" refers to a substance that can ultimately specifically inhibit STATS activity in an *in vivo* environment.

The term "patient" refers to any animal, preferably a mammal, and most preferably a human, that is about to receive or has received administration of the compound, according to the embodiments of the present invention. The term "mammal" includes any mammal. Examples of mammals include, but are not limited to, cows, horses, sheep, pigs, cats, dogs, mice, rats, domestic rabbits, guinea pigs, monkeys, humans, and the like; humans are most preferred.

The term "therapeutically effective amount" refers to an amount of a compound that, when administered to a patient, is sufficient to effectively treat the disease or condition described herein. "Therapeutically effective amount" varies depending on the compound, the condition and the severity thereof, and the age of the patient to be treated, but can be adjusted as needed by those skilled in the art.

On the basis of not departing from the general knowledge in the art, the preferred conditions described above can be arbitrarily combined to form preferred embodiments of the present invention.

The reagents and starting materials used in the present invention are all commercially available.

The positive effect of the present invention is that: the compound has a novel structure and has good inhibitory activity against STATS.

### BRIEF DESCRIPTION OF THE DRAWINGS

FIG. 1 shows a representative image of arterial plaques in a mouse.
FIG. 2 shows a typical image of the left ventricular outflow tract of a mouse.

### DETAILED DESCRIPTION

The present invention is further explained below by description of examples, but the present invention is not limited to these examples anyway. In the following examples, experimental methods with no conditions specified follow conventional methods and use conventional conditions, or conditions are selected according to the instructions for use of the commercial products.

Starting Material Preparation Example 1: Synthesis of Common Intermediate

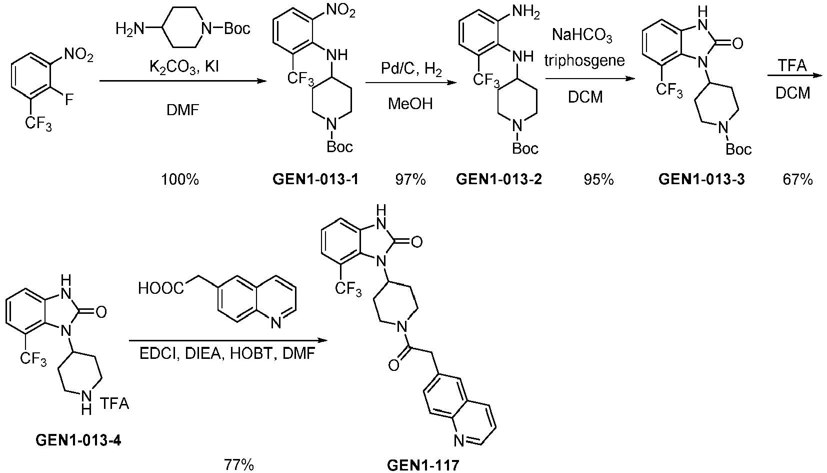

### Step 1. Synthesis of tert-butyl 4-((2-nitro-6-(trifluoromethyl)phenyl)amino)piperidine-1-carboxylate (GEN1-013-1)

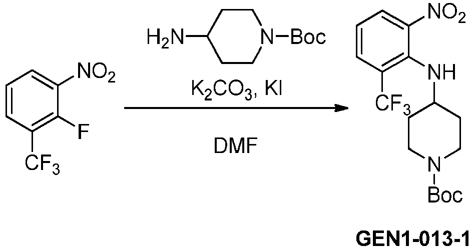

2-Fluoro-1-nitro-3-(trifluoromethyl)benzene (1.00 g, 4.80 mmol), tert-butyl 4-aminopiperidine-1-carboxylate (961 mg, 4.80 mmol) and K₂CO₃ (995 mg, 7.20 mmol) were suspended in DMF (10 mL), and the suspension was stirred at 85 °C for 2 h. After the reaction mixture was cooled to room temperature, water (50 mL) was added, and the mixture was extracted with EA (25 mL × 3). The combined organic layers were washed with water (25 mL) and brine (25 mL), dried over MgSO₄ and concentrated to give the desired compound (1.86 g, yield: 100%) as a yellow solid. ¹HNMR (400 MHz, CDCl₃): δ 8.08 (dd, *J=* 8.0, 1.2 Hz, 1H), 7.78 (dd, *J*= 7.6, 1.2 Hz, 1H), 6.96 (t, *J* = 8.0 Hz, 1H), 5.93 (d, *J =* 9.6 Hz, 1H), 4.10-3.93 (m, 2H), 3.46-3.36 (m, 1H), 2.84-2.78 (m, 2H), 1.94-1.91 (m, 2H), 1.45 (s, 9H), 1.39-1.29 (m, 2H).

### Step 2. Synthesis of tert-butyl 4-((2-amino-6-(trifluoromethyl)phenyl)amino)piperidine-1-carboxylate (GEN1-013-2)

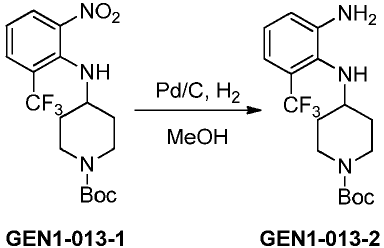

Pd/C (10%, 186 mg) was added under a N₂ atmosphere to a solution of tert-butyl 4-((2-nitro-6-(trifluoromethyl)phenyl)amino)piperidine-1-carboxylate (1.86 g, 4.78 mmol) in methanol (20 mL). The resulting mixture was degassed with H₂ and stirred overnight at 35 °C under a hydrogen atmosphere. The reaction mixture was filtered, and the filtrate was concentrated to give the desired compound (1.66 g, yield: 97%) as a brown oil. ¹HNMR (400 MHz, CDCl₃): δ 6.97 (dd, *J* = 7.6, 1.6 Hz, 1H), 6.92 (t, *J* = 8.0 Hz, 1H), 6.86 (dd, *J=* 8.0, 1.6 Hz, 1H), 4.11 (br s, 2H), 3.88 (s, 2H), 3.38-3.24 (m, 2H), 2.73-2.66 (m, 2H), 1.84-1.81 (m, 2H), 1.46 (s, 9H), 1.40-1.31 (m, 2H).

### Step 3. Synthesis of tert-butyl 4-(2-oxo-7-(trifluoromethyl)-2,3-dihydro-1H-benzo[d]imidazol-1-yl)piperidine-1-carboxylate (GEN1-013-3)

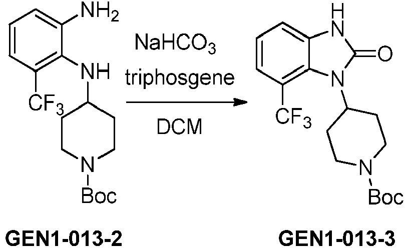

To a suspension of tert-butyl 4-((2-atnino-6-(trifluoromethyl)phenyl)atnino)piperidine-1-carboxylate (500 mg, 1.39 mmol) and NaHCOs (584 mg, 6.95 mmol) in DCM (25 mL) was added, at 7 °C, triphosgene (206 mg, 0.694 mmol). The mixture was stirred for 2 h. The reaction mixture was poured into water (50 mL) and extracted with DCM (25 mL × 3). The combined organic layers were washed with brine (25 mL), dried over MgSO₄ and concentrated to give the desired compound (510 mg, yield: 95%) as a white solid. ¹HNMR (400 MHz, CDCl₃): δ 10.16 (s, 1H), 7.40 (d, *J* = 8.0 Hz, 1H), 7.30 (d, *J* = 7.6 Hz, 1H), 7.14 (t, *J* = 8.0 Hz, 1H), 4.42-4.25 (m, 3H), 2.82-2.70 (m, 4H), 1.76-1.73 (m, 2H), 1.50 (s, 9H).

### Step 4. Synthesis of 1-(piperidin-4-yl)-7-(trifluoromethyl)-1H-benzo[d]imidazol-2(3H)-one (GEN1-013-4)

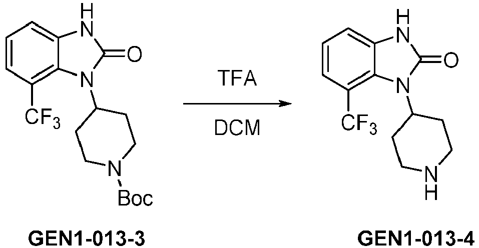

To DCM (6 mL) was added tert-butyl 4-(2-oxo-7-(trifluoromethyl)-2,3-dihydro-1H-benzo[d]imidazol-1-yl)piperidine-1-carboxylate (510 mg, 1.32 mmol), and TFA (2 mL) was added at room temperature. The mixture was stirred at room temperature overnight. The reaction mixture was concentrated, and the residue was dissolved in DCM (10 mL). To the solution was added Na₂CO₃ (sat.,10 mL), and the solution was stirred for 20 min. The mixture was filtered, and the filter cake was dried to give the desired compound (340 mg, yield: 67%) as a white solid. ¹HNMR (400 MHz, DMSO-*d*₆): δ 8.41 (br s, 1H), 7.37 (d, *J* = 8.0 Hz, 1H), 7.31 (d, *J* = 7.2 Hz, 1H), 7.18 (t, *J* = 8.0 Hz, 1H), 4.29-4.18 (m, 1H), 3.43-3.40 (m, 2H), 2.97-2.80 (m, 4H), 1.88-1.85 (m, 2H). MS (ESI) Rt = 1.50 min, *m*/*z* 286.3 [M+H]⁺, purity: 98% @ 254 nm, 100% @ 214 nm.

### Step 5. 1-(1-(2-(Quinolin-6-yl)acetyl)piperidin-4-yl)-7-(trifluoromethyl)-1H-benzo[d]imidazol-2(3H)-one (GEN1-117)

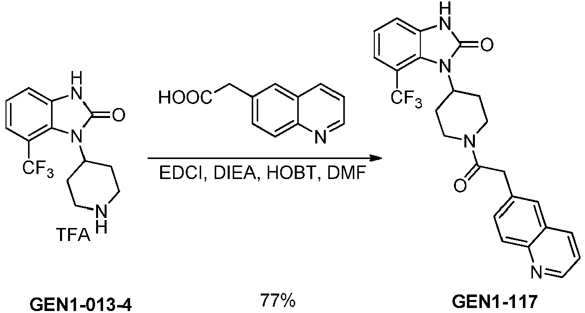

The compound 1-(piperidin-4-yl)-7-(trifluoromethyl)-1H-benzo[d]imidazol-2(3H)-one 2,2,2-trifluoroacetate (100 mg, 250 µmol, see the final report of GEN1-013-4 for its synthesis), EDCI (72 mg, 0.36 mmol), HOBT (51 mg, 0.38 mmol) and DIEA (162 mg, 1.25 mmol) were dissolved in DMF (2 mL), and 2-(6-quinolyl)acetic acid (56 mg, 0.30 mmol) was added. After the addition was complete, the resulting mixture was stirred at room temperature for 16 h. The mixture was then purified by preparative chromatography (preparative 5 Xbridge C₁₈ 5 µm 19 × 150 mm, 30-70% B; A: H₂O (0.1% NH₄HCO₃), B: CH₃CN; 214 nm, flow rate: 15 mL/min) to give the desired compound (88 mg, yield: 77%) as a white solid. ¹HNMR (400 MHz, DMSO-*d*₆): δ 11.45 (s, 1H), 8.87 (dd, *J* = 4.4, 1.6 Hz, 1H), 8.33 (d, *J* = 7.2 Hz, 1H), 7.97 (d, *J* = 8.4 Hz, 1H), 7.82 (s, 1H), 7.66 (dd, *J* = 8.8, 2.0 Hz, 1H), 7.52 (dd, *J* = 8.4, 4.4 Hz, 1H), 7.36 (d, *J=* 8.4 Hz, 1H), 7.28 (d, *J* = 7.2 Hz, 1H), 7.16 (t, *J* = 8.0 Hz, 1H), 4.64 (d, *J* = 10.0 Hz, 1H), 4.29-4.20 (m, 2H), 4.01-3.96 (m, 2H), 3.09-3.02 (m, 1H), 2.61-2.54 (m, 3H), 1.72 (s, 2H). LCMS (ESI): Rt = 3.694 min, *m*/*z* 455.1 [M+H]⁺; purity: 98.00% @ 254 nm, 99.57% @ 214 nm.

### Example 1

### (2-Oxo-3-(1-(2-(quinolin-6-yl)acetyl)piperidin-4-yl)-4-(trifluoromethyl)-2,3-dihydro-1H-benzo[d]imidazol-1-yl)methyl dihydrogen phosphate (GEN1-284)

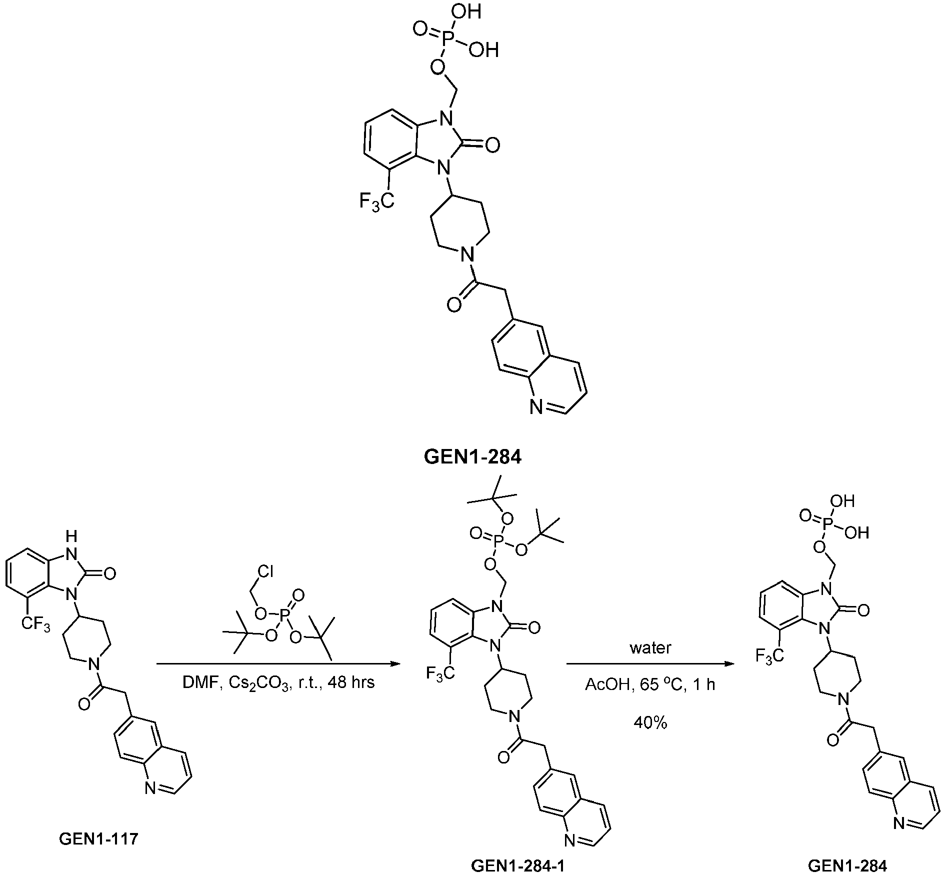

### Step 1. Di-tert-butyl ((2-oxo-3-(1-(2-(quinolin-6-yl)acetyl)piperidin-4-yl)-4-(trifluoromethyl)-2,3-dihydro-1H-benzo[d]imidazol-1-yl)methyl) phosphate (GEN1-284-1)

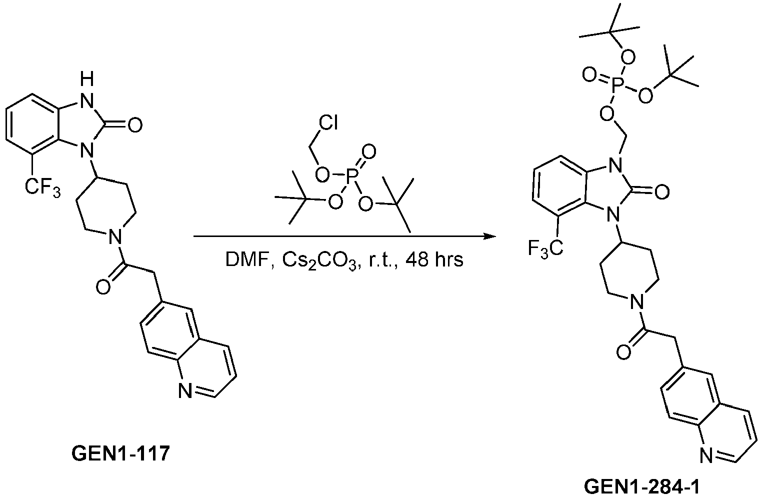

1-(1-(2-(Quinolin-6-yl)acetyl)piperidin-4-yl)-7-(trifluoromethyl)-1,3-dihydro-2H-benzo[d]imidazol-2-one (120 mg, 0.26 mmol) and cesium carbonate (258 mg, 0.79 mmol) were dissolved in DMF (2.0 mL). The resulting mixture was stirred at room temperature for 2.5 h under a nitrogen atmosphere. Di-tert-butyl (chloromethyl) phosphate (88 mg, 0.34 mmol) was then added, and the mixture was stirred at room temperature for another 48 h under a nitrogen atmosphere. The reaction mixture was poured into water (10 mL) and extracted with ethyl acetate (10 mL × 3). The combined organic layers were washed with water (25 mL) and saturated brine (25 mL), dried over anhydrous sodium sulfate and concentrated to give a pale yellow oil. The crude product was purified by silica gel column chromatography using (MeOH/(MeOH + DCM) = 5%) as an eluent to give di-tert-butyl ((2-oxo-3-(1-(2-(quinolin-6(-yl)acetyl)piperidin-4-yl)-4-(trifluoromethyl)-2,3-dihydro-1H-benzo[d]imidazol-1-yl)methyl) phosphate (188 mg) as a pale yellow oil. LCMS (ESI): Rt = 1.63 min, *m*/*z* 677.3 [M+H]⁺; purity: 63% @ 254 nm, 85% @ 214 nm.

### Step 2. (2-Oxo-3-(1-(2-(quinolin-6-yl)acetyl)piperidin-4-yl)-4-(trifluoromethyl)-2,3-dihydro-1H-benzo[d]imidazol-1-yl)methyl dihydrogen phosphate (GEN1-284)

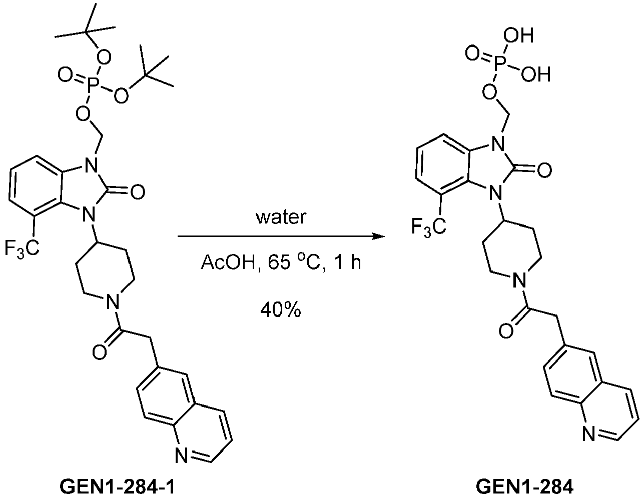

Di-tert-butyl ((2-oxo-3-(1-(2-(quinolin-6-yl)acetyl)piperidin-4-yl)-4-(trifluoromethyl)-2,3-dihydro-1H-benzo[d]imidazol-1-yl)methyl) phosphate (188 mg) was dissolved in a mixed solution of acetic acid (2.0 mL) and water (0.5 mL). The resulting mixture was stirred at 65 °C for 1.0 h. The resulting mixture was directly purified by reversed-phase flash chromatography (C₁₈ reversed-phase silica gel column; mobile phase CH₃CN, water, 0% to 70% gradient, 40 min; detection, UV 214 nm) to give the desired compound (60 mg, yield: 40%) as a white solid. ¹H NMR (400 MHz, DMSO-*d*₆) δ: 8.87 (d, *J=* 4.1 Hz, 1H), 8.35 (d, *J=* 8.2 Hz, 1H), 7.98 (d, *J* = 8.6 Hz, 1H), 7.83 (s, 1H), 7.69-7.59 (m, 2H), 7.57-7.43 (m, 2H), 7.29 (t, *J* = 8.0 Hz, 1H), 5.62 (d, *J* = 8.0 Hz, 2H), 4.63 (d, *J=* 12.6 Hz, 1H), 4.37-4.16 (m, 2H), 4.09-3.91 (m, 2H), 3.07 (t, *J* = 13.2 Hz, 1H), 2.69-2.50 (m, 3H), 1.75 (d, *J* = 12.1 Hz, 2H). LCMS (ESI): Rt = 1.16 min, *m*/*z* 565.2 [M+H]⁺; purity: one main peak.

### Example 2

### (2-Oxo-3-(1-(2-(quinolin-6-yl)acetyl)piperidin-4-yl)-4-(trifluoromethyl)-2,3-dihydro-1H-benzo[d]imidazol-1-yl)methyl disodium phosphate (GEN1-284salt)

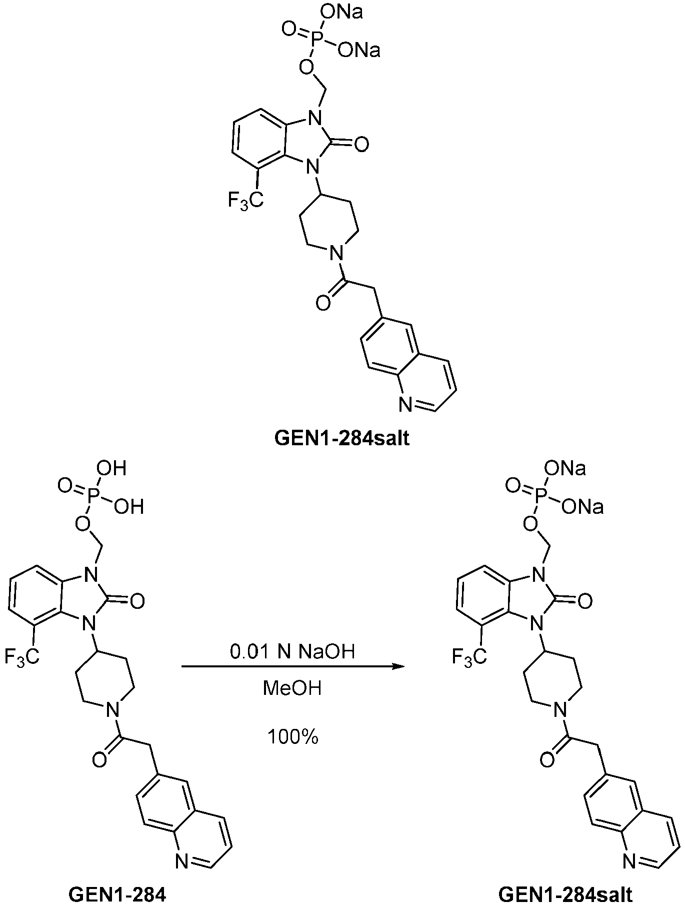

### Step 1. (2-Oxo-3-(1-(2-(quinolin-6-yl)acetyl)piperidin-4-yl)-4-(trifluoromethyl)-2,3-dihydro-1H-benzo[d]imidazol-1-yl)methyl disodium phosphate (GEN1-284salt)

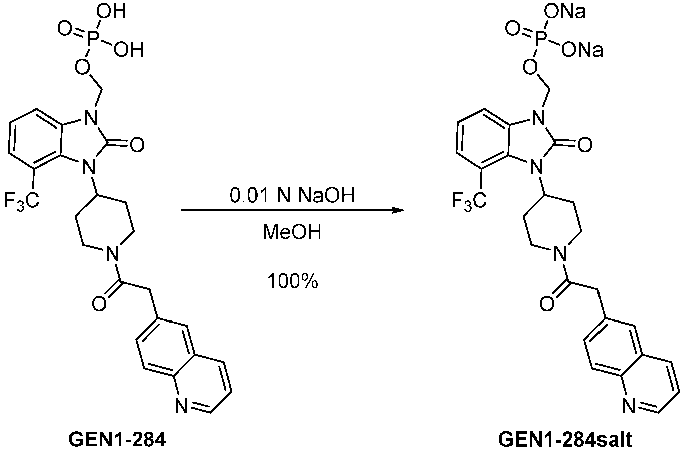

(2-Oxo-3-(1-(2-(quinolin-6-yl)acetyl)piperidin-4-yl)-4-(trifluoromethyl)-2,3-dihydro-1H-benzo[d]imidazol-1-yl)methyl dihydrogen phosphate (26.2 mg, 0.046 mmol) was dissolved in methanol (0.5 mL), and a 0.01 N standard aqueous sodium hydroxide solution (9.29 mL, 0.093 mmol) was added dropwise at 0 °C. The resulting mixture was stirred at 0 °C for 0.25 h. The resulting mixture was lyophilized to give (2-oxo-3-(1-(2-(quinolin-6-yl)acetyl)piperidin-4-yl)-4-(trifluoromethyl)-2,3-dihydro-1H-benzo[d]imidazol-1-yl)methyl disodium phosphate as a white solid (28 mg, yield: 100%). ¹HNMR (400 MHz, D₂O) δ: 8.72-8.65 (m, 1H), 8.23 (d, *J* = 8.3 Hz, 1H), 7.90 (d, *J =* 8.7 Hz, 1H), 7.66 (s, 1H), 7.57 (d, *J =* 8.3 Hz, 2H), 7.48-7.35 (m, 2H), 7.21 (d, *J =* 8.0 Hz, 1H), 5.56-5.40 (m, 2H), 4.55 (d, *J* = 13.6 Hz, 1H), 4.39-4.27 (m, 1H), 4.12 (d, *J* = 14.0 Hz, 1H), 4.04-3.91 (m, 2H), 3.13 (t, *J* = 13.2 Hz, 1H), 2.73 (t, *J* = 13.1 Hz, 1H), 2.53-2.20 (m, 2H), 1.81 (d, *J* = 12.6 Hz, 1H), 1.67 (d, *J =* 12.7 Hz, 1H). LCMS (ESI): Rt = 3.355 min (note: the conditions for the HPLC-MS analysis of this compound were different from those for the foregoing GEN1-284), *m*/*z* 565.1 [M+H]⁺; purity: 95.45% @ 254 nm, 81.76% @ 214 nm.

### Example 3

### 4-Chloro-1-(1-(2-(quinolin-6-yl)acetyl)piperidin-4-yl)-1,3-dihydro-2H-benzo[d]imidazol-2-one (GEN1-295)

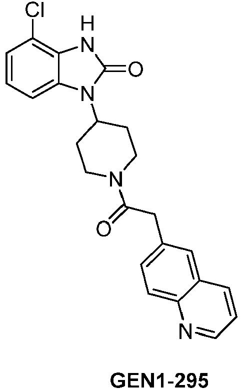

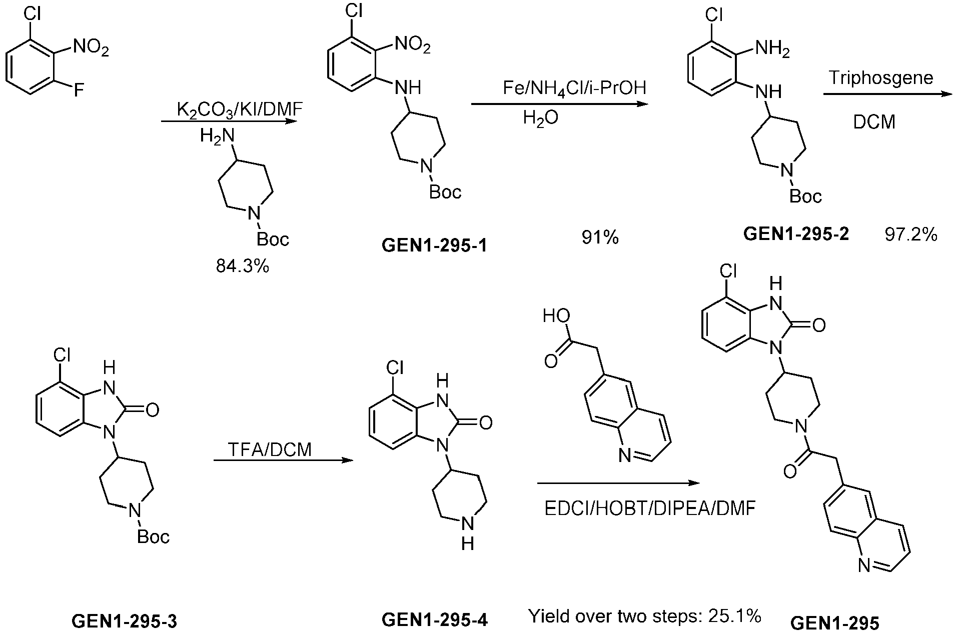

### Step 1. Synthesis of tert-butyl 4-((3-chloro-2-nitrophenyl)amino)piperidine-1-carboxylate (GEN1-295-1)

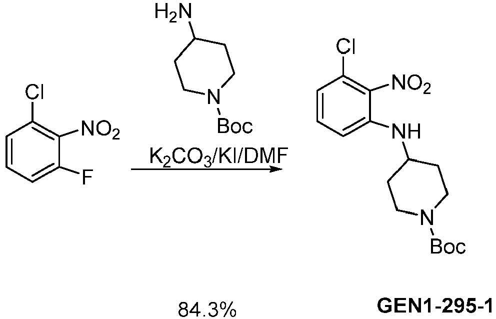

To a solution of 1-chloro-3-fluoro-2-nitrobenzene (702.16 mg, 3 mmol) and tert-butyl 4-aminopiperidine-1-carboxylate (880.23 mg, 4.4 mmol) in DMF (20 mL) were added, at room temperature, K₂CO₃ (1.1 g, 8 mmol) and KI (66.4 mg, 0.4 mmol). The resulting mixture was then stirred at 80 °C for 3 h. The reaction mixture was diluted with water (100 mL) and extracted with EtOAc (30 mL × 3). The combined organic phases were washed with brine (30 mL), dried over anhydrous Na₂SO₄ and filtered. The filtrate was concentrated and purified by silica gel column chromatography using (PE:EA = 3:1) as an eluent to give the desired compound (1.2 g, yield: 84.3%) as a yellow oil. MS (ESI) RT = 1.79 min, m/z 354 [M-H]⁻, purity: 90.58% @ 254 nm, 73.24% @ 214 nm.

### Step 2. Synthesis of tert-butyl 4-((2-amino-3-chlorophenyl)amino)piperidine-1-carboxylate (GEN1-295-2)

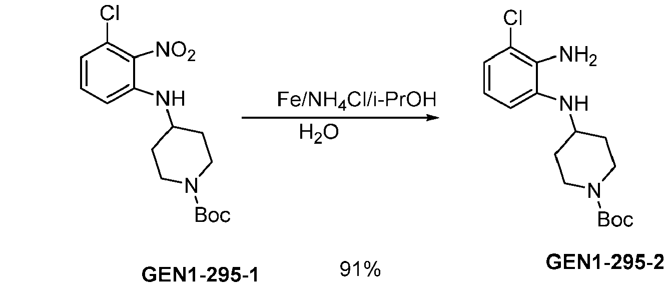

To a suspension of tert-butyl 4-((3-chloro-2-nitrophenyl)atnino)piperidine-1-carboxylate (1.2 g, 3.372 mmol) and NH₄Cl (3.607 g, 67.44 mmol) in i-PrOH (25 mL) and H₂O (5 mL) was added, at room temperature, Fe (1.888 g, 33.72 mmol). The resulting mixture was stirred at room temperature for 16 h. The reaction mixture was filtered, and the filtrate was concentrated to give a crude product. The crude product was purified by silica gel column chromatography using (PE:EA= 1:1) as an eluent to give the desired compound (1.0 g, yield: 91%) as a brown solid. MS (ESI) RT = 1.58 min, purity: 100% @ 254 nm, 100% @ 214 nm.

### Step 3. Synthesis of tert-butyl 4-(4-chloro-2-oxo-2,3-dihydro-1H-benzo[d]imidazol-1-yl)piperidine-1-carboxylate (GEN1-295-3)

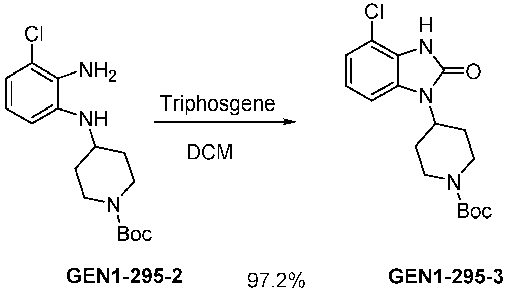

To a solution of tert-butyl 4-((2-amino-3-chlorophenyl)amino)piperidine-1-carboxylate (200 mg, 0.614 mmol) and NaHCOs (515.76 mg, 6.14 mmol) in DCM (5 mL) was added, under a nitrogen atmosphere, triphosgene (72.86 mg, 0.246 mmol), with the temperature controlled at 0 °C. The resulting mixture was stirred at room temperature for 16 h. The reaction mixture was filtered, and the filtrate was concentrated to give a crude product. The crude product was purified by silica gel column chromatography using (PE:EA = 1:1) as an eluent to give the desired compound (210 mg, yield: 97.2%) as a brown solid. MS (ESI) RT = 1.50 min, purity: 64.2% @ 254 nm, 89.68% @ 214 nm.

### Step 4. Synthesis of 4-chloro-1-(piperidin-4-yl)-1,3-dihydro-2H-benzo[d]imidazol-2-one (GEN1-295-4)

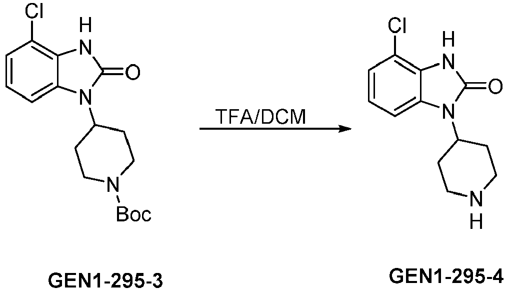

To a solution of tert-butyl 4-(4-chloro-2-oxo-2,3-dihydro-1H-benzo[d]imidazol-1-yl)piperidine-1-carboxylate (100 mg, 0.284 mmol) in DCM (10 mL) was added, at room temperature, TFA (4 mL). The resulting mixture was stirred at room temperature for 0.5 h. The reaction solution was concentrated. The resulting residue was dissolved again in ACN (40 mL), and the solution was concentrated again. The resulting crude product (100 mg) could be directly used in the next step without purification. MS (ESI) RT = 1.36 min, m/z 252.2 [M+H]⁺, purity: 100% @ 254 nm, 100% @ 214 nm.

### Step 5. Synthesis of 4-chloro-1-(1-(2-(quinolin-6-yl)acetyl)piperidin-4-yl)-1,3-dihydro-2H-benzo[d]imidazol-2-one (GEN1-295)

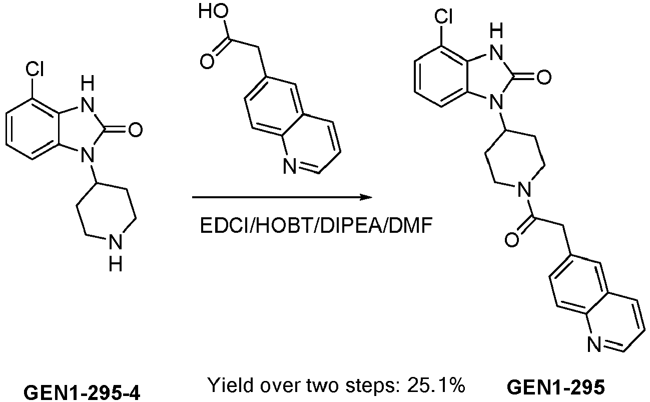

To a solution of 4-chloro-1-(piperidin-4-yl)-1,3-dihydro-2H-benzo[d]imidazol-2- (100 mg, crude, 0.284 mmol), 2-(quinolin-6-yl)acetic acid (63.84 mg, 0.341 mmol), HOBT (57.56 mg, 0.426 mmol) and EDCI (81.66 mg, 0.341 mmol) in DMF (2 mL) was added, at room temperature, DIPEA (146.8 mg, 1.136 mmol). The resulting mixture was stirred at room temperature for 1 h. The crude product was purified through a reversed-phase chromatography column. Purification conditions: C18 silica gel column; mobile phase ACN: H₂O, gradient: 5% to 50%; 45 min, monitoring wavelength: 214 nm. The desired compound (30 mg, yield over two steps: 25.1%) was obtained as a white solid. ¹H NMR (400 MHz, DMSO-d6) δ 11.41 (s, 1H), 8.96-8.78 (m, 1H), 8.37 (d, J = 8.3 Hz, 1H), 8.00 (d, J = 8.6 Hz, 1H), 7.88 (s, 1H), 7.71 (dd, J = 8.8, 1.9 Hz, 1H), 7.56-7.49 (m, 1H), 7.05-6.82 (m, 3H), 4.61 (d, J = 13.1 Hz, 1H), 4.48-4.32 (m, 1H), 4.16 (d, J = 13.9 Hz, 1H), 4.10-3.92 (m, 2H), 3.19 (t, J = 13.0 Hz, 1H), 2.71 (t, J = 12.8 Hz, 1H), 2.17-1.87 (m, 2H), 1.76-1.57 (m, 2H). MS (ESI) RT = 1.32 min, m/z 421.2 [M+H]⁺, purity: 100% @ 254 nm, 100% @ 214 nm.

### Example 4

### 1-(1-(1-(2-(1H-Indol-5-yl)acetyl)piperidin-4-yl)-7-(trifluoromethyl)-1,3-dihydro-2H-benzo[d]imidazol-2-one (GEN1-296)

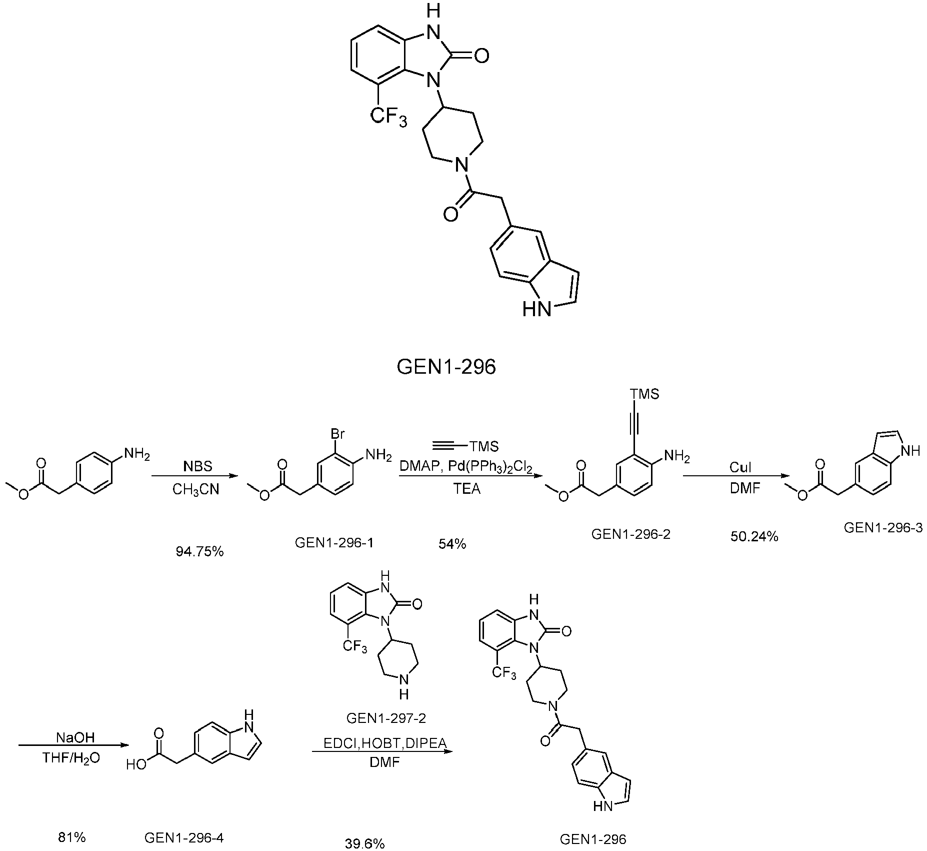

### Step 1. Synthesis of methyl 2-(4-amino-3-bromophenyl)acetate (GEN1-296-1)

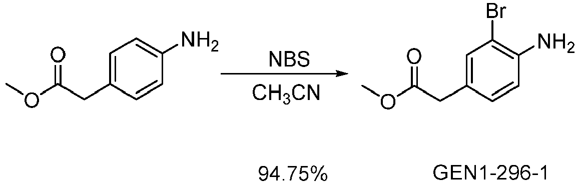

To a solution of methyl 2-(4-aminophenyl)acetate (3 g, 18.16 mmol) in ACN (20 mL) was added, under a nitrogen atmosphere, NBS (3.23 g, 18.16 mmol). The resulting mixture was stirred at room temperature for 16 h. The reaction solution was filtered, and the filtrate was concentrated. The residue was purified by silica gel column chromatography using (PE:EA = 5:1) as an eluent to give the desired compound (4.2 g, yield: 94.75%) as a pale yellow oil. MS (ESI) RT = 1.36 min, m/z 244.0 [M+H]⁺, purity:83.91% @ 254 nm, 71.46% @ 214 nm.

### Step 2. Synthesis of methyl 2-(4-amino-3-((trimethylsilyl)ethynyl)phenyl)acetate (GEN1-296-2)

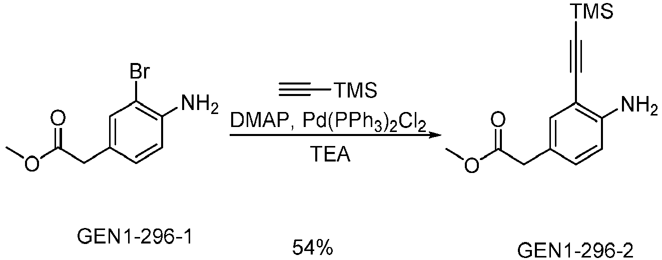

To a solution of methyl 2-(4-amino-3-bromophenyl)acetate (2.0 g, 8.19 mmol), trimethylsilylacetylene (8.125 g, 81.9 mmol) and DMAP (100 mg, 0,819 mmol) in TEA (10 mL) was added, at room temperature under a nitrogen atmosphere, Pd(PPh₃)₂Cl₂ (575 mg, 0.819 mmol). The resulting mixture was stirred at 70 °C for 16 h. The resulting mixture was concentrated, diluted by addition of water (50 mL) and extracted with DCM (50 mL × 3). The combined organic layers were washed with brine (50 mL), dried over anhydrous Na₂SO₄ and filtered. The filtrate was concentrated, and the residue was purified through a silica gel column (PE:EA = 20: 1) to give the desired compound (1.16 g, yield: 54%) as a yellow oil.

### Step 3. Synthesis of methyl 2-(1H-indol-5-yl)acetate (GEN1-296-3)

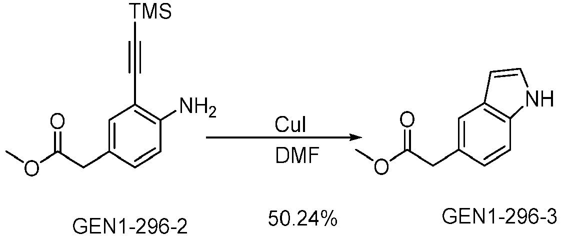

To a solution of methyl 2-(4-amino-3-(((trimethylsilyl)ethynyl)phenyl)acetate (1.1 g, 4.2 mmol) in DMF (20 mL) was added, at room temperature under a nitrogen atmosphere, CuI (80 mg, 0.42 mmol). The resulting mixture was stirred at 90 °C for 16 h. The reaction was then quenched with water (50 mL). The mixture was extracted with DCM (50 mL × 3). The combined organic phases were washed with brine (20 mL × 2), dried over Na₂SO₄, filtered and concentrated to give a crude product. The crude product was purified through a reversed-phase chromatography column. Purification conditions: C18 silica gel column; mobile phase ACN: H₂O, gradient: 5% to 60% 50 min, monitoring wavelength: 214 nm. The desired compound (400 mg, yield: 50.24%) was obtained as a yellow oil. MS (ESI) RT = 2.343 min, m/z 190.1 [M+H]⁺, purity:55.31% @ 254 nm, 40.54% @ 214 nm.

### Step 4. Synthesis of 2-(1H-indol-5-yl)acetic acid (GEN1-295-4)

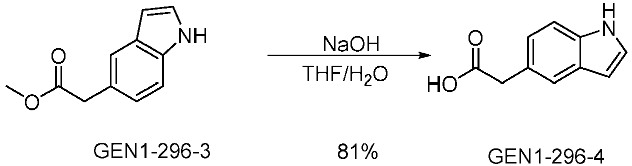

To a solution of methyl 2-(1H-indol-5-yl)acetate (400 mg, 2.114 mmol) in THF (10 mL) and H₂O (5 mL) was added, at room temperature, NaOH (169.12 mg, 4.228 mmol). The resulting mixture was stirred at room temperature for 16 h. The mixture was then adjusted to pH 4 and purified through a reversed-phase chromatography column. Purification conditions: C18 silica gel column; mobile phase ACN: H₂O, gradient: 5% to 50%; 45 min, monitoring wavelength: 214 nm. The desired compound (300 mg, yield: 81%) was obtained as a white solid. MS (ESI) RT = 1.68 min, m/z 176.1 [M+H]⁺, purity:97.25% @ 254 nm, 80.46% @ 214 nm.

### Step 5. Synthesis of 1-(1-(1-(2-(1H-indol-5-yl)acetyl)piperidin-4-yl)-7-(trifluoromethyl)-1,3-dihydro-2H-benzo[d]imidazol-2-one (GEN1-296)

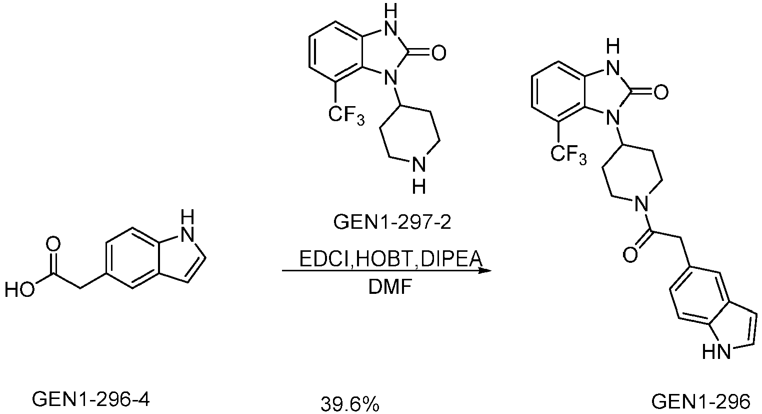

To a solution of 1-(piperidin-4-yl)-7-(trifluoromethyl)-1,3-dihydro-2H-benzo[d]imidazol-2-one (81.4 mg, 0.2854 mmol), 2-(1H-indol-5-yl)acetic acid (50 mg, 0.2854 mmol), HOBT (57.84 mg, 0.4281 mmol) and EDCI (82.07 mg, 0.4281 mmol) in DMF (1 mL) was added, at room temperature, DIPEA (258.5 mg, 0.2 mmol). The resulting mixture was stirred at room temperature for 1 h. The crude product was purified through a reversed-phase chromatography column. Purification conditions: C18 silica gel column; mobile phase ACN: H₂O, gradient: 5% to 50%; 40 min, monitoring wavelength: 214 nm. The desired compound (50 mg, yield: 39.6%) was obtained as a white solid. ¹H NMR (400 MHz, Methanol-d4) δ 7.48 (s, 1H), 7.44-7.36 (m, 2H), 7.33-7.28 (m, 1H), 7.26-7.16 (m, 2H), 7.06 (dd, J = 8.4, 1.7 Hz, 1H), 6.44 (dd, J = 3.1, 0.9 Hz, 1H), 4.86-4.78 (m, 1H), 4.52-4.38 (m, 1H), 4.34-4.25 (m, 1H), 3.93 (d, J = 4.4 Hz, 2H), 3.13-3.01 (m, 1H), 2.79-2.52 (m, 3H), 1.88-1.68 (m, 2H). MS (ESI) RT = 2.63 min, m/z 443.2 [M+H]⁺, purity: 100% @ 254 nm, 93.97% @ 214 nm.

### Example 5

### 1-(1-(2-(3-(Dimethylamino)phenyl)acetyl)piperidin-4-yl)-7-(trifluoromethyl)-1,3-dihydro-2H-benzo[d]imidazol-2-one(GEN1-297)

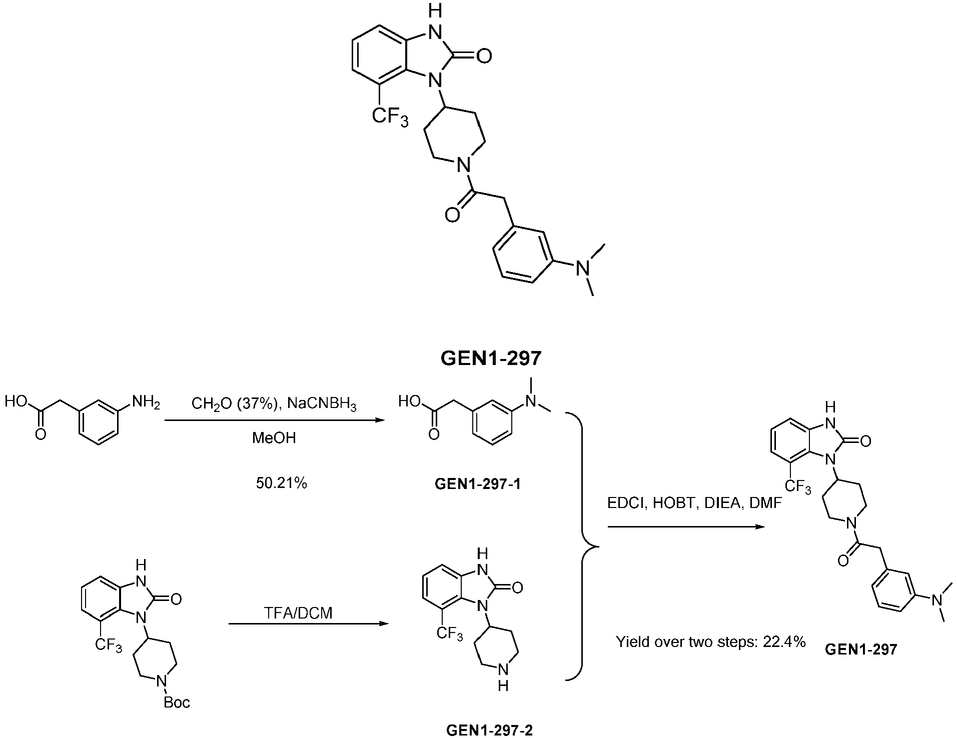

### Step 1. Synthesis of 2-(3-(dimethylamino)phenyl)acetic acid (GEN1-297-1)

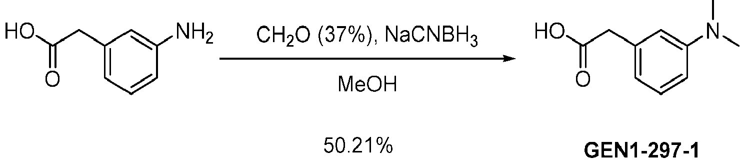

To a solution of 2-(3-aminophenyl)acetic acid (151.17 mg, 1 mmol) in MeOH (3 mL) was added, at 0 °C, 37% formaldehyde (202.91 mg, 2.5 mmol). The resulting mixture was stirred at room temperature for 6 h. The mixture was purified through a reversed-phase chromatography column. Purification conditions: C18 silica gel column; mobile phase ACN: H₂O, gradient: 5% to 50%; 45 min, monitoring wavelength: 214 nm. The desired compound (90 mg, yield: 50.21%) was obtained. MS (ESI) RT = 0.27 min, m/z 180.1 [M+H]⁺, purity: 100% @ 254 nm, 100% @ 214 nm.

### Step 2. 1-(Piperidin-4-yl)-7-(trifluoromethyl)-1,3-dihydro-2H-benzo[d]imidazol-2-one (GEN1-297-2)

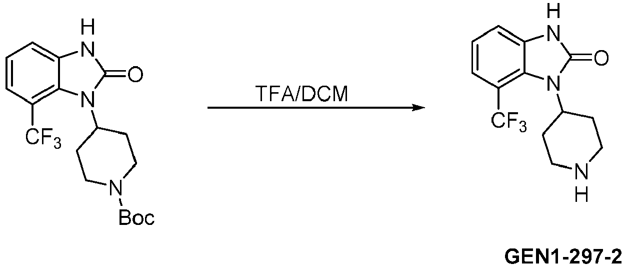

To a solution of tert-butyl 4-(2-oxo-7-(trifluoromethyl)-2,3-dihydro-1H-benzo[d]imidazol-1-yl)piperidine-1-carboxylate (200 mg, 0.52 mmol) in DCM (10 mL) was added, at 0 °C, TFA (2 mL). The resulting mixture was stirred at room temperature for 0.5 h. The reaction mixture was then concentrated, and the residue was redissolved in DCM (20 mL). The solution was concentrated again to give a crude product as a yellow solid. The crude product could be directly used in the next step without purification.

### Step 3. Synthesis of 1-(1-(2-(3-(dimethylamino)phenyl)acetyl)piperidin-4-yl)-7-(trifluoromethyl)-1,3-dihydro-2H-benzo[d]imidazol-2-one (GEN1-297)

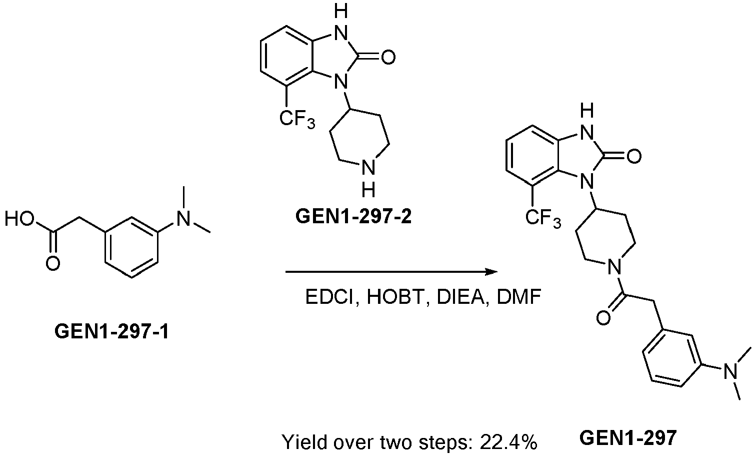

To a solution of 2-(3-(dimethylamino)phenyl)acetic acid (200 mg, crude, 0.5 mmol), 1-(piperidin-4-yl)-7-(trifluoromethyl)-1,3-dihydro-2H-benzo[d]imidazol-2-one (90 mg, 0.5 mmol), HOBT (101.4 mg, 0.75 mmol) and EDCI (143.78 mg, 0.75 mmol) in DMF (1 mL) was added, at room temperature, DIPEA (193.86 mg, 1.5 mmol). The resulting mixture was stirred at room temperature for 1 h. The mixture was purified through a reversed-phase chromatography column. Purification conditions: C18 silica gel column; mobile phase ACN: H₂O, gradient: 5% to 50%; 45 min, monitoring wavelength: 214 nm. The desired compound (50 mg, yield over two steps: 22.4%) was obtained as a white solid. ¹H NMR (400 MHz, DMSO-d6) δ 11.47 (s, 1H), 7.38 (d, J = 8.1 Hz, 1H), 7.30 (d, J = 7.7 Hz, 1H), 7.23-7.09 (m, 2H), 6.66-6.54 (m, 3H), 4.65 (d, J = 10.0 Hz, 1H), 4.34-4.09 (m, 2H), 3.79-3.54 (m, 2H), 2.99 (t, J = 13.1 Hz, 1H), 2.61-2.56(m, 1H), 2.52-2.42(m, 2H), 1.78-1.64 (m, 2H). MS (ESI) RT = 2.60 min, m/z 447.2 [M+H]⁺, purity: 100% @ 254 nm, 93.4% @ 214 nm.

### Example 6

### 1-(1-(2-(Benzo[b]thiophen-5-yl)acetyl)piperidin-4-yl)-7-(trifluoromethyl)-1,3-dihydro-2H-benzo[d]imidazol-2-one (GEN1-298)

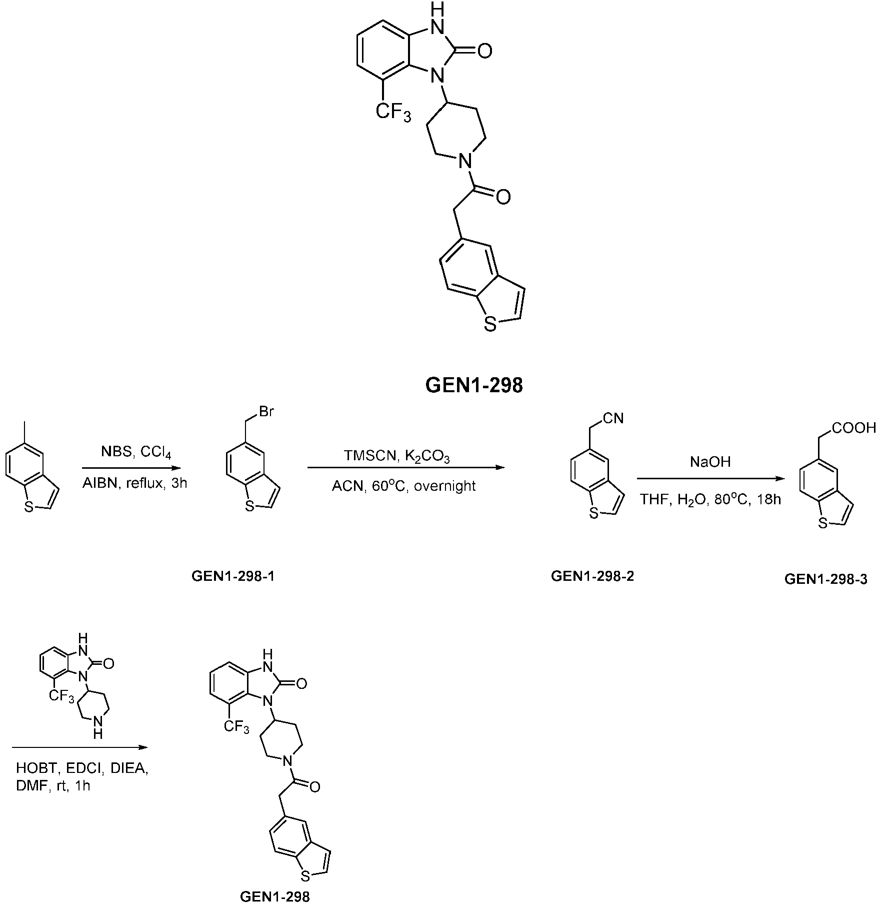

### Step 1. 5-(Bromomethyl)benzo[b]thiophene (GEN1-298-1)

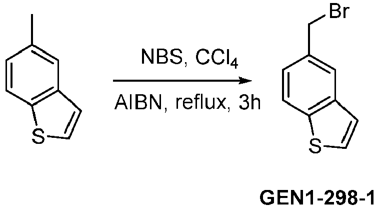

To a solution of 5-methylbenzo[b]thiophene (280 mg, 1.89 mmol) in CCl₄ (12 mL) were added NBS (370 mg, 2.08 mmol) and AIBN (34 mg, 0.11 mmol). The mixture was stirred and heated at reflux for 3 h and then cooled to room temperature overnight. The mixture was filtered, and the filtrate was dried *in vacuo* by evaporation at 40 °C. The mixture was filtered, and the residue was treated with n-hexane and heated until it dissolved, and then cooled. The solid was then separated by filtration to give 5-(bromomethyl)benzo[b]thiophene (490 mg, yield: 86%) as a white solid. TLC:PE = 100%, Rf = 0.6

### Step 2. 2-(Benzo[b]thiophen-5-yl)acetonitrile (GEN1-298-2)

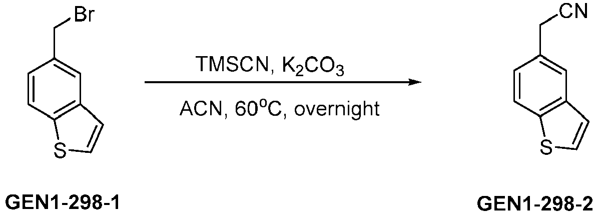

To a solution of 5-(bromomethyl)benzo[b]thiophene (250 mg, 1.1 mmol) in ACN (10 mL) were added, at 0 °C, K₂CO₃ (3.05 mg, 2.2 mmol) and TMSCN (164 mg, 1.7 mmol). The mixture was stirred at 60 °C overnight. The reaction mixture was poured into water (20 mL) and extracted with EA (20 mL × 3). The combined organic layers were washed with water (20 mL) and brine (20 mL), dried over Na₂SO₄ and concentrated to give a yellow oil. The crude product was purified by reversed-phase flash chromatography (C₁₈ reversed-phase silica gel column; mobile phase ACN, water, 10% to 85% gradient; 30 min; detection, UV 214 nm) to give 2-(benzo[b]thiophen-5-yl)acetonitrile (84 mg, yield: 44%) as a white solid. MS (ESI) RT = 2.67 min, m/z 174.1 [M+H]⁺, purity: 46.06% @ 254 nm, 83.97% @ 214 nm.

### Step 3. 2-(Benzo[b]thiophen-5-yl)acetic acid (GEN1-298-3)

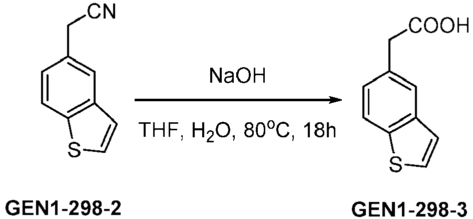

To a mixed solution of 2-(benzo[b]thiophen-5-yl)acetonitrile (60 mg, 0.35 mmol) in THF (5 mL) and H₂O (5 mL) was added, under a nitrogen atmosphere, NaOH (190 mg, 4.75 mmol). The mixture was stirred at 80 °C for 18 h. The mixture was then diluted with H₂O (20 mL) and washed with EA (20 mL). The aqueous phase was adjusted to pH 4 with an aqueous hydrochloric acid solution (2 M) and extracted with EA (20 mL × 3). The organic phases were dried over Na₂SO₄ and concentrated to give 2-(benzo[b]thiophen-5-yl)acetic acid (57 mg, yield: 86%) as a white solid. MS (ESI) RT = 2.35 min, m/z 193.0 [M+H]⁺, purity: 100% @ 254 nm, 96.39% @ 214 nm.

### Step 4. 1-(1-(2-(Benzo[b]thiophen-5-yl)acetyl)piperidin-4-yl)-7-(trifluoromethyl)-1,3-dihydro-2H-benzo[d]imidazol-2-one (GEN1-298)

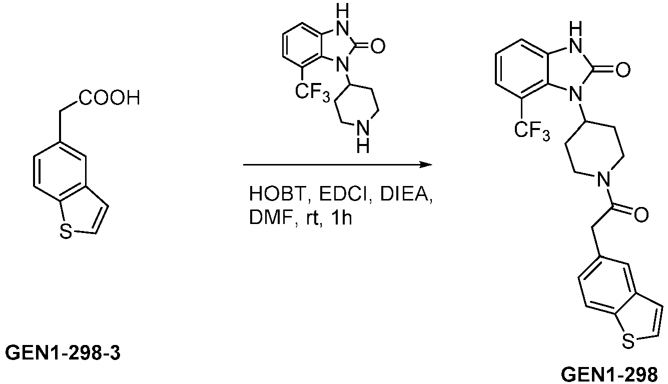

To a mixture of 2-(benzo[b]thiophen-5-yl)acetic acid (57 mg, 0.30 mmol) in DMF (5 mL) were added 1-(piperidin-4-yl)-7-(trifluoromethyl)-1,3-dihydro-2H-benzo[d]imidazol-2-one (85 mg, 0.30 mmol), EDCI (68 mg, 0.36 mmol), HOBT (48 mg, 0.36 mmol) and DIEA (77 mg, 0.60 mmol). The mixture was stirred at room temperature for 1 h. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 1-(1-(2-(benzo [b]thiophen-5-yl)acetyl)piperidin-4-yl)-7-(trifluoromethyl)-1,3-dihydro-2H-benzo [d]imidazol-2-one (25 mg, yield: 18%) as a white solid. ¹H NMR (400 MHz, DMSO-*d*₆) δ 11.46 (s, 1H), 7.93 (d, *J* = 8.4 Hz, 1H), 7.77 - 7.73 (m, 2H), 7.43 (d, *J* = 5.6 Hz, 1H), 7.35 (d, *J* = 8.0 Hz, 1H), 7.27 - 7.23 (m, 2H), 7.15 (t, *J* = 8.0 Hz, 1H), 4.62 (d, *J* = 10.4 Hz, 1H), 4.31 - 4.15 (m, 2H), 3.89 (q, *J* = 15.2 Hz, 2H), 3.00 (t, *J* = 12.8 Hz, 1H), 2.63 - 2.51 (m, 3H), 1.83 - 1.63 (m, 2H). MS (ESI) RT = 2.98 min, m/z 460.2 [M+H]⁺, purity: 96.67% @ 254 nm, 95.17%@ 214 nm.

### Example 7

### 1-(1-(2-(Isoquinolin-6-yl)acetyl)piperidin-4-yl)-7-(trifluoromethyl)-1,3-dihydro-2H-benzo[d]imidazol-2-one (GEN1-299)

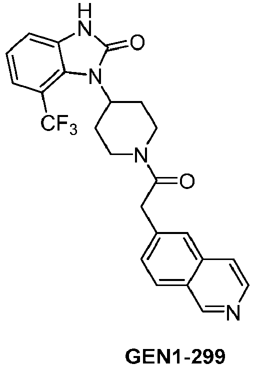

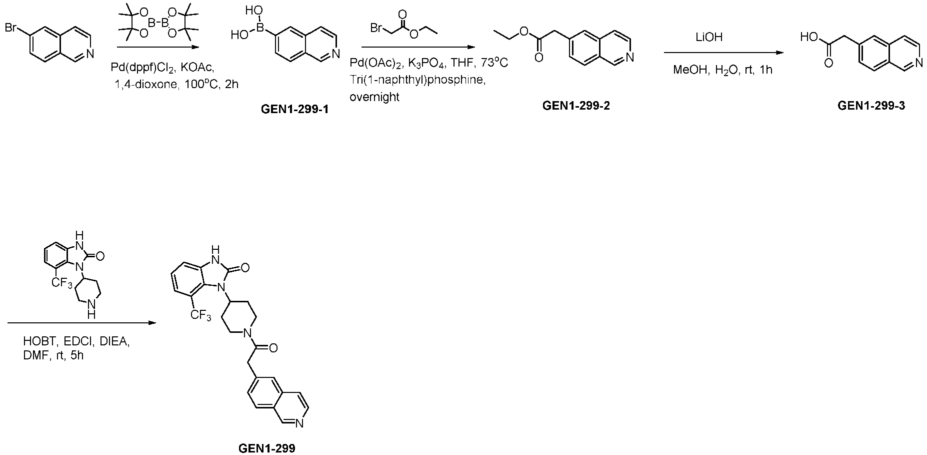

### Step 1. Isoquinolin-6-ylboronic acid (GEN1-299-1)

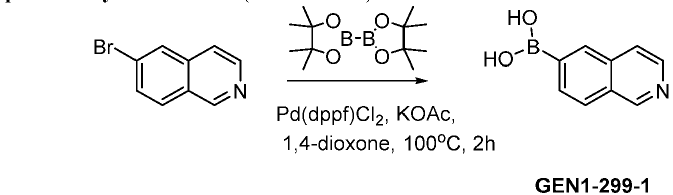

To a solution of 6-bromoisoquinoline (1 g, 4.8 mmol) in 1,4-dioxane (10 mL) were added KOAc (1.41 g, 14.4 mmol), 4,4,4',4',5,5,5,5'-octamethyl-2,2'-bis(1,3,2-dioxaborolane) (1.83 g, 7.2 mmol) and Pd(dppf)Cl₂·CH₂Cl₂ (392 mg, 0.48 mmol). The mixture was stirred at 100 °C for 2 h. The reaction mixture was poured into water (100 mL) and extracted with EA (100 mL × 3). The combined organic layers were washed with water (100 mL) and brine (50 mL), dried over Na₂SO₄ and concentrated to give a yellow oil. The crude product was purified by reversed-phase flash chromatography (C₁₈ reversed-phase silica gel column; mobile phase ACN, water, 10% to 85% gradient; 30 min; detection, UV 214 nm) to give isoquinolin-6-ylboronic acid (590 mg, yield: 71%). MS (ESI) RT = 0.43 min, m/z 174.1 [M+H]⁺, purity: 7.59% @ 254 nm, 63.95% @ 214 nm.

### Step 2. Ethyl 2-(isoquinolin-6-yl)acetate (GEN1-299-2)

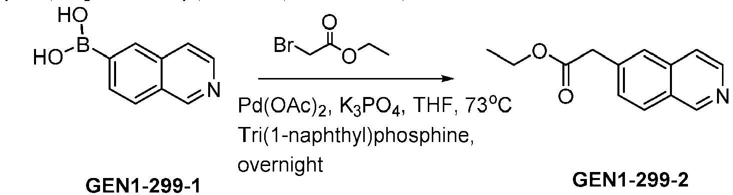

To a solution of Pd(OAc)₂ (27 mg, 0.12 mmol) in THF (10 mL) were added K₃PO₄ (1.27 g, 5.99 mmol), isoquinolin-6-ylboronic acid (300 mg, 1.73 mmol), ethyl 2-bromoacetate (200 mg, 1.2 mmol) and tris(1-naphthyl)phosphine (150 mg, 0.36 mmol). The mixture was stirred at 73 °C overnight. The reaction mixture was poured into water (20 mL) and extracted with EA (50 mL × 3). The combined organic layers were washed with water (50 mL) and brine (50 mL), dried over Na₂SO₄ and concentrated to give a crude product. The crude product was purified by reversed-phase flash chromatography (C₁₈ reversed-phase silica gel column; mobile phase ACN, water, 10% to 85% gradient; 30 min; detection, UV 214 nm) to give ethyl 2-(isoquinolin-6-yl)acetate (49 mg, yield: 19%) as a yellow liquid. MS (ESI) RT = 1.50 min, m/z 216.1 [M+H]⁺, purity: 50.64% @ 254 nm, 84.37% @ 214 nm.

### Step 3. 2-(Isoquinolin-6-yl)acetic acid (GEN1-299-3)

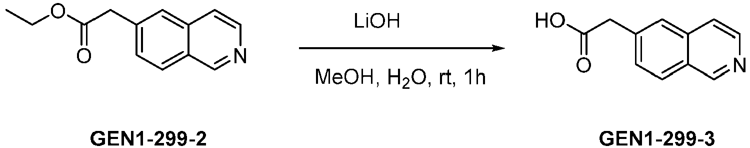

To a solution of ethyl 2-(isoquinolin-6-yl)acetate (49 mg, 0.23 mmol) in MeOH (8 mL) and H₂O (2 mL) was added LiOH (24 mg, 1 mmol). The mixture was stirred at room temperature for 1 h. The mixture was then concentrated and diluted with H₂O (20 mL) and washed with EA (20 mL). The aqueous phase was adjusted to pH 4 with an aqueous hydrochloric acid solution (2 M) and extracted with EA (20 mL × 3). The organic phases were dried over Na₂SO₄ and concentrated to give 2-(isoquinolin-6-yl)acetic acid (42 mg, yield: 99%) as a white solid. MS (ESI) RT = 0.59 min, m/z 188.1 [M+H]⁺, purity: 100% @ 254 nm, 43.80% @ 214 nm.

### Step 4. 1-(1-(2-(Isoquinolin-6-yl)acetyl)piperidin-4-yl)-7-(trifluoromethyl)-1,3-dihydro-2H-benzo[d]imidazol-2-one (GEN1-299)

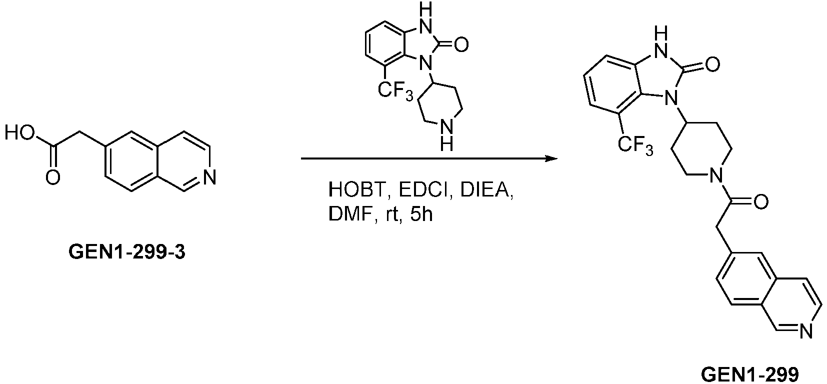

To a solution of 2-(isoquinolin-6-yl)acetic acid (42 mg, 0.22 mmol) in DMF (3 mL) were added 1-(piperidin-4-yl)-7-(trifluoromethyl)-1,3-dihydro-2H-benzo[d]imidazol-2-one (70 mg, 0.25 mmol), EDCI (52 mg, 0.27 mmol), HOBT (36 mg, 0.27 mmol) and DIEA (58 mg, 0.45 mmol). The mixture was stirred at room temperature for 5 h. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 1-(1-(2-(isoquinolin-6-yl)acetyl)piperidin-4-yl)-7-(trifluoromethyl)-1,3-dihydro-2H-benzo [d]imidazol-2-one (40 mg, yield: 39%) as a yellow solid. ¹H NMR (400 MHz, DMSO-*d₆*) δ 11.46 (s, 1H), 9.27 (s, 1H), 8.48 (d, *J* = 5.6 Hz, 1H), 8.07 (d, *J* = 8.4 Hz, 1H), 7.89-7.73 (m, 2H), 7.58 (dd, *J* = 8.4, 1.6 Hz, 1H), 7.36 (d, *J* = 8.0 Hz, 1H), 7.27 (d, *J* = 7.6 Hz, 1H), 7.16 (t, *J* = 8.0 Hz, 1H), 4.63 (d, *J=* 11.2 Hz, 1H), 4.24 (dt, *J* = 17.6, 10.8 Hz, 2H), 4.02 (d, *J* = 2.4 Hz, 2H), 3.06 (t, *J* = 13.2 Hz, 1H), 2.66-2.50 (m, 3H), 1.72 (d, *J=* 12.0 Hz, 2H). MS (ESI) RT =2.54 min, m/z 455.2 [M+H]⁺, purity: 100% @ 254 nm, 99.57% @ 214 nm.

### Example 8

### 1-(1-(2-(2-(2-Methylquinolin-6-yl)acetyl)piperidin-4-yl)-7-(trifluoromethyl)-1,3-dihydro-2H-benzo[d]imidazol-2-one (GEN1-300)

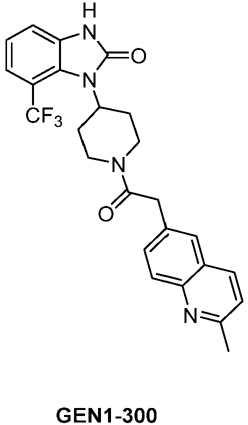

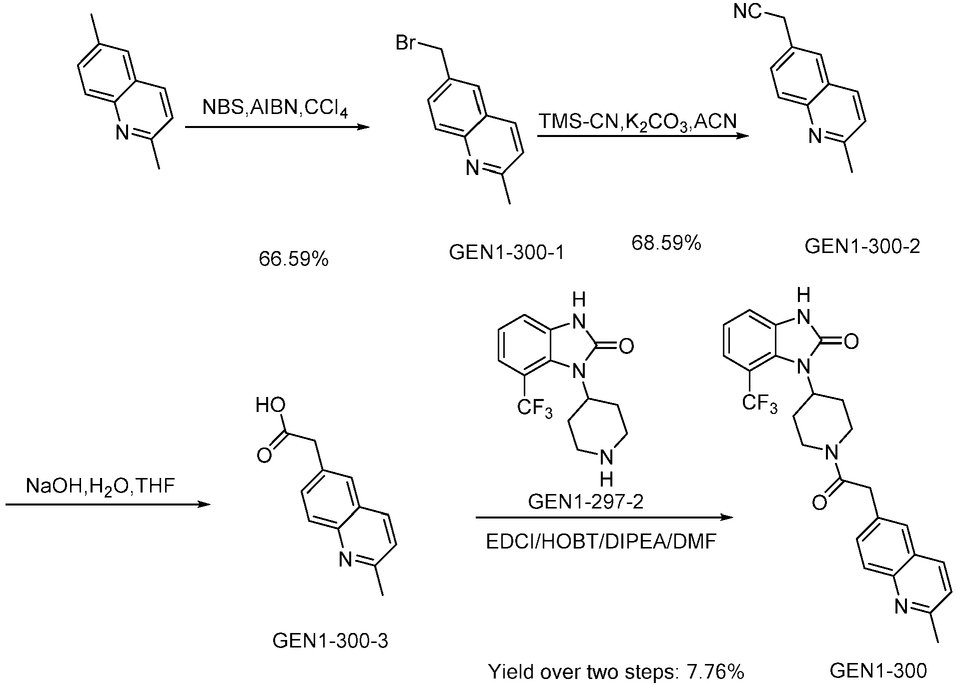

### Step 1. Synthesis of 6-(bromomethyl)-2-methylquinoline (GEN1-300-1)

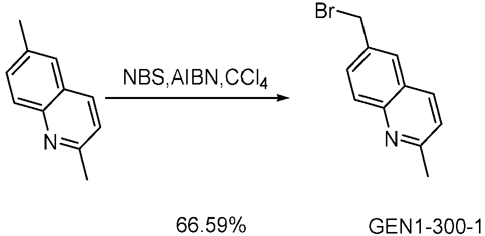

To a solution of 2,6-dimethylquinoline (1.0 g, 6.36 mmol) and AIBN (104.44 mg, 0.636 mmol) in CCl₄ (35 mL) was added, at room temperature, NBS (1.245 g, 7 mmol). The resulting mixture was stirred at 77 °C for 3 h. The reaction solution was quenched with water (50 mL). The resulting mixture was extracted with DCM (40 mL × 3). The combined organic phases were washed with brine (20 mL × 2), dried over Na₂SO₄, filtered and concentrated to give a brown solid. The crude product was purified by silica gel column chromatography using (PE:EA = 3:1) as an eluent to give the desired compound (1.0 g, yield: 66.59%) as a pale yellow solid. MS (ESI) RT = 1.602 min, m/z 236 [M+H]⁺, purity: 94.84% @ 254 nm.

### Step 2. 2-(2-Methylquinolin-6-yl)acetonitrile (GEN1-300-2)

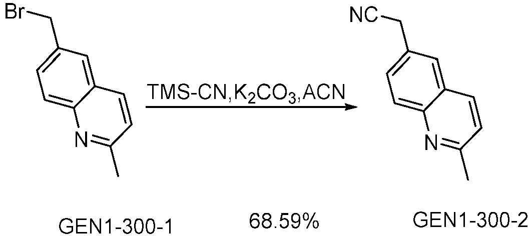

To a suspension of 6-(bromomethyl)-2-methylquinoline (236.11 mg, 1 mmol) and K₂CO₃ (158.94 mg, 1.15 mmol) in ACN (5 mL) was added, at room temperature under a nitrogen atmosphere, TMS-CN (143.85 mg, 1.45 mmol). The resulting mixture was stirred at 60 °C for 3 h. The mixture was filtered, and the filtrate was concentrated to give a crude product. The crude product was purified by silica gel column chromatography using (PE:EA = 3:1) as an eluent to give the desired compound (125 mg, yield: 68.59%) as a white solid. MS (ESI) RT = 0.91 min, m/z 183.1 [M+H]⁺, purity: 78.01% @ 214 nm.

### Step 3. Synthesis of 2-(2-methylquinolin-6-yl)acetic acid (GEN1-300-3)

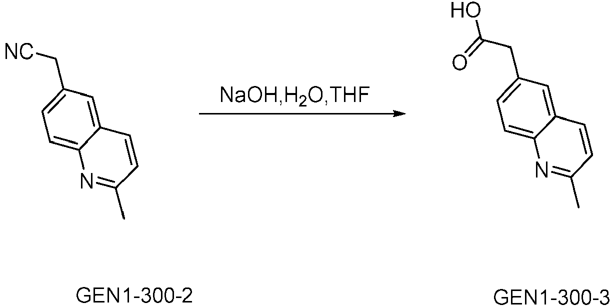

To a solution of 2-(2-methylquinolin-6-yl)acetonitrile (100 mg, 0.554 mmol) in H₂O (2.5 mL) and THF (2.5 mL) was added, at room temperature, NaOH (33.25 mg, 0.831 mmol). The resulting mixture was stirred at 90 °C for 3 h. The reaction mixture was quenched with water (25 mL). The mixture was extracted with EtOAc (15 mL × 3). The aqueous phase was adjusted to pH 4 with 1 N HCl and then extracted with EtOAc (15 mL × 3). The combined organic phases were washed with brine (20 mL × 2), dried over Na₂SO₄, filtered and concentrated to give the desired compound (110 mg, crude). The crude product was directly used in the next step. MS (ESI) RT = 0.571 min, m/z 202.1 [M+H]⁺, purity: 83.06% @ 214 nm.

### Step 4. Synthesis of 1-(1-(2-(2-(2-Methylquinolin-6-yl)acetyl)piperidin-4-yl)-7-(trifluoromethyl)-1,3-dihydro-2H-benzo[d]imidazol-2-one (GEN1-300-3)

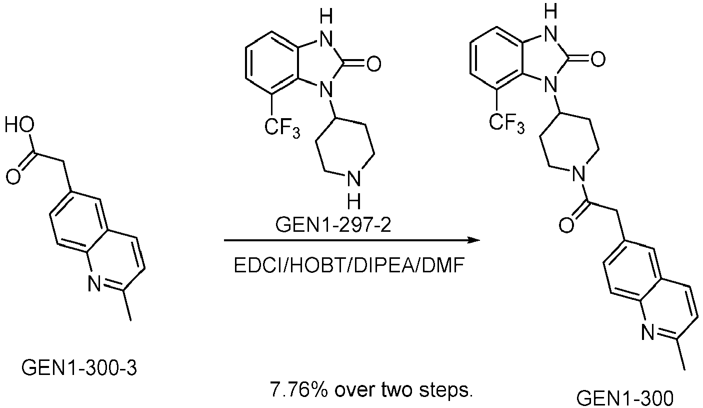

To a solution of 1-(piperidin-4-yl)-7-(trifluoromethyl)-1,3-dihydro-2H-benzo[d]imidazol-2-one (156.9 mg, 0.55 mmol), 2-(2-methylquinolin-6-yl)acetic acid (110 mg, 0.55 mmol), EDCI (158.2 mg, 0.825 mmol) and HOBT (114.5 mg, 0.825 mmol) in DMF (5 mL) was added, at room temperature, DIPEA (142 mg, 1.1 mmol). The resulting mixture was stirred at room temperature for 2 h. The reaction was quenched with water (50 mL). The mixture was extracted with EtOAc (30 mL × 3). The combined organic phases were washed with brine (20 mL × 2), dried over Na₂SO₄, filtered and concentrated to give a brown solid. The crude product was purified through a reversed-phase chromatography column. Purification conditions: C18 silica gel column; mobile phase ACN: H₂O, gradient: 5% to 50%; 45 min, monitoring wavelength: 214 nm. The desired compound (20 mg, yield over two steps: 7.76%) was obtained as a white solid. ¹H NMR (400 MHz, DMSO-d6) δ 11.46 (s, 1H), 8.20 (d, J = 8.4 Hz, 1H), 7.86 (d, J = 8.6 Hz, 1H), 7.75 (d, J = 1.9 Hz, 1H), 7.59 (dd, J = 8.6, 2.0 Hz, 1H), 7.43-7.31 (m, 2H), 7.27 (d, J = 7.7 Hz, 1H), 7.15 (t, J = 7.9 Hz, 1H), 4.63 (d, J = 11.2 Hz, 1H), 4.32-4.15 (m, 2H), 3.96 (d, J = 4.3 Hz, 2H), 3.04 (t, J = 13.2 Hz, 1H), 2.64 (s, 3H), 2.62-2.51 (m, 3H), 1.78-1.63 (m, 2H). MS (ESI) RT = 1.930 min, m/z 469.2 [M+H]⁺, purity: 81.51% @ 214 nm.

### Example 9

### 1-(1-(2-(4-Chloroquinolin-6-yl)acetyl)piperidin-4-yl)-7-(trifluoromethyl)-1,3-dihydro-2H-benzo[d]imidazol-2-one (GEN1-301)

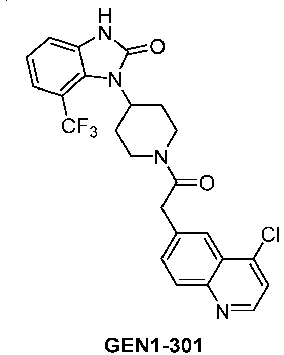

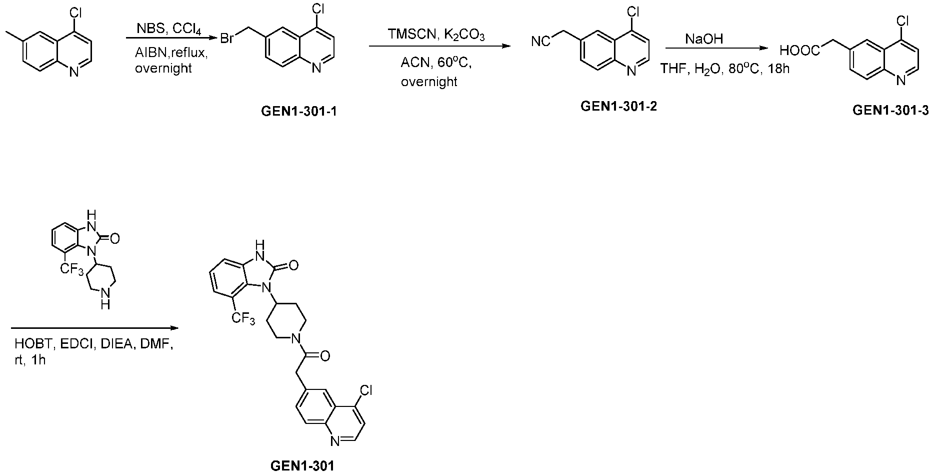

### Step 1. 6-(Bromomethyl)-4-chloroquinoline (GEN1-301-1)

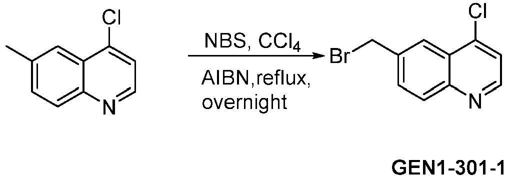

To a solution of 4-chloro-6-methylquinoline (400 mg, 2.26 mmol) in CCl4 (20 mL) were added NBS (442 mg, 2.49 mmol) and AIBN (37 mg, 0.23 mmol). The mixture was stirred at reflux overnight. The mixture was filtered, and the filtrate was dried *in vacuo* by evaporation at 40 °C. The reaction mixture was poured into water (10 mL) and extracted with EA (20 mL × 3). The combined organic layers were washed with water (50 mL) and brine (50 mL), dried over Na2SO4 and concentrated to give a yellow oil. The crude product was purified by silica gel column chromatography using (PE:EA = 2:1) as an eluent to give 6-(bromomethyl)-4-chloroquinoline (316 mg, yield: 55%) as a white solid. MS (ESI) RT = 2.92 min, m/z 255.9 [M+H]+, purity: 95.13% @ 254 nm, 98.35% @ 214 nm.

### Step 2. 2-(4-Chloroquinolin-6-yl)acetonitrile (GEN1-301-2)

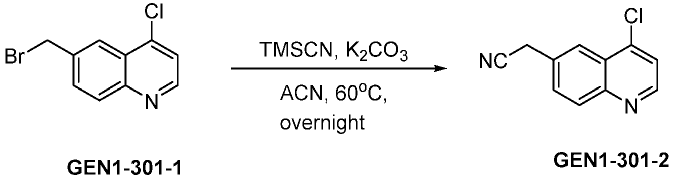

To a solution of 6-(bromomethyl) -4-chloroquinoline (316 mg, 1.24 mmol) in ACN (6 mL) were added, at 0 °C, K₂CO₃ (274 mg, 1.98 mmol) and TMSCN (184 mg, 1.86 mmol). The mixture was stirred at 60 °C overnight. The reaction mixture was poured into water (10 mL) and extracted with EA (20 mL × 3). The combined organic layers were washed with water (20 mL) and brine (20 mL), dried over Na₂SO₄ and concentrated to give a yellow liquid. The crude product was purified by reversed-phase flash chromatography (C₁₈ reversed-phase silica gel column; mobile phase ACN, water, 10% to 85% gradient; 30 min; detection, UV 214 nm) to give 2-(4-chloroquinolin-6-yl)acetonitrile (140 mg, yield: 56%) as a yellow liquid. MS (ESI) RT = 2.27 min, m/z 203.0 [M+H]⁺, purity: 77.17% @ 254 nm, 77.68% @ 214 nm.

### Step 3. 2-(4-Chloroquinolin-6-yl)acetic acid (GEN1-301-3)

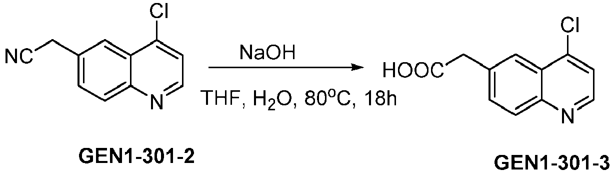

To a mixture of 2-(4-chloroquinolin-6-yl)acetonitrile (140 mg, 0.7 mmol) in THF (2.2 mL) and H₂O (2.2 mL) was added NaOH (111 mg, 2.8 mmol). The mixture was stirred at 80 °C for 18 h. The mixture was then diluted with H₂O (10 mL) and washed with EA (10 mL). The aqueous phase was adjusted to pH 4 with an aqueous hydrochloric acid solution (2 M) and extracted with EA (10 mL × 3). The organic phases were dried over Na₂SO₄ and concentrated to give 2-(4-chloroquinolin-6-yl)acetic acid (100 mg, yield: 65%) as a red solid. MS (ESI) RT = 1.92 min, m/z 222.0 [M+H]⁺, purity: 73.66% @ 254 nm, 95.15% @ 214 nm.

### Step 4. 1-(1-(2-(4-Chloroquinolin-6-yl)acetyl)piperidin-4-yl)-7-(trifluoromethyl)-1,3-dihydro-2H-benzo[d]imidazol-2-one (GEN1-301)

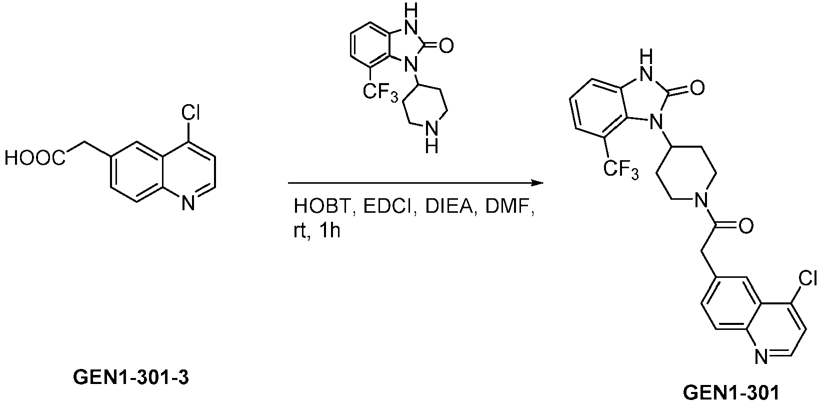

To a solution of 2-(4-chloroquinolin-6-yl)acetic acid (100 mg, 0.45 mmol) in DMF (5 mL) were added 1-(piperidin-4-yl)-7-(trifluoromethyl)-1,3-dihydro-2H-benzo[d]imidazol-2-one (129 mg, 0.45 mmol), EDCI (104 mg, 0.54 mmol), HOBT (73 mg, 0.54 mmol) and DIEA (117 mg, 0.9 mmol). The mixture was stirred at room temperature for 1 h. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 1-(1-(2-(4-chloroquinolin-6-yl)acetyl)piperidin-4-yl)-7-(trifluoromethyl)-1,3-dihydro-2H-benzo [d]imidazol-2-one (30 mg, yield: 14%) as a red solid. ¹H NMR (400 MHz, DMSO-*d₆*) δ 11.45 (s, 1H), 8.81 (d, *J* = 4.8 Hz, 1H), 8.12 - 8.00 (m, 2H), 7.83 - 7.71 (m, 2H), 7.36 (dd, *J* = 8.0, 1.2 Hz, 1H), 7.32 - 7.24 (m, 1H), 7.16 (t, *J* = 8.0 Hz, 1H), 4.62 (d, *J* = 12.0 Hz, 1H), 4.34 - 4.18 (m, 2H), 4.09 (s, 2H), 3.07 (t, *J* = 12.8 Hz, 1H), 2.56 (d, *J* = 12.4 Hz, 3H), 1.73 (d, *J=* 11.2 Hz, 2H). MS (ESI) RT = 2.73 min, m/z 489.2 [M+H]⁺, purity: 92.20% @ 254 nm, 99.14% @ 214 nm.

### Example 10

### 1-(1-(2-(2-Chloroquinolin-6-yl)acetyl)piperidin-4-yl)-7-(trifluoromethyl)-1,3-dihydro-2H-benzo[d]imidazol-2-one (GEN1-302)

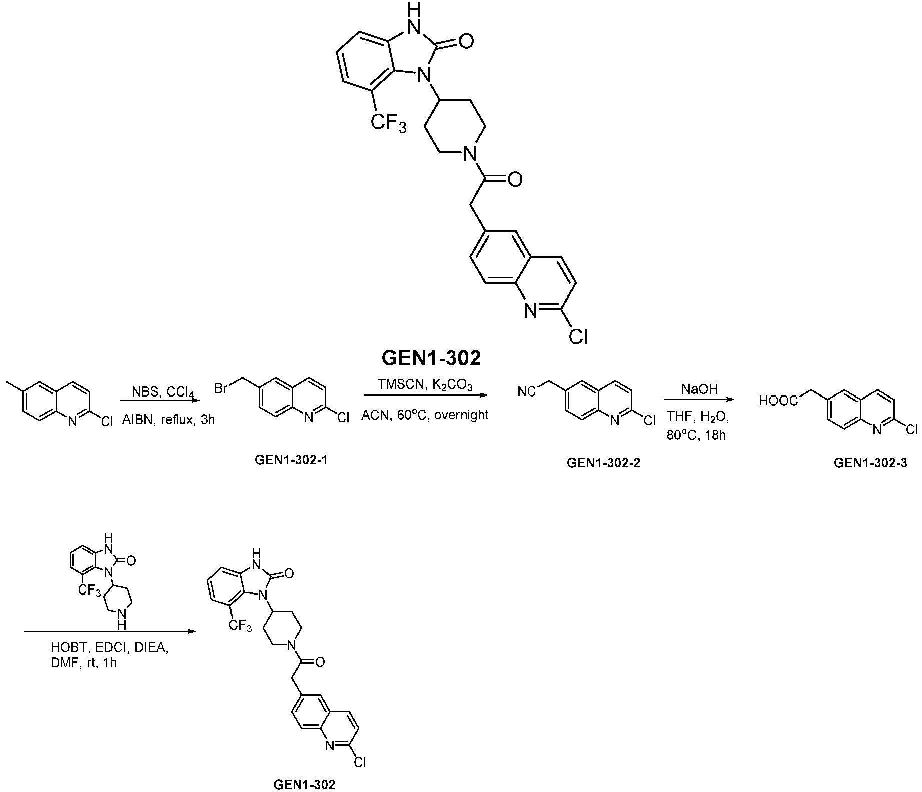

### Step 1. 6-(Bromomethyl)-2-chloroquinoline (GEN1-302-1)

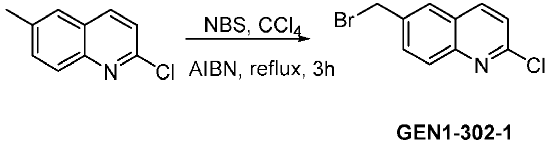

To a solution of 2-chloro-6-methylquinoline (200 mg, 1.13 mmol) in CCl₄ (6 mL) were added NBS (221 mg, 1.24 mmol) and AIBN (19 mg, 0.11 mmol). The mixture was stirred and heated at reflux for 3 h and then cooled to room temperature overnight. The mixture was filtered, and the filtrate was dried *in vacuo* by evaporation at 40 °C. The reaction mixture was poured into water (10 mL) and extracted with EA (20 mL × 3). The combined organic layers were washed with water (50 mL) and brine (50 mL), dried over Na₂SO₄ and concentrated to give a yellow liquid. The crude product was purified by silica gel column chromatography using (PE:EA = 2:1) as an eluent to give 6-(bromomethyl)-2-chloroquinoline (187 mg, yield: 65%) as a white solid. MS (ESI) RT = 3.02 min, m/z 255.9 [M+H]⁺, purity: 100% @ 254 nm, 100% @ 214 nm.

### Step 2. 2-(2-Chloroquinolin-6-yl)acetonitrile (GEN1-302-2)

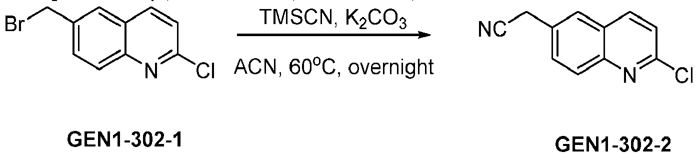

To a solution of 6-(bromomethyl)-2-chloroquinoline (187 mmg, 0.73 mmol) in ACN (6 mL) were added, at 0 °C, K₂CO₃ (162 mg, 1.17 mmol) and TMCSN (109 mg, 1.10 mmol). The mixture was stirred at 60 °C overnight. The reaction mixture was poured into water (10 mL) and extracted with EA (20 mL × 3). The combined organic layers were washed with water (20 mL) and brine (20 mL), dried over Na₂SO₄ and concentrated to give a yellow liquid. The crude product was purified by reversed-phase flash chromatography (C₁₈ reversed-phase silica gel column; mobile phase ACN, water, 10% to 85% gradient; 30 min; detection, UV 214 nm) to give 2-(2-chloroquinolin-6-yl)acetonitrile (120 mg, yield: 81%) as a yellow liquid. MS (ESI) RT = 2.46 min, m/z 203.0 [M+H]⁺, purity: 100% @ 254 nm, 100% @ 214 nm.

### Step 3. 2-(2-Chloroquinolin-6-yl)acetic acid (GEN1-302-3)

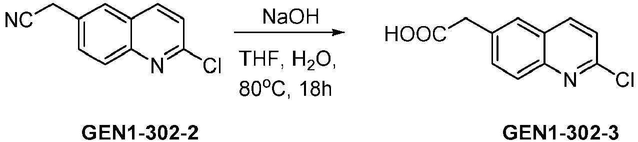

In a sealed tube, to 2-(2-chloroquinolin-6-yl)acetonitrile (60 mg, 0.3 mmol) in THF (1 mL) and H2O (1 mL) was added NaOH (48 mg, 1.2 mmol). The mixture was stirred at 80 °C for 18 h under N₂. The mixture was then diluted with H₂O (10 mL) and washed with EA (10 mL). The aqueous phase was adjusted to pH 4 with an aqueous hydrochloric acid solution (2 M) and extracted with EA (10 mL × 3). The organic phases were dried over Na₂SO₄ and concentrated to give 2-(2-chloroquinolin-6-yl)acetic acid (48 mg, yield: 73%) as a white solid.
TLC:MeOH/(DCM + MeOH) = 10%, Rf = 0.5

### Step 4. 1-(1-(2-(2-Chloroquinolin-6-yl)acetyl)piperidin-4-yl)-7-(trifluoromethyl)-1,3-dihydro-2H-benzo[d]imidazol-2-one (GEN1-302)

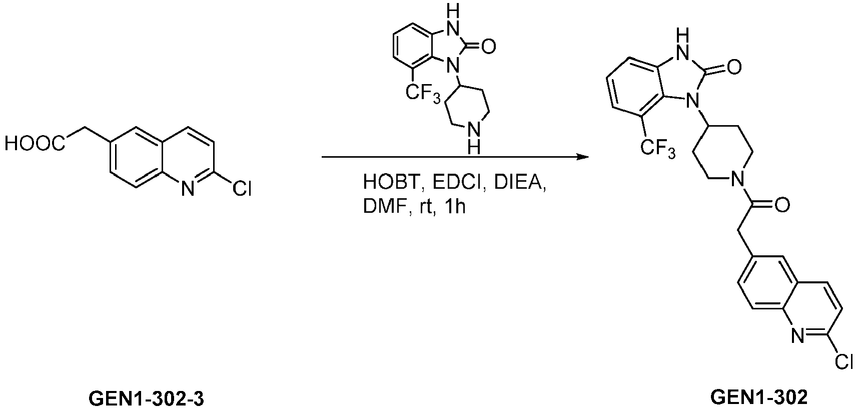

To 2-(2-chloroquinolin-6-yl)acetic acid (48 mg, 0.22 mmol) in DMF (5 mL) were added 1-(piperidin-4-yl)-7-(trifluoromethyl)-1,3-dihydro-2H-benzo[d]imidazol-2-one (62 mg, 0.22 mmol), EDCI (50 mg, 0.26 mmol), HOBT (35 mg, 0.26 mmol) and DIEA (56 mg, 0.44 mmol). The mixture was stirred at room temperature for 1 h. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 1-(1-(2-(2-chloroquinolin-6-yl)acetyl)piperidin-4-yl)-7-(trifluoromethyl)-1,3-dihydro-2H-benzo[d imidazol-2-one (20 mg, yield: 19%) as a white solid. ¹H NMR (400 MHz, DMSO-*d₆*) δ 11.46 (s, 1H), 8.42 (d, *J =* 8.8 Hz, 1H), 7.99 - 7.83 (m, 2H), 7.72 (dd, *J* = 8.8, 2.0 Hz, 1H), 7.57 (d, *J* = 8.8 Hz, 1H), 7.43 - 7.31 (m, 1H), 7.27 (d*, J* = 7.6 Hz, 1H), 7.16 (t, *J* = 8.0 Hz, 1H), 4.62 (d, *J* = 12.0 Hz, 1H), 4.24 (q, *J* = 14.0, 12.4 Hz, 2H), 4.01 (d*, J* = 2.4 Hz, 2H), 3.10 - 2.98 (m, 1H), 2.56 (d, *J* = 11.2 Hz, 3H), 1.71 (d, *J =* 12.0 Hz, 2H). MS (ESI) RT = 2.87 min, m/z 489.2 [M+H]⁺, purity: 100% @ 254 nm, 99.44% @ 214 nm.

### Example 11

### 1-(1-(2-(8-Methylquinolin-6-yl)acetyl)piperidin-4-yl)-7-(trifluoromethyl)-1,3-dihydro-2H-benzo[d]imidazol-2-one (GEN1-303)

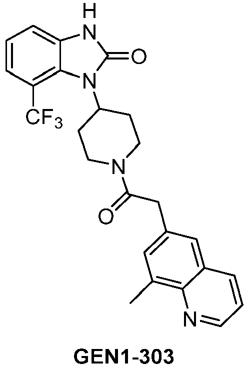

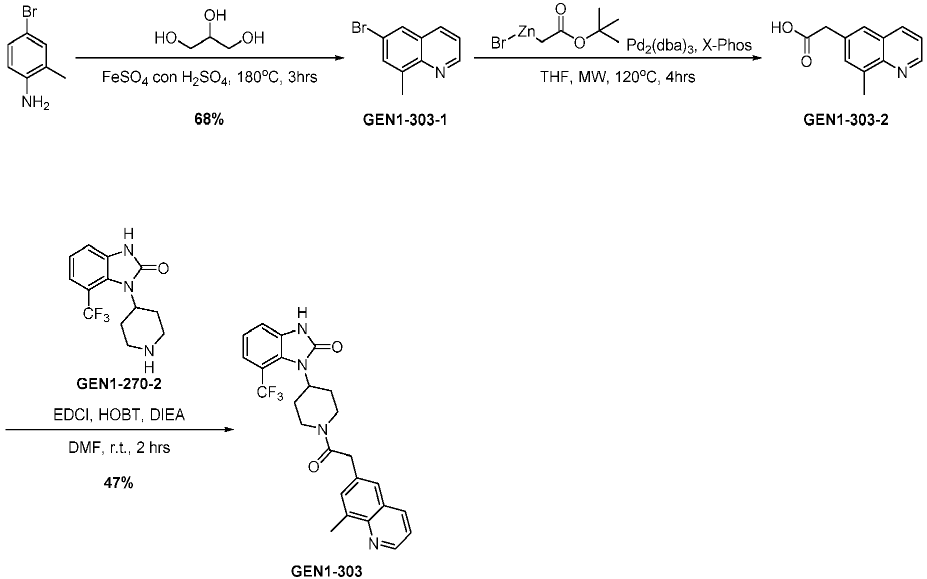

### Step 1. 6-Bromo-8-methylquinoline (GEN1-303-1)

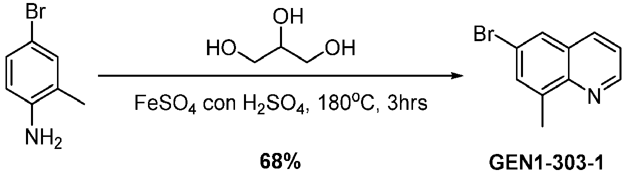

To a mixed solution of 4-bromo-2-methylaniline (1.0 g, 5.40 mmol), Fe₂SO₄ 7H₂O (531 mg, 1.59 mmol), PhNO₂ (406 mg, 3.30 mmol) and glycerol (2.04 g, 22.17 mmol) in DMF (3.0 mL) was slowly added dropwise concentrated sulfuric acid (1.15 mL). The mixture was heated at reflux and stirred for 3 h. The reaction mixture was cooled and poured into 20 mL of ice water, and saturated NaHCO₃ was added to the solution until pH = 8. The solution was filtered and extracted with dichloromethane. The dichloromethane layers were combined, dried over Na₂SO₄ and concentrated. The crude product was purified by silica gel column chromatography using (EA/(EA + PE) = 15%) as an eluent to give 6-bromo-8-methylquinoline (808 mg, yield: 68%) as a pale yellow solid. MS (ESI) RT = 3.025 min, m/z 222.0 [M+H]⁺, purity: 100% @ 254 nm, 100% @ 214 nm.

### Step 2. 2-(8-Methylquinolin-6-yl)acetic acid (GEN1-303-2)

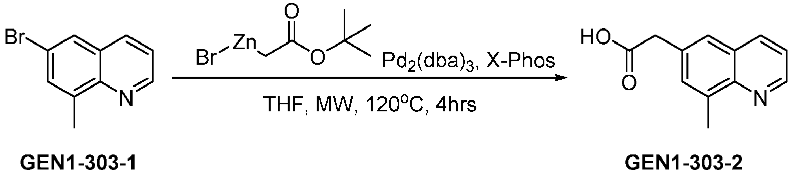

6-Bromo-8-methylquinoline (50 mg, 0.23 mmol), Pd₂(dba)₃ (41 mg, 0.04 mmol) and X-Phos (43 mg, 0.09 mmol) were dissolved in a solution of (2-(tert-butoxy)-2-oxoethyl)zinc(II) bromide (1.8 mL, 0.90 mmol, 0.5 N) in THF under an argon atmosphere. The resulting mixture was reacted in a microwave reactor at 120 °C for 4 h. The reaction mixture was filtered, and the filter cake was washed with THF (10 mL × 3). The filtrate was concentrated under reduced pressure to give a crude product (404 mg), which could be directly used in the next step without further purification. MS (ESI) RT = 1.202 min, m/z 202.1 [M+H]⁺, purity: 66% @ 254 nm, 60% @ 214 nm.

### Step 3. 1-(1-(2-(8-Methylquinolin-6-yl)acetyl)piperidin-4-yl)-7-(trifluoromethyl)-1,3-dihydro-2H-benzo[d]imidazol-2-one (GEN1-303)

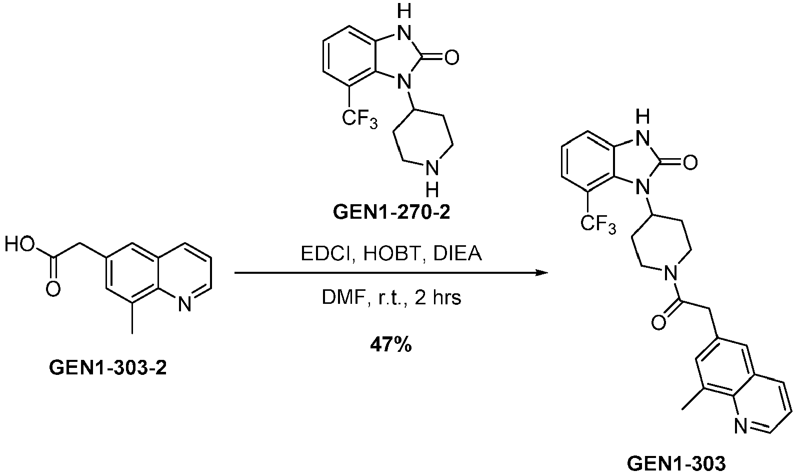

To a solution of 1-(piperidin-4-yl)-7-(trifluoromethyl)-1,3-dihydro-2H-benzo[d]imidazol-2-one (390 mg, 1.37 mmol), 2-(8-methylquinolin-6-yl)acetic acid (250 mg, 1.24 mmol), EDCI (394 mg, 2.05 mmol) and HOBT (277 mg, 2.05 mmol) in DMF (4.0 mL) was added dropwise DIEA (353 mg, 2.74 mmol). The resulting mixture was stirred at room temperature for 2 h. The reaction mixture was purified by reversed-phase flash chromatography (C₁₈ reversed-phase silica gel column; mobile phase CH₃CN, water, 5% to 70% gradient, 50 min; detection, UV 214 nm) to give the desired compound (33 mg, yield: 47%) as a white solid. ¹H NMR (400 MHz, DMSO-*d₆*) δ: 11.47 (s, 1H), 8.91-8.85 (m, 1H), 8.30 (d, *J* = 8.4, 1.7 Hz, 1H), 7.64 (s, 1H), 7.55-7.47 (m, 2H), 7.34 (d, *J* = 8.0 Hz, 1H), 7.26 (d, *J* = 7.7 Hz, 1H), 7.14 (t, *J* = 7.9 Hz, 1H), 4.64 (d, *J* = 10.5 Hz, 1H), 4.30-4.14 (m, 2H), 3.94 (s, 2H), 3.04 (t, *J* = 13.2 Hz, 1H), 2.71 (s, 3H), 2.63-2.51 (m, 3H), 1.70 (t, *J* = 11.4 Hz, 2H). MS (ESI) RT = 2.305 min, m/z 469.2 [M+H]⁺, purity: 100% @ 254 nm, 98.8% @ 214 nm.

### Example 12

### 6-(2-Oxo-2-(4-(2-oxo-7-(trifluoromethyl)-2,3-dihydro-1H-benzo[d]imidazol-1-yl)piperidin-1-yl)ethyl)quinoline-4-carbonitrile (GEN1-304)

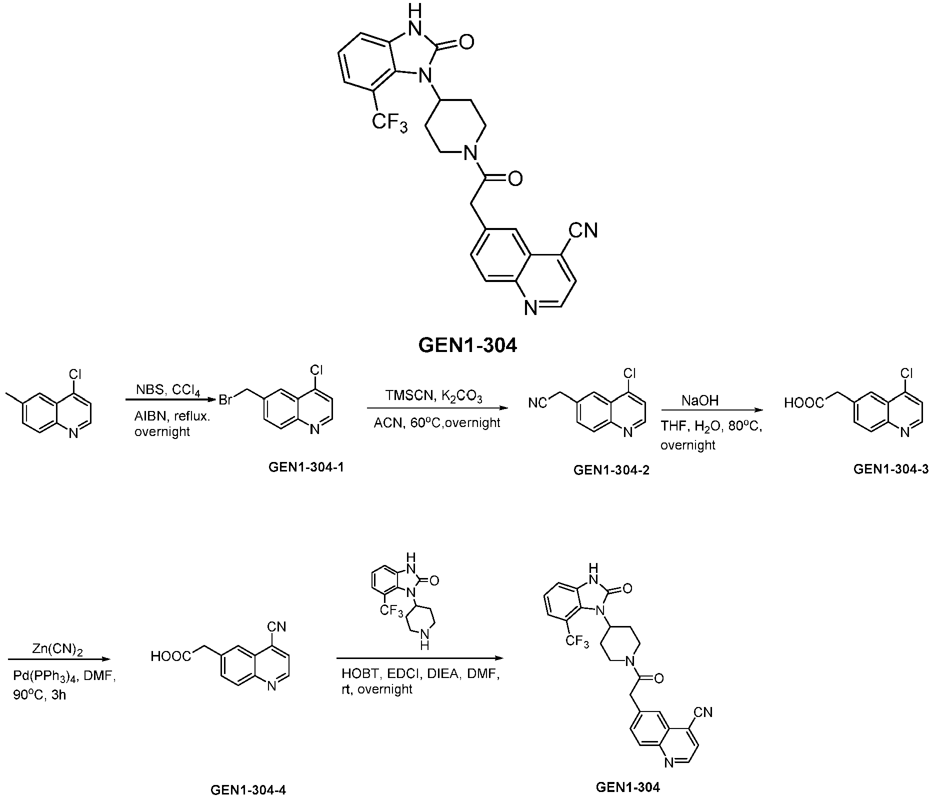

### Step 1. 6-(Bromomethyl)-4-chloroquinoline (GEN1-304-1)

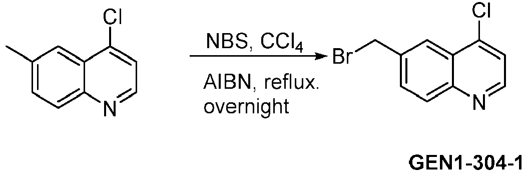

To a solution of 4-chloro-6-methylquinoline (400 mg, 2.26 mmol) in CCl₄ (20 mL) were added NBS (442 mg, 2.49 mmol) and AIBN (37 mg, 0.23 mmol). The mixture was stirred and heated at reflux overnight. The mixture was filtered, and the filtrate was dried *in vacuo* by evaporation at 40 °C. The reaction mixture was poured into water (10 mL) and extracted with EA (20 mL × 3). The combined organic layers were washed with water (50 mL) and brine (50 mL), dried over Na₂SO₄ and concentrated to give a yellow liquid. The crude product was purified by silica gel column chromatography using (PE:EA = 2:1) as an eluent to give 6-(bromomethyl)-4-chloroquinoline (353 mg, yield: 61%) as a white solid. MS (ESI) RT = 2.93 min, m/z 255.9 [M+H]+, purity: 72.29% @ 254 nm, 49.39% @ 214 nm.

### Step 2. 2-(4-Chloroquinolin-6-yl)acetonitrile (GEN1-304-2)

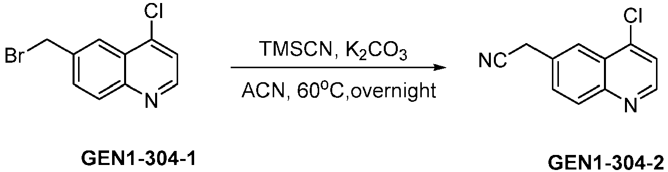

To a solution of 6-(bromomethyl) -4-chloroquinoline (353 mg, 1.38 mmol) in ACN (6 mL) were added, at 0 °C, K₂CO₃ (306 mg, 2.22 mmol) and TMCSN (206 mg, 2.08 mmol). The mixture was stirred at 60 °C overnight. The reaction mixture was poured into water (10 mL) and extracted with EA (20 mL × 3). The combined organic layers were washed with water (20 mL) and brine (20 mL), dried over Na₂SO₄ and concentrated to give a yellow liquid. The crude product was purified by reversed-phase flash chromatography (C₁₈ reversed-phase silica gel column; mobile phase ACN, water, 10% to 85% gradient; 30 min; detection, UV 214 nm) to give 2-(4-chloroquinolin-6-yl)acetonitrile (180 mg, yield: 64%) as a yellow liquid. MS (ESI) RT = 2.27 min, m/z 203.0 [M+H]⁺, purity: 79.08% @ 254 nm, 68.48% @ 214 nm.

### Step 3. 2-(4-Chloroquinolin-6-yl)acetic acid (GEN1-304-3)

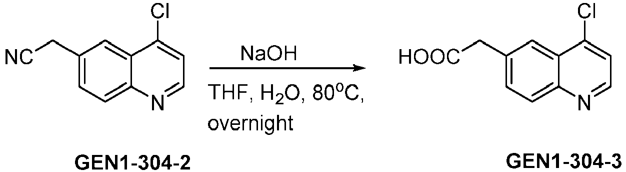

In a sealed tube, to 2-(4-chloroquinolin-6-yl)acetonitrile (80 mg, 0.40 mmol) in THF (1.6 mL) and H₂O (1.6 mL) was added NaOH (63 mg, 1.58 mmol). The mixture was stirred at 80 °C overnight. The mixture was then diluted with H₂O (10 mL) and washed with EA (10 mL). The aqueous phase was adjusted to pH 4 with an aqueous hydrochloric acid solution (2 M) and extracted with EA (10 mL × 3). The organic phases were dried over Na₂SO₄ and concentrated to give 2-(4-chloroquinolin-6-yl)acetic acid (75 mg, yield: 86%) as a red solid. MS (ESI) RT = 1.91 min, m/z 222.0 [M+H]⁺, purity: 34.16% @ 254 nm, 43.93% @ 214 nm.

### Step 4. 2-(4-Cyanoquinolin-6-yl)acetic acid (GEN1-304-4)

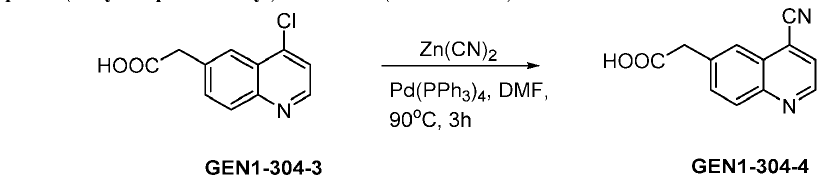

To a solution of 2-(4-chloroquinolin-6-yl)acetic acid (75 mg, 0.34 mmol) in DMF (6 mL) were added Zn(CN)₂ (44 mg, 0.38 mmol) and Pd(PPh₃)₄ (78 mg, 0.07 mmol). The mixture was stirred at 90 °C for 3 h. The reaction mixture was poured into water (10 mL) and extracted with EA (20 mL × 3). The combined organic layers were washed with water (50 mL) and brine (50 mL), dried over Na₂SO₄ and concentrated to give a yellow oil. The crude product was purified by reversed-phase flash chromatography (C₁₈ reversed-phase silica gel column; mobile phase ACN, water, 10% to 85% gradient; 30 min; detection, UV 214 nm) to give 2-(4-cyanoquinolin-6-yl)acetic acid (38 mg, yield: 53%) as a yellow solid. MS (ESI) RT = 1.88 min, m/z 213.1 [M+H]⁺, purity: 7.02% @ 254 nm, 20.19% @ 214 nm.

### Step 5. 6-(2-Oxo-2-(4-(2-oxo-7-(trifluoromethyl)-2,3-dihydro-1H-benzo[d]imidazol-1-yl)piperidin-1-yl)ethyl)quinoline-4-carbonitrile (GEN1-304)

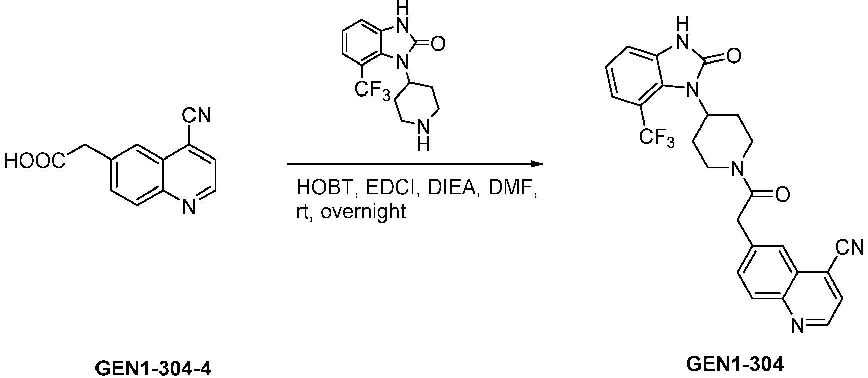

To 2-(4-cyanoquinolin-6-yl)acetic acid (38 mg, 0.18 mmol) in DMF (6 mL) were added 1-(piperidin-4-yl)-7-(trifluoromethyl)-1,3-dihydro-2H-benzo[d]imidazol-2-one (56 mg, 0.2 mmol), EDCI (41 mg, 0.21 mmol), HOBT (29 mg, 0.21 mmol) and DIEA (46 mg, 0.36 mmol). The mixture was stirred at room temperature overnight. The mixture was then diluted with H₂O (20 mL) and extracted with EA (20 mL × 3). The combined organic layers were washed with water (20 mL) and brine (20 mL), dried over Na₂SO₄ and concentrated to give a yellow liquid. The crude product was purified by reversed-phase flash chromatography (C₁₈ reversed-phase silica gel column; mobile phase ACN, water, 10% to 85% gradient; 30 min; detection, UV 214 nm) to give 6-(2-oxo-2-(4-(2-oxo-7-(trifluoromethyl)-2,3-dihydro-1H-benzo[d]imidazol-1-yl)piperidin]-1-yl)ethyl)quinoline-4-carbonitrile (60 mg, yield: 70%) as a white solid. ¹H NMR (400 MHz, DMSO-*d₆*) δ 11.45 (s, 1H), 9.08 (d, *J* = 4.4 Hz, 1H), 8.25-8.09 (m, 2H), 8.03 (s, 1H), 7.85 (d, *J* = 8.8 Hz, 1H), 7.36 (d, *J* = 8.0 Hz, 1H), 7.27 (d, *J* = 7.6 Hz, 1H), 7.16 (t, *J* = 8.0 Hz, 1H), 4.62 (d, *J* = 12.0 Hz, 1H), 4.25 (d, *J* = 12.8 Hz, 2H), 4.15 (s, 2H), 3.07 (t, *J* = 13.2 Hz, 1H), 2.77-2.53 (m, 3H), 1.72 (d, *J* = 12.0 Hz, 2H). MS (ESI) RT = 2.66 min, m/z 480.2 [M+H]⁺, purity: 100% @ 254 nm, 100% @ 214 nm.

### Example 13

### 1-(1-(2-(2-(2-Methoxyquinolin-6-yl)acetyl)piperidin-4-yl)-7-(trifluoromethyl)-1,3-dihydro-2H-benzo[d]imidazol-2-one (GEN1-305)

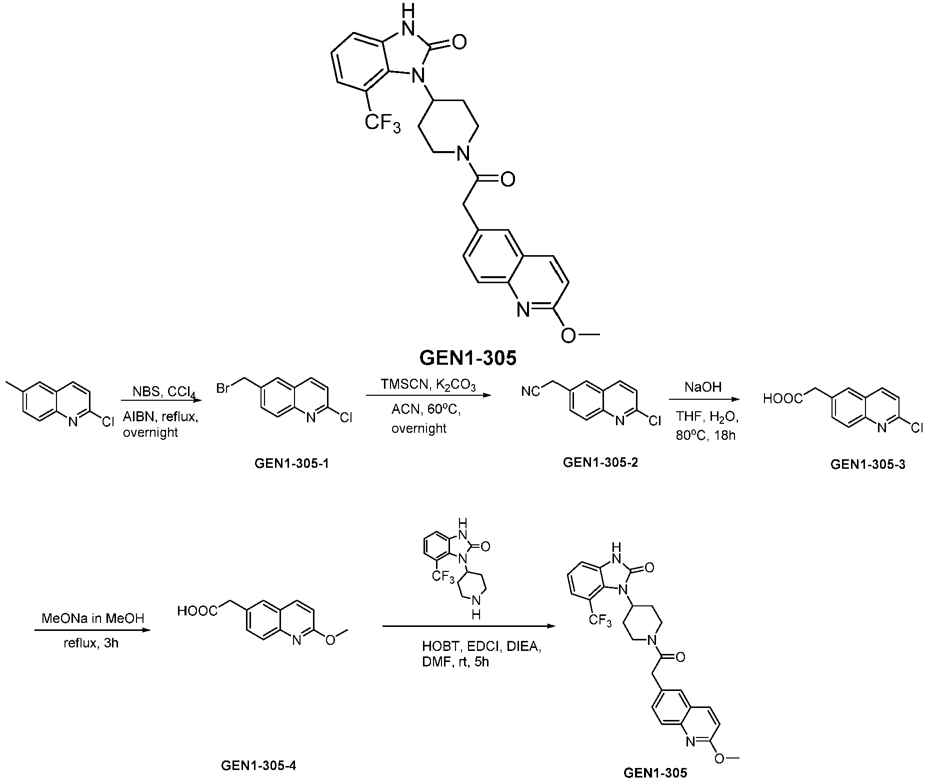

### Step 1. 6-(Bromomethyl)-2-chloroquinoline (GEN1-305-1)

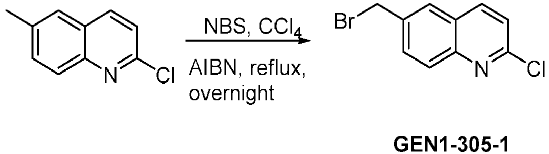

To a solution of 2-chloro-6-methylquinoline (400 mg, 2.26 mmol) in CCl₄ (20 mL) were added NBS (442 mg, 2.48 mmol) and AIBN (37 mg, 0.23 mmol). The mixture was stirred and heated at reflux overnight. The mixture was filtered, and the filtrate was dried *in vacuo* by evaporation at 40 °C. The reaction mixture was poured into water (10 mL) and extracted with EA (20 mL × 3). The combined organic layers were washed with water (50 mL) and brine (50 mL), dried over Na₂SO₄ and concentrated to give a yellow liquid. The crude product was purified by silica gel column chromatography using (PE:EA = 2:1) as an eluent to give 6-(bromomethyl)-2-chloroquinoline (399 mg, yield: 69%) as a white solid. MS (ESI) RT = 3.02 min, m/z 255.9 [M+H]⁺, purity: 95.79% @ 254 nm, 23.70% @ 214 nm.

### Step 2. 2-(2-Chloroquinolin-6-yl)acetonitrile (GEN1-305-2)

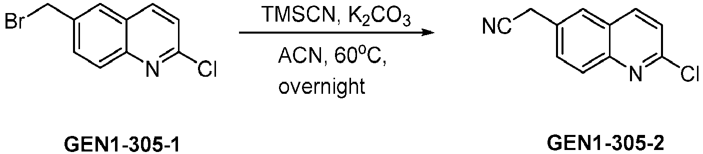

To a solution of 6-(bromomethyl) -2-chloroquinoline (399 mg, 1.56 mmol) in ACN (6 mL) were added, at 0 °C, K₂CO₃ (345 mg, 2.5 mmol) and TMSCN (232 mg, 2.34 mmol). The mixture was stirred at 60 °C overnight. The reaction mixture was poured into water (10 mL) and extracted with EA (20 mL × 3). The combined organic layers were washed with water (20 mL) and brine (20 mL), dried over Na₂SO₄ and concentrated to give a yellow liquid. The crude product was purified by reversed-phase flash chromatography (C₁₈ reversed-phase silica gel column; mobile phase ACN, water, 10% to 85% gradient; 30 min; detection, UV 214 nm) to give 2-(2-chloroquinolin-6-yl)acetonitrile (250 mg, yield: 79%) as a yellow solid. MS (ESI) RT = 2.47 min, m/z 203.0 [M+H]⁺, purity: 80.78% @ 254 nm, 75.07% @ 214 nm.

### Step 3. 2-(2-Chloroquinolin-6-yl)acetic acid (GEN1-305-3)

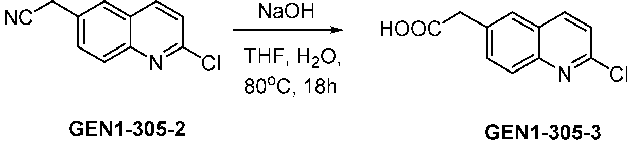

In a sealed tube, to a mixture of 2-(2-chloroquinolin-6-yl)acetonitrile (100 mg, 0.5 mmol) in THF (1.6 mL) and H₂O (1.6 mL) was added NaOH (79 mg, 1.98 mmol). The mixture was stirred at 80 °C for 18 h. The mixture was then diluted with H₂O (10 mL) and washed with EA (10 mL). The aqueous phase was adjusted to pH 4 with an aqueous hydrochloric acid solution (2 M) and extracted with EA (10 mL × 3). The organic phases were dried over Na₂SO₄ and concentrated to give 2-(2-chloroquinolin-6-yl)acetic acid (100 mg, yield: 92%) as a white solid. MS (ESI) RT = 2.18 min, m/z 222.0 [M+H]⁺, purity: 50.98% @ 254 nm, 14.63% @ 214 nm.

### Step 4. 2-(2-Methoxyquinolin-6-yl)acetic acid (GEN1-305-4)

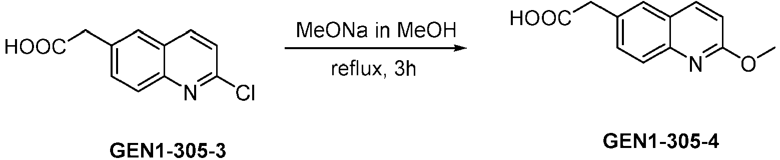

2-(2-Chloroquinolin-6-yl)acetic acid (100 mg, 0.45 mmol) was added to a solution of MeONa in MeOH (4 mL), and the mixture was heated at reflux for 3 h. The mixture was then diluted with H₂O (10 mL) and washed with EA (10 mL). The aqueous phase was adjusted to pH 4 with an aqueous hydrochloric acid solution (2 M) and extracted with EA (10 mL × 3). The organic phases were dried over Na₂SO₄ and concentrated to give 2-(2-methoxyquinolin-6-yl)acetic acid (87 mg, yield: 84%) as a white solid. MS (ESI) RT = 2.21 min, m/z 218.1 [M+H]⁺, purity: 78.77%: @ 254 nm, 85.78% @ 214 nm.

### Step 5. 1-(1-(2-(2-(2-Methoxyquinolin-6-yl)acetyl)piperidin-4-yl)-7-(trifluoromethyl)-1,3-dihydro-2H-benzo[d]imidazol-2-one (GEN1-305)

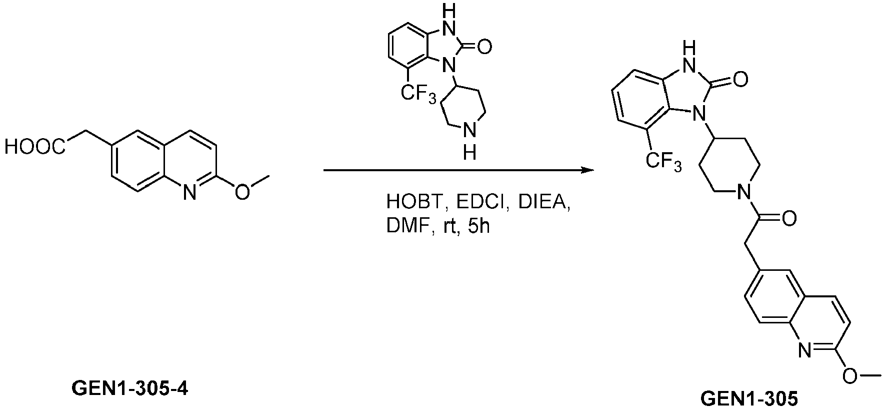

To 2-(2-methoxyquinolin-6-yl)acetic acid (87 mg, 0.40 mmol) in DMF (5 mL) were added 1-(piperidin-4-yl)-7-(trifluoromethyl)-1,3-dihydro-2H-benzo[d]imidazol-2-one (126 mg, 0.44 mmol), EDCI (92 mg, 0.48 mmol), HOBT (65 mg, 0.48 mmol) and DIEA (103 mg, 0.80 mmol). The mixture was stirred at room temperature for 5 h. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 1-(1-(2-(2-(2-methoxyquinolin-6-yl)acetyl)piperidin-4-yl)-7-(trifluoromethyl)-1,3-dihydro-2H-benzo[d]imidazol-2-one (50 mg, yield: 26%) as a white solid. ¹H NMR (400 MHz, DMSO-*d₆*) δ 11.45 (s, 1H), 8.20 (d, *J* = 8.8 Hz, 1H), 7.73 (d, *J* = 8.0 Hz, 2H), 7.55 (dd, *J* = 8.8, 2.0 Hz, 1H), 7.36 (d, *J* = 8.0 Hz, 1H), 7.27 (d, *J* = 7.6 Hz, 1H), 7.15 (t*, J* = 8.0 Hz, 1H), 7.00 (d, *J* = 8.8 Hz, 1H), 4.63 (d, *J* = 11.2 Hz, 1H), 4.30-4.14 (m, 2H), 3.95 (d, *J* = 22.8 Hz, 5H), 3.03 (t, *J* = 13.2 Hz, 1H), 2.55 (d, *J* = 10.8 Hz, 3H), 1.72 (d, *J* = 10.8 Hz, 2H). MS (ESI) RT = 2.91 min, m/z 485.3 [M+H]⁺, purity: 95.75% @ 254 nm, 99.64% @ 214 nm.

### Example 14

### 1-(1-(2-(5-Chloroquinolin-6-yl)acetyl)piperidin-4-yl)-7-(trifluoromethyl)-1,3-dihydro-2H-benzo[d]imidazol-2-one (GEN1-306)

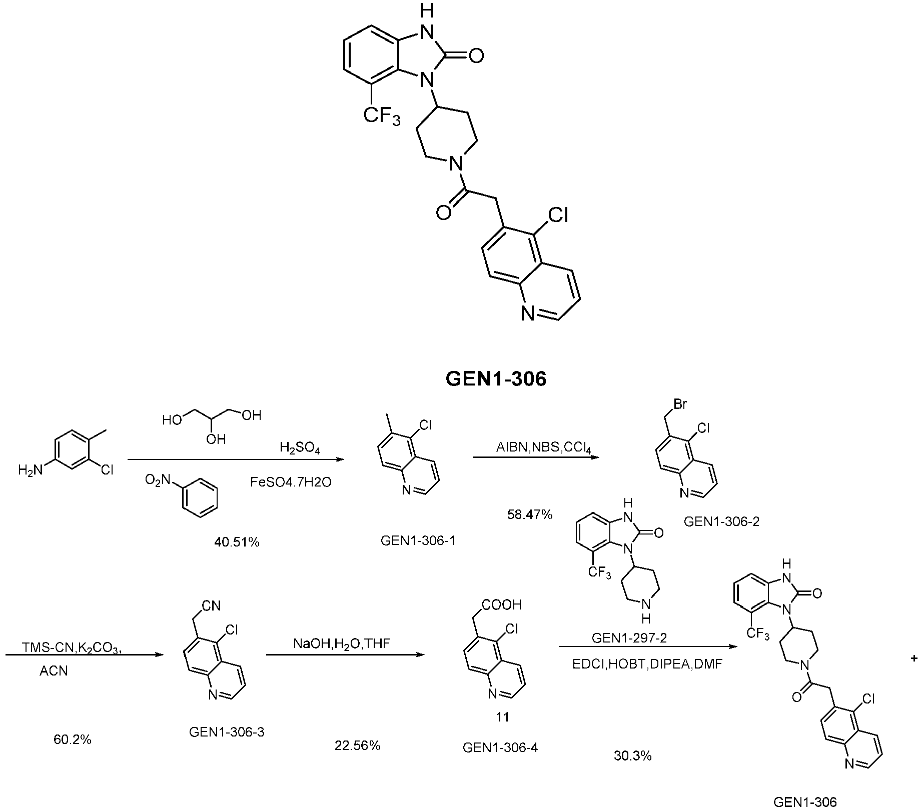

### Step 1. Synthesis of 5-chloro-6-methylquinoline (GEN1-306-1)

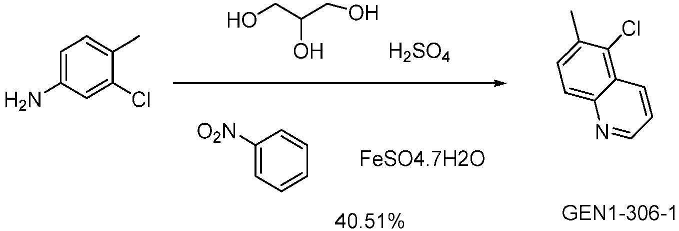

To a mixture of 3-chloro-4-methylaniline (3.05 g, 21.54 mmol), propane-1,2,3-triol (8.16 g, 88.6 mmol) and FeSO₄.7H₂O (2.417 g, 8.695 mmol) in nitrobenzene (1.624 g, 13.19 mmol) was added, at room temperature, H₂SO₄ (4.6 mL, 86.16 mmol). The resulting mixture was stirred at 140 °C for 3 h. The reaction was quenched with ice water and made basic with sodium bicarbonate solution. The resulting mixture was extracted with DCM (80 mL × 3). The combined organic phases were washed with brine (20 mL × 2), dried over Na₂SO₄, filtered and concentrated to give a brown solid. The crude product was purified by silica gel column chromatography (PE:EA = 4:1) to give the desired compound (1.55 g, yield: 40.51%) as a yellow solid. MS (ESI) RT = 2.73 min, m/z 178.1 [M+H]⁺, purity: 100% @ 254 nm, 63.19%, @ 214 nm.

### Step 2. 6-(Bromomethyl)-5-chloroquinoline (GEN1-306-2)

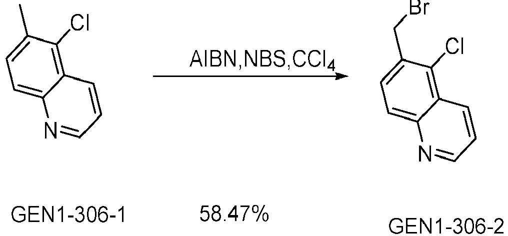

To a mixture of 5-chloro-6-methylquinoline (532.89 mg, 3 mmol) and AIBN (49.26 mg, 0.3 mmol) in CCl₄ (10 mL) was added, at room temperature under a nitrogen atmosphere, NBS (560.64 mg, 3.15 mmol). The mixture was then stirred at 75 °C for 6 h. The reaction solution was quenched with water (50 mL). The mixture was extracted with DCM (40 mL × 3). The combined organic phases were washed with brine (20 mL × 2), dried over Na₂SO₄, filtered and concentrated to give a yellow solid. The crude product was purified by silica gel column chromatography using (PE:EA = 3:1) as an eluent to give the desired compound (450 mg, yield: 58.47%) as a white solid. MS (ESI) RT = 2.873 min, m/z 255.9 [M+H]⁺, purity: 97.68% @ 254 nm.

### Step 3. Synthesis of 2-(5-chloroquinolin-6-yl)acetonitrile (GEN1-306-3)

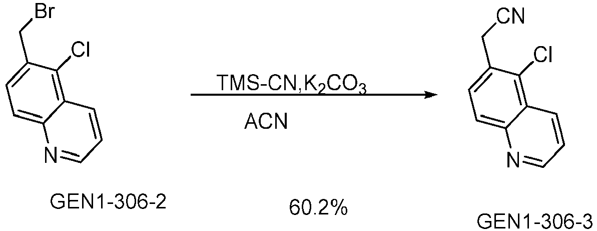

To a suspension of 6-(bromomethyl)-5-chloroquinoline (256.53 mg, 1 mmol) and K₂CO₃ (158.94 mg, 1.15 mmol) in ACN (5 mL) was added, at room temperature under a nitrogen atmosphere, TMS-CN (143.85 mg, 1.45 mmol). The resulting mixture was stirred at 60 °C for 3 h. The mixture was filtered. The filtrate was concentrated and purified by silica gel column chromatography using (PE:EA = 3:1 to 1:1) as an eluent to give the desired compound (122 mg, yield: 60.2%) as a white solid. MS (ESI) RT = 2.210 min, m/z 203.0 [M+H]⁺, purity: 100% @ 254 nm.

### Step 4. Synthesis of 2-(5-chloroquinolin-6-yl)acetic acid (GEN1-306-4)

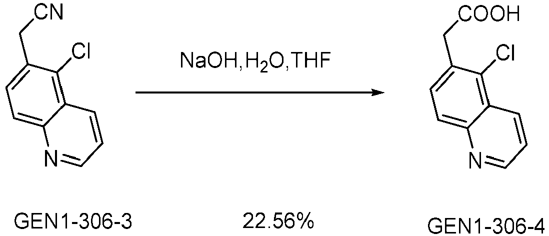

To a solution of 2-(5-chloroquinolin-6-yl)acetonitrile (122 mg, 0.6 mmol) in THF (1 mL) and H₂O (1 mL) was added NaOH (96 mg, 2.4 mmol). The resulting mixture was stirred at 80 °C for 6 h. The mixture was then stirred at 100 °C for 6 h. The solution was adjusted to pH 4 and concentrated. The reaction solution was purified by C18 column chromatography (ACN:H₂O = 0% to 50%) within 45 min to give the desired compound (30 mg, yield: 22.56%) as a white solid. MS (ESI) RT = 1.810 min, m/z 222.0 [M+H]⁺, purity: 16.6% @ 254 nm, 31.8% @ 214 nm.

### Step 5. Synthesis of 1-(1-(2-(5-chloroquinolin-6-yl)acetyl)piperidin-4-yl)-7-(trifluoromethyl)-1,3-dihydro-2H-benzo[d]imidazol-2-one (GEN1-306)

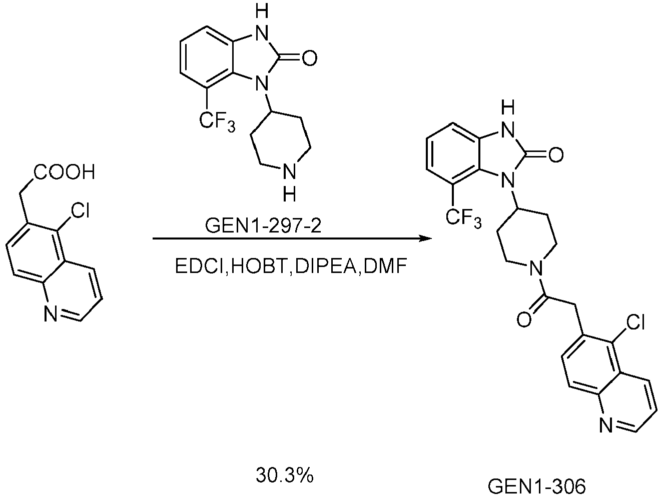

To a solution of 1-(piperidin-4-yl)-7-(trifluoromethyl)-1,3-dihydro-2H-benzo[d]imidazol-2-one (38.6 mg, 0.135 mmol), 2-(5-chloroquinolin-6-yl)acetic acid (30 mg, 0.135 mmol), EDCI (38.82 mg, 0.2025 mmol) and HOBT (27.36 mg, 0.2025 mmol) in DMF (5 mL) was added DIPEA (34.87 mg, 0.27 mmol). The resulting mixture was stirred at room temperature for 2 h. The reaction was quenched with water (50 mL). The mixture was extracted with EA (30 mL × 3). The combined organic phases were washed with brine (20 mL × 2), dried over Na₂SO₄ and concentrated to give a brown solid. The mixture was then filtered. The filtrate was concentrated, and the crude product was purified by reversed-phase chromatography. Purification conditions: C18 silica gel column; mobile phase ACN: H₂O, gradient: 5% to 50%; 45 min, monitoring wavelength: 214 nm. The desired compound (20 mg, yield: 30.3%) was obtained as a white solid. ¹H NMR (400 MHz, DMSO-d6) δ 11.57 (s, 1H), 9.03 (dd, J = 4.2, 1.6 Hz, 1H), 8.66 (dt, J = 8.7, 1.1 Hz, 1H), 8.04 (d, J = 8.6 Hz, 1H), 7.80 (d, J = 8.7 Hz, 1H), 7.75 (dd, J = 8.6, 4.2 Hz, 1H), 7.42 (dd, J = 8.1, 1.2 Hz, 1H), 7.35 (dd, J = 7.8, 1.1 Hz, 1H), 7.22 (t, J = 7.9 Hz, 1H), 4.64 (d, J = 12.2 Hz, 1H), 4.42-4.08 (m, 4H), 3.27-3.12 (m, 1H), 2.85-2.58 (m, 3H), 1.91-1.68 (m, 2H). MS (ESI):Rt = 2.687 min; *m*/*z* 489.2 [M+H]⁺ purity: 100% @254 nm, 99.82% @214 nm.

### Example 15

### 1-(1-(2-(8-Chloroquinolin-6-yl)acetyl)piperidin-4-yl)-7-(trifluoromethyl)-1,3-dihydro-2H-benzo[d]imidazol-2-one (GEN1-307)

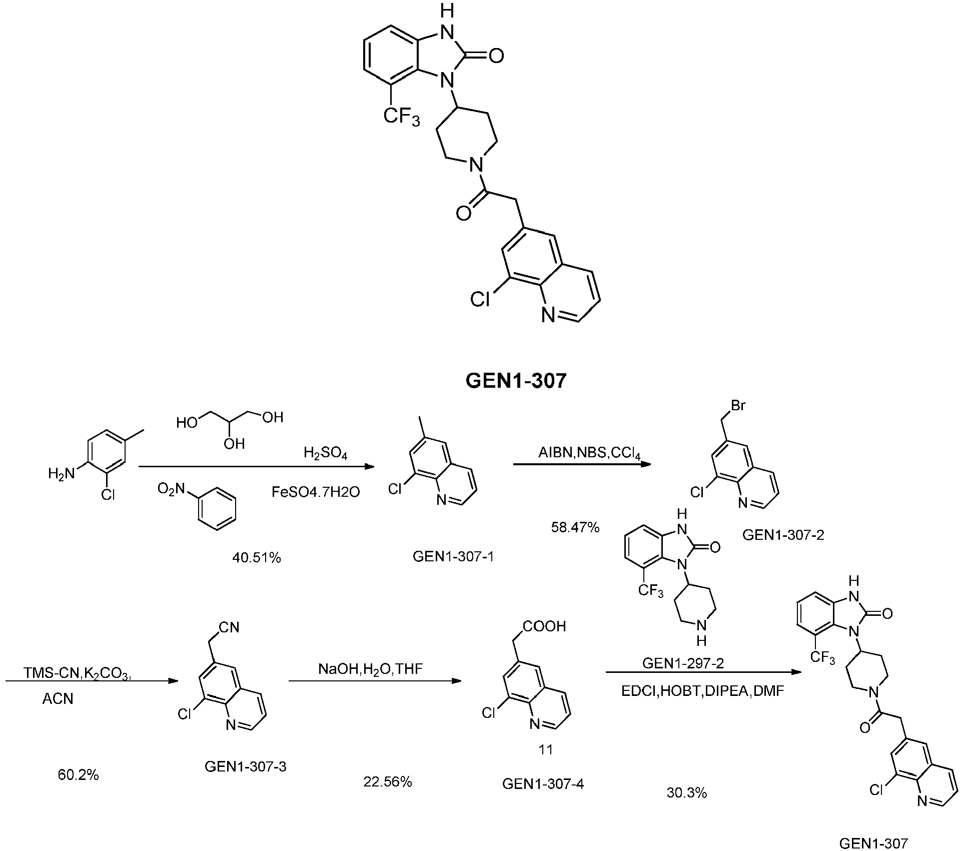

### Step 1. Synthesis of 8-chloro-6-methylquinoline (GEN1-307-1)

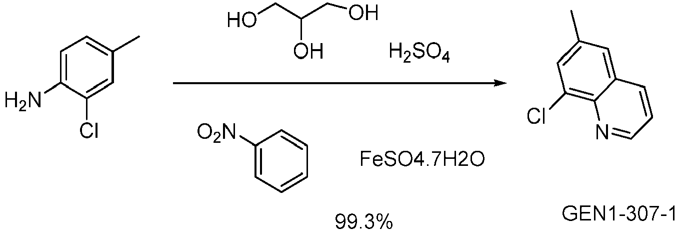

To a mixture of 2-chloro-4-methylaniline (3.05 g, 21.54 mmol), propane-1,2,3-triol (8.16 g, 88.6 mmol) and FeSO₄.7H₂O (2.417 g, 8.695 mmol) in nitrobenzene (1.624 g, 13.19 mmol) was added, at room temperature, H₂SO₄ (4.6 mL, 86.16 mmol). The mixture was then stirred at 140 °C for 3 h. The reaction solution was quenched with ice water and made basic with sodium bicarbonate solution. The mixture was extracted with DCM (80 mL × 3). The combined organic phases were washed with brine (20 mL × 2), dried over Na₂SO₄, filtered and concentrated to give a brown solid. The crude product was purified by column chromatography on silica gel (PE:EA= 3:1) to give the desired compound (3.8 g, yield: 99.3%) as a yellow solid. MS (ESI) RT = 2.568 min, m/z 178.1 [M+H]⁺, purity: 94.19% @ 254 nm.

### Step 2. 6-(Bromomethyl)-8-chloroquinoline (GEN1-307-2)

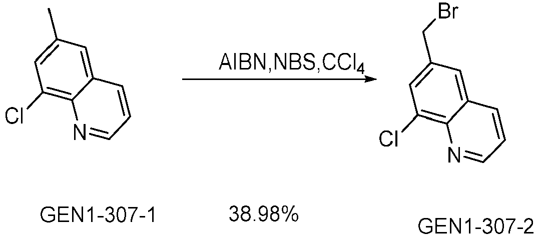

To a mixture of 8-chloro-6-methylquinoline (1.066 g, 6 mmol) and AIBN (98.53 mg, 0.6 mmol) in CCl₄ (30 mL) was added, at room temperature under a nitrogen atmosphere, NBS (1.12 g, 6.3 mmol). The resulting mixture was stirred at 75 °C for 6 h. The reaction was concentrated and purified by silica gel column chromatography using (PE:EA = 3:1) as an eluent to give the desired compound (600 mg, yield: 38.98%) as a pale yellow solid. MS (ESI) RT = 2.697 min, m/z 255.9 [M+H]⁺, purity: 93.17% @ 254 nmm.

### Step 3. Synthesis of 2-(8-chloroquinolin-6-yl)acetonitrile (GEN1-306-3)

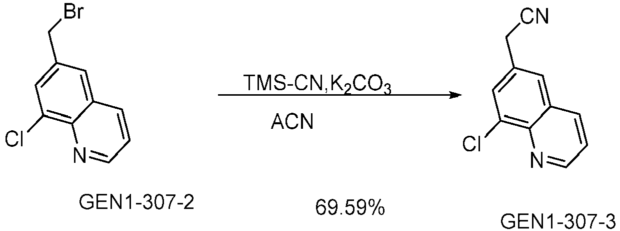

To a suspension of 6-(bromomethyl)-8-chloroquinoline (600 mg, 2.34 mmol) and K₂CO₃ (371.9 mg, 2.69 mmol) in ACN (10 mL) was added, at room temperature under a nitrogen atmosphere, TMS-CN (464.3 mg, 4.68 mmol). The resulting mixture was stirred at 60 °C for 16 h. To the mixture were then added K₂CO₃ (371.9 mg, 2.69 mmol) and TMS-CN (464.3 mg, 4.68 mmol). The resulting mixture was stirred at 60 °C for 6 h. The mixture was filtered. The filtrate was concentrated and purified by silica gel column chromatography using (PE:EA = 3:1 to 1:1) as an eluent to give the desired compound (330 mg, yield: 69.59%) as a pale yellow solid. MS (ESI) RT = 2.116 min, m/z 203.0 [M+H]⁺, purity: 96.59% @ 254 nm.

### Step 4. Synthesis of 2-(8-chloroquinolin-6-yl)acetic acid (GEN1-307-4)

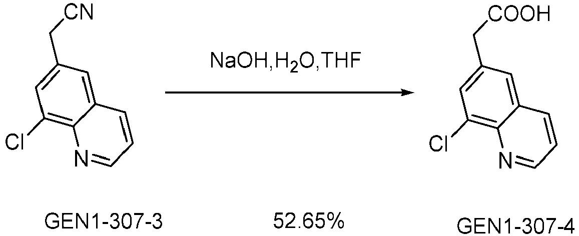

To a solution of 2-(8-chloroquinolin-6-yl)acetonitrile (330 mg, 1.628 mmol) in THF (5 mL) and H2O (5 mL) was added NaOH (260.48 mg, 6.512 mmol). The resulting mixture was stirred at 80 °C for 48 consecutive hours. The solution was adjusted to pH 4 and concentrated. The residual crude product was purified by reversed-phase chromatography. Purification conditions: C18 silica gel column; mobile phase ACN: H₂O, gradient: 5% to 50%; 45 min, monitoring wavelength: 214 nm. The desired compound (190 mg, yield: 52.65%) was obtained as a white solid. MS (ESI) RT = 1.811 min, m/z 222.0 [M+H]⁺, purity: 41.9% @ 254 nm, 33.25% @ 214 nm.

### Step 5. Synthesis of 1-(1-(2-(8-chloroquinolin-6-yl)acetyl)piperidin-4-yl)-7-(trifluoromethyl)-1,3-dihydro-2H-benzo[d]imidazol-2-one (GEN1-307)

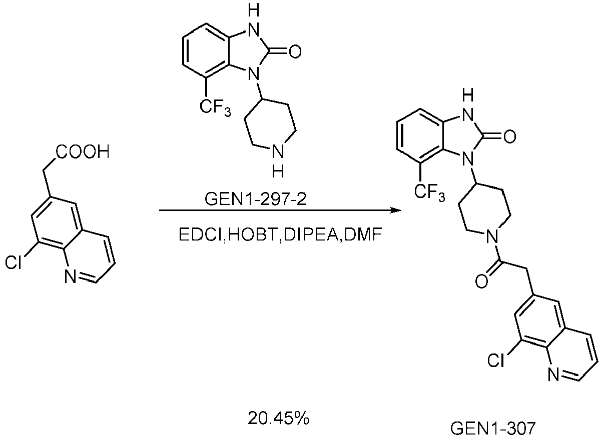

To a solution of 1-(piperidin-4-yl)-7-(trifluoromethyl)-1,3-dihydro-2H-benzo[d]imidazol-2-one (57.05 mg, 0.2 mmol), 2-(8-chloroquinolin-6-yl)acetic acid (44.32 mg, 0.2 mmol), EDCI (57.51 mg, 0.3 mmol) and HOBT (40.54 mg, 0.3 mmol) in DMF (2 mL) was added DIPEA (51.66 mg, 0.4 mmol). The resulting mixture was stirred at room temperature for 2 h. The reaction was quenched with water (30 mL). The mixture was extracted with EA (20 mL × 3). The combined organic phases were washed with brine (20 mL × 2), dried over Na₂SO₄, filtered and concentrated to give a brown solid. The crude product was purified through a reversed-phase chromatography column. Purification conditions: C18 silica gel column; mobile phase ACN: H₂O, gradient: 5% to 50%; 45 min, monitoring wavelength: 214 nm. The desired compound (20 mg, yield: 20.45%) was obtained as a white solid. ¹H NMR (400 MHz, DMSO-d6) δ 11.51 (s, 1H), 9.02 (dd, J = 4.2, 1.7 Hz, 1H), 8.45 (dd, J = 8.4, 1.7 Hz, 1H), 7.87 (dd, J = 17.5, 1.8 Hz, 2H), 7.67 (dd, J = 8.3, 4.2 Hz, 1H), 7.39 (dd, J = 8.2, 1.2 Hz, 1H), 7.31 (dd, J = 7.8, 1.2 Hz, 1H), 7.19 (t, J = 7.9 Hz, 1H), 4.65 (d, J = 12.0 Hz, 1H), 4.37-4.17 (m, 2H), 4.04 (s, 2H), 3.11 (t, J = 13.2 Hz, 1H), 2.72-2.54 (m, 3H), 1.84-1.67 (m, 2H). MS (ESI) RT = 2.639 min, m/z 489.2 [M+H]⁺, purity: 100% @ 254 nm, 99.53% @ 214 nm.

### Example 16

### 7-(Trifluoromethyl)-1-(1-(2-(2-(8-(trifluoromethyl)quinolin-6-yl)acetyl)piperidin-4-yl)-1,3-dihydro-2H-benzo[d]imidazol-2-one (GEN1-308)

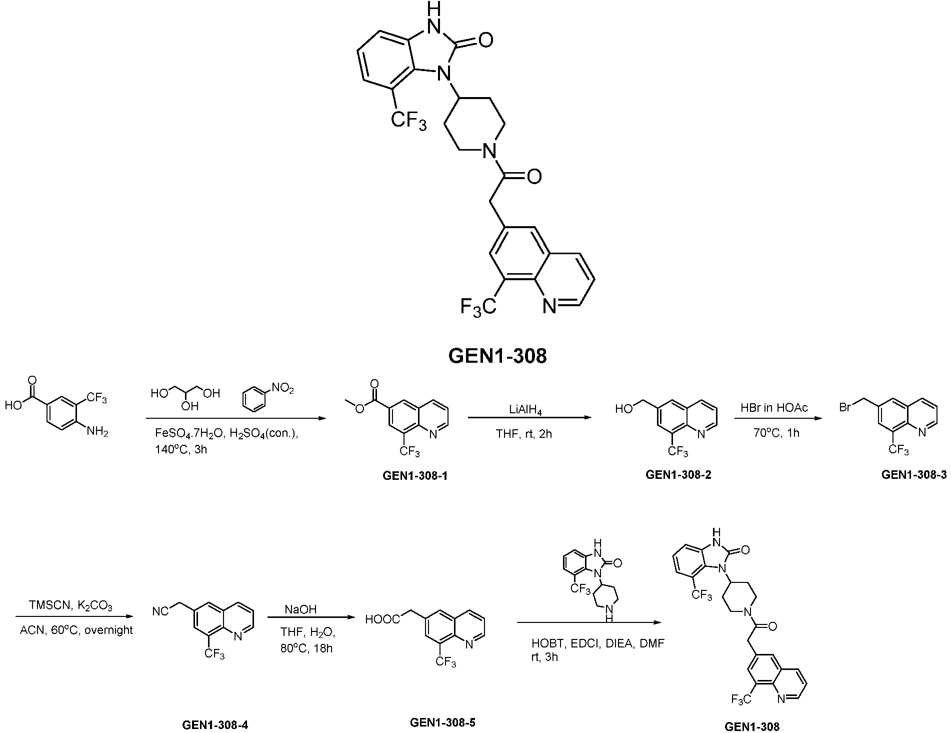

### Step 1. Methyl 8-(trifluoromethyl)quinoline-6-carboxylate (GEN1-308-1)

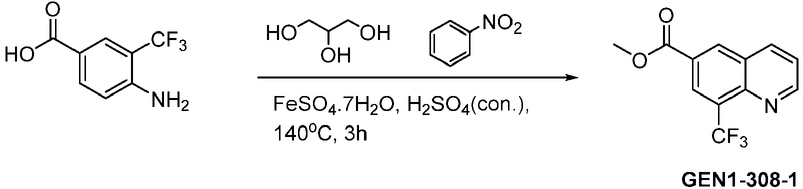

To a mixture of 4-amino-3-(trifluoromethyl)benzoic acid (1.8 g, 8.78 mmol), nitrobenzene (648 mg, 5.27 mmol), glycerol (3.22 g, 35 mmol) and FeSO₄.7H₂O (972 mg, 3.49 mmol) was added dropwise H₂SO₄ (concentrated) (3.44 g, 35 mmol). The mixture was stirred at 140 °C for 3 h. The mixture was then cooled to 60 °C, MeOH (3 mL) was added, and the mixture was stirred at 60 °C overnight. The mixture was concentrated and diluted with water (10 mL), then adjusted at 0 °C to pH 8 with saturated sodium bicarbonate solution and extracted with EA (100 mL × 3). The combined organic layers were washed with water (50 mL) and brine (50 mL), dried over Na₂SO₄ and concentrated to give a yellow liquid. The crude product was purified by reversed-phase flash chromatography (C₁₈ reversed-phase silica gel column; mobile phase ACN, water, 10% to 85% gradient; 30 min; detection, UV 214 nm) to give methyl 8-(trifluoromethyl)quinoline-6-carboxylate (460 mg, yield: 21%) as a yellow solid. MS (ESI) RT = 2.84 min, m/z 256.1 [M+H]⁺, purity: 59.60% @ 254 nm, 73.06% @ 214 nm.

### Step 2. (8-(Trifluoromethyl)quinolin-6-yl)methanol (GEN1-308-2)

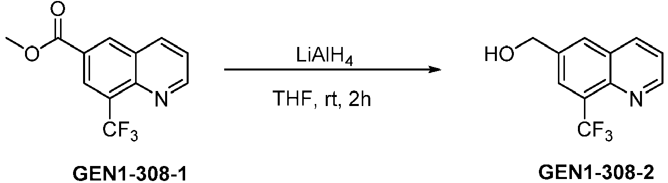

To a solution of methyl 8-(trifluoromethyl)quinoline-6-carboxylate (240 mg, 0.94 mmol) in THF (6 mL) was added, at 0 °C, LiAlH₄ (29 mg, 0.76 mmol). The mixture was stirred at room temperature for 2 h. Na₂SO₄.10H₂O was then added at 0 °C until no bubbles were produced, and the mixture was filtered. The filtrate was concentrated to give crude (8-(trifluoromethyl)quinolin-6-yl)methanol (228 mg) as a yellow liquid. MS (ESI) RT = 2.15 min, m/z 228.1 [M+H]⁺, purity: 90.52% @ 254 nm, 78.64% @ 214 nm.

### Step 3. 6-(Bromomethyl)-8-(trifluoromethyl)quinoline (GEN1-308-3)

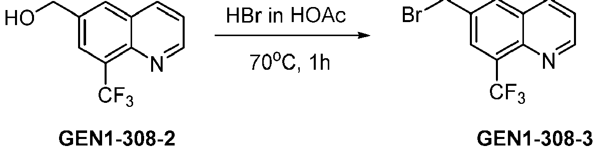

(8-(Trifluoromethyl)quinolin-6-yl)methanol (228 mg, 1.0 mmol) was dissolved in 45% HBr in HOAc (4 mL), and the solution was heated to 70 °C and stirred for 1 h. The crude product was purified by reversed-phase flash chromatography (C₁₈ reversed-phase silica gel column; mobile phase ACN, water, 10% to 85% gradient; 30 min; detection, UV 214 nm) to give 6-(bromomethyl)-8-(trifluoromethyl)quinoline (189 mg, yield: 65%) as a white solid. MS (ESI) RT = 2.99 min, m/z 290.0 [M+H]⁺, purity: 86.75% @ 254 nm, 83.95% @ 214 nm.

### Step 4. 2-(8-(Trifluoromethyl)quinolin-6-yl)acetonitrile (GEN1-308-4)

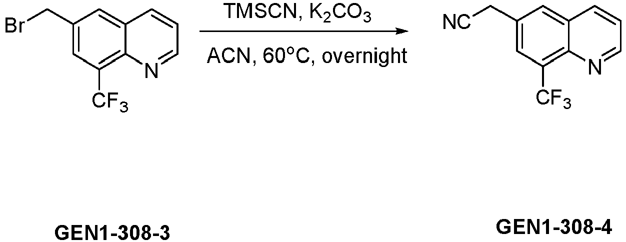

To a solution of 6-(bromomethyl)-8-(trifluoromethyl)quinoline (189 mg, 0.74 mmol) in ACN (10 mL) were added, at 0 °C, K₂CO₃ (144 mg, 1.04 mmol) and TMSCN (97 mg, 0.98 mmol). The mixture was stirred at 60 °C overnight. The reaction mixture was poured into water (10 mL) and extracted with EA (20 mL × 3). The combined organic layers were washed with water (20 mL) and brine (20 mL), dried over Na₂SO₄ and concentrated to give a crude product. The crude product was purified by reversed-phase flash chromatography (C18 reversed-phase silica gel column; mobile phase ACN, water, 10% to 85% gradient; 30 min; detection, UV 214 nm) to give 2-(8-(trifluoromethyl)quinolin-6-yl)acetonitrile (105 mg, yield: 68%) as a white solid. MS (ESI) RT = 2.50 min, m/z 237.1 [M+H]⁺, purity: 76.07% @ 254 nm, 16.36% @ 214 nm.

### Step 5. 2-(8-(Trifluoromethyl)quinolin-6-yl)acetic acid (GEN1-308-5)

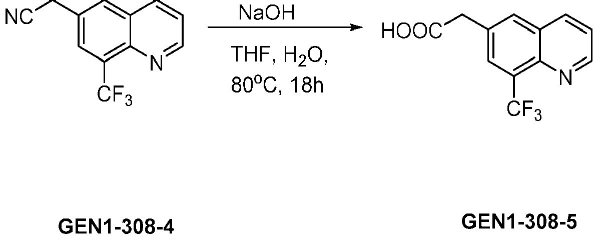

To a mixture of 2-(8-(trifluoromethyl)quinolin-6-yl)acetonitrile (105 mg, 0.44 mmol) in THF (1.6 mL) and H₂O (1.6 mL) was added NaOH (71 mg, 1.78 mmol). The mixture was stirred at 80 °C for 18 h, then diluted with H₂O (20 mL) and washed with EA (20 mL). The aqueous phase was adjusted to pH 4 with an aqueous hydrochloric acid solution (2 M) and extracted with EA (20 mL × 3). The organic phases were dried over Na₂SO₄ and concentrated to give crude 2-(8-(trifluoromethyl)quinolin-6-yl)acetic acid (118 mg) as a white solid. MS (ESI) RT = 2.22 min, m/z 256.1 [M+H]⁺, purity: 74.63% @ 254 nm, 76.86% @ 214 nm.

### Step 6. 7-(Trifluoromethyl)-1-(1-(2-(2-(8-(trifluoromethyl)quinolin-6-yl)acetyl)piperidin-4-yl)-1,3-dihydro-2H-benzo[d]imidazol-2-one (GEN1-308)

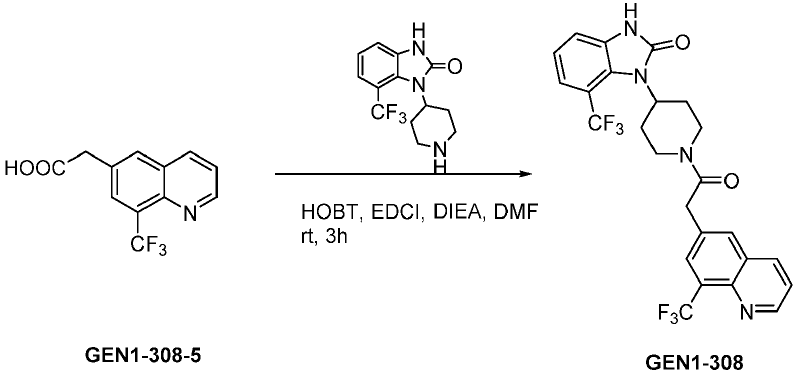

To a solution of 2-(8-(trifluoromethyl)quinolin-6-yl)acetic acid (50 mg, 0.2 mmol) in DMF (3 mL) were added 1-(piperidin-4-yl)-7-(trifluoromethyl)-1,3-dihydro-2H-benzo[d]imidazol-2-one (60 mg, 0.21 mmol), EDCI (45 mg, 0.23 mmol), HOBT (35 mg, 0.25 mmol) and DIEA (50 mg, 0.39 mmol). The mixture was stirred at room temperature for 3 h. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 7-(trifluoromethyl)-1-(1-(2-(8-(trifluoromethyl)quinolin-6-yl)acetyl)piperidin-4-yl)-1,3-dihydro-2H-benzo[d]imidazol-2-one (46 mg, yield: 45%) as a white solid. ¹H NMR (400 MHz, DMSO-*d₆*) δ 11.47 (s, 1H), 9.02 (dd, *J* = 4.0, 1.6 Hz, 1H), 8.49 (dd, *J* = 8.4, 2.0 Hz, 1H), 8.22-8.02 (m, 2H), 7.68 (dd, *J* = 8.4, 4.0 Hz, 1H), 7.37 (dd, *J* = 8.0, 1.2 Hz, 1H), 7.28 (d, *J* = 7.6 Hz, 1H), 7.16 (t, *J* = 8.0 Hz, 1H), 4.62 (d, *J* = 12.4 Hz, 1H), 4.27 (q, *J* = 13.2, 12.4 Hz, 2H), 4.10 (s, 2H), 3.18-3.02 (m, 1H), 2.78-2.54 (m, 3H), 1.76 (d, *J* = 12.0 Hz, 2H). MS (ESI) RT = 2.90 min, m/z 523.2 [M+H]⁺, purity: 100% @ 254 nm, 99.76% @ 214 nm.

### Example 17

### 1-(1-(2-(4-(4-Hydroxyquinolin-6-yl)acetyl)piperidin-4-yl)-7-(trifluoromethyl)-1,3-dihydro-2H-benzo[d]imidazol-2-one (GEN1-309)

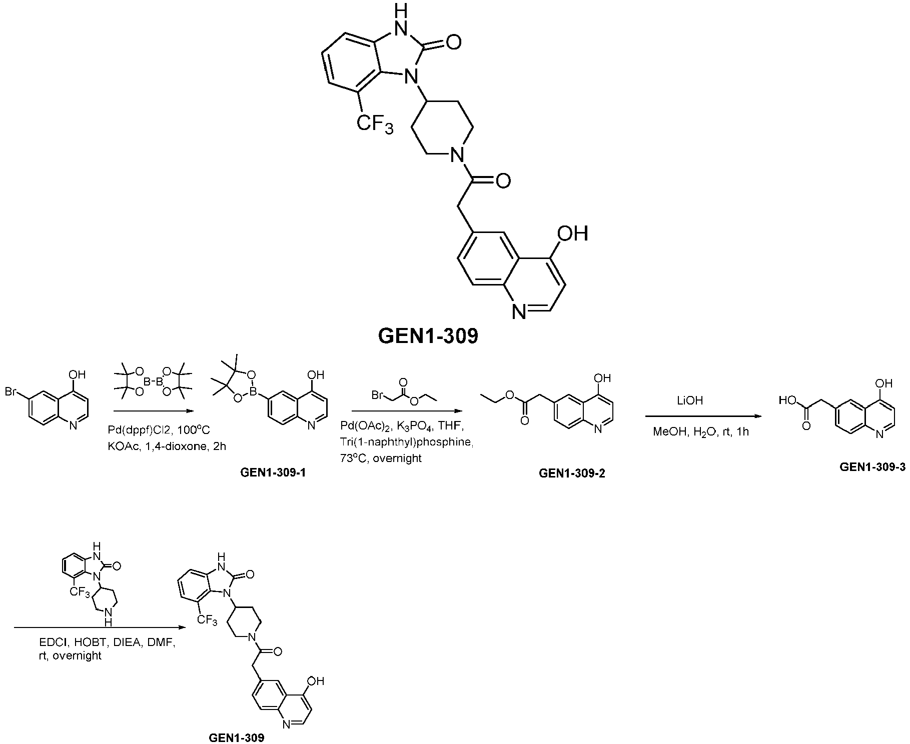

### Step 1. 6-(4,4,5,5-Tetramethyl-1,3,2-dioxaborolan-2-yl)quinolin-4-ol (GEN1-309-1)

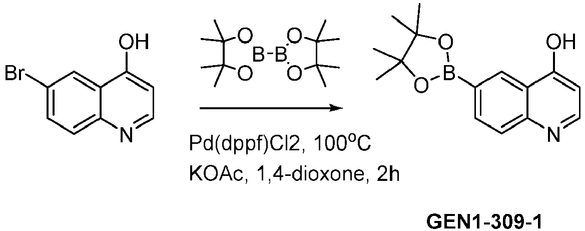

To a solution of 6-bromoquinolin-4-ol (3 g, 13.4 mmol) in 1,4-dioxane (30 mL) were added KOAc (3.9 g, 39.8 mmol), 4,4,4',4',5,5,5',5'-octamethyl-2,2'-bis(1,3,2-dioxaborolane) (5.1 g, 20.1 mmol) and Pd(dppf)Cl₂.CH₂Cl₂ (1.095 g, 1.34 mmol). The mixture was stirred at 100 °C for 2 h. The reaction mixture was filtered, and the filter cake was dissolved in EA (300 mL). The solution was then filtered, and the filtrate was concentrated to give 6-(4,4,5,5-tetramethyl-1,3,2-dioxaborolan-2-yl)quinolin-4-ol (1.5 g, yield: 41%) as a yellow solid. MS (ESI) RT = 2.16 min, m/z 271.9 [M+H]⁺, purity: 88.43% @ 254 nm, 92.31% @ 214 nm.

### Step 2. Ethyl 2-(4-hydroxyquinolin-6-yl)acetate (GEN1-309-2)

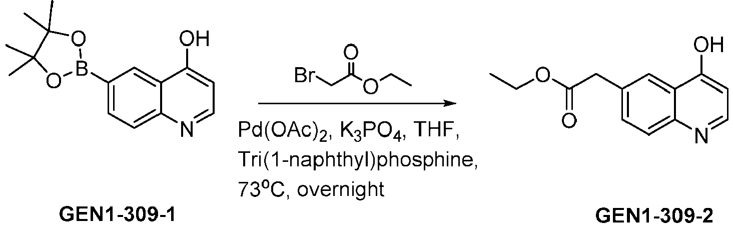

To a solution of Pd(OAc)₂ (13 mg, 0.06 mmol) in THF (5 mL) were added K₃PO₄ (635 mg, 2.99 mmol), 6-(4,4,5,5-tetramethyl-1,3,2-dioxaborolan-2-yl)quinolin-4-ol (243 mg, 0.9 mmol), ethyl 2-bromoacetate (100 mg, 0.6 mmol) and tris(1-naphthyl)phosphine (74 mg, 0.18 mmol). The mixture was stirred at 73 °C overnight. Four identical reactions were carried out simultaneously. The four reactions were combined and filtered, and the filtrate was concentrated to give a crude product. The crude product was purified by reversed-phase flash chromatography (C₁₈ reversed-phase silica gel column; mobile phase ACN, water, 10% to 85% gradient; 30 min; detection, UV 214 nm) to give ethyl 2-(4-hydroxyquinolin-6-yl)acetate (80 mg, yield: 14%) as a white solid. MS (ESI) RT = 1.85 min, m/z 231.9 [M+H]⁺, purity: 17.58% @ 254 nm, 48.70% @ 214 nm.

### Step 3. 2-(4-Hydroxyquinolin-6-yl)acetic acid (GEN1-309-3)

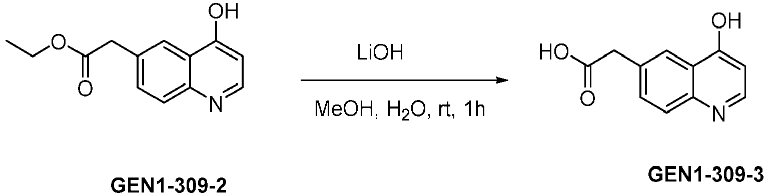

To a mixture of ethyl 2-(4-hydroxyquinolin-6-yl)acetate (80 mg, 0.35 mmol) in MeOH (8 mL) and H₂O (2 mL) was added LiOH (40 mg, 1.67 mmol). The mixture was stirred at room temperature for 1 h. The crude product was purified by reversed-phase flash chromatography (C₁₈ reversed-phase silica gel column; mobile phase ACN, water, 10% to 35% gradient; 30 min; detection, UV 214 nm) to give 2-(4-hydroxyquinolin-6-yl)acetic acid (130 mg, crude) as a white solid. MS (ESI) RT = 1.08 min, m/z 203.9 [M+H]⁺, purity: 17.35% @ 254 nm, 55.18% @ 214 nm.

### Step 4. 1-(1-(2-(4-(4-Hydroxyquinolin-6-yl)acetyl)piperidin-4-yl)-7-(trifluoromethyl)-1,3-dihydro-2H-benzo[d]imidazol-2-one (GEN1-309)

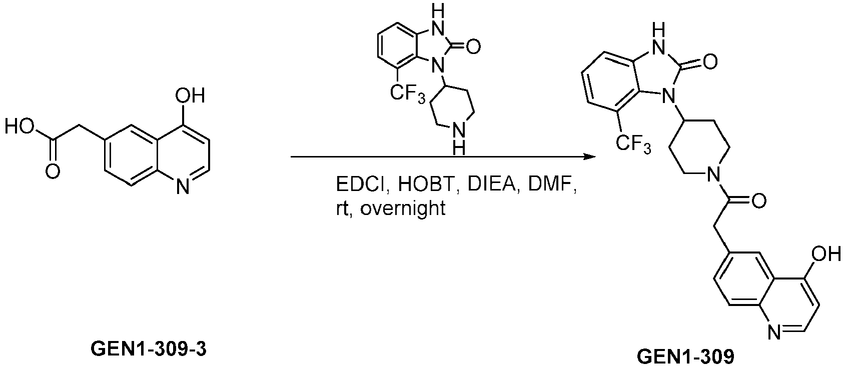

To 2-(4-hydroxyquinolin-6-yl)acetic acid (100 mg, crude) in DMF (6 mL) were added 1-(piperidin-4-yl)-7-(trifluoromethyl)-1,3-dihydro-2H-benzo[d]imidazol-2-one (93 mg, 0.33 mmol), EDCI (68 mg, 0.35 mmol), HOBT (48 mg, 0.35 mmol) and DIEA (77 mg, 0.60 mmol). The mixture was stirred at room temperature overnight. The mixture was then diluted with water (20 mL) and filtered to give a crude product as a white solid. The crude product was purified by reversed-phase flash chromatography (C₁₈ reversed-phase silica gel column; mobile phase ACN, water, 10% to 85% gradient; 30 min; detection, UV 214 nm) to give 1-(1-(2-(4-(4-hydroxyquinolin-6-yl)acetyl)piperidin-4-yl)-7-(trifluoromethyl)-1,3-dihydro-2H-benzo [d]imidazol-2-one (60 mg, yield: 26%) as a white solid. ¹H NMR (400 MHz, DMSO-*d₆*) δ 11.50 (s, 1H), 8.18 (dd, *J* = 8.0, 1.6 Hz, 1H), 7.88 (d, J = 7.6 Hz, 1H), 7.77 - 7.65 (m, 1H), 7.43 (d, J = 8.8 Hz, 1H), 7.42 - 7.34 (m, 2H), 7.30 (d, *J* = 7.6 Hz, 1H), 7.18 (t, *J* = 8.0 Hz, 1H), 6.07 (d, *J* = 7.6 Hz, 1H), 5.58 - 5.14 (m, 2H), 4.49 (d, *J* = 12.4 Hz, 1H), 4.40 - 4.28 (m, 1H), 4.16 (d, *J* = 13.2 Hz, 1H), 3.21 (t, *J* = 13.2 Hz, 1H), 2.94 - 2.74 (m, 1H), 2.73 - 2.53 (m, 2H), 1.86 (d, *J* = 12.4 Hz, 1H), 1.76 (d, *J* = 12.0 Hz, 1H). MS (ESI) RT = 2.32 min, m/z 471.2 [M+H]⁺, purity: 0% @ 254 nm, 99.77% @ 214 nm.

### Example 18

### 6-(2-Oxo-2-(4-(2-oxo-7-(trifluoromethyl)-2,3-dihydro-1H-benzo[d]imidazol-1-yl)piperidin-1-yl)ethyl)quinoline-8-carbonitrile (GEN1-310)

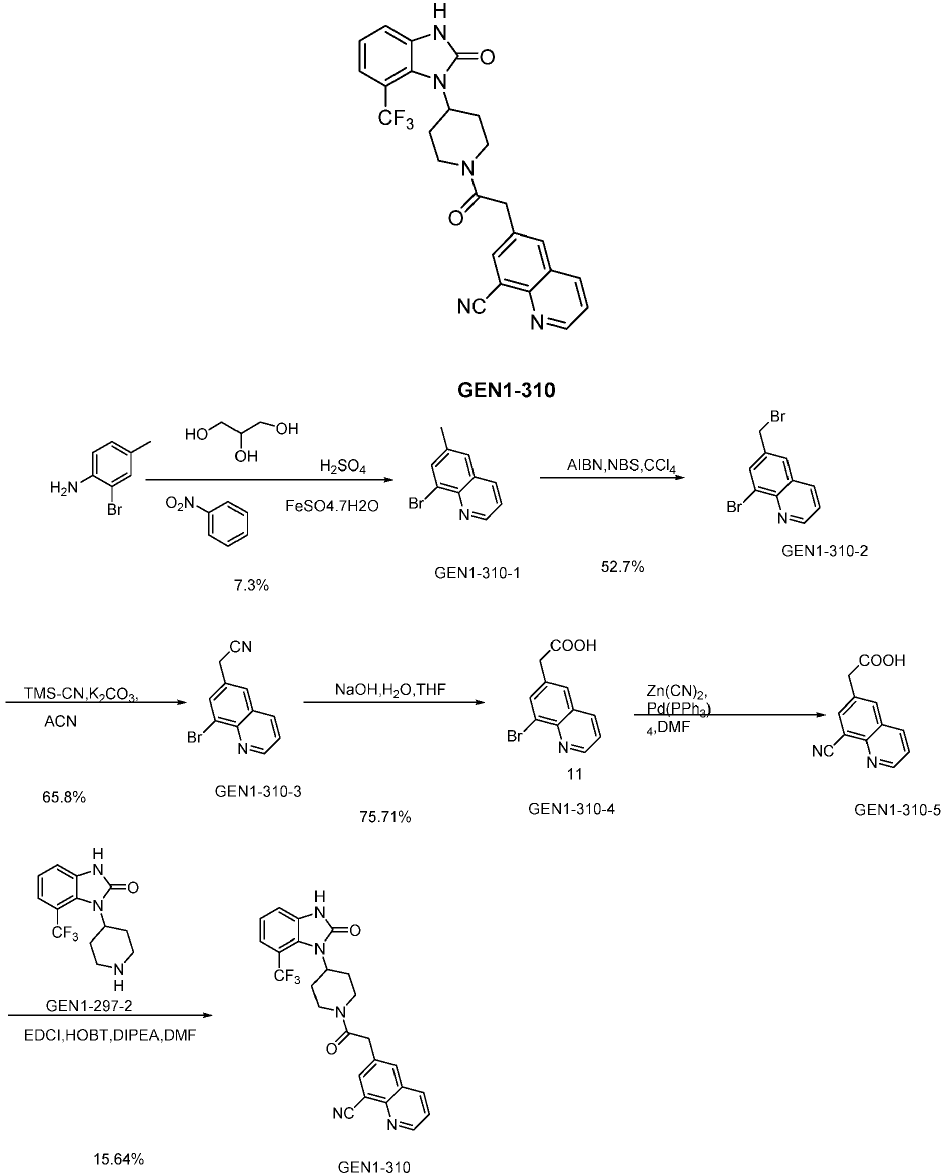

### Step 1. Synthesis of 8-bromo-6-methylquinoline (GEN1-310-1)

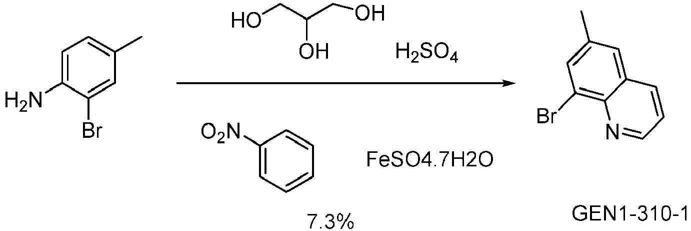

To a mixture of 2-bromo-4-methylaniline (4.007 g, 21.54 mmol), propane-1,2,3-triol (8.16 g, 88.6 mmol) and FeSO₄.7H₂O (2.417 g, 8.695 mmol) in nitrobenzene (1.624 g, 13.19 mmol) was added, at room temperature, H₂SO₄ (4.6 mL, 86.16 mmol). The mixture was then stirred at 128 °C for 3 h. The reaction solution was quenched with ice water and made basic with sodium bicarbonate solution. The mixture was extracted with DCM (80 mL × 3). The combined organic phases were washed with brine (20 mL × 2), dried over Na₂SO₄, filtered and concentrated to give a brown solid. The crude product was purified by column chromatography on silica gel (PE:EA = 3:1) to give the desired compound (350 mg, yield: 7.3%) as a yellow solid. MS (ESI) RT = 2.704 min, m/z 222.0 [M+H]⁺, purity: 94.38% @ 254 nm.

### Step 2. 8-Bromo-6-(bromomethyl)quinoline (GEN1-310-2)

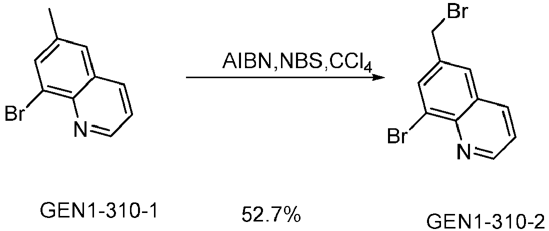

To a solution of 8-bromo-6-methylquinoline (350 mg, 1.576 mmol) and AIBN (25.88 mg, 0.1576 mmol) in CCl₄ (10 mL) was added, at room temperature under a nitrogen atmosphere, NBS (294.5 mg, 1.655 mmol). The resulting mixture was stirred at 70 °C for 8 h. The mixture was concentrated and purified by silica gel column chromatography using (PE:EA = 3:1) as an eluent to give the desired compound (250 mg, yield: 52.7%) as a white solid. MS (ESI) RT = 2.794 min, m/z 301.9 [M+H]⁺, purity: 88.77% @ 254 nm.

### Step 3. Synthesis of 2-(8-bromoquinolin-6-yl)acetonitrile (GEN1-310-3)

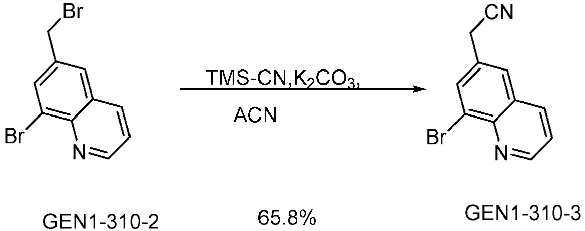

To a suspension of 8-bromo-6-(bromomethyl)quinoline (250 mg, 0.83 mmol) and K₂CO₃ (131.92 mg, 0.955 mmol) in acetonitrile (5 mL) was added, at room temperature under a nitrogen atmosphere, TMS-CN (164.69 mg, 1.66 mmol). The resulting mixture was stirred at 60 °C for 16 h. The mixture was filtered. The filtrate was concentrated and purified by silica gel column chromatography using (PE:EA = 3:1 to 1:1) as an eluent to give the desired compound (135 mg, yield: 65.8%) as a pale yellow solid. MS (ESI) RT = 2.217 min, m/z 247.0 [M+H]⁺, purity: 67.39% @ 214 nm.

### Step 4. Synthesis of 2-(8-bromoquinolin-6-yl)acetic acid (GEN1-310-4)

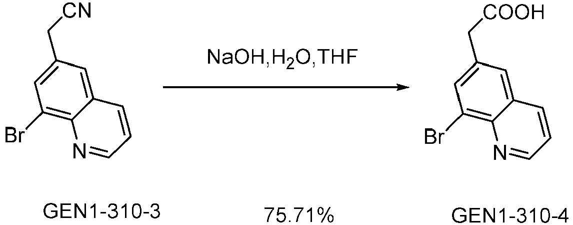

To a solution of 2-(8-bromoquinolin-6-yl)acetonitrile (135 mg, 0.546 mmol) in THF (10 mL) and H₂O (30 mL) was added NaOH (87.36 mg, 2.184 mmol). The resulting mixture was stirred at 80 °C for 16 h. The solution was adjusted to pH 4 and concentrated. The residual crude product was purified by reversed-phase chromatography. Purification conditions: C18 silica gel column; mobile phase ACN: H₂O, gradient: 5% to 50%; 45 min, monitoring wavelength: 214 nm. The desired compound (110 mg, yield: 75.71%) was obtained as a white solid. MS (ESI) RT = 1.913 min, m/z 266.0 [M+H]⁺, purity: 93.87% @ 254 nm.

### Step 5. Synthesis of 2-(8-cyanoquinolin-6-yl)acetic acid (GEN1-310-5)

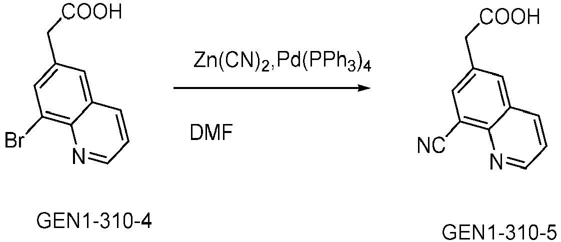

To a solution of 2-(8-bromoquinolin-6-yl)acetic acid (110 mg, 0.413 mmol) and Zn(CN)₂ (48.49 mg, 0.413 mmol) in anhydrous DMF (5 mL) was added, at room temperature under a nitrogen atmosphere, Pd(PPh₃)₄ (71.43 mg, 0.062 mmol). The resulting mixture was stirred at 90 °C for 16 h. The reaction solution was purified by reversed-phase chromatography. Purification conditions: C18 silica gel column; mobile phase ACN: H₂O, gradient: 5% to 50%; 45 min, monitoring wavelength: 214 nm. The desired compound (100 mg, 50% purity) was obtained as a white solid. MS (ESI) RT = 1.759 min, m/z 212.9 [M+H]⁺, purity: 54.22% @ 254 nm.

### Step 6. Synthesis of 6-(2-oxo-2-(4-(2-oxo-7-(trifluoromethyl)-2,3-dihydro-1H-benzo[d]imidazol-1-yl)piperidin-1-yl)ethyl)quinoline-8-carbonitrile (GEN1-310)

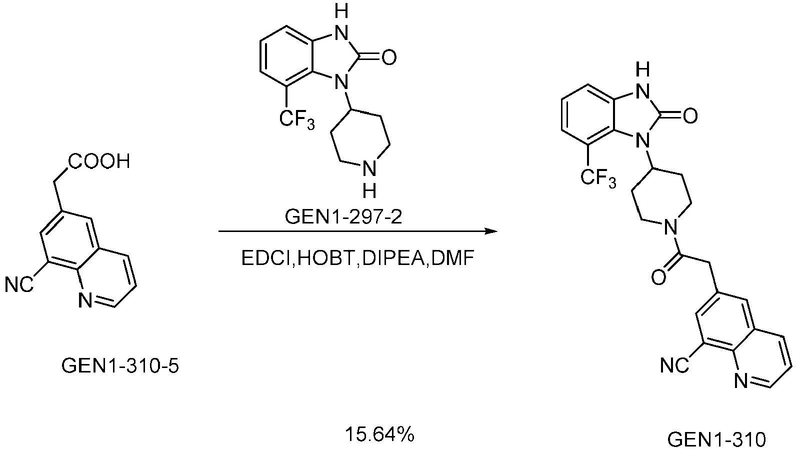

To a solution of 1-(piperidin-4-yl)-7-(trifluoromethyl)-1,3-dihydro-2H-benzo[d]imidazol-2-one (150 mg, crude, 0.4 mmol), 2-(8-bromoquinolin-6-yl)acetic acid (84.9 mg, 0.4 mmol), EDCI (115 mg, 0.6 mmol) and HOBT (81 mg, 0.6 mmol) in DMF (5 mL) was added DIPEA (206.6 mg, 1.6 mmol). The resulting mixture was stirred at room temperature for 24 h. The reaction solution was purified by reversed-phase chromatography. Purification conditions: C18 silica gel column; mobile phase ACN: H₂O, gradient: 5% to 50%; 45 min, monitoring wavelength: 214 nm. The desired product (30 mg, yield: 15.64%) was obtained as a white solid. ¹H NMR (400 MHz, DMSO-d6) δ 11.49 (s, 1H), 9.08 (dd, J = 4.3, 1.7 Hz, 1H), 8.54 (dd, J = 8.4, 1.6 Hz, 1H), 8.29 (d, J = 1.9 Hz, 1H), 8.21 (d, J= 1.9 Hz, 1H), 7.74 (dd, J = 8.3, 4.2 Hz, 1H), 7.39 (dd, J= 8.1, 1.3 Hz, 1H), 7.30 (d, J = 7.6 Hz, 1H), 7.19 (t, J = 7.9 Hz, 1H), 4.64 (d, J = 12.2 Hz, 1H), 4.35-4.22 (m, 2H), 4.10 (s, 2H), 3.13 (t, J = 13.0 Hz, 1H), 2.71-2.54 (m, 3H), 1.86-1.70 (m, 2H). MS (ESI) RT = 2.597 min, m/z 480.2 [M+H]⁺, purity: 100% @ 254 nm.

### Example 19

### 1-(1-(2-(7-Methylquinolin-6-yl)acetyl)piperidin-4-yl)-7-(trifluoromethyl)-1,3-dihydro-2H-benzo[d]imidazol-2-one (GEN1-311)

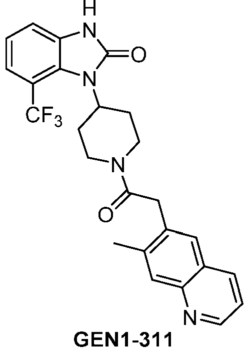

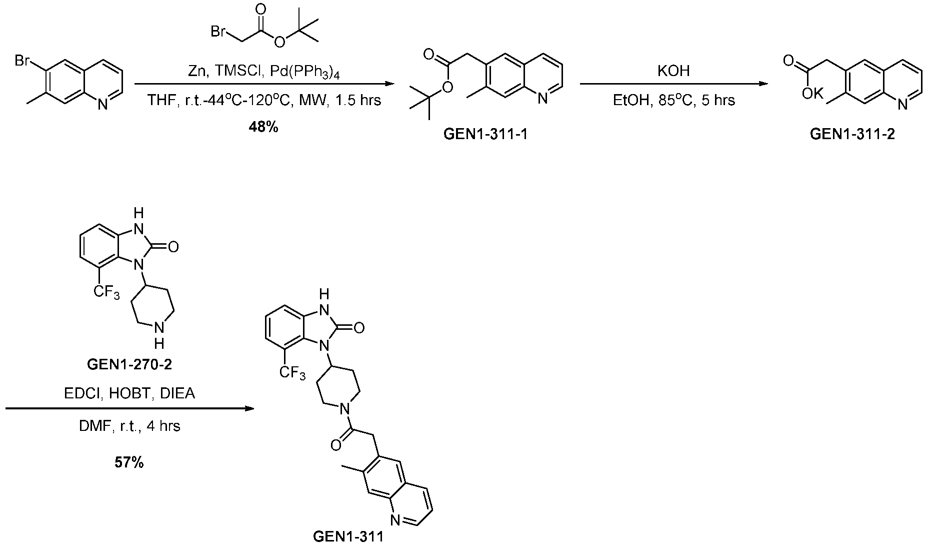

### Step 1. Tert-butyl 2-(7-methylquinolin-6-yl)acetate (GEN1-311-1)

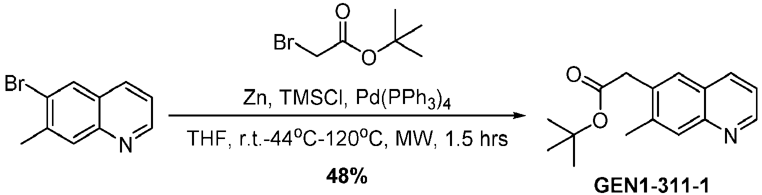

To a solution of Zn (192 mg, 3.00 mmol) in THF (0.8 mL) was added, under a nitrogen atmosphere, TMCSC1 (16 mg, 0.14 mmol). The resulting mixture was stirred at room temperature for 20 min. To the solution was added dropwise a solution of tert-butyl 2-bromoacetate (290 mg, 1.50 mmol) in THF (2.0 mL). The resulting mixture was stirred at 45 °C for 20 min. To the solution were added 6-bromo-7-methylquinoline (100 mg, 0.46 mmol) and Pd(PPh₃)₄ (208 mg, 0.18 mmol). The mixture was reacted in a microwave reactor at 120 °C for 30 min (repeated 3 times). The reaction mixture was purified by reversed-phase flash chromatography (C₁₈ reversed-phase silica gel column; mobile phase CH₃CN, water, 0% to 70% gradient, 50 min; detection, UV 214 nm) to give tert-butyl 2-(7-methylquinolin-6-yl)acetate (57 mg, yield: 48%) as a yellow solid. MS (ESI) RT = 2.932 min, m/z 258.1 [M+H]⁺, purity: 64% @ 254 nm, 82% @ 214 nm.

### Step 2. Potassium 2-(7-methylquinolin-6-yl)acetate (GEN1-311-2)

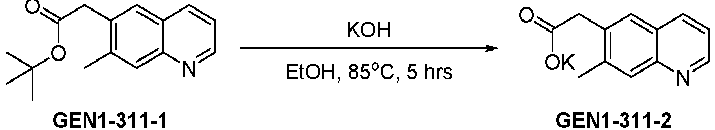

To a solution of tert-butyl 2-(7-methylquinolin-6-yl) acetate (55 mg, 0.21 mmol) in EtOH (1.0 mL) were added KOH (18 mg, 0.32 mmol) and water (1.0 mL). The resulting mixture was stirred at 85 °C for 5 h. The mixture was concentrated to give a crude product (78 mg), which could be directly used in the next step without further purification. MS (ESI) RT = 1.070 min, m/z 202.1 [M+H]⁺, purity: 89% @ 214 nm.

### Step 3. 1-(1-(2-(7-Methylquinolin-6-yl)acetyl)piperidin-4-yl)-7-(trifluoromethyl)-1,3-dihydro-2H-benzo[d]imidazol-2-one (GEN1-311)

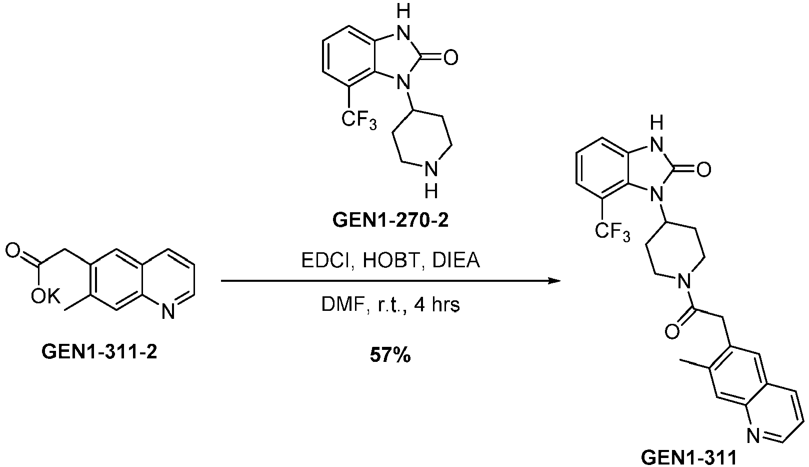

To a solution of 1-(piperidin-4-yl)-7-(trifluoromethyl)-1,3-dihydro-2H-benzo[d]imidazol-2-one (71 mg, 0.25 mmol), potassium 2-(7-methylquinolin-6-yl)acetate (78 mg, crude), EDCI (72 mg, 0.38 mmol) and HOBT (50 mg, 0.37 mmol) in DMF (2.0 mL) was added dropwise DIEA (96 mg, 0.74 mmol). The resulting mixture was stirred at room temperature for 4 h. The reaction mixture was added dropwise to water (20 mL). The solid precipitate was collected by filtration. The crude product was purified by trituration with MeOH and THF to give 1-(1-(2-(7-methylquinolin-6-yl)acetyl)piperidin-4-yl)-7-(trifluoromethyl)-1,3-dihydro-2H-benzo[d]imidazol-2-one (66 mg, yield: 57%) as a white solid. ¹H NMR (400 MHz, DMSO-*d₆*) δ: 11.49 (s, 1H), 8.82 (s, 1H), 8.29 (d, *J* = 8.1 Hz, 1H), 7.82 (s, 1H), 7.68 (s, 1H), 7.49-7.09 (m, 4H), 4.65 (s, 1H), 4.40-4.10 (m, 2H), 4.08-3.83 (m, 2H), 3.19-3.05 (m, 1H), 2.79-2.54 (m, 3H), 2.43 (s, 3H), 1.75 (s, 2H). MS (ESI) RT = 2.607 min, m/z 469.2 [M+H]⁺, purity: 100% @ 254 nm, 97% @ 214 nm.

### Example 20

### 7-(Trifluoromethyl)-1-(1-(2-(3-(3-(trifluoromethyl)quinolin-6-yl)acetyl)piperidin-4-yl)-1,3-dihydro-2H-benzo[d]imidazol-2-one (GEN1-312)

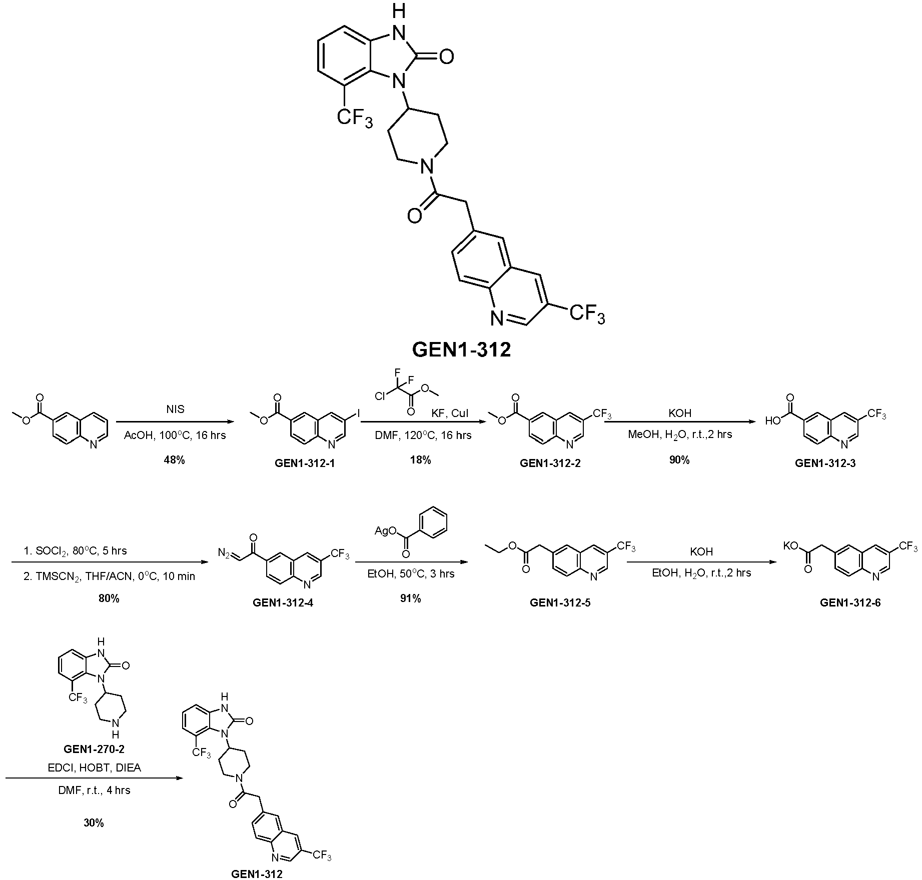

### Step 1. Methyl 3-iodoquinoline-6-carboxylate (GEN1-312-1)

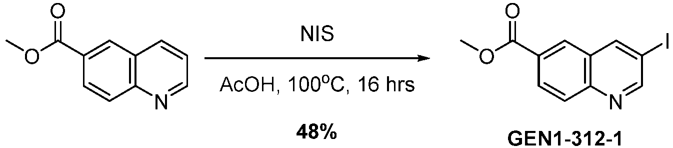

To a solution of methyl quinoline-6-carboxylate (1.0 g, 5.35 mmol) in AcOH (9.0 mL) was added, under a nitrogen atmosphere, NIS (1.68 g, 7.49 mmol). The resulting mixture was stirred at 100 °C for 16 h. The reaction mixture was concentrated under reduced pressure. The crude product was purified by silica gel column chromatography using (EA/(PE + EA) = 20%) as an eluent to give methyl 3-iodoquinoline-6-carboxylate (800 mg, yield: 48%) as a pale yellow oil. ¹H NMR (400 MHz, Chloroform-*d*) δ: 9.10 (d, *J* = 2.1 Hz, 1H), 8.62 (d, *J* = 2.1 Hz, 1H), 8.45 (d, *J* = 1.8 Hz, 1H), 8.30 (dd, *J* = 8.8, 1.9 Hz, 1H), 8.09 (d, *J* = 8.8 Hz, 1H), 3.99 (s, 3H).MS (ESI) RT = 2.953 min, m/z 313.9 [M+H]⁺, purity: 96% @ 254 nm, 89% @ 214 nm.

### Step 2. Methyl 3-(trifluoromethyl)quinoline-6-carboxylate (GEN1-312-2)

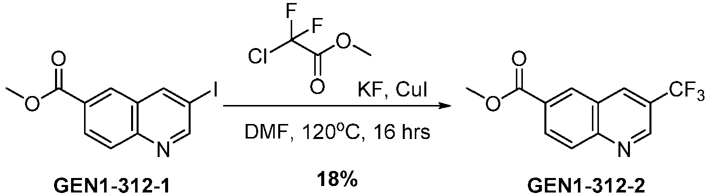

To a solution of methyl 3-iodoquinoline-6-carboxylate (400 mg, 1.28 mmol) in DMF (7.0 mL) were added, under an argon atmosphere, methyl 2-chloro-2,2-difluoroacetate (736 mg, 5.11 mmol), KF (148 mg, 2.55 mmol) and CuI (485 mg, 2.55 mmol). The resulting mixture was stirred at 120 °C for 16 h under an argon atmosphere, cooled and filtered. The filtrate was concentrated, and the crude product was purified by silica gel column chromatography using (EA/(PE + EA) = 10%) as an eluent to give methyl 3-(trifluoromethyl)quinoline-6-carboxylate (60 mg, yield: 18%) as a pale yellow oil. ¹H NMR (400 MHz, DMSO-*d₆*) δ: 9.32 (d, *J* = 2.3 Hz, 1H), 9.19 (d, *J* = 2.4 Hz, 1H), 8.92 (d, *J* = 1.9 Hz, 1H), 8.39 (dd, *J* = 8.9, 2.0 Hz, 1H), 8.25 (d, *J* = 8.8 Hz, 1H), 3.96 (s, 3H). MS (ESI) RT = 2.901 min, m/z 256.0 [M+H]⁺, purity: 87% @ 254 nm, 92% @ 214 nm.

### Step 3. 3-(Trifluoromethyl)quinoline-6-carboxylic acid (GEN1-312-3)

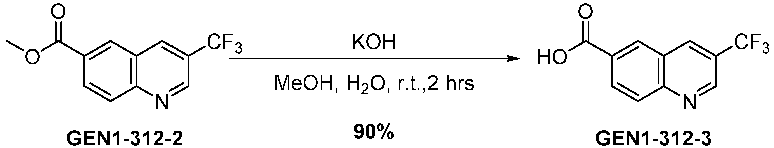

To a solution of methyl 3-(trifluoromethyl)quinoline-6-carboxylate (55 mg, 0.21 mmol) in methanol (2.0 mL) were added KOH (15 mg, 0.27 mmol) and water (0.2 mL). The resulting mixture was stirred at room temperature for 2 h under a nitrogen atmosphere. The reaction mixture was purified by reversed-phase flash chromatography (C₁₈ reversed-phase silica gel column; mobile phase CH₃CN, water (0.1% formic acid), 0% to 70% gradient, 50 min; detection, UV 214 nm) to give the desired compound (47 mg, yield: 90%) as a white solid. MS (ESI) RT = 2.387 min, m/z 344.0 [M+H]⁺, purity: 99% @ 254 nm, 96% @ 214 nm.

### Step 4. 2-Diazo-1-(3-(trifluoromethyl)quinolin-6-yl)ethan-1-one (GEN1-312-4)

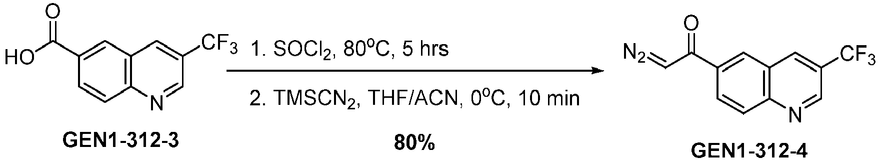

3-(Trifluoromethyl)quinoline-6-carboxylic acid (47 mg, 0.20 mmol) was dissolved in SOCl₂ (2.0 mL) under a nitrogen atmosphere, and the solution was stirred at 80 °C for 5 h, then cooled and concentrated. The resulting residue was dissolved in THF/MeCN (1.0/1.0 mL), and a solution of TMSCHN₂ (2.0 M in hexane, 0.24 mL) in THF/MeCN (2.0/2.0 mL) was added at 0 °C. The mixture was stirred for 10 min and then concentrated. The crude product was purified by silica gel column chromatography using (EA/(PE + EA) = 20%) as an eluent to give the desired product (41.3 mg, yield: 80%) as a pale yellow solid. MS (ESI) RT = 2.599 min, m/z 266.0 [M+H]⁺, purity: 100% @ 254 nm, 92% @ 214 nm.

### Step 5. Ethyl 2-(3-(trifluoromethyl)quinolin-6-yl)acetate (GEN1-312-5)

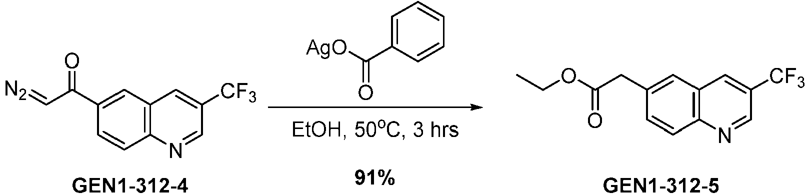

To a solution of 2-diazo-1-(3-(trifluoromethyl)quinolin-6-yl)ethan-1-one (31 mg, 0.12 mmol) in ethanol (4.0 mL) was added, under a nitrogen atmosphere, silver benzoate (35 mg, 0.15 mmol). The resulting mixture was stirred at 50 °C for 3 h under a nitrogen atmosphere. The reaction mixture was filtered, and the filtrate was concentrated. The crude product was purified by silica gel column chromatography using (EA/(PE + EA) = 8%) as an eluent to give the desired product (30 mg, yield: 91%) as a white solid. MS (ESI) RT = 2.935 min, m/z 284.1 [M+H]⁺, purity: 63% @ 254 nm, 90% @ 214 nm.

### Step 6. Potassium 2-(3-(trifluoromethyl)quinolin-6-yl)acetate (GEN1-312-6)

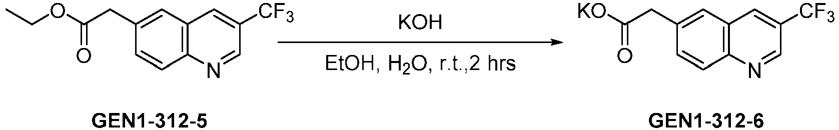

To a solution of ethyl 2-(3-(trifluoromethyl)quinolin-6-yl)acetate (30 mg, 0.11 mmol) in ethanol (2.0 mL) were added KOH (7.1 mg, 0.13 mmol) and water (0.2 mL). The resulting mixture was stirred at room temperature for 2 h under a nitrogen atmosphere. The resulting mixture was concentrated *in vacuo* to give a crude product (40 mg), which could be directly used in the next step without further purification.

### Step 7. 7-(Trifluoromethyl)-1-(1-(2-(3-(3-(trifluoromethyl)quinolin-6-yl)acetyl)piperidin-4-yl)-1,3-dihydro-2H-benzo[d]imidazol-2-one (GEN1-312)

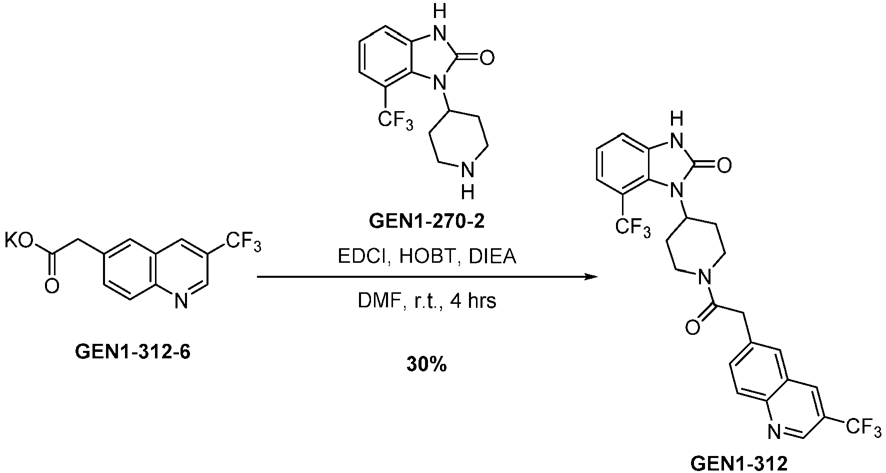

To a solution of 1-(piperidin-4-yl)-7-(trifluoromethyl)-1,3-dihydro-2H-benzo[d]imidazol-2-one (36 mg, 0.13 mmol), potassium 2-(3-(trifluoromethyl)quinolin-6-yl)acetate (40 mg), EDCI (30 mg, 0.16 mmol) and HOBT (21 mg, 0.16 mmol) in DMF (1.0 mL) was added dropwise DIEA (27 mg, 0.21 mmol). The resulting mixture was stirred at room temperature for 4 h. The reaction mixture was purified by reversed-phase flash chromatography (C₁₈ reversed-phase silica gel column; mobile phase CH₃CN, water, 0% to 70% gradient, 50 min; detection, UV 214 nm) to give the desired compound (16 mg, yield: 30%) as a white solid. ¹H NMR (400 MHz, DMSO-*d₆*) δ: 11.44 (s, 1H), 9.15 (d, *J* = 2.3 Hz, 1H), 8.90 (s, 1H), 8.11 (d*, J* = 8.7 Hz, 1H), 8.03 (d*, J* = 1.9 Hz, 1H), 7.86 (dd, *J* = 8.7, 1.9 Hz, 1H), 7.36 (d, *J* = 8.0 Hz, 1H), 7.28 (d, *J* = 7.7 Hz, 1H), 7.17 (d, *J* = 7.9 Hz, 1H), 4.63 (d, *J* = 11.8 Hz, 1H), 4.35-4.16 (m, 2H), 4.13-3.98 (m, 2H), 3.15-3.02 (m, 1H), 2.69-2.50 (m, 3H), 1.80-1.65 (m, 2H). MS (ESI) RT = 2.927 min, m/z 523.1 [M+H]⁺, purity: 100% @ 254 nm, 99.5% @ 214 nm.

### Example 21

### 1-(1-(2-(7-Chloroquinolin-6-yl)acetyl)piperidin-4-yl)-7-(trifluoromethyl)-1,3-dihydro-2H-benzo[d]imidazol-2-one (GEN1-313)

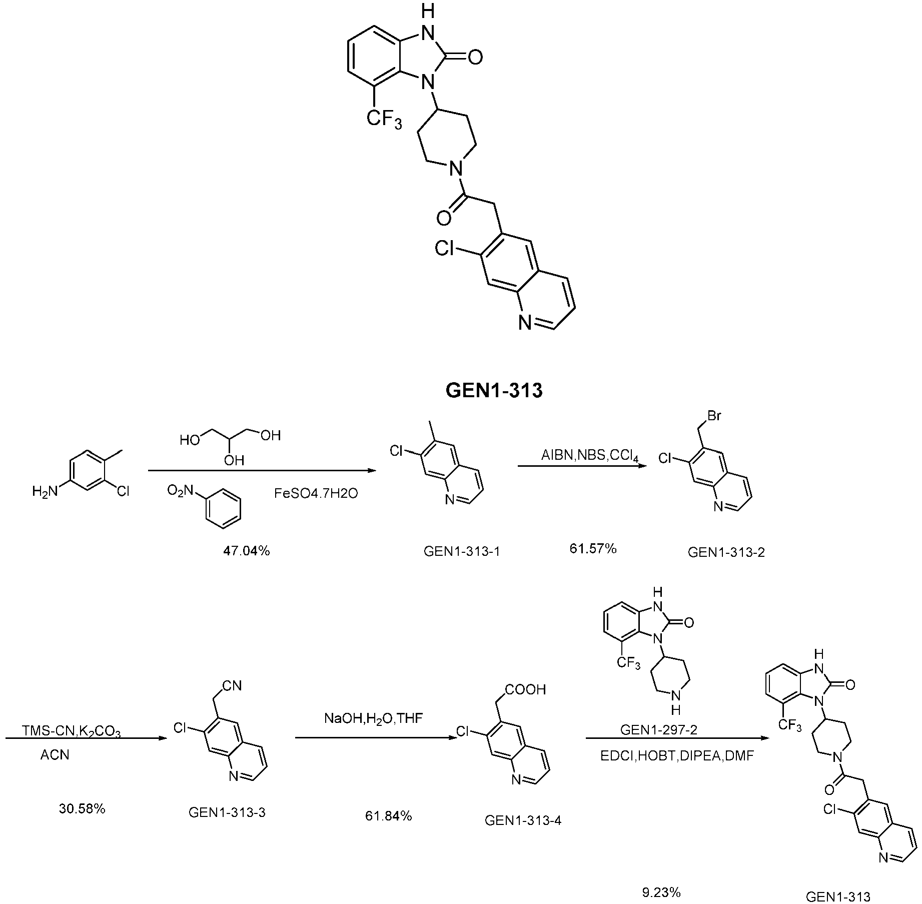

### Step 1. Synthesis of 7-chloro-6-methylquinoline (GEN1-313-1)

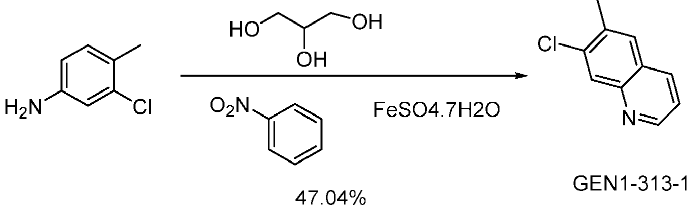

To a mixture of 3-chloro-4-methylaniline (3.05 g, 21.54 mmol), propane-1,2,3-triol (8.16 g, 88.6 mmol), FeSO₄.7H₂O (2.417 g, 8.695 mmol) and nitrobenzene (1.624 g, 13.19 mmol) was added, at room temperature, H₂SO₄ (4.6 mL, 86.16 mmol). The resulting mixture was stirred at 140 °C for 3 h. The reaction was then quenched with ice water and made basic with sodium bicarbonate solution. The mixture was extracted with DCM (80 mL × 3). The combined organic phases were washed with brine (20 mL × 2), dried over Na₂SO₄ and concentrated to give a brown solid. The crude product was purified by column chromatography on silica gel (PE:EA = 4:1) to give the desired compound (1.8 g, yield: 47.04%) as a yellow solid. MS (ESI) RT = 2.49 min, m/z 178.1 [M+H]⁺, purity: 42.61% @ 214 nm.

### Step 2. 6-(Bromomethyl)-7-chloroquinoline (GEN1-313-2)

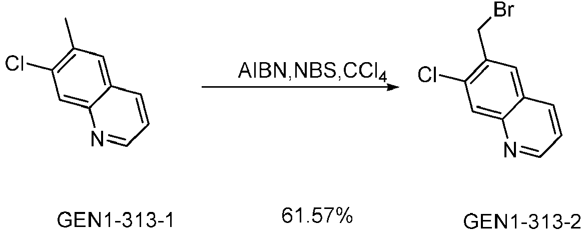

To a mixture of 7-chloro-6-methylquinoline (1.8 g, 10.13 mmol) and AIBN (1.894 g, 10.64 mmol) in CCl4 (50 mL) was added, at room temperature under a nitrogen atmosphere, NBS (1.894 g, 10.64 mmol). The resulting mixture was stirred at 75 °C for 6 h. The reaction was concentrated and purified by column chromatography on silica gel (PE:EA = 3:1) to give the desired compound (1.6 g, yield: 61.57%) as a yellow solid. MS (ESI) RT = 2.748 min, m/z 255.9 [M+H]⁺, purity: 92.51% @ 254 nm.

### Step 3. Synthesis of 2-(7-chloroquinolin-6-yl)acetonitrile (GEN1-313-3)

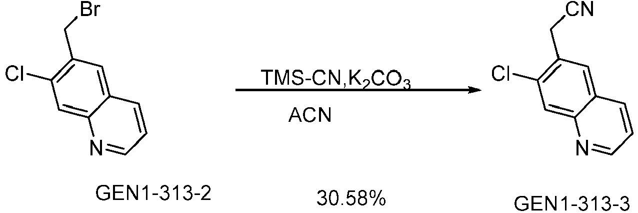

To a microwave tube were added, at room temperature under a nitrogen atmosphere, 6-(bromomethyl)-7-chloroquinoline (600 mg, 2.34 mmol), K₂CO₃ (743.57 mg, 5.38 mmol), ACN (20 mL) and TMSCN (928.6 mg, 9.36 mmol). The resulting mixture was stirred at 75 °C for 48 h under a sealed condition. The resulting mixture was filtered. The filtrate was concentrated and purified by silica gel column chromatography (PE:EA = 3:1 to 1:1) to give the desired compound (145 mg, yield: 30.58%) as a pale yellow solid. MS (ESI) RT = 2.146 min, m/z 203.1 [M+H]⁺, purity: 90.74% @ 254 nm.

### Step 4. Synthesis of 2-(7-chloroquinolin-6-yl)acetic acid (GEN1-313-4)

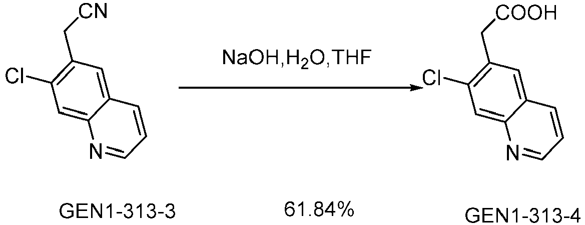

To a mixture of 2-(7-chloroquinolin-6-yl)acetonitrile (145 mg, 0.715 mmol) in THF (8 mL) and H₂O (8 mL) was added NaOH (114.49 mg, 2.862 mmol). The resulting mixture was stirred at 80 °C for 16 h. The solution was adjusted to pH 4 and concentrated. The resulting residue was purified by C18 column chromatography (ACN:HzO = 0% to 50%) over 45 min to give the desired product (98 mg, yield: 61.84%) as a white solid. MS (ESI) RT = 1.648 min, m/z 222.0 [M+H]⁺, purity: 55.48% @ 254 nm.78.1% @ 214 nm.

### Step 5. Synthesis of 1-(1-(2-(7-chloroquinolin-6-yl)acetyl)piperidin-4-yl)-7-(trifluoromethyl)-1,3-dihydro-2H-benzo[d]imidazol-2-one (GEN1-313)

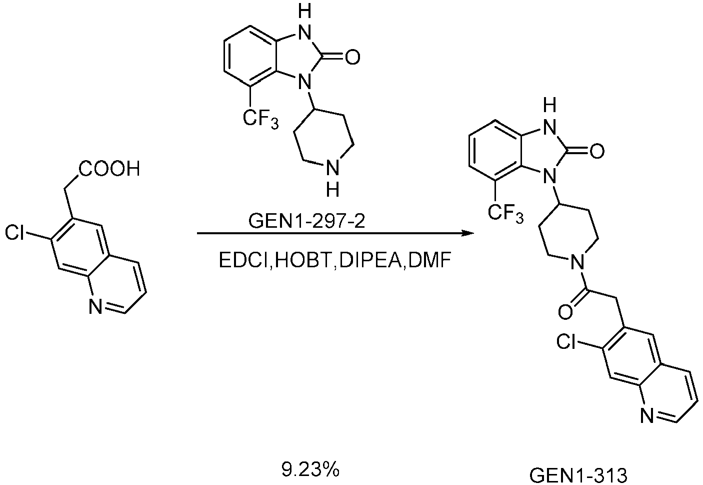

To a solution of 1-(piperidin-4-yl)-7-(trifluoromethyl)-1,3-dihydro-2H-benzo[d]imidazol-2-one (126.37 mg, 0.443 mmol), 2-(7-chloroquinolin-6-yl)acetic acid (98 mg, 0.443 mmol), EDCI (127.48 mg, 0.665 mmol) and HOBT (89.86 mg, 0.665 mmol) in DMF (5 mL) was added DIPEA (114.43 mg, 0.886 mmol). The resulting mixture was stirred at room temperature for 24 h. The reaction solution was purified by silica gel column chromatography (EA:MeOH, 0% to 10%) to give the desired compound (20 mg, yield: 9.23%) as a white solid. ¹H NMR (400 MHz, DMSO-d6) δ 11.48 (s, 1H), 9.04-8.86 (m, 1H), 8.39 (d, J = 8.3 Hz, 1H), 8.24-7.90 (m, 2H), 7.68-7.52 (m, 1H), 7.33 (dd, J = 32.8, 7.9 Hz, 3H), 7.17 (t, J = 7.8 Hz, 1H), 4.88-4.50 (m, 1H), 4.39-4.20 (m, 2H), 4.17-3.92 (m, 2H), 3.16 (t, J = 13.2 Hz, 1H), 2.88-2.53 (m, 3H), 1.85-1.65 (m, 2H). MS (ESI) RT = 2.636 min, m/z 489.1 [M+H]⁺, purity: 100% @ 254 nm.95.78% @ 214 nm.

### Example 22

### 1-(1-(2-(Quinolin-6-yl)acetyl)piperidin-4-yl)-1H-benzo[d]imidazol-2(3H)-one

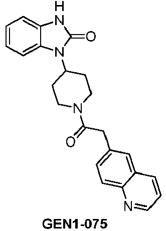

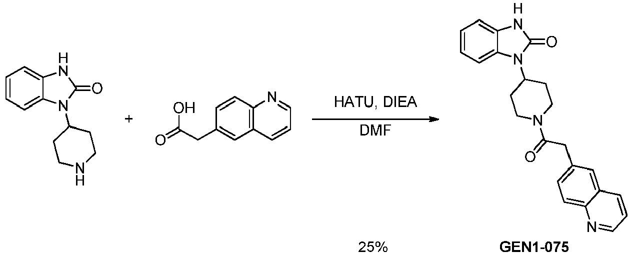

### Step 1. Synthesis of 1-(1-(2-(quinolin-6-yl)acetyl)piperidin-4-yl)-1H-benzo[d]imidazol-2(3H)-one (GEN1-075)

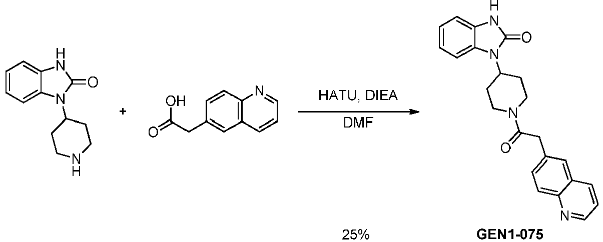

To a solution of 2-(6-quinolyl)acetic acid (187 mg, 0.999 mmol), 3-(4-piperidinyl)-1H-benzimidazol-2-one (217 mg, 0.999 mmol) and HATU (494 mg, 1.30 mmol) in DMF (4 mL) was added DIEA (387 mg, 2.99 mmol). The mixture was stirred at 25 °C for 6 h and then purified through a C18 column (CH₃CN:water = 5% to 50%) to give the desired compound (100 mg, yield: 25%) as a white solid. ¹H NMR (400 MHz, CDCl₃): δ 9.54 (s, 1H), 8.91 (s, 1H), 8.15 (t, *J* = 8.4 Hz, 2H), 7.78 (s, 1H), 7.71 (d, *J* = 8.4 Hz, 1H), 7.44-7.40 (m, 1H), 7.05-6.98 (m, 2H), 6.89 (t, *J* = 7.6 Hz, 1H), 6.76 (d, *J* = 8.0 Hz, 1H), 4.93 (d, *J* = 13.2 Hz, 1H), 4.57-4.46 (m, 1H), 4.13 (d, *J* = 13.6 Hz, 1H), 4.02 (s, 2H), 320 (t, *J* = 12.4 Hz, 1H), 2.73 (t, *J* = 14.8 Hz, 1H), 2.33-2.23 (m, 1H), 2.03-1.93 (m, 1H), 1.89 (d, *J* = 12.8 Hz, 1H), 1.78-1.73 (m, 1H). MS (ESI): Rt = 2.639 min, *m*/*z* 387.2, [M+H]⁺; purity: 99.84% @ 214 nm, 96.55% @ 254 nm.

### Example 23

### 3-[1-[2-(1H-Indol-5-yl)acetyl]-4-piperidinyl]-1H-benzimidazol-2-one

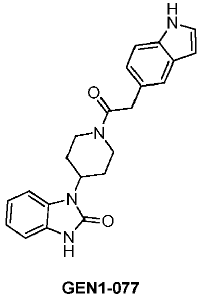

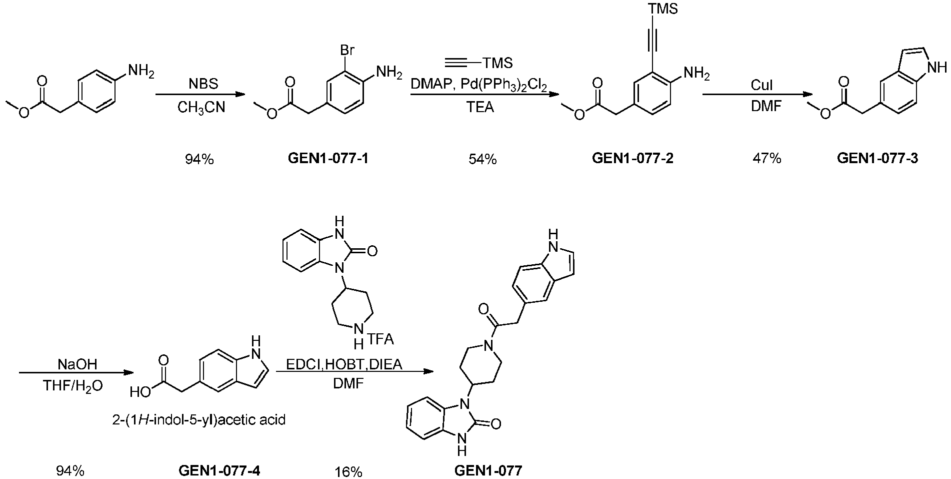

### Step 1. Synthesis of methyl 2-(4-amino-3-bromophenyl)acetate

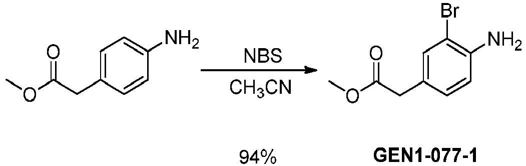

To a solution of methyl 2-(4-aminophenyl)acetate (3.00 g, 18.2 mmol) in acetonitrile (10 mL) was added dropwise a solution of 1-bromopyrrolidine-2,5-dione (3.23 g, 18.2 mmol) in acetonitrile (10 mL), and the mixture was stirred at room temperature overnight. The resulting mixture was filtered, and the filtrate was concentrated. The residue was purified through a silica gel column (petroleum ether:EtOAc = 5:1) to give the desired compound (4.15 g, yield: 94%) as a pale yellow oil. MS (ESI) Rt = 1.43 min, *m*/*z* 245.0 [M+H]⁺; purity: 100% @ 254 nm, 100% @ 214 nm.

### Step 2. Synthesis of methyl 2-(4-amino-3-((trimethylsilyl)ethynyl)phenyl)acetate

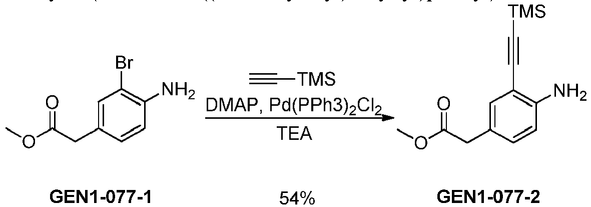

To a solution of methyl 2-(4-amino-3-bromophenyl)acetate (2.00 g, 8.19 mmol), ethynyltrimethylsilane (8.05 g, 81.9 mmol) and DMAP (100 mg, 0.819 mmol) in Et3N (10 mL) was added Pd(PPh3)2Cl2 (575 mg, 0.819 mmol). The mixture was stirred at 70 °C overnight under a nitrogen atmosphere. The resulting mixture was concentrated, and water (50 mL) was added. DCM (50 mL × 3) was added to extract the desired compound. The combined organic layers were washed with brine (50 mL), dried over anhydrous Na₂SO₄ and filtered. The filtrate was concentrated, and the residue was purified through a silica gel column (petroleum ether:EtOAc = 20:1) to give the desired compound (1.16 g, yield: 54%) as a yellow oil. MS (ESI) Rt = 1.71 min, *m*/*z* 262.2 [M+H]⁺; purity: 100% @ 254 nm, 100% @ 214 nm.

### Step 3. Synthesis of methyl 2-(1H-indol-5-yl)acetate

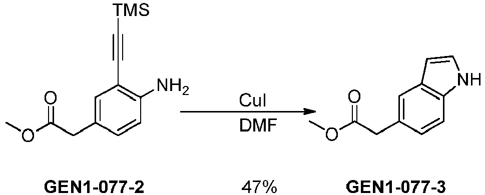

To a mixture of methyl 2-(4-amino-3-(((trimethylsilyl)ethynyl)phenyl)acetate (1.16 g, 4.44 mmol) in DMF (10 mL) was added CuI (85 mg, 0.446 mmol). The mixture was stirred at 90 °C for 24 h under a nitrogen atmosphere. The resulting mixture was cooled to room temperature and poured into water (30 mL). DCM (50 mL × 3) was added to extract the desired compound. The combined organic layers were washed with brine (50 mL), dried over anhydrous Na₂SO₄ and concentrated. The residue was purified by RP-HPLC (45-65% B; A: H₂O, B: CH₃CN) to give the desired compound (393 mg, yield: 47%) as a yellow oil. MS (ESI) Rt = 1.43 min, *m*/*z* 188.2 [M-H]-; purity: 100% @ 254 nm, 100% @ 214 nm.

### Step 4. Synthesis of 2-(1H-indol-5-yl)acetic acid

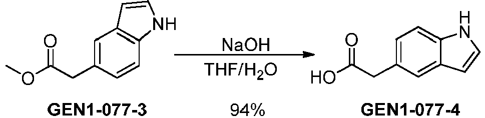

To a solution of methyl 2-(1H-indol-5-yl)acetate (393 mg, 2.08 mmol) in THF (6 mL) and H₂O (3 mL) was added NaOH (166 mg, 4.15 mmol). The mixture was stirred at 50 °C overnight. The resulting mixture was concentrated, and water (15 mL) was added. The mixture was acidified to pH 1 with an aqueous HCl solution (12 M), and DCM (50 mL × 3) was added to extract the desired compound. The combined organic solutions were washed with brine (50 mL), dried over anhydrous Na₂SO₄ and concentrated to give the desired compound (341 mg, yield: 94%) as an ochre solid. MS (ESI) Rt = 1.142 min, *m*/*z* 176.0 [M+H]⁺; purity: 100% @ 214 nm.

### Step 5. Synthesis of 3-[1-[2-(1H-Indol-5-yl)acetyl]-4-piperidinyl]-1H-benzimidazol-2-one (GEN1-077)

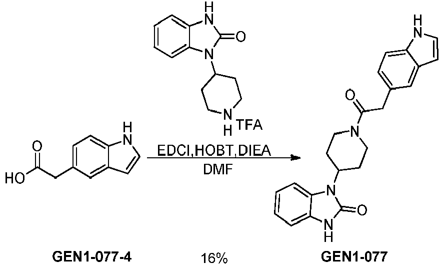

To a solution of 1-(piperidin-4-yl)-1H-benzo[d]imidazol-2(3H)-1-one 2,2,2-trifluoroacetate (254 mg, 0.767 mmol), EDCI (164 mg, 0.856 mmol), HOBT (116 mg, 0.859 mmol) and DIEA (369 mg, 2.86 mmol) in DMF (3 mL) was added 2-(1H-indol-5-yl)acetic acid (100 mg, 0.571 mmol). The mixture was stirred at room temperature overnight. The resulting mixture was poured into water (20 mL), and the mixture was filtered. The precipitate was washed with water (10 mL) and recrystallized from MeOH. The precipitate was then completely dissolved in DMSO, and hot water (2 mL) was added dropwise. The mixture was filtered, and the residue was dried to give the desired compound (35 mg, yield: 16%) as a white solid. ¹HNMR (400 MHz, DMSO-*d*₆): δ 11.02 (s, 1H), 10.79 (s, 1H), 7.48 (s, 1H), 7.37 (d, *J* = 8.4 Hz, 1H), 7.31 (s, 1H), 7.05 (d*, J* = 8.0 Hz, 1H), 6.92-6.89 (m, 2H), 6.85-6.81 (m, 1H), 6.75-6.73 (m, 1H), 6.41 (s, 1H), 4.60 (d, *J* = 12.8 Hz, 1H), 4.40-4.34 (m, 1H), 4.15-4.09 (m, 1H), 3.89-3.75 (m, 2H), 3.14-3.08 (m, 1H), 2.66 (t, *J* = 12.0 Hz, 1H), 2.02-1.93 (m, 1H), 1.78-1.70 (m, 1H), 1.65-1.63 (m, 1H), 1.50-1.47 (m, 1H). MS (ESI) Rt = 3.835 min, *m*/*z* 375.2 [M+H]⁺; purity: 97.76% @ 254 nm, 99.07% @ 214 nm.

### Example 24

### 1-(1-(2-(4-(Dimethylamino)phenyl)acetyl)piperidin-4-yl)-7-(trifluoromethyl)-1,3-dihydro-2H-benzo[d]imidazol-2-one (GEN1-118)

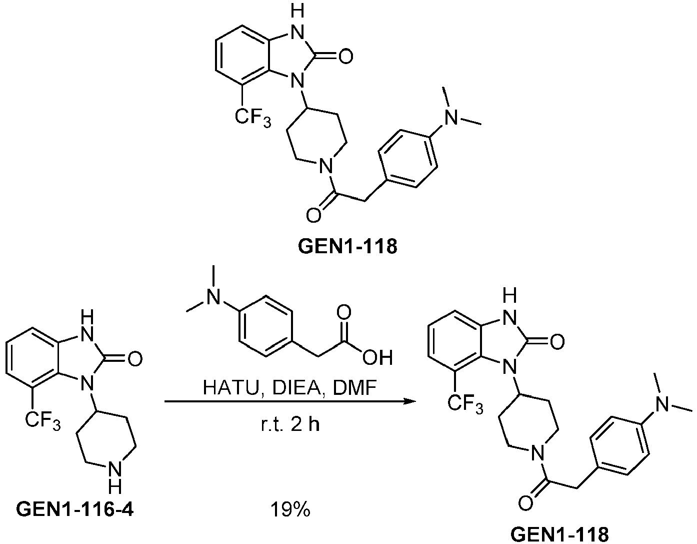

### Step 1. 1-(1-(2-(4-(Dimethylamino)phenyl)acetyl)piperidin-4-yl)-7-(trifluoromethyl)-1,3-dihydro-2H-benzo[d]imidazol-2-one (GEN1-118)

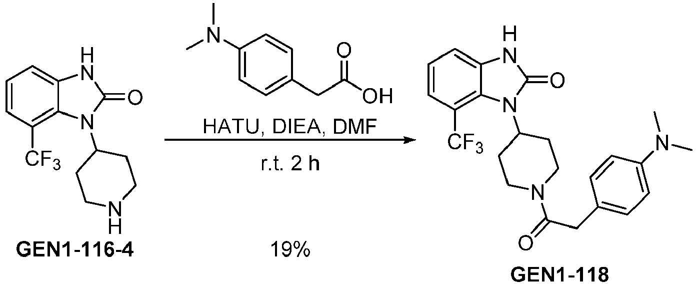

The compound 1-(piperidin-4-yl)-7-(trifluoromethyl)-1,3-dihydro-2H-benzo[d]imidazol-2-one (71 mg, 0.25 mmol), HATU (140 mg, 0.37 mmol) and DIEA (129 mg, 1.00 mmol) were dissolved in DMF (1.5 mL), and 2-(4-(dimethylamino)phenyl)acetic acid (46 mg, 0.25 mmol) was added. After the addition was complete, the resulting mixture was stirred at room temperature for 5 h. The reaction mixture was added dropwise to water (50 mL), and the mixture was filtered to give a white solid. The crude product was purified by Prep-TLC (MeOH/(MeOH + DCM) = 60%) to give the desired compound (21.1 mg, yield: 19%) as a white solid. ¹H NMR (400 MHz, DMSO-d₆): δ 11.45 (s, 1H), 7.36 (d, *J* = 8.2, 1H), 7.27 (d, *J* = 7.8 Hz, 1H), 7.16 (t, *J* = 7.9 Hz, 1H), 7.06 (d, *J* = 8.6 Hz, 2H), 6.67 (d, *J* = 8.4 Hz, 2H), 4.67-4.54 (m, 1H), 4.29-4.07 (m, 2H), 3.74-3.49 (m, 2H), 2.93 (t, *J* = 13.1 Hz, 1H), 2.86 (s, 6H), 2.56-2.51 (m, 1H), 2.49-2.33 (m, 2H), 1.75-1.59 (m, 2H). MS (ESI): Rt = 1.62 min, *m*/*z* 446.9 [M + H]⁺, purity: 100% @ 254 nm, 100% @ 214 nm.

### Example 25

### 1-(1-(2-(Quinoxalin-6-yl)acetyl)piperidin-4-yl)-7-(trifluoromethyl)-1,3-dihydro-2H-benzo[d]imidazol-2-one (GEN1-140)

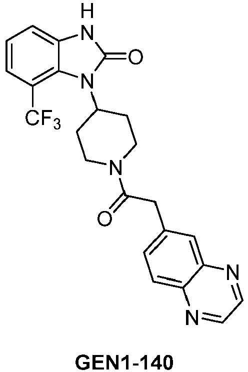

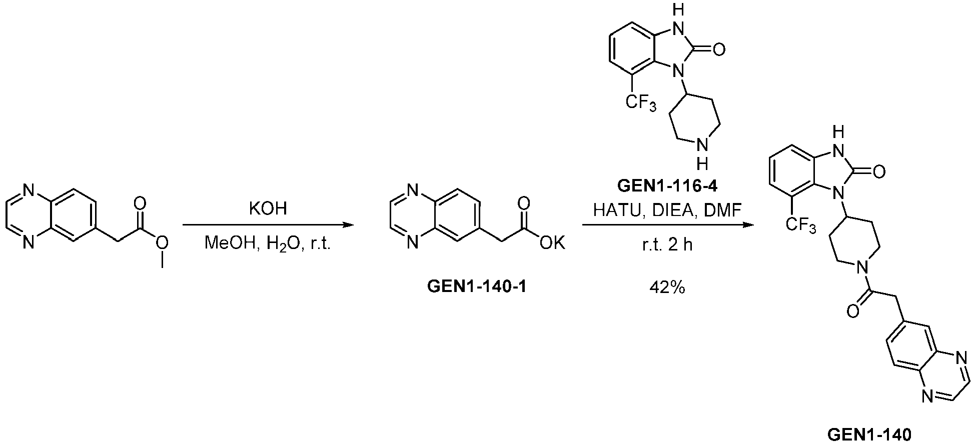

### Step 1. Potassium 2-(quinoxalin-6-yl)acetate (GEN1-140-1)

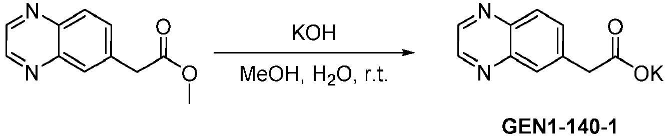

To a solution of methyl 2-(quinoxalin-6-yl)acetate (100 mg, 0.50 mmol) in MeOH (2 mL) was added a solution of KOH (28 mg, 0.50 mmol) in water (0.2 mL). The resulting mixture was stirred at room temperature for 5 h. The resulting mixture was concentrated. The crude product was directly used in the next step without further purification. TLC:MeOH/(DCM + MeOH) = 5%, Rf = 0.6

### Step 2. 1-(1-(2-(Quinoxalin-6-yl)acetyl)piperidin-4-yl)-7-(trifluoromethyl)-1,3-dihydro-2H-benzo[d]imidazol-2-one (GEN1-140)

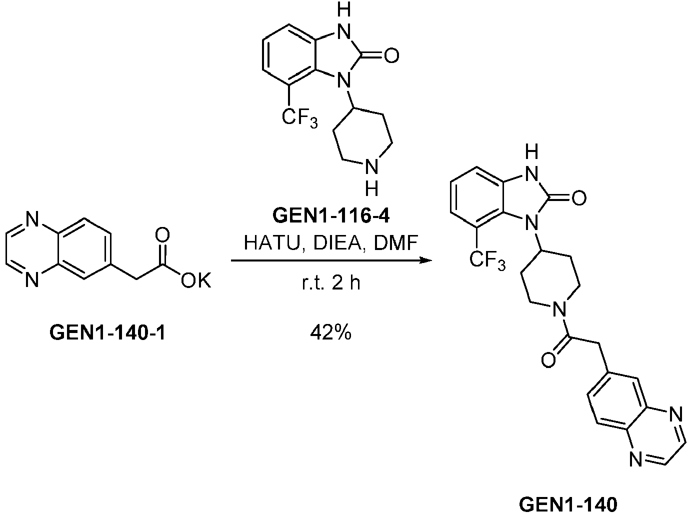

To a solution of potassium 2-(quinoxalin-6-yl)acetate (102 mg, crude), 1-(piperidin-4-yl)-7-(trifluoromethyl)-1,3-dihydro-2H-benzo[d]imidazol-2-one (140 mg, 0.49 mmol) and HATU (280 mg, 0.74 mmol) in DMF (2.5 mL) was added DIEA (125 mg, 0.98 mmol). The resulting mixture was stirred at room temperature for 2 h. The reaction mixture was added dropwise to water (20 mL). The solid precipitate was collected by filtration. The crude product was purified by reversed-phase flash chromatography (C₁₈ reversed-phase silica gel column; mobile phase CH₃CN, water, 10% to 70% gradient, 40 min; detection, UV 214 nm) to give the desired compound (94.3 mg, yield: 42%) as a white solid. ¹H NMR (400 MHz, DMSO-d₆): δ 11.45 (s, 1H), 8.96-8.89 (m, 2H), 8.05 (d, *J* = 8.6 Hz, 1H), 8.01-7.96 (m, 1H), 7.81-7.72 (m, 1H), 7.36 (d, *J* = 8.2, 1H), 7.30-7.24 (m, 1H), 7.16 (t, *J* = 7.9 Hz, 1H), 4.62 (d, *J* = 12.5 Hz, 1H), 4.35-4.20 (m, 2H), 4.18-3.97 (m, 2H), 3.06 (t, *J* = 13.2 Hz, 1H), 2.67-2.51 (m, 2H), 2.48-2.43 (m, 1H), 1.82-1.61 (m, 2H). MS (ESI): Rt = 1.42 min, *m*/*z* 456.1 [M + H]⁺, purity: 100% @ 254 nm, 100% @ 214 nm.

### Comparative Example 1

### 1-(1-(2-Phenylacetyl)piperidin-4-yl)-1H-benzo[d]imidazol-2(3H)-one (X4)

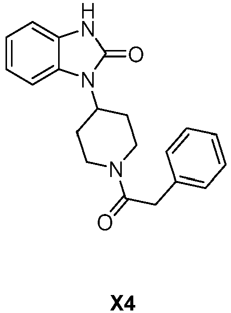

### Step 12.1. Synthesis of 1-(1-(2-phenylacetyl)piperidin-4-yl)-1H-benzo[d]imidazol-2(3H)-one (X4)

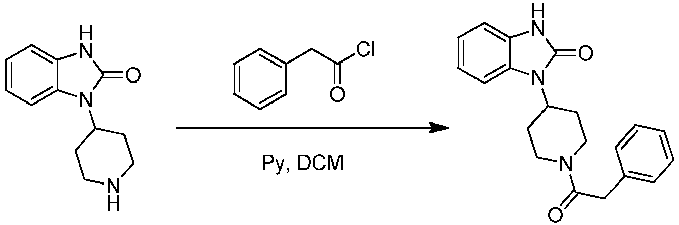

To DCM (40 mL) were added 1-(piperidin-4-yl)-1H-benzo[d]imidazol-2(3H)-one hydrochloride (1.50 g, 5.90 mmol) and pyridine (1.87 g, 23.6 mmol), and 2-phenylacetyl chloride (2.28 g, 14.8 mmol) was added dropwise at 0 °C. The reaction mixture was stirred at room temperature for 16 h, diluted with water and then extracted with DCM. The combined organic layers were dried over Na₂SO₄ and concentrated under reduced pressure to give a crude product. The crude product was purified by high performance liquid chromatography (10-95% aqueous CH₃CN solution) to give the desired white solid (1.46 g, yield: 63%). ¹H NMR (400 MHz, CDCl₃) δ 10.82 (s, 1 H), 7.38-7.21 (m, 5 H), 6.96-6.95 (m, 4H), 4.57 (d, *J* = 13.2 Hz, 1 H), 4.45-4.35 (m, 1 H), 4.09 (d, *J* = 13.6 Hz, 1 H), 3.84-3.74 (m, 2 H), 3.19-3.08 (m, 1 H), 2.73-2.62 (m, 1 H), 2.11-1.88 (m, 2 H), 1.72-1.54 (m, 2 H). MS (ESI) *m*/*z* 336.1 [M + H]⁺, purity: 97.9% @ 254 nm, 99.9% @ 214 nm. Comparative Example 2

### 1-(1-(2-(4-Methoxyphenyl)acetyl)piperidin-4-yl)-1H-benzo[d]imidazol-2(3H)-one

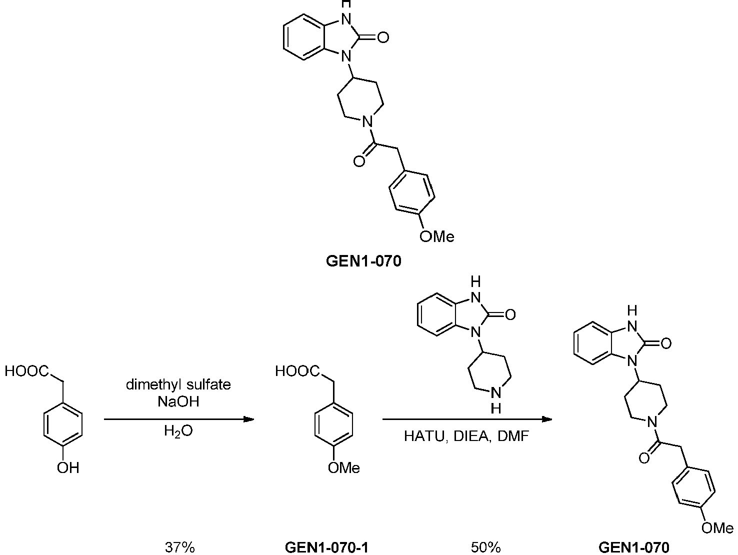

### 1.1. Synthesis of 2-(4-methoxyphenyl)acetic acid (GEN1-070-1)

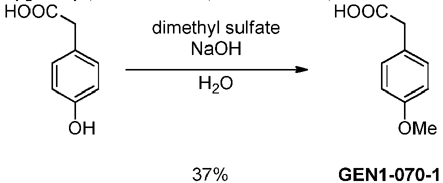

To a solution of 2-(4-hydroxyphenyl)acetic acid (1.00 g, 6.57 mmol) and NaOH (1.58 g, 39.4 mmol) in water (15 mL) was added dropwise dimethyl sulfate (2.07 g, 16.4 mmol). The resulting mixture was stirred at 45 °C for 16 h. The resulting mixture was acidified with concentrated hydrochloric acid. HCl was added until pH = 1-2, and the mixture was filtered. The filter cake was washed with water (5 mL × 2) to give the desired compound (401 mg, yield: 37%) as a white solid. ¹HNMR (400 MHz, DMSO-*d*₆): δ 12.15 (br s, 1H), 7.16 (d, *J* = 8.8 Hz, 2H), 6.86 (d, *J* = 8.4 Hz, 2H), 3.73 (s, 3H), 3.48 (s, 2H).

### Step 2. Synthesis of 1-(1-(2-(4-methoxyphenyl)acetyl)piperidin-4-yl)-1H-benzo[d]imidazol-2(3H)-one (GEN1-070)

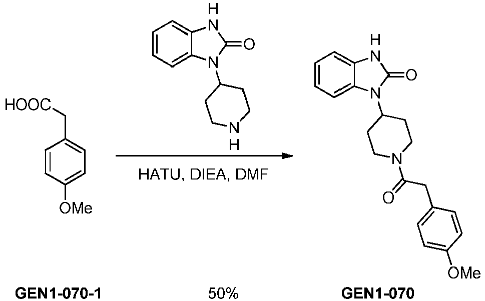

To a solution of 3-(4-piperidinyl)-1H-benzimidazol-2-one (157 mg, 0.723 µmol), 2-(4-methoxyphenyl)acetic acid (100 mg, 0.602 mmol) and DIEA (233 mg, 1.80 mmol) in DMF (1.5 mL) was added HATU (343 mg, 0.902 mmol), and the mixture was stirred at room temperature for 16 h. The resulting mixture was purified by preparative chromatography. HPLC (waters-3 sunfire C18 5 µm 19 × 150 mm, 20%-50% B; A: H₂O (0.1% TFA), B: CH₃CN; UV: 214 nm, flow rate: 15 mL/min). Saturated Na₂CO₃ was then added to basify the mixture to pH 8. The mixture was extracted with EtOAc (20 mL × 3), and the combined organic layers were concentrated to give the desired compound (111 mg, yield: 50%) as a white solid. ¹HNMR (400 MHz, CDCl₃): δ 9.23-9.13 (m, 1H), 7.26-7.24 (m, 2H), 7.08-6.99 (m, 3H), 6.91 (d, *J* = 8.8 Hz, 2H), 6.83 (d, *J* = 7.6 Hz, 1H), 4.88 (d, *J* = 13.2 Hz, 1H), 4.56-4.48 (m, 1H), 4.06 (d, *J* = 14 Hz, 1H), 3.78 (s, 3H), 3.76 (s, 2H), 3.18-3.11 (m, 1H), 2.72-2.65 (m, 1H), 2.27-2.16 (m, 1H), 1.96-1.81 (m, 2H), 1.72-1.67 (m, 1H). MS (ESI): Rt = 3.095 min, *m*/*z* 366.2 [M+H]⁺; purity: 99.31% @ 254 nm, 99.64% @ 214 nm.

### Comparative Example 3

### 1-(1-(1-(2-(1H-Indol-3-yl)acetyl)piperidin-4-yl)-7-(trifluoromethyl)-1,3-dihydro-2H-benzo[d]imidazol-2-one (GEN1-169)

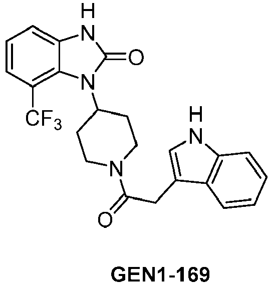

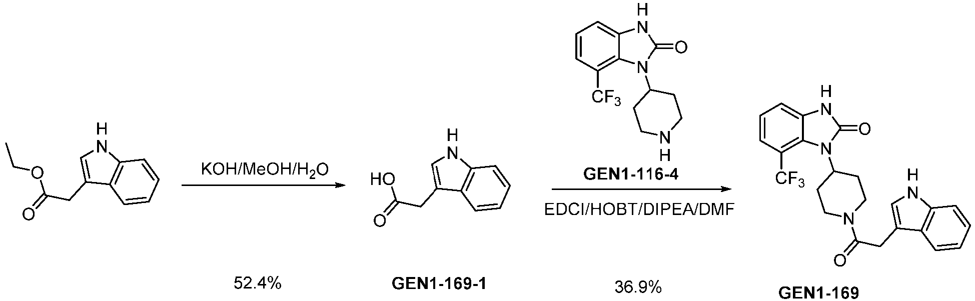

### Step 1. Synthesis of 2-(1H-indol-3-yl)acetic acid (GEN1-218-1)

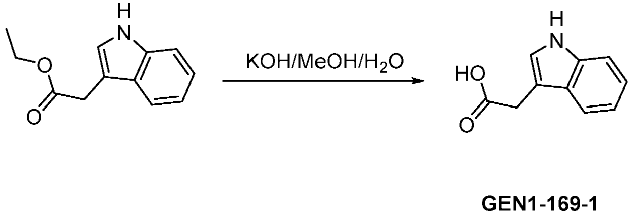

To a suspension of ethyl 2-(1H-indol-3-yl)acetate (100 mg, 0.49 mmol) in MeOH (5 mL) and H₂O (5 mL) was added, at room temperature, KOH (41.2 mg, 0.735 mmol). The resulting mixture was stirred at room temperature for 3 h. The reaction mixture was diluted with water (30 mL), and the pH was adjusted to 4 with 1 N HCl. The mixture was then extracted with EtOAc (30 mL × 3). The combined organic phases were washed with brine (30 mL), then dried over anhydrous Na₂SO₄ and filtered. The filtrate was concentrated to give the desired compound (45 mg, yield: 52.4%) as a white solid. MS (ESI): Rt = 0.26 min, m/z 176.2, [M + H]⁺;

### Step 2. Synthesis of 1-(1-(1-(2-(1H-indol-3-yl)acetyl)piperidin-4-yl)-7-(trifluoromethyl)-1,3-dihydro-2H-benzo[d]imidazol-2-one (GEN1-169)

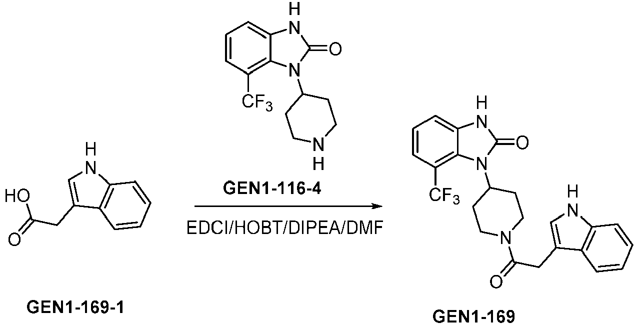

To a solution of 1-(piperidin-4-yl)-7-(trifluoromethyl)-1,3-dihydro-2H-benzo[d]imidazol-2-one (50 mg, 0.175 mmol, see GEN1-116-4 for its synthesis), 2-(1H-indol-3-yl)acetic acid (44.8 mg, 0.21 mmol), HOBT (35.5 mg, 0.263 mmol) and DIEA (45.2 mg, 0.35 mmol) in DMF (1 mL) was added EDCI (50.4 mg, 0.263 mmol). The resulting mixture was stirred at room temperature for 1 h. The reaction mixture was diluted with water (30 mL) and extracted with EtOAc (20 mL × 3). The combined organic phases were washed with brine (30 mL), then dried over anhydrous Na₂SO₄ and filtered. The filtrate was concentrated to give a crude product. The crude product was purified through a reversed-phase chromatography column. Purification conditions: C18 silica gel column; mobile phase ACN: H₂O, gradient: 5% to 50%; 45 min, monitoring wavelength: 214 nm. The desired compound (28.6 mg, yield: 36.9%) was obtained as a white solid. ¹H NMR (400 MHz, DMSO-d6) δ 11.5-11.34(m,1H), 10.91 (s, 1H), 7.57 (d, J = 7.9 Hz, 1H), 7.41-7.31 (m, 2H), 7.28 (d, J = 7.6 Hz, 1H), 7.24-7.20 (m, 1H), 7.19-7.12 (m, 1H), 7.07 (t, J = 7.5 Hz, 1H), 6.97 (t, J = 7.4 Hz, 1H), 4.70-4.54 (m, 1H), 4.30-4.16 (m, 2H), 3.92-3.70 (m, 2H), 2.98 (t, J = 13.0 Hz, 2H), 2.71-2.56 (m, 1H), 2.47-2.31 (m, 2H), 1.74-1.64 (m, 2H). MS (ESI): Rt = 1.50 min, m/z 443.2, [M + H]⁺; purity: 100% @ 254 nm.

### Effect Example 1: HTRF Experiment

### Experimental materials:

A multifunctional microplate reader (TECAN; INFINITE F PLEX);
96-well cell culture plates (Bioyong), HTRF 384-well low volume plates (Cisbio; Cat#66PL384025);
Human interferon β (IFN-β) (GenScript; Cat#P01574), STATS phospho-Y694 kits (Cisbio; Cat#64AT5PEG).

### Experimental procedure:

Hela cells (American Type Culture Collection (ATCC), CCL-2) were cultured in a 96-well plate with DMEM containing 5% fetal bovine serum at a density of 3 × 10⁴ cells/well. The cells were incubated overnight in a 37 °C, 5% carbon dioxide humidified incubator. The next day, the original DMEM in the 96-well plate was blotted, and equal volumes of medium containing different concentrations of compounds (stock solutions of compounds prepared with dimethyl sulfoxide were diluted to desired concentrations in medium) were added for 2 h of pretreatment. The plate was left to stand in a 37 °C incubator. Human interferon β was then added to a final concentration of 10 nM for stimulation. The plate was gently shaken so the contents were well mixed, and the plate was then left to stand in a 37 °C incubator for 30 min.

The medium was removed, and the plate was washed once with an appropriate volume of PBS and then blotted dry. Then 1× lysis buffer was added at 40 µL/well, and the plate was left to stand at room temperature for 30 min (preparation of 1 mL of 1× lysis buffer: mixing well 740 µL of ddH₂O with 250 µL of 4× lysis buffer and 10 µL of Blocking reagent). After 30 min, the cells were repeatedly pipetted until the lysis buffer was not viscous (bubbles were avoided) so that sufficient lysis was achieved.

In an HTRF 384-well low volume plate, 4 µL of an antibody premix (containing two antibodies: Cryptate and d2: the two antibodies were each 20-fold diluted with detection buffer and were then mixed in equal volume to form the antibody premix) and 16 µL of the cell lysate were added to each well. The plate was sealed with a sealing membrane and then incubated overnight at room temperature in a dark place.

After the overnight incubation, signals at 665 nm and 620 nm were detected separately on a multifunctional microplate reader. The calculation formula for the STATS Y694 phosphorylation assay is: Ratio = [(signal 665 nm) / (signal 620 nm)] × 10⁴. A dimethyl sulfoxide-only treatment group was set as a negative control (Ratio_{Negative}), and an interferon β stimulation group without compound addition was set as a positive control (Ratio_{Positive}). The inhibition rates of different samples against STATS Y694 phosphorylation were normalized: inhibition rate = [1 - (Ratio_{Experiment} - Ratio_{Negative}) / (Ratio_{Positive} - Ratio_{Negative})] × 100. The results are shown in Table 1.

**Table 1. Quantitative data for biochemical-level inhibitory activity against cells (HTRF experiment)**

| **Compound No.** | **Inhibition rate @10 µM** | **IC₅₀/µM** |
|---|---|---|
| GEN1-169 | 13.47% | - |
| GEN1-077 | 34.25% | - |
| GEN1-117 | 89.31% | - |
| GEN1-118 | 27.57% | - |
| GEN1-140 | 53.79% | - |
| GEN1-284 | 80.21% | 3.9 |
| GEN1-284 salt | 82.46% | 2.6 |
| GEN1-298 | 51.98% | - |
| GEN1-300 | 72.83% | 7.6 |
| GEN1-301 | 80.70% | 8.8 |
| GEN1-302 | 80.37% | 7.0 |
| GEN1-303 | 91.91% | 5.5 |
| GEN1-304 | 40.76% | - |
| GEN1-305 | 63.05% | - |
| GEN1-306 | 95.64% | - |
| GEN1-312 | 55.50% | - |

| | | |
|---|---|---|
| Note: "-" means not measured. | | |

### Effect Example 2: Comparison of PK Profiles

Method: SD rats from acceptable sources (N = 3, female, 6-8 weeks, weighing 200-230 g, purchased from JH Laboratory Animal Co. LTD, SCXK (SH) 2017-0012 20170012001153) or beagles (N = 3, not experimentally naive animals, female, weighing 6.55-8.62 kg, purchased from Beijing Marshall Biotech Co. Ltd., quality inspection number SCXK (BJ) 2016-0001 110318201100068072) were selected and administered, by intravenous injection or oral gavage (3 animals per route), corresponding doses of the test compound GEN1-117 or GEN1-284s (the sample for intravenous injection was dissolved in a solution of 10% DMSO, 10% Soltol HS15 and 80% PBS, and the sample for oral administration was dissolved in a 0.5% aqueous methylcellulose solution). Peripheral blood samples (150 µL) were taken (at 0, 0.083, 0.25, 0.5, 1, 2, 4, 8 and 24 h for the intravenous injection route; at 0, 0.25, 0.5, 1, 2, 4, 8 and 24 h for the oral injection route; into K2-EDTA anticoagulant tubes; centrifuged at 2000 g at 4 C for 5 min within 15 min), and plasma was separated and subjected to LC-MS analysis (LC-MS/MS-37, triple Quad 6500) using the conventional method with diclofenac as an internal standard. Mass spectrometry conditions: ESI cation, MRM detection:

| Name of compound | Parent (m/z) / Daughter (m/z) |
|---|---|
| GEN1-284s | Q1/Q3 Masses: 565.00/467.00 Da |
| GEN1-117 | Q1/Q3 Masses: 455.10/286.20 Da |
| Diclofenac | Q1/Q3 Masses: 296.20/214.20 Da |

### HPLC conditions: mobile phase A: 0.1% FA in water, mobile phase B: 0.1% FA in ACN

| Time (min) | Mobile phase (%) |
|---|---|
| 0.20 | 10 |
| 1.10 | 95 |
| 1.80 | 95 |
| 1.81 | 10 |
| 2.30 | End |

### Chromatography column: Waters ACQUITY UPLC HSS T3 (2.1 × 50 mm, 1.8 µm)

| | |
|---|---|
| Flow rate: | 0.60 mL/min |

### Column temperature: 60 °C

| Retention time: | | |
|---|---|---|
| | GEN1-284s | 1.08 min |
| | GEN1-117 | 1.18 min |
| | Diclofenac | 1.41 min |

A test sample standard curve was prepared from 1, 2, 10, 30, 100, 300, 1000, 2700 and 3000 ng/mL (50% ACN in water), and 3 µL of standard-curve solution was mixed with 57 µL of same-species blank plasma. A test plasma sample (60 µL) was well mixed with 6 µL of 10% FA in water, and 30 µL of the mixture was taken and well mixed with 200 µL of the internal standard compound diclofenac (40 ng/mL in ACN). After the resulting mixture was vortexed and shaken for 1 min and centrifuged at 5800 rpm for 10 min, 100 µL of the supernatant was transferred to a new test plate, and 1 µL of solution was loaded on an LC-MS/MS system.

**Table 2. The PK profile of GEN1-117 in rats:**

| **Route/dose of administration** | **Pharmacokinetic parameter** | **Test compound GEN1-117** |
|---|---|---|
| i.v. (1mg/kg) | t_{1/2} (h) | 4.20 |
| | Vdₛₛ (L/kg) | 2.75 |
| | Cl (L/h/kg) | 0.62 |
| | AUC₀₋ₗₐₛₜ (h*ng/mL) | 1641 |
| p.o. (10mg/kg) | Cₘₐₓ (ng/mL) | 42.8 |
| | t_{1/2} (h) | 4.28 |
| | Tₘₐₓ (h) | 4.00 |
| | AUC₀₋ₗₐₛₜ (h*ng/mL) | 423 |
| | F% | 2.6% |

**Table 3. The PK profile of GEN1-284s in rats:**

| **Route and dose of administration** | **Pharmacokinetic parameter** | **GEN1-117** | **GEN1-284s** |
|---|---|---|---|
| i.v.(1mg/kg) GEN1-284s | t_{1/2} (h) | 4.16 | 0.39 |
| | Vdₛₛ (L/kg) | NA | 0.047 |
| | Cl (L/h/kg) | NA | 0.374 |
| | AUC₀₋ₗₐₛₜ (h*ng/mL) | 1509 | 2746 |
| p.o. (10mg/kg) GEN1-284s | Cₘₐₓ (ng/mL) | 2443 | 6.04 |
| | t_{1/2} (h) | 3.82 | 0.95 |
| | Tₘₐₓ (h) | 2.00 | 0.42 |
| | AUC₀₋ₗₐₛₜ (h*ng/mL) | 14608 | 5.72 |
| | F% | 96.3% | - |

**Table 4. The PK profile of GEN1-284s in beagles:**

| **Route and dose of administration** | **Pharmacokinetic parameter** | **GEN1-117** | **GEN1-284s** |
|---|---|---|---|
| i.v.(0.5mg/kg) GEN1-284s | t_{1/2} (h) | 4.06 | 0.085 |
| | Vdₛₛ (L/kg) | NA | 0.155 |
| | Cl (L/h/kg) | NA | 2.61 |
| | AUC₀₋ₗₐₛₜ (h*ng/mL) | 1997 | 207 |
| p.o. (5mg/kg) GEN1-284s | Cₘₐₓ (ng/mL) | 2773 | ND |
| | t_{1/2} (h) | 4.18 | ND |
| | Tₘₐₓ (h) | 1.33 | ND |
| | AUC₀₋ₗₐₛₜ (h*ng/mL) | 17936 | ND |
| | F% | 89.8% | - |

### Effect Example 3. Evaluation of Inhibitory Capacities Against hERG Ion Channel

A CHO cell line stably transfected with cDNA and expressing hERG channels of P15 was used for the study. Cells were maintained in Petri dishes or flasks at 37 °C in a humidified incubator with 5% CO2 and cultured in a medium containing Ham's F12, 10% (v/v) heat-inactivated FBS, 100 µg/mL hygromycin B and 100 µg/mL geneticin (from Invitrogen). The cells were allowed to grow and reach a confluency of about 80-90% under the above conditions. Before the experiment, the cells were treated with Detachin (from Genlantis) for 3-5 min, at 37 °C, then gently mixed well with medium at room temperature, and titrated 15-20 times with a pipette. Then the cells were resuspended in a serum-free medium containing CHO-S-SFM II medium (from Invitrogen) buffered with HEPES (25 mM). The cells used in QPatch study must meet the following criteria: under microscopy examination, the majority of cells in suspension should be single and isolated; their viability should be greater than 95%, with only a small amount of debris and cell clumps (which may clog the holes in QPlate during whole-cell clamp recording); the cell density should be within the range of 3-8 × 106 cells/mL in the final suspension before application to the QPatch stir chamber. Cells under such conditions can be used for recording only within four hours of being harvested.

Data acquisition was performed using automated QPatch (Sophion Biosciences, Denmark). The cells were voltage-clamped at a holding potential of -80 mV. The hERG current was activated by depolarization at +20 mV for 5 s, after which the current was activated back to -50 mV for 2.5 s to remove the inactivation and observe the outward tail current. The maximum amount of tail current was used to determine hERG current amplitude.

| Reagent | External solution (mM) | Internal solution (mM) |
|---|---|---|
| CaCl2 | 2 | 5.374 |
| MgCl2 | 1 | 1.75 |
| KCl | 4 | 120 |
| NaCl | 145 | - |
| Glucose | 10 | - |
| HEPES | 10 | 10 |
| EGTA | | 5 |
| Na-ATP | - | 4 |
| pH | 7.4 (adjusted with NaOH). | 7.25 (adjusted with KOH), |
| Osmotic pressure | ∼305 mOsm | ∼295 mOsm |

Six doses (30, 10, 3, 1, 0.3 and 0.1 µM) were selected to obtain a fitted curve and IC⁵⁰. The compound was prepared into serial dilutions in 3, 1, 0.3 and 0.1 mM DMSO vehicle to the final µM ranges before the QPatch test. 5-10 minutes' ultrasonication and vortexing were generally used for solution to help completely dissolve the compound. The final DMSO concentration was 0.1% or lower, except that for the 30 µM point, the DMSO concentration was about 0.3%. The IC50 evaluation doses of the positive control cisapride were 3, 1, 0.3, 0.1, 0.03 and 0.01 µM.

After achieving break-in (whole-cell) configuration, the cells were recorded for 120 s to assess current stability. The voltage settings described above were then applied to the cells once every 15 s throughout the whole process. Only stable cells with recording parameters above threshold were allowed to enter the drug assessment procedure. All the experiments were conducted at room temperature (about 25 °C).

An external solution containing 0.1% DMSO (vehicle) was applied to the cells to establish a baseline. After allowing the current to stabilize for 3 min, the compound was added. The compound solution was added and the cells were kept in the test solution until the effect of the compound reached a steady state or for a maximum of 4 min. For dose response assays, the compound was applied cumulatively to the cells from low to high concentrations. Washing with external solution was performed after compound testing. The positive control cisapride was used in the experiments to test the same batch of cells used for test compounds to ensure the normal response and the good quality of the cells.

**Table 5**

| | 1.1 GEN1-117 | | | | |
|---|---|---|---|---|---|
| Concentration (µM) | | % of hERG inhibition | | Mean % hERG inhibition | SD* |
| | | Cell 1 | Cell 2 | | |
| 0.1 | | 3.84 | -0.21 | 1.81 | 2.87 |
| 0.3 | | 3.95 | 4.17 | 4.06 | 0.16 |
| 1 | | 7.99 | 13.39 | 10.69 | 3.81 |
| 3 | | 11.01 | 13.35 | 12.18 | 1.65 |
| 10 | 12.75 | 19.32 | 16.04 | 4.64 | |
| 30 | 12.80 | 19.26 | 16.03 | 4.57 | |

| | | | | | |
|---|---|---|---|---|---|
| * Sample testing was repeated in different cells to ensure that single concentration standard error was below 10. Outliers are not listed in the report. | | | | | |

**Table 6**

| | 1.2 GEN1-284S | | | | |
|---|---|---|---|---|---|
| Concentration (µM) | | % percent hERG inhibition | | Mean percent hERG inhibition | SD* |
| | | Cell 1 | Cell 2 | | |
| 0.1 | | 2.22 | 8.28 | 5.25 | 4.29 |
| 0.3 | | 10.22 | 2.25 | 6.24 | 5.64 |
| 1 | | 2.88 | 0.00 | 1.44 | 2.04 |
| 3 | | 0.00 | 2.66 | 1.33 | 1.88 |
| 10 | | 9.28 | 3.41 | 6.34 | 4.15 |
| 30 | | 19.05 | 8.86 | 13.95 | 7.21 |

| | | | | | |
|---|---|---|---|---|---|
| *Sample testing was repeated in different cells to ensure that single concentration standard error was below 10. Outliers are not listed in the report. Conclusion: Both GEN1-117 and GEN1-284s have no significant inhibitory activity against hERG. | | | | | |

### Effect Example 4: Evaluation of Therapeutic Effect of GEN1-117 on Rat AIA Model

**Abstract:** Arthritis is a chronic inflammatory disease. It is particularly important to construct an arthritis disease model for pathological and pharmacological research. An AIA animal model can be easily induced by subcutaneously administering one injection of adjuvant antigen into the base of the tail of a susceptible breed, and it can produce immune responses. Therefore, the model can be constructed using this method, so that studies on the effectiveness of drugs can be carried out.

**Objective:** GEN1-117 has the function of inhibiting STATS activation. This experiment was intended to evaluate the therapeutic effect of GEN1-117 on an adjuvant arthritis rat *in vivo* model.

**Method:** A tubercle bacillus-induced arthritis model was constructed using female Lewis rats (6-8 weeks) by the standard method. After 9 days, during the disease, tofacitinib or a control agent was orally administered every day, and the GEN1-117 compound was intraperitoneally injected every day. Body weight was measured daily, foot swelling and right hind paw volume were measured once every two days, and the therapeutic effect of the test compound was evaluated. At the end of the *in vivo* experiment, all the animals were euthanized.

**Results:** the disease model was successfully constructed; GEN1-117 has a significant therapeutic effect in reducing the severity of the disease. Both the foot swelling and the right hind paw volume were significantly smaller than those of the control group.

**Conclusion:** GEN1-117 has a good *in-vivo* therapeutic effect on arthritis in AIA rats.

### 1. Objective

This experiment was intended to evaluate the therapeutic effect of the test compound GEN1-117 on the adjuvant arthritis rat *in vivo* model.

### 2. Experimental standards

This project was implemented in GenEros Biopharma (Hangzhou) Ltd. All the animal experiments involved in the implementation complied with the standard procedures for the use of laboratory animals and the guide for the care and use of laboratory animals provided by IACUC of GenEros, and the animal welfare requirements of the Office of Laboratory Animal Welfare. This experiment is a non-GLP experiment.

### 3. Experimental materials

### 3.1. Instruments

Analytical balance, model: SQP, manufacturer: Beijing Sartorius Scientific Instruments Co., Ltd.; Electronic balance, model: MP5002, manufacturer: Changzhou Tianzhiping Instruments and Equipment Co., Ltd.;
Constant-temperature magnetic stirrer, model: 85-2, manufacturer: Shanghai Sile Instruments Co., Ltd. Mini vortex mixer, model: XW-80A, manufacturer: Shanghai Huxi Analysis Instrument Factory Co., Ltd. High-frequency numerical control ultrasonic cleaner, model: KQ-50TDB, manufacturer: Kunshan Ultrasonic Instruments Co., Ltd.
Centrifuge, model: 5424 R, manufacturer: Eppendorf.
Stereo microscope: model: MZ6, manufacturer: Leica.
Digital camera, model: EOS800D, manufacturer: Canon.
Olympus microscope, model: Olympus BX43, manufacturer: Japan.

### 3.2. Test drugs

**Test compound:** GEN1-117, storage condition: -20 °C (high-concentration stock solution, powder); working solutions can be freshly prepared and temporarily stored at 2-8 °C for 1 day.

Tofacitinib, purchased from Meilunbio (Lot: M0422A), storage condition: 4 °C.

### 4. Vehicle and compound preparation

Positive drug: tofacitinib: dissolved in PBS or normal saline.

Test compound: high-concentration stock solution in DMSO, diluted to appropriate concentrations with PBS or normal saline before use.

### 5. Laboratory animals

### 5.1. Use of animals

Female Lewis rats, 7-8 weeks old, purchased from Vital River Laboratory Animal Technology Co., Ltd. (Beijing, China).

### 5.2. Housing of animals

The animals used in the following experiments were all Lewis rats. After arrival at the facility of GenEros Biopharma Ltd., they were housed in an animal room under strictly controlled environmental conditions. The temperature of the room was maintained at 20-24 °C and the humidity was maintained at 30-70%. The lighting of the animal room was controlled by an electronic timer switch system: on for 12 h and off for 12 h every day (switched on at 6:00 AM and off at 18:00 PM). The animals were given *ad libitum* access to food and water.

### 6. Experimental method

### 6.1. Experimental design

Arthritis was induced by inoculation of the rats with complete Freund's adjuvant (CFA). On day 0, 0.1 mL of cfa containing 10 mg/mL M. tuberculosis in paraffin oil was intradermally injected into the left hind paws of the rats. On day 9, group design was performed. Administration was performed according to the following design. Treatment started with the first injection and continued for 14 days. Foot swelling and right hind paw volume were measured once every two days; body weight was measured daily. After 14 days, all the animals were euthanized.

### 6.2. Grouping and administration

A total of 37 animals were selected by screening in this experiment, and the grouping is shown in Table 7:

**Table 7. Grouping and treatment of animals**

| Group | Dose (mg/kg) | Number | Animal | Route of administration | Time of administration |
|---|---|---|---|---|---|
| Control group | -- | 5 | AIA | PO | 14 days |
| Vehicle group (0.5% methylcellulose) | -- | 8 | AIA | PO | 14 days |
| Positive group (tofacitinib) | 10 | 12 | AIA | PO | 14 days |
| GEN1-117 | 10 | 12 | AIA | PO | 14 days |

### 6.3. Statistics

Data collection and statistical graphing were performed using Excel and GraphPad Prism software 5.0. Data are expressed as Mean ± S.E.M. One-way ANOVA Dennett's test and t-test were used. P < 0.05 indicates a statistically significant difference between groups.

### 7. Results

### Pharmacodynamic study of test drug in AIA rat model

In the treatment of rats of the AIA arthritis disease model with the GEN1-117 drug, the volume of the hind paws of the rats significantly decreased on days 17-23 after drug intervention compared to the control rats (Table 8).

**Table 8**

| | Hind paw volume data of the AIA rat model | | | | | | | | | | | |
|---|---|---|---|---|---|---|---|---|---|---|---|---|
| Days | Vehicle | | | | | | | | | | | |
| 9 | 1.12 | 1.31 | 1.62 | 1.49 | 1.27 | 1.25 | 1.26 | 1.08 | | | | |
| 11 | 1.06 | 1.03 | 1.27 | 1.09 | 1.27 | 1.08 | 1.55 | 1.19 | | | | |
| 13 | 1.25 | 1.46 | 1.6 | 1.76 | 1.52 | 1.83 | 2.25 | 1.21 | | | | |
| 15 | 1.36 | 1.04 | 1.79 | 2.32 | 1.7 | 2.11 | 2.14 | 1.49 | | | | |
| 17 | 1.46 | 1.95 | 2.15 | 2.03 | 1.53 | 2.35 | 2.77 | 1.91 | | | | |
| 19 | 2.51 | 2.25 | 3.04 | 2.38 | 2.61 | 3.35 | 2.93 | 1.8 | | | | |
| 21 | 1.98 | 2.32 | 3.05 | 2.99 | 2.01 | 3.1 | 3.5 | 2.06 | | | | |
| 23 | 1.91 | 2.33 | 3.19 | 3.5 | 2.15 | 2.52 | 3.2 | 2.48 | | | | |
| | | | | | | | | | | | | |

| Days | Tofacitinib 10 mg/kg | | | | | | | | | | | |
|---|---|---|---|---|---|---|---|---|---|---|---|---|
| 9 | 2.1 | 1.42 | 1.4 | 1.21 | 1.35 | 1.24 | 1.1 | 1.36 | | | | |
| 11 | 2 | 1.56 | 1.23 | 1.03 | 1.53 | 1.02 | 1.46 | 1.01 | | | | |
| 13 | 2.59 | 1.92 | 1.72 | 1.16 | 1.39 | 1.11 | 1.83 | 1.47 | | | | |
| 15 | 1.43 | 2.54 | 2.22 | 1.31 | 1.33 | 1.65 | 2.45 | 1.18 | | | | |
| 17 | 2.5 | 2.45 | 2.08 | 1.17 | 2.21 | 1.73 | 2.16 | 0.94 | | | | |
| 19 | 2.81 | 2.85 | 1.96 | 1.46 | 2.31 | 2.33 | 1.83 | 1.42 | | | | |
| 21 | 2.49 | 2.2 | 2.17 | 1.18 | 1.27 | 1.36 | 1.96 | 1.22 | | | | |
| 23 | 2.74 | 1.9 | 1.91 | 1.23 | 1.17 | 1.64 | 1.92 | 1.18 | | | | |
| | | | | | | | | | | | | |

| Days | GEN1-117 10 mg/kg | | | | | | | | | | | |
|---|---|---|---|---|---|---|---|---|---|---|---|---|
| 9 | 1.23 | 1.18 | 1.15 | 1.31 | 1.35 | 1.12 | 1.38 | 1.68 | 1.27 | 1.41 | 1.46 | 1.38 |
| 11 | 1.19 | 1.17 | 1.18 | 1.13 | 1.3 | 1.08 | 1.18 | 1.92 | 1.23 | 1.3 | 1.65 | 0.97 |
| 13 | 1.25 | 1.26 | 0.84 | 1.38 | 1.26 | 1.03 | 1.25 | 2.32 | 1.28 | 1.93 | 2.35 | 1.08 |
| 15 | 1.27 | 1.1 | 1.38 | 1.08 | 1.27 | 1.11 | 1.26 | 2.93 | 1.66 | 2.03 | 2.2 | 1.04 |
| 17 | 1.14 | 1.12 | 1.28 | 1.06 | 1.41 | 1.09 | 1.03 | 2.61 | 1.88 | 2.03 | 1.62 | 0.88 |
| 19 | 1.42 | 1.25 | 1.37 | 1.26 | 1.65 | 1.28 | 1.38 | 2.43 | 2.78 | 3.18 | 2.26 | 1.39 |
| 21 | 1 | 0.86 | 1.09 | 1.08 | 1.33 | 1.04 | 1.14 | 2.65 | 2.14 | 3.15 | 1.95 | 1.08 |
| 23 | 0.99 | 0.88 | 0.93 | 0.96 | 1.26 | 1.08 | 1.08 | 2.87 | 1.89 | 3.36 | 2.21 | 1.25 |

The body weight of the rats from the experimental group significantly decreased on day 9 compared to the rats from the control group. The rats from the GEN1-117 intervention group gained significant weight (Table 9).

**Table 9**

| | The effects of the drugs on body weight (g) | | | | | | | | | | | |
|---|---|---|---|---|---|---|---|---|---|---|---|---|
| Days | vehicle | | | | | | | | | | | |
| 9 | 155 | 165 | 171 | 169 | 165 | 161 | 159 | 138 | | | | |
| 10 | 154 | 164 | 171 | 164 | 166 | 158 | 156 | 135 | | | | |
| 11 | 150 | 164 | 164 | 161 | 162 | 154 | 157 | 134 | | | | |
| 12 | 147 | 160 | 155 | 156 | 157 | 151 | 151 | 130 | | | | |
| 13 | 148 | 156 | 151 | 153 | 153 | 150 | 148 | 131 | | | | |
| 14 | 146 | 152 | 153 | 152 | 151 | 149 | 152 | 129 | | | | |
| 15 | 146 | 150 | 154 | 149 | 152 | 147 | 151 | 131 | | | | |
| 16 | 148 | 154 | 150 | 148 | 150 | 148 | 149 | 129 | | | | |
| 17 | 150 | 152 | 151 | 148 | 152 | 146 | 148 | 129 | | | | |
| 18 | 154 | 155 | 153 | 149 | 152 | 146 | 150 | 128 | | | | |
| 19 | 156 | 152 | 154 | 151 | 154 | 151 | 153 | 130 | | | | |
| 20 | 156 | 144 | 148 | 140 | 155 | 148 | 140 | 127 | | | | |
| 21 | 155 | 152 | 143 | 149 | 152 | 148 | 151 | 130 | | | | |
| 22 | 153 | 152 | 148 | 146 | 153 | 150 | 151 | 133 | | | | |
| 23 | 158 | 152 | 146 | 147 | 158 | 147 | 153 | 132 | | | | |

| Days | Tofacitinib 10 mg/kg | | | | | | | | | | | |
|---|---|---|---|---|---|---|---|---|---|---|---|---|
| 9 | 167 | 156 | 146 | 168 | 151 | 154 | 161 | 167 | | | | |
| 10 | 165 | 153 | 142 | 168 | 150 | 152 | 160 | 166 | | | | |
| 11 | 162 | 154 | 140 | 166 | 145 | 151 | 158 | 167 | | | | |
| 12 | 155 | 149 | 136 | 159 | 145 | 149 | 155 | 168 | | | | |
| 13 | 154 | 148 | 136 | 157 | 140 | 167 | 154 | 150 | | | | |
| 14 | 151 | 144 | 135 | 154 | 140 | 147 | 153 | 164 | | | | |
| 15 | 150 | 145 | 134 | 152 | 139 | 146 | 151 | 162 | | | | |
| 16 | 153 | 142 | 136 | 152 | 141 | 145 | 147 | 160 | | | | |
| 17 | 156 | 147 | 138 | 151 | 142 | 146 | 146 | 163 | | | | |
| 18 | 155 | 148 | 141 | 151 | 143 | 148 | 148 | 164 | | | | |
| 19 | 160 | 151 | 144 | 159 | 145 | 153 | 154 | 166 | | | | |
| 20 | 159 | 150 | 136 | 160 | 145 | 152 | 153 | 165 | | | | |
| 21 | 161 | 150 | 148 | 162 | 149 | 155 | 154 | 166 | | | | |
| 22 | 165 | 150 | 150 | 163 | 149 | 158 | 158 | 169 | | | | |
| 23 | 165 | 156 | 150 | 160 | 151 | 159 | 158 | 173 | | | | |

| Days | GEN1-117 10 mg/kg | | | | | | | | | | | |
|---|---|---|---|---|---|---|---|---|---|---|---|---|
| 9 | 166 | 153 | 145 | 169 | 175 | 168 | 169 | 164 | 154 | 154 | 153 | 158 |
| 10 | 156 | 143 | 135 | 159 | 165 | 158 | 158 | 158 | 152 | 151 | 155 | 152 |
| 11 | 156 | 140 | 132 | 155 | 164 | 158 | 155 | 157 | 146 | 150 | 153 | 152 |
| 12 | 158 | 143 | 137 | 159 | 164 | 160 | 156 | 162 | 148 | 153 | 154 | 155 |
| 13 | 157 | 145 | 143 | 161 | 167 | 165 | 161 | 169 | 149 | 158 | 155 | 159 |
| 14 | 164 | 151 | 150 | 170 | 173 | 170 | 164 | 172 | 150 | 160 | 155 | 166 |
| 15 | 162 | 151 | 154 | 176 | 174 | 174 | 170 | 175 | 149 | 159 | 151 | 169 |
| 16 | 165 | 156 | 155 | 180 | 177 | 179 | 175 | 176 | 147 | 164 | 158 | 135 |
| 17 | 169 | 160 | 161 | 189 | 180 | 180 | 179 | 184 | 150 | 170 | 152 | 181 |
| 18 | 177 | 166 | 165 | 190 | 186 | 187 | 187 | 190 | 150 | 178 | 156 | 190 |
| 19 | 184 | 173 | 173 | 176 | 193 | 186 | 186 | 192 | 155 | 176 | 160 | 193 |
| 20 | 190 | 182 | 185 | 188 | 199 | 184 | 183 | 187 | 152 | 188 | 160 | 191 |
| 21 | 196 | 192 | 179 | 183 | 197 | 193 | 201 | 195 | 153 | 188 | 161 | 200 |
| 22 | 202 | 203 | 176 | 197 | 207 | 197 | 211 | 193 | 154 | 198 | 162 | 213 |
| 23 | 207 | 215 | 188 | 210 | 214 | 205 | 217 | 195 | 153 | 198 | 160 | 222 |

| Days | control | | | | | | | | | | | |
|---|---|---|---|---|---|---|---|---|---|---|---|---|
| 9 | 176 | 181 | 201 | 170 | 175 | | | | | | | |
| 10 | 175 | 203 | 174 | 170 | 182 | | | | | | | |
| 11 | 211 | 173 | 178 | 187 | 180 | | | | | | | |
| 12 | 189 | 182 | 176 | 214 | 186 | | | | | | | |
| 13 | 190 | 215 | 183 | 176 | 186 | | | | | | | |
| 14 | 215 | 187 | 185 | 193 | 178 | | | | | | | |
| 15 | 184 | 195 | 176 | 188 | 218 | | | | | | | |
| 16 | 184 | 172 | 193 | 178 | 223 | | | | | | | |
| 17 | 225 | 194 | 194 | 189 | 183 | | | | | | | |
| 18 | 227 | 185 | 193 | 191 | 192 | | | | | | | |
| 19 | 231 | 199 | 200 | 192 | 189 | | | | | | | |
| 20 | 230 | 197 | 186 | 197 | 192 | | | | | | | |
| 21 | 232 | 196 | 201 | 187 | 198 | | | | | | | |
| 22 | 196 | 191 | 197 | 236 | 204 | | | | | | | |

Compared to the control rats, the rats from the GEN1-117 group showed significant reductions in clinical scores on days 13-23, indicating that the GEN1-117 drug can relieve the AIA rat arthritis disease model and has similar efficacy to the positive drug tofacitinib (Table 10).

**Table 10**

| | The effects of the drugs on clinical scores | | | | | | | | | | | |
|---|---|---|---|---|---|---|---|---|---|---|---|---|
| Days | vehicle | | | | | | | | | | | |
| 9 | 0 | 0 | 0 | 0 | 0 | 0 | 0 | 0 | | | | |
| 11 | 0 | 0 | 0.5 | 0 | 0 | 0 | 0.5 | 0 | | | | |
| 13 | 1 | 0.5 | 5 | 3.83 | 3.17 | 0.83 | 2.5 | 1 | | | | |
| 15 | 1 | 1 | 7.7 | 4.8 | 4.2 | 2 | 3 | 1.3 | | | | |
| 17 | 1.5 | 2.5 | 8.2 | 7.3 | 4.5 | 4.3 | 5.5 | 3 | | | | |
| 19 | 2.3 | 2.3 | 10.3 | 7.8 | 4.3 | 5.5 | 7.5 | 3.5 | | | | |
| 21 | 2 | 4 | 9 | 8.5 | 5.5 | 6.5 | 8.5 | 3.5 | | | | |
| 23 | 1.5 | 2.5 | 9 | 9 | 3.5 | 6.5 | 6.5 | 2.5 | | | | |

| Days | Tofacitinib 10 mg/kg | | | | | | | | | | | |
|---|---|---|---|---|---|---|---|---|---|---|---|---|
| 9 | 1 | 0 | 0 | 0 | 0 | 0 | 0 | 0 | | | | |
| 11 | 1.5 | 0.5 | 0.5 | 0 | 0.5 | 0.25 | 0.5 | 0 | | | | |
| 13 | 3.3 | 1 | 1.5 | 1 | 1 | 0.5 | 1.2 | 0.7 | | | | |
| 15 | 3.5 | 3 | 2 | 1.5 | 2.5 | 0.8 | 2 | 0.7 | | | | |
| 17 | 3 | 2 | 1.8 | 1.5 | 2.3 | 1.3 | 2 | 0.3 | | | | |
| 19 | 2.8 | 2 | 1.3 | 0.3 | 1.5 | 1 | 1.3 | 0 | | | | |
| 21 | 2 | 1.5 | 1.3 | 0 | 1.3 | 1 | 1.5 | 0 | | | | |
| 23 | 3 | 1 | 6 | 0.5 | 1 | 1 | 1 | 0 | | | | |

| Days | GEN1-117 10 mg/kg | | | | | | | | | | | |
|---|---|---|---|---|---|---|---|---|---|---|---|---|
| 9 | 0 | 0 | 0 | 0 | 0 | 0 | 0 | 1 | 0 | 0.17 | 1 | 0 |
| 11 | 0 | 0 | 0 | 0 | 0 | 0 | 0 | 1.5 | 0.5 | 0.75 | 2.5 | 0 |
| 13 | 0 | 0 | 0 | 0.17 | 0 | 0 | 0 | 1.67 | 1 | 1 | 3 | 0 |
| 15 | 0 | 0 | 0.17 | 0 | 0.17 | 0.17 | 0.17 | 2 | 3.5 | 2 | 5.5 | 0 |
| 17 | 0 | 0 | 0 | 0 | 0 | 0 | 0 | 1.83 | 3.5 | 1.83 | 5 | 0 |
| 19 | 0 | 0 | 0.5 | 0 | 0 | 0 | 0 | 2 | 4.25 | 2.5 | 4 | 0 |
| 21 | 0 | 0 | 0 | 0 | 0.5 | 0 | 0 | 2 | 4.17 | 2.67 | 5.33 | 0 |
| 23 | 0 | 0 | 0 | 0 | 0.5 | 0 | 0 | 2.5 | 4 | 3 | 4 | 0 |

Compared to the scores of the control rats, the maximum arthritis index score of the rats from the GEN1-117 group shows that the GEN1-117 drug can effectively relieve the AIArat arthritis disease model and has similar efficacy to the positive drug tofacitinib (Table 11).

**Table 11**

| The arthritis index scores of rats | | |
|---|---|---|
| vehicle | Tofacitinib 10 mg/kg | GEN1-117 10 mg/kg |
| 9.3 | 20.1 | 0 |
| 12.8 | 11 | 0 |
| 49.7 | 14.4 | 0.67 |
| 41.23 | 4.8 | 0.17 |
| 25.17 | 10.1 | 1.17 |
| 25.63 | 5.85 | 0.17 |
| 34 | 9.5 | 0.17 |
| 14.8 | 1.7 | 14.5 |
| | | 20.92 |
| | | 14.08 |
| | | 30 |
| | | 0 |

### 8. Conclusion

In this study, the results show that daily intraperitoneal injection of the STATS inhibitor GEN1-117 dose-dependently inhibited the increase in hind paw volume and clinical score ofAIArats. Through this experiment, it can be concluded that GEN1-117 can significantly alleviate disease progression in the AIA model.

### Effect Example 5: Evaluation of Therapeutic Effect of GENl-284s on Mouse CIA Model

### 1. Objective

This experiment was intended to evaluate the *in vivo* efficacy of the compound GEN1-284s in the collagen-induced arthritis model.

### 2. Experimental materials and equipment

### 2.1. Experimental materials

Bovine type II collagen (CII): Sichuan University, catalog No. 20190618
Acetic acid: Sigma (St. Louis, MO, USA), catalog No. A8976
Complete Freund's adjuvant: Sigma, catalog No. F5881
Tofacitinib: Dalian Meilunbio Co. Ltd., catalog No. MB3358-1g-MLNE

### 2.2. Test drugs

### (1) Test compound

Name: GEN1-284s
Source: client
Lot number: E00144-18156-019P5
Appearance: white solid
Purity: 95.06%
Storage: at 4 °C
Vehicle: PBS
Preparation method: The compound was prepared once every 3 days, by directly dissolving it in PBS.

### (2) Control Compound

Name: tofacitinib
Source: Dalian Meilunbio Co. Ltd.
Storage: at -20 °C
Vehicle: 0.5% MC
Preparation method: The test sample was weighed out, 0.5% MC was added, and ultrasonication was performed for 20-30 min to help dissolution.

### 2.3. Experimental instruments

Anaesthetic machine: Raymain, RM-HSIV-u
High-speed homogenizer: IKA, T10 basic
Compound weighing scale: Sartorius, CPA225D
Animal weighing scale: electronic balance from Changzhou Tianzhiping, YH2000

### 2.4. Laboratory animals and housing conditions

| Animal species: | DBA/1 mice |
|---|---|
| Supplier: | Beijing Vital River Laboratory Animal Co., Ltd. |
| Certificate number: | 110011201107916352 |
| Sex and body weight: | male, 14-16 g |
| Housing place: | WuXi AppTec animal housing center |
| Length of acclimatization: | 7 days |
| Temperature: | 20∼26°C |
| Humidity: | 40∼70% |
| Lighting: | Fluorescent lamp, on (08:00-20:00)/off (20:00-08:00), 12 h/12 h |
| Housing density: | 5 mice/cage |
| Food: | given *ad libitum* access to food (radiation-sterilized feed, Jiangsu Medicience) |
| Water: | given *ad libitum access* to water (prepared using Molecular (ultra-)pure water system) |

The use of animals described in this experimental report was examined and approved by the Institutional Animal Care and Use Committee (IACUC) of WuXi AppTec.

### 3. Experimental method

### 3.1. Preparation of experimental drugs

Vehicle: PBS, stored at 4 °C for later use.

GEN1-284s: 5, 15 and 30 mg/mL solutions of the compound GEN1-284s were prepared in vehicle.

### 3.2. Arthritis model induction

### Preparation of acetic acid

2 N acetic acid was diluted to 100 mM, filtered through a 0.22 µm filter membrane and stored at 4 °C.

### Preparation of CII solution

Bovine type II collagen (CII) was dissolved in a 100 mM acetic acid solution, and the resulting solution was stored overnight at 4 °C. The concentration of collagen was 8 mg/mL.

### Preparation of emulsion

The CII solution that had been stored overnight was mixed with an equal volume of complete Freund's adjuvant, and the mixture was homogenized on ice using a high-speed homogenizer at 30,000 rpm for about 60 min until the solution turned into a stable emulsion.

### Arthritis induction

50 DBA/1 mice were all immunized. The day of the first immunization was recorded as day 0, and the subsequent days were numbered in sequence. DBA/1 mice were anesthetized with isoflurane, and 50 µL of the prepared collagen emulsion (containing 200 µg of CII) was injected subcutaneously into the tail (2-3 cm from the base of the tail). On day 21, an equal volume of collagen emulsion was injected into the tail in the same manner. Mice from the normal group were not immunized.

### 3.3. Administration

On day 28, when the mean clinical score reached about 0.5, the mice were grouped by weight and score according to the experimental protocol, such that the two criteria were substantially identical for each group. The grouping is shown in Table 12. The 50 mice were divided into 5 groups. Mice with large deviations in body weight and score were excluded, and finally, 8 mice remained in each group.

The dose administered to each group is shown in Table 12. The volume for gavage administration was 10 mL/kg, and the administration continued for a total of 14 days.

**Table 12. Grouping and dosage design**

| Group | Test drug | Number | Route of administration | Concentration | Dose | Frequency of administration |
|---|---|---|---|---|---|---|
| | | | | mg/mL | mg/kg | |
| G2 | Vehicle^{a} | 8 | p.o. | N/A | N/A | QD, 14 days |
| G3 | Tofacitinib^{b} | 8 | p.o. | 1 | 10 | BID, 14 days |
| G4 | GEN1-284s^{a} | 8 | p.o. | 5 | 50 | QD, 14 days |
| G5 | GEN1-284s^{a} | 8 | p.o. | 15 | 150 | QD, 14 days |
| G6 | GEN1-284s^{a} | 8 | p.o. | 50-30 | 500-300° | QD, 500, 5 days |
| | | | | | | 300, 9 days |
| a PBS | | | | | | |
| b 0.5% MC | | | | | | |
| c Explanation of dose adjustment | | | | | | |
| On day 28, grouping and administration began. | | | | | | |
| On day 29, 3 mice in the high-dose group died, and the dissection results showed that: the esophagi were intact, the digestive tracts of the animals melted, and there were formed stools in the colons. The greatest abnormality was in the lungs: the lobes of the lungs were all dark red. No obvious abnormality was found in other organs. | | | | | | |
| On day 32, 1 mouse in the high-dose group died, and the dissection results showed that: the major abnormality was in the lungs, and no obvious abnormality was found in other organs. | | | | | | |
| On day 33, the dose was adjusted to 300 mg/kg. | | | | | | |

### 3.4. Measurement of indicators for onset of arthritis

Clinical observation: DBA/1 mice were observed daily for basic health and changes in body weight from before immunization to day 21 after immunization (recorded once a week). After day 22, the mice were observed daily for health, morbidity and changes in body weight (recorded at least three times a week) until the end of the experiment.

Clinical scoring: After day 22, the mice were observed daily for morbidity. When the mice began to have the disease (the clinical symptoms of arthritis appeared), scoring was performed on a scale of 0-4 according to the severity of the disease (swelling with redness, deformed joints); the highest score was 4 for each limb and was 16 for each animal. The scoring criteria are shown in Table 13. Scoring was performed at least three times each week ^{[3]}.

**Table 13. Clinical scoring criteria for arthritis**

| Score | Clinical symptom |
|---|---|
| 0 | No erythema and swelling with redness |
| 1 | Erythema or mild swelling with redness near the tarsal bones or at the ankle or metatarsus, or erythema and swelling with redness at one toe |
| 2 | Mild erythema and swelling at the ankle and metatarsus, swelling with redness and erythema at two or more toes |
| 3 | Moderate erythema and swelling at the ankle, wrist and metatarsus |
| 4 | Severe swelling with redness at all of the ankle, wrist, metatarsus and toes |

### 3.5. Pathological analysis

At the end of the experiment, the mice were euthanized. The right hind limbs of the mice were taken, soaked in 10% formalin solution, decalcified with formic acid solution, embedded in paraffin, sectioned, H&E stained, and observed under a microscope. The degree of damage to the joints was evaluated in terms of inflammatory cell infiltration, pannus formation, cartilage damage and bone resorption, and the damage was scored on a scale of 0-4. The scoring criteria for each aspect are shown in Table 14.

**Table 14. Pathological scoring criteria for arthritis**

| Lesion | Characteristics of lesion | Score |
|---|---|---|
| Inflammatory cell infiltration | No visible inflammatory cells | 0 |
| | Subsynovial cell fibrosis, and minimal cell infiltration | 1 |
| | Synovial cell proliferation, and slight monocyte infiltration | 2 |
| | Synovial cell proliferation, and massive monocyte, plasma cell and lymphocyte infiltration | 3 |
| | Massive inflammatory cell infiltration around joints, tissue fibrosis, and synovial membrane thickening | 4 |
| Pannus formation | No visible pannus formation | 0 |
| | Minimal pannus formation at the cartilage margin | 1 |
| | Fibrous hyperplasia between cartilages, and slight pannus formation at the articular margin | 2 |
| | Pannus formation on 50% of the surface of articular cartilage | 3 |
| | Pannus formation visible on the whole surface of articular cartilage | 4 |
| Cartilage damage | No visible cartilage damage | 0 |
| | Articular chondrocyte proliferation | 1 |
| | Loss of chondrocyte matrix, and small amounts of damaged chondrocytes | 2 |
| | Fibrous hyperplasia around joints, and large amounts of damaged chondrocytes | 3 |
| | Massive fibrous hyperplasia between articular cartilages, and cartilage erosion | 4 |
| Bone resorption | No visible bone resorption | 0 |
| | Minimal bone resorption visible at the synovial margin | 1 |
| | Slight osteoclast formation visible in small areas of bone tissue | 2 |
| | Local subchondral bone tissue with bone resorption | 3 |
| | Bone resorption in large areas of bone tissue, accompanied by cartilage erosion | 4 |

### 4. Statistical processing

Experimental data were expressed using Mean ± SEM, clinical score and body weight were subjected to two way ANOVA, and AUC and pathology score were subjected to one way ANOVA; p < 0.05 was considered a significant difference.

### 5. Experimental results and discussion

### 5.1. Clinical score

This experiment evaluated the improving effect of the compound GEN1-284s in the mouse arthritis (CIA) model on clinical scores. A mouse CIA model was constructed by subcutaneously injecting CII collagen emulsion into the tails of the mice on day 0 and day 21 of the experiment, and the mice began to show the clinical symptoms of arthritis on day 25 after the first immunization (day 4 after the second immunization). On day 28, grouping and administration began. The mean clinical score of the vehicle control group gradually increased and reached 7.5 on day 41, indicating successful construction of the collagen-induced arthritis model. The positive control tofacitinib treatment group began to significantly inhibit the score of arthritis on day 34. The compound GEN1-284s at doses of 50 mg/kg and 150 mg/kg began to consistently and significantly inhibit the score of arthritis on day 32 and day 34, respectively. By the end of the experiment, the clinical score of the vehicle group was 7.5, while the clinical scores of the GEN1-284s low- and medium-dose groups were as low as 3.1 (p < 0.0001) and 4.4 (p < 0.001), respectively.

**Table 15. Statistical analysis of the clinical score data of the collagen-induced arthritis mice**

| Day | G1 vehicle Control group | G2 Tofacitinib 10mg/kg | G3 GEN1-284s 50mg/kg | G4 GEN1-284s 150mg/kg | G5 GEN1-284s 300mg/kg |
|---|---|---|---|---|---|
| 21 | 0.0±0.00 | 0.0±0.00 | 0.0±0.00 | 0.0±0.00 | 0.0±0.00 |
| 25 | 0.0±0.00 | 0.3±0.16 | 0.3±0.16 | 0.0±0.00 | 0.0±0.00 |
| 28 | 0.6±0.26 | 0.8±0.31 | 0.6±0.26 | 0.6±0.26 | 0.6±0.26 |
| 29 | 1.3±0.45 | 1.0±0.42 | 0.8±0.25 | 0.9±0.40 | 0.6±0.24 |
| 32 | 3.5±0.57 | 1.8±0.49 | 1.3±0.31* | 2.0±0.42 | 2.0±0.58 |
| 34 | 4.6±0.71 | 2.5±0.65* | 1.8±0.56*** | 2.5±0.57* | 2.5±0.87 |
| 36 | 5.9±0.67 | 2.8±0.75*** | 2.4±0.84**** | 3.4±0.78** | 4.0±1.15 |
| 39 | 7.0±0.42 | 3.4±0.80**** | 2.9±0.90**** | 3.9±0.93*** | 5.5±1.19 |
| 41 | 7.5±0.42 | 3.5±0.87**** | 3.1±0.90**** | 4.4±0.92*** | 6.3±1.38 |
| Mean ± SEM, *p < 0.05, **p < 0.01, ***p< 0.001, ****p < 0.0001 vs. vehicle control group, two-way ANOVA | | | | | |

The area under curve (AUC) was calculated by analyzing the clinical score curve of each animal in each group, and the inhibition rate of each dosing group relative to the vehicle control group was calculated on the basis of the mean AUC of each group. The GEN1-284s 50 mg/kg dosing group significantly inhibited the score of arthritis, and the inhibition rate reached 56.9%, which is superior to the 47.0% inhibition rate of the tofacitinib group.

### 5.2. Body weight

The mean body weight of each of the dosing groups showed no significant difference after onset compared to the vehicle group (Table 16).

**Table 16. Statistical analysis of body weight data**

| Day | G1 vehicle control group | G2 Tofacitinib 10mg/kg | G3 GEN1-284s 50mg/kg | G4 GEN1-284s 150mg/kg | G5 GEN1-284s 300mg/kg |
|---|---|---|---|---|---|
| 21 | 20.63±0.60 | 20.39±0.43 | 20.64±0.23 | 20.40±0.23 | 20.18±0.29 |
| 25 | 20.71±0.66 | 20.54±0.45 | 20.73±0.24 | 20.53±0.27 | 20.43±0.27 |
| 28 | 20.64±0.73 | 20.63±0.49 | 20.65±0.25 | 20.64±0.24 | 20.65±0.34 |
| 29 | 20.28±0.62 | 20.2±0.44 | 20.49±0.25 | 20.48±0.29 | 20.08±0.49 |
| 32 | 19.39±0.54 | 19.91±0.50 | 19.66±0.28 | 20.06±0.25 | 19.48±1.02 |
| 34 | 19.95±0.54 | 20.39±0.44 | 20.41±0.31 | 20.41±0.41 | 19.30±1.05 |
| 36 | 19.75±0.46 | 20.26±0.43 | 20.54±0.36 | 20.70±0.35 | 19.60±0.81 |
| 39 | 19.71±0.52 | 20.31±0.34 | 20.48±0.27 | 20.40±0.29 | 19.80±0.96 |
| 41 | 20.94±0.36 | 21.04±0.44 | 20.95±0.20 | 20.91±0.26 | 20.30±0.78 |
| Mean ± SEM, ns (no significance) vs. vehicle control group, two-way ANOVA | | | | | |

### 5.3. Pathological results

The infiltration of monocytes, plasma cells and lymphocytes in joints, pannus hyperplasia on the surface of cartilage and damaged cartilage could be observed in the mice from the vehicle control group via H&E staining. The positive control tofacitinib treatment group showed no significant lesions in joint tissues. The total score of the GEN1-284s 50 mg/kg dosing group was lower than that of the vehicle control group.

### 6. Experimental conclusion

The construction of the mouse CIA model was successful in that severe clinical symptoms of arthritis were induced by collagen in the mice from the vehicle control group, and the later histopathological analysis of joints also showed cell infiltration and damage to bone tissue; however, the positive control group tofacitinib had a significant improving effect on the symptoms of arthritis and lesions in tissues.

The compound GEN1-284s at doses of 50 mg/kg and 150 mg/kg began to consistently and significantly inhibit the score of arthritis on day 32 and day 34, respectively. The GEN1-284s 50 mg/kg dosing group's score curve AUC inhibition rate reached 56.9%, which is superior to the 47.0% inhibition rate of the tofacitinib group. In the case of administering the compound GEN1-284s at doses of 50 mg/kg, 150 mg/kg and 300 mg/kg, the mice showed good tolerance and did not significantly lose weight by the end of the experiment. In the case of administering the compound GEN1-284s at a dose of 500 mg/kg, death was caused among the mice.

From the histomorphological viewpoint, damage was induced by collagen to the joints of the mice from the vehicle control group. The 50 mg/kg dosing group of the test compound GEN1-284s had a tendency to relieve arthritis in animals.

### Testing was repeated for dose exploration

The above method was used.

The doses were adjusted to GEN1-284s (10, 30, 50 mg/kg, qd, po).

### Conclusion

The construction of the mouse CIA model was successful in that severe clinical symptoms of arthritis were induced by collagen in the mice from the vehicle control group, and the later histopathological analysis of joints also showed cell infiltration and damage to bone tissue; however, the positive control group tofacitinib had a significant improving effect on the symptoms of arthritis and lesions in tissues.

The compound GEN1-284s at doses of 10, 30 and 50 mg/k began to consistently and significantly inhibit the score of arthritis on day 32 and day 34, respectively, and showed no significant difference from the tofacitinib positive group.

In the case of administering the compound GEN1-284s at doses of 10, 30 and 50 mg/k, the mice showed good tolerance and did not significantly lose weight by the end of the experiment.

**Table 17. Clinical scores**

| Day | G1 solvent control group | G2 Tofacitinib 10mg/kg | G3 GEN1-284s 10mg/kg | G4 GEN1-284s 30mg/kg | G5 GEN1-284s 50mg/kg |
|---|---|---|---|---|---|
| | Mean ± SEM | Mean ± SEM | Mean ± SEM | Mean ± SEM | Mean ± SEM |
| 21 | 0.0±0.00 | 0.0±0.00 | 0.0±0.00 | 0.0±0.00 | 0.0±0.00 |
| 24 | 0.0±0.00 | 0.0±0.00 | 0.0±0.00 | 0.0±0.00 | 0.0±0.00 |
| 26 | 0.3±0.16 | 0.3±0.16 | 0.3±0.16 | 0.3±0.16 | 0.4±0.18 |
| 28 | 0.6±0.18 | 0.6±0.18 | 0.6±0.18 | 0.6±0.18 | 0.6±0.18 |
| 31 | 3.3±0.41 | 1.8±0.37 | 2.5±0.57 | 2.1±0.69 | 1.9±0.67 |
| 33 | 5.9±0.72 | 3.1±0.52 | 4.1±0.99 | 3.6±1.13 | 3.4±1.15 |
| 35 | 8.1±1.09 | 3.6±0.56 ** | 5.6±1.29 | 5.1±1.25 | 4.8±1.36 * |
| 38 | 10.0±1.21 | 4.6±0.75 *** | 6.9±1.55 | 6.0±1.40 ** | 5.9±1.54 ** |
| 40 | 10.6±1.27 | 5.1±0.90 **** | 7.5±1.69 | 6.9±1.53 * | 6.6±1.60 ** |
| 42 | 11.6±1.07 | 5.5±0.87 **** | 7.9±1.79 * | 8.1±1.26 * | 6.9±1.55 ** |
| *p < 0.05, **p < 0.01, ***p < 0.001, ****p < 0.0001 vs. solvent control group, two-way ANOVA | | | | | |

**Table 18**

| | Day35 | Day38 | Day40 | Day42 |
|---|---|---|---|---|
| G1 Vehicle vs. G2 Tofacitinib BID | ** | *** | **** | **** |
| G1 Vehicle vs. G3 GEN1-284s 10mpk | ns | ns | ns | * |
| G1 Vehicle vs. G4 GEN1-284s 30mpk | ns | ** | * | * |
| G1 Vehicle vs. G5 GEN1-284s 50mpk | * | ** | ** | ** |

| | | | | |
|---|---|---|---|---|
| ^{∗}P < 0.05, ^{∗∗}P < 0.01, ^{∗∗∗}P < 0.001, ^{∗∗∗∗}P < 0.0001. Comparing to Vehicle group, Two-way ANOVA. | | | | |

**Table 19. Changes in bodv weight**

| Day | G1 solvent control group | G2 Tofacitinib 10mg/kg | G3 GEN1-284s 10mg/kg | G4 GEN1-284s 30mg/kg | G5 GEN1-284s 50mg/kg |
|---|---|---|---|---|---|
| | Mean ± SEM | Mean ± SEM | Mean ± SEM | Mean ± SEM | Mean ± SEM |
| 21 | 20.81±0.22 | 20.41±0.27 | 20.64±0.17 | 20.25±0.34 | 20.44±0.30 |
| 24 | 20.18±0.35 | 19.69±0.37 | 20.40±0.28 | 19.74±0.33 | 19.66±0.23 |
| 26 | 20.06±0.23 | 19.59±0.36 | 20.19±0.41 | 19.79±0.34 | 19.70±0.26 |
| 28 | 19.33±0.44 | 19.25±0.50 | 19.34±0.52 | 19.48±0.54 | 19.60±0.43 |
| 31 | 18.40±0.57 | 18.98±0.53 | 18.89±0.44 | 19.20±0.56 | 18.88±0.52 |
| 33 | 18.44±0.52 | 19.14±0.43 | 18.65±0.48 | 19.50±0.61 | 18.91±0.59 |
| 35 | 18.49±0.58 | 19.03±0.46 | 19.04±0.47 | 19.68±0.61 | 19.09±0.53 |
| 38 | 18.76±0.58 | 18.98±0.45 | 19.54±0.38 | 19.46±0.56 | 19.10±0.52 |
| 40 | 19.14±0.44 | 19.56±0.32 | 19.85±0.28 | 20.06±0.53 | 19.31±0.43 |
| 42 | 19.30±0.45 | 19.69±0.40 | 19.90±0.31 | 19.78±0.44 | 19.39±0.39 |
| ns (no significance) vs. solvent control group, two-way ANOVA | | | | | |

### Effect Example 6: Preliminary Safety Evaluation of GENl-284s by Single- and Multiple-Administration

### Acute Toxicity Testing in Mice

### Abstract

**Objective:** to evaluate the toxic dose of the compound GEN1-284s in female BALB/c mice.

### Materials and method:

Part I: Single administration. A total of 6 mice were used. They were randomly divided into 2 groups: the vehicle control and GEN1-284s (1000 mg/kg), dosed once by oral gavage and observed for 6 days, and changes in body weight and activities were recorded.

Part II: Multiple administrations. This experiment used a total of 6 mice. They were divided into two groups of 3: a GEN1-284s-1000 mg/kg group and a GEN1-284s-300 mg/kg group, and were intragastrically given GEN1-284s for 14 consecutive days at 200 µL each. The state and survival of the mice were observed every day, and their body weights were recorded. Two mice died 2 h after GEN1-284s-1000 mg/kg administration.

The dose was changed to 600 mg/kg, and the experiment was continued.

### Results:

**Part I: The single administration of 1000 mg/kg GEN1-284s did not affect the body weight or activity of the BALB/c mice, and the results could not be distinguished from those of the vehicle control group, indicating that this dose can be well tolerated by mice.**

Part II: The multiple-administration results show that GEN1-284s-1000 mg/kg is highly toxic to female BALB/c mice: death was caused among the mice 2 h after administration; the continuous administration of GEN1-284s-600 mg/kg resulted in great toxicity to the mice: all died within 5 days; the continuous administration of GEN1-284s-300 mg/kg did not affect the survival of the mice and caused no significant change in body weight.

**Conclusion:** The continuous administration of GEN1-284s-300 mg/kg did not affect the survival of the mice and caused no significant change in body weight. Therefore, this dose has low toxicity to female BALB/c mice and will not affect survival after 14 consecutive days of administration.

### 1. Objective

This experiment was intended to evaluate the toxic dose of the compound GEN1-284s in BALB/c mice.

### 2. Experimental standards

This project was implemented in GenEros Biopharma (Hangzhou) Ltd. All the animal experiments involved in the implementation complied with the standard procedures for the use of laboratory animals and the guide for the care and use of laboratory animals provided by IACUC of GenEros, and the animal welfare requirements of the Office of Laboratory Animal Welfare. This experiment is a non-GLP experiment.

### 3. Experimental materials

### 3.1. Instruments

Analytical balance, model: SQP, manufacturer: Beijing Sartorius Scientific Instruments Co., Ltd.;

### 3.2. Test drug and reagent

### Test compound:

GEN1-284s, storage condition: -20 °C (high-concentration stock solution, powder); working solutions can be freshly prepared and temporarily stored at 2-8 °C for 1 day.

### Reagent:

1×PBS, manufacturer: Hyclone, Lot: AE29449011, storage condition: 4 °C.

### 4. Laboratory animals

### 4.1. Use of animals

12 female BALB/c mice, 6 w, purchased from Beijing Vital River Laboratory Animal Co., Ltd.

### 4.2. Housing of animals

The animals used in the following experiments were all BALB/c mice. After arrival at the facility of GenEros Biopharma Ltd., they were housed in an animal room under strictly controlled environmental conditions. The temperature of the room was maintained at 20-24 °C and the humidity was maintained at 30-70%. The lighting of the animal room was controlled by an electronic timer switch system: on for 12 h and off for 12 h every day (switched on at 6:00 AM and off at 18:00 PM). The animals were given *ad libitum* access to food and water.

### 5. Experimental method

### 5.1. Experimental design

### Part I:

This experiment used a total of 6 mice. They were randomly divided into two groups of 3: a vehicle group and a GEN1-284s-1000 mg/kg group, and were intragastrically dosed once on day 0 at 200 µL each. The state and survival of the mice were observed every day, and their body weights were recorded.

### Part II:

This experiment used a total of 6 mice. They were divided into two groups of 3: a GEN1-284s-1000 mg/kg group and a GEN1-284s-300 mg/kg group, and were intragastrically given GEN1-284s for 14 consecutive days at 200 µL each. The state and survival of the mice were observed every day, and their body weights were recorded. Two mice died 2 h after GEN1-284s-1000 mg/kg administration. The dose was changed to 600 mg/kg, and the experiment was continued.

### 5.2. Grouping and administration

### Part I

| Group | Number of animals | Route of administration | Number of doses |
|---|---|---|---|
| Vehicle control | 3 | P.O | 1, day 0 |
| GEN1-284s-1000mg/kg | 3 | P.O | 1, day 0 |

### Part II

| Group | Number of animals | Route of administration | Time of administration |
|---|---|---|---|
| GEN1-284s-300mg/kg. | 3 | P.O | 1-14 days |
| GEN1-284s-1000 mg/kg, changed to GEN1-284s-600 mg/kg on day 2 | 3 | P.O | 1-14 days |

Each mouse was intragastrically administered GEN1-284s for 14 consecutive days in a volume of 200 µL/20 g. The state and survival of the mice were observed every day, and their body weights were recorded. Two mice died 2 h after GEN1-284s-1000 mg/kg administration. The dose was changed to 600 mg/kg, and the experiment was continued.

### 5.3. Indicators

Changes in body weight and survival of the mice were observed during 6 days after the single administration, and changes in body weight and the survival of the mice were observed during 14 consecutive days of high-dose GEN1-284s administration.

### 5.4. Statistics

Data collection and statistical graphing were performed using Excel and GraphPad Prism software 5.0. Data are expressed as Mean ± S.E.M.

### 6. Results

### 6.1. Mouse survival observation

### Part I:

After administration, no abnormality was seen in the survival of the mice.

### Part II:

### States of the mice:

Day 1: the mice from the GEN1-284s-300 mg/kg group were normal, and 2 mice from the GEN1-284s-1000 mg/kg group died 2 h after administration.

Day 2: the mice from the GEN1-284s-300 mg/kg group were normal, the GEN1-284s-1000 mg/kg was changed to GEN1-284s-600 mg/kg, and administration was continued; the mice were in acceptable states.

Day 3: the mice from the GEN1-284s-300 mg/kg group were normal, and those from the GEN1-284s-600 mg/kg group were in bad states.

Day 4: the mice from the GEN1-284s-300 mg/kg group were normal, and 1 mouse from the GEN1-284s-600 mg/kg group died: all died by this time.

Days 5-14: the mice from the GEN1-284s-300 mg/kg group were normal and showed no significant change in body weight.

### 6.2. Survival of mice and changes in body weight

### Part I:

**Table 20. Changes in body weight after the single administration of GEN1-284s to mice in the acute toxicity test**

| | Control group | | | GEN1-284s 1000mg/kg | | |
|---|---|---|---|---|---|---|
| Day | F1 | F2 | F3 | F4 | F5 | F6 |
| 0 | 21.66 | 19.84 | 21.27 | 23.5 | 21.2 | 21.1 |
| 1 | 22.3 | 20.7 | 22.1 | 23.8 | 21.5 | 22 |
| 2 | 22.7 | 20.4 | 21.7 | 23.5 | 21.9 | 21.7 |
| 3 | 22.4 | 20.2 | 22.2 | 24.1 | 21.8 | 20.3 |
| 4 | 22.5 | 20.2 | 22.3 | 24.3 | 21.8 | 22.7 |
| 5 | 22 | 21 | 22.9 | 23.8 | 21.6 | 23.4 |
| 6 | 22 | 20.4 | 22.1 | 23.4 | 21.6 | 22.8 |

### Part II:

From the survival results, the GEN1-284s 600 mg/kg group showed higher toxicity: all of the mice died within 5 days, while the continuous administration of GEN1-284s 300 mg/kg did not affect the survival of the mice. From the body weight results in Table 21, it can be seen that the continuous administration of GEN1-284s did not significantly affect the body weight of the mice.

**Table 21**

| Changes in body weight of the mice in the toxicity test | | | | |
|---|---|---|---|---|
| Days | 284s-300mg/kg | | 284s-600mg/kg | |
| 1 | 16.966667 | 0.6658328 | 17.366667 | 0.9291573 |
| 2 | 16.933333 | 0.5507571 | 16.9 | |
| 3 | 16.866667 | 0.4932883 | 17.3 | |
| 4 | 17.166667 | 0.3511885 | 17.3 | |
| 5 | 17.166667 | 0.6658328 | | |
| 6 | 17.233333 | 0.6658328 | | |
| 7 | | | | |
| 8 | 17.466667 | 0.1527525 | | |
| 9 | 17.166667 | 0.4163332 | | |
| 10 | 17.1 | 0.5567764 | | |
| 11 | 17.3 | 0.3605551 | | |
| 12 | 17.233333 | 0.5033223 | | |
| 13 | 17.466667 | 0.6806859 | | |
| 14 | 17.466667 | 0.5859465 | | |

### 7. Conclusion

### In conclusion:

The single administration of GEN1-284s 1000 mg/kg did not affect the survival and body weight of the animals.

In the case of multiple administrations, GEN1-284s-1000 mg/kg is highly toxic to female BALB/c mice: death was caused among the mice 2 h after administration; the continuous administration of GEN1-284s-600 mg/kg resulted in great toxicity to the mice: all died within 5 days; the continuous administration of GEN1-284s-300 mg/kg did not affect the survival of the mice and caused no significant change in body weight, and therefore, this dose has low toxicity to female BALB/c mice and will not affect survival after 14 consecutive days of administration.

### Effect Example 7: Evaluation of GENl-284s in Atherosclerosis Model

### Abstract

Atherosclerosis is a chronic inflammatory disease of arteries, and because its pathogenesis is not clear, it is particularly important to successfully construct an AS disease model for pathological and pharmacological research. The atherosclerosis model caused by an apolipoprotein E gene knockout mouse is due to genetic damage, and the characteristics of lesions in the model are closer to those in humans. Apoe KO mice can spontaneously show symptoms of dyslipidemia after eating a high-fat diet and can produce intimal plaques close to those in humans.

**Objective:** This experiment used an Apoe gene knockout mouse atherosclerosis model induced by a high-fat diet to evaluate the effect of the compound GEN1-284s on blood lipids and arterial plaque pathological changes in mice of the atherosclerosis model.

**Method:** Apoe KO mice were tested for basal blood lipid levels and divided into a normal control group and a high-fat group. After 8 weeks of housing, the mice from the high-fat group were divided into 4 groups of about 8: a normal diet group (Chow), a high-fat diet model group (HFD), an Atorvastatin positive group (HFD+atorvastatin 5 mpk, QD), and a GEN1-284s group (284-60 mpk, QD). Oral administration was performed for 8 consecutive weeks, and the effect of the compound on the following indicators in Apoe KO mice was observed: 4 blood lipid tests (TC, TG, HDL-C and LDL-C), aortic plaque En-face staining, and pathological oil red staining of the cardiac outflow tract.

**Results:** The results show that after an 8 weeks' high-fat diet, the mice were orally administered the test compound until week 16. Compared to the normal group on a normal diet, a high-fat diet will lead to dyslipidemia in mice (there are upward trends in TCHO and LDL-C and a downward trend in HDL-C), and for arterial plaque accumulation and lesions in the cardiac outflow tract, treatment with the compound GEN1-284s can effectively reduce the formation of arterial plaques, partially relieve dyslipidemia and reduce arterial plaque accumulation, and has a significant improving effect on cardiac outflow tract plaques.

**Conclusion:** In conclusion: GEN1-284s can partially lower blood lipid levels, reduce the formation of arterial plaques and left ventricle outflow tract plaques, and help lesions in the cardiac outflow tract, and the effect is similar to that of the positive drug atorvastatin.

### 1. Objective

This experiment used an Apoe gene knockout mouse model of atherosclerosis induced by a high-fat diet to evaluate the effect of different doses of GEN1-284s on blood lipids, arterial plaques and lesions in the cardiac outflow tract in mice of the atherosclerosis model.

### 2. Experimental standards

This project was implemented in GenEros Biopharma (Hangzhou) Ltd. All the animal experiments involved in the implementation complied with the standard procedures for the use of laboratory animals and the guide for the care and use of laboratory animals provided by IACUC of GenEros, and the animal welfare requirements of the Office of Laboratory Animal Welfare. This experiment is a non-GLP experiment.

### 3. Experimental materials

### 3.1. Instruments

Analytical balance, model: SQP, manufacturer: Beijing Sartorius Scientific Instruments Co., Ltd.; Electronic balance, model: MP5002, manufacturer: Changzhou Tianzhiping Instruments and Equipment Co., Ltd.;
Constant-temperature magnetic stirrer, model: 85-2, manufacturer: Shanghai Sile Instruments Co., Ltd. Mini vortex mixer, model: XW-80A, manufacturer: Shanghai Huxi Analysis Instrument Factory Co., Ltd. High-frequency numerical control ultrasonic cleaner, model: KQ-50TDB, manufacturer: Kunshan Ultrasonic Instruments Co., Ltd.

Centrifuge, model: 5424 R, manufacturer: Eppendorf.

Stereo microscope: model: MZ6, manufacturer: Leica.

Hitachi 7180 biochemistry automatic analyzer, model: 7180, manufacturer: Japan.

Digital camera, model: EOS800D, manufacturer: Canon.

Cryomicrotome, model: Leica CM1950, manufacturer: Germany.

Olympus microscope, model: Olympus BX43, manufacturer: Japan.

### 3.2. Test drugs and reagents

**Test compound:** GEN1-284s, storage condition: -20 °C (high-concentration stock solution, powder); working solutions can be freshly prepared and temporarily stored at 2-8 °C for 1 day.

Atorvastatin, manufacturer: Sigma, Lot#: LRAA9204, storage condition: 4 °C.

**Reagents:** DMSO, storage condition: room temperature.

PEG300, manufacturer: Shanghai Yuanye Biotech Co., Ltd., storage condition: room temperature.

0.9% sodium chloride injection: Cisen Pharmaceutical Co., Ltd.

4% paraformaldehyde, manufacturer: Beyotime, storage condition: -20 °C.

Isopropanol, manufacturer: Genetimes, storage condition: room temperature.

Oil red O solution, manufacturer: Sigma, storage condition: 4 °C.

Total cholesterol test kit, manufacturer: Applygen Biopharmaceutical, storage condition: 4 °C.

Triglyceride test kit, manufacturer: Applygen Biopharmaceutical, storage condition: 4 °C.

HDL-C test kit, manufacturer: Applygen Biopharmaceutical, storage condition: 4 °C.

LDL-C test kit, manufacturer: Applygen Biopharmaceutical, storage condition: 4 °C.

### 4. Vehicle and compound preparation

Positive drug: atorvastatin calcium: prepared as a suspension in normal saline and well mixed before administration; volume for administration: 10 mL/kg

### 5. Laboratory animals

### 5.1. Use of animals

56 C57BL/6 Apoe KO mice, male, 7-8 weeks old, purchased from Vital River Laboratory Animal Technology Co., Ltd. (Beijing, China).

### 5.2. Housing of animals

The animals used in the following experiments were all C57BL/6 mice. After arrival at the facility of GenEros Biopharma Ltd., they were housed in an animal room under strictly controlled environmental conditions. The temperature of the room was maintained at 20-24 °C and the humidity was maintained at 30-70%. The temperature and humidity of the animal room were monitored in real time using a hygrothermograph and recorded twice daily (once in the morning and once in the afternoon). The lighting of the animal room was controlled by an electronic timer switch system: on for 12 h and off for 12 h every day (switched on at 6:00 AM and off at 18:00 PM). The animals were given *ad libitum* access to food and water. The normal control group was put on a normal diet, and all the other groups were put on a high-fat diet (D 12079Bi, Research Diets Inc., NJ, US).

### 6. Experimental method

### 6.1. Experimental design

Mice from the groups except for the normal diet control group (Chow) were fed with high-fat feed every day for 16 consecutive days. From week 9, each group was orally administered solvent or a corresponding test compound, and body weight data were recorded every day for 8 consecutive weeks. After the first 8 weeks' high-fat diet, blood samples were taken and centrifuged to separate serum, and the serum was tested for blood lipid levels (TCHO, TG, LDL-C and HDL-C). The mice were randomly grouped according to the TCHO results and body weight. During the subsequent 8 weeks of administration, blood lipid tests (TCHO, TG, LDL-C and HDL-C) were run once every 4 weeks.

During the experiment, the living states of the animals were observed, and abnormal states were recorded. After the experiment ended, endpoint treatment and sample collection were performed.

### 6.2. Grouping and administration

A total of 56 animals were selected by screening in this experiment, and the animals other than those in the normal control group were divided into 7 groups of 8. The grouping is shown in Table 22:

**Table 22. Grouping and treatment of animals**

| Group | Type | Dose (mg/kg) | Number | Animal | Diet | Route of administration | Time of administration |
|---|---|---|---|---|---|---|---|
| 1 | Normal control | -- | n=8 | Apoe-/- | Chow diet | PO | 8W |
| | | | | | | QD | |
| 2 | Model control | -- | n=8 | Apoe-/- | High fat diet | PO | 8W |
| | | | | | | QD | |
| 3 | Atorvastatin ATV | 5mpk | n=8 | Apoe-/- | High fat diet | PO | 8W |
| | | | | | | QD | |
| 4 | 284-60mpk | High dose 60 mpk | n=8 | Apoe-/- | High fat diet | PO | 8W |
| | | | | | | QD | |

### 6.3. Endpoint indicators

### 6.3.1. Blood lipid tests:

Mice were fasted overnight, and blood (150 µL) was collected via mandibular blood sampling or eye ball removal (for endpoint only) and left at room temperature for 2 h. The blood was centrifuged at 4 °C at 7000 rpm for 10 min, and serum was pipetted, quick-frozen in dry ice and then stored at -80 °C before analysis. The serum was tested for the biochemical indicators TCHO, TG, LDL-C and HDL-C using kits and a microplate reader.

### 6.3.2. Sample taking and testing:

### 6.3.2.1. Aortic plaque En-face staining and plaque area analysis:

At the end of the experiment, the mice were euthanized by CO2 inhalation, and their hearts and aortas (from the aortic arch to the iliac artery) were isolated. The isolated artery was dissected longitudinally from the aortic arch to the iliac artery, fixed in 4% paraformaldehyde for 30 min, synchronized with 60% isopropanol for 10 min, then stained in 60% oil red for 30 min, and then washed with 60% isopropanol 3 times for 5 min each time. Finally, the stained aorta was washed with deionized water and photographed. The proportion of red plaque areas was measured using Image Pro Plus 6.0 (Media Cybernetics, MD, US).

### 6.3.2.2. HE staining for pathological examination (pathological sections) of atheromatous plaque areas of animals:

Mouse heart samples were fixed overnight in 4% paraformaldehyde solution and dehydrated in sucrose solution. Then, the samples were embedded with OCT (Sakura, Japan) under a stereo microscope and made into 10 µm frozen sections. The sections were stained with oil red ORO and then subjected to routine pathological examinations. Images of the outflow tract were acquired under an optical microscope at 4× magnification, and high-quality morphological observations were carried out using an eyepiece with 20× magnification. The percentage of intimal lesions in the cardiac outflow tract was calculated. The detection criteria followed the criteria for histological classification of human atherosclerotic disease, which are described in detail below:

**Table 23. Histological classification of atherosclerotic disease**

| **Type** | **Histological description** | **Age of onset and clinical relevance*** |
|---|---|---|
| Type I lesion | Macrophages and foam cells are | Infants and children (during the first |
| (early stage) | produced. | decade); |
| | | No clinical symptoms of the disease appear. |
| Type II lesion (fatty streak stage) | Intracellular lipids accumulate, forming fatty streaks. | Infants, children and adolescents (during the first two decades); |
| | | No clinical symptoms of the disease appear. |
| Type III lesion (intermediate stage) | Type II lesion plus small areas of extracellular lipid deposition. | Adults (during the third decade); |
| | | No clinical symptoms of the disease appear. |
| Type IV lesion (atheromatous plaque stage) | Type II lesion plus formation of large lipid cores. | Adults (during the third decade); there is a risk of developing the disease, and clinical symptoms of the disease may appear. |
| Type V lesion (fibrous plaque stage) | Formation of large lipid cores plus calcification and fibrosis. | Elderly people (after thirty years); |
| | | there is a risk of developing the disease, and clinical symptoms of the disease may appear. |
| Type VI lesion (Compound lesion stage) | Surface damage, hematoma, hemorrhage or thrombosis. | Elderly people (after thirty years); |
| | | there is a risk of developing the disease, and clinical symptoms of the disease may appear. |

| | | |
|---|---|---|
| *The last column comes from human clinical studies. | | |

Type V lesions are classified into 3 types:
Type Va: large lipid cores plus fibrotic tissues;
Type Vb: lipid cores and fibrosis plus calcification; and
Type Vc: significantly fewer lipid cores, plus fibrotic tissues.

Type VI lesions are classified into 3 types:
Type VIa: fissures in lesion surface;
Type VIb: hemorrhage; and
Type VIc: thrombus deposition.

The area of plaques in the left ventricular outflow tract was measured using a microscope, and the proportion of plaques in the left ventricular outflow tract was calculated; this value reflects the degree of stenosis in the cardiac outflow tract vessel.

### 6.4. Statistics

Data collection and statistical graphing were performed using Excel and GraphPad Prism software 5.0. Data are expressed as Mean ± S.E.M. One-way ANOVA Dennett's test and t-test were used. P < 0.05 indicates a statistically significant difference between groups.

### 7. Results

### 7.1. Effect of test drugs on blood lipids in atherosclerosis mice

A long-term high-fat diet will lead to dyslipidemia. Blood lipid levels can be evaluated by regularly testing serum for TCHO, TG, LDL-C and HDL-C. As shown in Table 24 to Table 27, there are upward trends in TCHO, TG and LDL-C and a downward trend in HDL-C in the mice from groups other than the normal group after feeding with high-fat feed. After oral administration of atorvastatin, dyslipidemia was relieved. The mice had been orally administered GEN1-284s since week 8, and dyslipidemia resulting from the high-fat diet was partially ameliorated compared to the HFD group. This indicates that oral administration of GEN1-284s can partially ameliorate dyslipidemia (TCHO, TG, HDL-C and LDL-C) in mice resulting from the high-fat diet.

**Table 24**

| | The effect of compounds on blood lipids (CHO) in Apo E-/- mice | | | | | | | |
|---|---|---|---|---|---|---|---|---|
| Time | Chow | | | | | | | |
| 1 | 1.295363 | 0.429939 | | | | | | |
| 2 | | | | | | | | |
| 3 | | | | | | | | |
| 4 | 1.111207 | 1.202392 | 1.585226 | 1.221932 | 0.830415 | 0.990351 | 1.248709 | |

| Time | HFD | | | | | | | |
|---|---|---|---|---|---|---|---|---|
| 1 | 0.604158 | 1.466341 | 1.235399 | 1.024715 | 1.040111 | 0.629278 | | |
| 2 | 0.726632 | 1.133671 | 1.196239 | 1.598357 | 0.586031 | 0.807477 | 0.951593 | |
| 3 | 0.778468 | 1.105998 | 1.473888 | 0.994234 | 0.395055 | 0.977159 | 1.275196 | |
| 4 | 1.349302 | 1.088773 | 1.043904 | 0.826796 | | 0.75298 | 0.938245 | |

| Time | HFD+ATV-5mpk | | | | | | | |
|---|---|---|---|---|---|---|---|---|
| 1 | 1.194883 | 1.06118 | 1.216761 | 1.074955 | 1.358568 | | 0.78486 | 0.503678 |
| 2 | 0.846845 | 0.920661 | 0.799041 | 1.023299 | 0.604309 | 0.860203 | 0.847548 | 0.893244 |
| 3 | 1.237166 | 0.929815 | 1.587204 | 1.424215 | 1.118416 | 0.761393 | 1.277525 | 1.057102 |
| 4 | 1.182853 | 1.199498 | 1.343512 | 1.672792 | 1.000482 | 1.219761 | 0.756598 | 1.397789 |

| Time | HFD+284-60mpk | | | | | | | |
|---|---|---|---|---|---|---|---|---|
| 1 | 0.798635 | 0.804307 | 0.856168 | 0.787291 | 0.827807 | | 1.372343 | 1.184348 |
| 2 | 1.233498 | 0.869342 | 1.164603 | 0.921364 | 0.610636 | 1.042984 | 0.99307 | 1.18007 |
| 3 | 1.387737 | 0.844439 | 1.353586 | 0.78002 | 1.42732 | 1.40714 | 1.241046 | 1.476992 |
| 4 | 1.589568 | 1.257393 | 1.396342 | 0.493898 | 0.547451 | 1.047522 | 1.02943 | 1.174168 |

**Table 25**

| | The effect of compounds on blood lipids (TG) in Apo E-/- mice | | | | | | | |
|---|---|---|---|---|---|---|---|---|
| Time | Chow | | | | | | | |
| 1 | | | 0.836458 | | | | | |
| 2 | | | | | | | | |
| 3 | | | | | | | | |
| 4 | 0.936085 | 1.179616 | 1.062049 | 1.076045 | 0.796126 | 1.062049 | 1.174017 | |

| Time | HFD | | | | | | | |
|---|---|---|---|---|---|---|---|---|
| 1 | 0.643637 | 0.902875 | 1.391357 | 1.009998 | 0.875023 | 1.17711 | | |
| 2 | 0.774273 | 1.208023 | 1.165975 | 1.524483 | 0.83845 | 0.769847 | 0.718948 | |
| 3 | 0.782221 | 1.131689 | 1.657266 | 1.068399 | 0.465774 | 0.702421 | 1.192227 | |
| 4 | | 1.126431 | 1.216005 | 1.062049 | 0.91929 | 0.717748 | 0.958479 | |

| Time | HFD+ATV-5mpk | | | | | | | |
|---|---|---|---|---|---|---|---|---|
| 1 | 1.03785 | 0.845028 | 1.136404 | 1.282091 | 0.875023 | | 1.001428 | 0.930727 |
| 2 | 1.203597 | 0.944675 | 1.095159 | 1.11065 | 0.747717 | 0.993361 | 0.763208 | 0.93361 |
| 3 | 1.340819 | 0.961083 | 1.459143 | 1.456391 | 0.950076 | 1.062896 | 1.577467 | 0.658394 |
| 4 | 1.51552 | 1.428744 | 2.380472 | 2.164934 | 1.213206 | 1.476331 | 0.510607 | 0.994868 |

| Time | HFD+284-60mpk | | | | | | | |
|---|---|---|---|---|---|---|---|---|
| 1 | | 0.433675 | 0.630782 | 0.626497 | 0.755045 | 0.746475 | 0.995001 | 1.031423 |
| 2 | 1.050899 | 0.780912 | 1.130567 | 1.050899 | 1.303182 | 1.008852 | 1.451454 | 1.117289 |
| 3 | 0.837255 | 0.801483 | 1.448136 | 0.765711 | 0.922559 | 1.222495 | 1.943445 | 1.200482 |
| 4 | 1.05925 | 0.667362 | 1.09284 | 0.728945 | 1.028459 | 0.759736 | 1.227202 | 1.277588 |

**Table 26**

| | The effect of compounds on blood lipids (HDL-C) in Apo E-/- mice | | | | | | | |
|---|---|---|---|---|---|---|---|---|
| Time | Chow | | | | | | | |
| 1 | | | 0.880356 | 1.699329 | | 2.680074 | | |
| 2 | | | | | | | | |
| 3 | | | | | | | | |
| 4 | 1.41543 | 0.621333 | 1.591897 | 2.408053 | 0.841915 | 0.687507 | 5.319746 | |

| Time | HFD | | | | | | | |
|---|---|---|---|---|---|---|---|---|
| 1 | 0.384928 | 1.188735 | 0.708473 | 0.425371 | 2.311031 | 0.981464 | | |
| 2 | 0.949822 | 1.269893 | 1.282364 | 1.257423 | 0.696259 | 0.710808 | 0.833432 | |
| 3 | 0.97015 | 0.921931 | 1.934533 | 0.938004 | 0.487959 | 1.066589 | 0.680835 | |
| 4 | | 0.863974 | 1.856596 | 1.062499 | 0.687507 | 0.621333 | 0.90809 | |

| Time | HFD+ATV-5mpk | | | | | | | |
|---|---|---|---|---|---|---|---|---|
| 1 | 0.430426 | 1.163458 | 1.734716 | 1.562833 | 1.618443 | | 1.289842 | 1.026962 |
| 2 | 1.178444 | 0.769003 | 0.586105 | 0.766924 | 0.644299 | 0.802257 | 0.858373 | 0.966449 |
| 3 | 0.712982 | 0.841566 | 0.648689 | 1.291611 | 1.1791 | 0.857639 | 0.712982 | 0.857639 |
| 4 | 0.952207 | 1.062499 | 0.643391 | 4.724173 | 0.665449 | 1.371314 | 0.687507 | 0.841915 |

| Time | HFD+284-60mpk | | | | | | | |
|---|---|---|---|---|---|---|---|---|
| 1 | 1.370729 | 0.531534 | 1.057295 | 0.99663 | 1.123015 | | 1.021907 | 1.153347 |
| 2 | 2.122031 | 0.993468 | 0.978919 | 1.032957 | 0.81057 | 1.037114 | 1.826901 | 1.565024 |
| 3 | 2.239921 | 0.536178 | 0.905858 | 0.857639 | 1.163027 | 1.018369 | 1.018369 | 1.066589 |
| 4 | 1.04044 | 0.5331 | 1.238965 | 0.819857 | 0.577216 | 0.511041 | 0.555158 | 1.569839 |

**Table 27**

| | The effect of compounds on blood lipids (LDL-C) in Apo E-/- mice | | | | | | | |
|---|---|---|---|---|---|---|---|---|
| Time | Chow | | | | | | | |
| 1 | 0.290503 | 0.709497 | 0.916201 | | | | | |
| 2 | | | | | | | | |
| 3 | | | | | | | | |
| 4 | 0.739936 | 0.966134 | 1.08115 | 1.272843 | 1.556549 | 0.84345 | 1.142492 | |

| Time | HFD | | | | | | | |
|---|---|---|---|---|---|---|---|---|
| 1 | 0.536313 | 0.837989 | 2.027933 | 0.960894 | 0.837989 | 0.798883 | | |
| 2 | 0.591346 | 1.155678 | 0.953297 | 1.723902 | 0.766483 | 0.855998 | 0.953297 | |
| 3 | 0.618296 | 0.780231 | 1.604627 | 0.927445 | 0.125131 | 1.302839 | 1.64143 | |
| 4 | 1.614057 | 1.138658 | 1.119489 | 0.383386 | 0.797444 | 0.946965 | | |

| Time | HFD+ATV-5mpk | | | | | | | |
|---|---|---|---|---|---|---|---|---|
| 1 | 1.100559 | 1.061453 | -0.07821 | 1.122905 | 1.296089 | 0.675978 | 1.005587 | |
| 2 | 0.723672 | 0.813187 | 0.661401 | 1.342491 | 0.603021 | 0.57967 | 1.151786 | 1.233517 |
| 3 | 0.975289 | 0.802313 | 0.87224 | 0.647739 | 0.76551 | 0.456362 | 1.218191 | 1.42061 |
| 4 | 1.050479 | 1.675399 | 0.908626 | 0.851118 | 1.31885 | 0.360383 | 0.601917 | 1.123322 |

| Time | HFD+284-60mpk | | | | | | | |
|---|---|---|---|---|---|---|---|---|
| 1 | 0.687151 | 1.011173 | 0.765363 | 0.519553 | 0.715084 | 1.050279 | 1.586592 | |
| 2 | 1.373627 | 0.68086 | 2.128664 | 1.097299 | 0.918269 | 1.393087 | 1.144002 | 0.937729 |
| 3 | 1.218191 | 0.621977 | 1.64143 | 0.901682 | 1.494216 | 1.214511 | 2.62776 | 1.405888 |
| 4 | 1.176997 | 0.935463 | 0.851118 | 0.172524 | 0.479233 | 1.161661 | 1.092651 | 0.50607 |

### 7.2. Effect of test drugs on arterial plaques in atherosclerosis mice

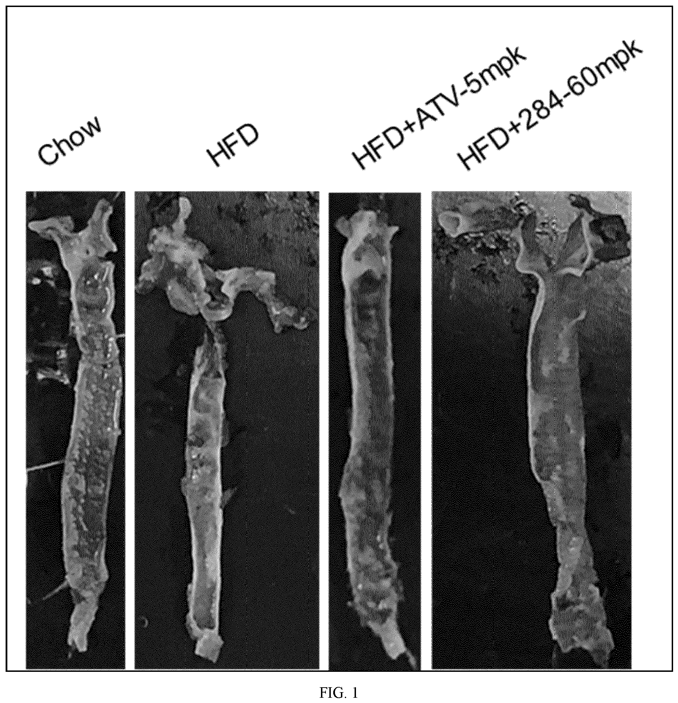

Lesions of atherosclerosis are closely correlated with thrombosis. As shown in Table 28 and FIG. 1, the HFD group after eating the high-fat diet had a much larger arterial plaque area than the normal control group. After oral administration of atorvastatin, the proportion of the arterial plaque area in the mice was significantly reduced to a level that was much lower than that of the HFD group. The test compound GEN1-284s significantly reduced the formation of atherosclerotic plaques, and its effect was comparable to that of atorvastatin.

**Table 28**

| The effect of compounds on atherosclerotic plaques in mice | | | |
|---|---|---|---|
| Chow | HFD | HFD+ATV-5mpk | HFD+284-60mpk |
| 2.926497 | 13.27125 | 8.608643 | 8.803075 |
| 6.437509 | 15.73307 | 9.550616 | 8.747435 |
| 3.607385 | 26.99193 | 9.286414 | 3.496609 |
| 2.798883 | 18.21609 | 13.92162 | 13.32327 |
| 3.7686 | 23.066 | 13.85783 | 11.93195 |
| 2.718915 | | 10.97725 | 14.51266 |
| 2.90204 | | 13.51589 | 15.35919 |
| | | | 6.043146 |

### 7.3. Effect of test compounds on plaques in cardiac outflow tract

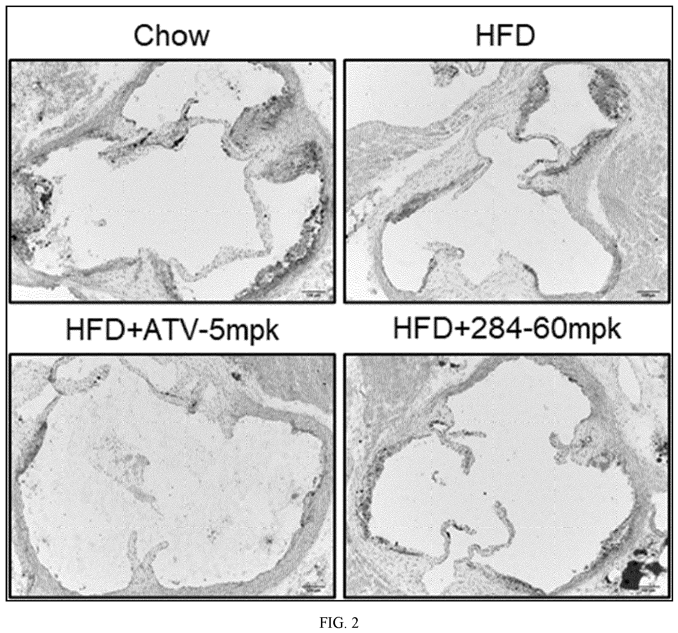

Dyslipidemia can cause accumulation of plaques within the left ventricular outflow tract and thereby apoptosis. Morphological changes in the left ventricular outflow tract and the proportion of outflow tract plaques to the outflow tract cross-section were evaluated. As shown in Table 29 and FIG. 2, the measurement of the cross-section of the left ventricular outflow tract showed that the HFD group had more outflow tract plaques than the normal control group. The atorvastatin group had significantly fewer plaques than the HFD group. GEN1-284s has a certain ameliorating effect on outflow tract plaques.

**Table 29**

| The effect of compounds on plaques in the cardiac outflow tracts of mice | | | |
|---|---|---|---|
| Chow | HFD | HFD+ATV-5mpk | HFD+284-60mpk |
| 2.473071 | 27.1089 | 3.770563 | 0.6250491 |
| 14.10743 | | 2.279487 | 2.394451 |
| 13.78163 | 7.171735 | 8.380182 | 7.368202 |
| 10.42742 | | 1.923165 | 3.172364 |
| 2.075372 | 14.65993 | 8.459556 | 8.443544 |
| 8.082601 | 16.19419 | 9.071092 | 8.651694 |
| 0.2222984 | | 7.843952 | 1.277743 |
| | | 1.61803 | |

### 8. Conclusion

In conclusion: GEN1-284s can reduce the formation of atherosclerotic plaques to some extent, and its effect is similar to that of the positive drug atorvastatin.

### Effect Example 8: Evaluation of GENl-284s in IMQ-Induced Psoriasis Model

### Abstract

Psoriasis is a chronic inflammatory skin disease with a long course and has a tendency to recur. Some patients have it almost all their lives. The disease mainly afflicts young adults and greatly affects the physical and mental health of patients. The clinical manifestations mainly include erythema and scales. It could occur over the whole body and is more commonly seen on the scalp and limb extension sides. It usually becomes aggravated in the winter. IMQ was applied to the exposed skin on the back of mice for six consecutive days to simulate the development of psoriasis.

**Objective:** This experiment used a Balb/c mouse model of IMQ-induced psoriasis to evaluate the effect of the compound GEN1-284s on red and swollen skin and scale pathological changes in mice with psoriasis.

**Method:** This experiment used a total of 19 mice. A natural skin observation control (n = 1). 2 cm² of hair was shaved off the backs of 30 mice on day 0, the stubble was removed with a mild depilatory cream, and the original skin was photographed. 62.5 mg of IMQ was applied to each of the mice. There was a mock group (n = 6), a DEX (3 mg/kg, PO., qD) group (n = 6), and a GEN1-284s (30 mg/kg, PO, qD) group (n = 6). On the day of modeling, administration was performed in a volume of 100 µL/20 g body weight 4 h after IMQ application. The PASI scoring method was used to score the erythema, swelling and scaling on the skin of the mice. The experiment took a total of 6 days. The first day was set to day 0. IMQ application and administration were not performed on day 5. The mice were sacrificed with carbon dioxide at the end of the experiment.

**Results:** The results show that after 5 days of IMQ induction, the mice were orally administered the test compound until day 6. Compared to the skin of a normal mouse, the skin of the mice from the CTR group was swollen and red and scaled significantly. The redness and scaling of the skin could be effectively reduced after treatment with the compound GEN1-284s.

**Conclusion:** In conclusion: GEN1-284s can partially reduce the redness, swelling and scaling of psoriatic skin, and its effect is similar to that of the positive drug dexamethasone.

### 1. Objective

This experiment used a Balb/c mouse model of IMQ-induced psoriasis to evaluate the ameliorating effect of different doses of GEN1-284s on the swelling, redness and scaling of the skin of the mice of the psoriasis model.

### 2. Experimental standards

This project was implemented in GenEros Biopharma (Hangzhou) Ltd. All the animal experiments involved in the implementation complied with the standard procedures for the use of laboratory animals and the guide for the care and use of laboratory animals provided by IACUC of GenEros, and the animal welfare requirements of the Office of Laboratory Animal Welfare. This experiment is a non-GLP experiment.

### 3. Experimental materials

### 3.1. Instruments

Analytical balance, model: SQP, manufacturer: Beijing Sartorius Scientific Instruments Co., Ltd.;
Electronic balance, model: MP5002, manufacturer: Changzhou Tianzhiping Instruments and Equipment Co., Ltd.;
Constant-temperature magnetic stirrer, model: 85-2, manufacturer: Shanghai Sile Instruments Co., Ltd.
Digital camera, model: EOS800D, manufacturer: Canon.

### 3.2. Test drugs and reagents

**Test compound:** GEN1-284s, storage condition: -20 °C (high-concentration stock solution, powder); working solutions can be freshly prepared and temporarily stored at 2-8 °C for 1 day.

Dexamethasone, manufacturer: Sigma, Lot: WXBC8102V, storage condition: 4 °C.

**Reagents:** DMSO, storage condition: room temperature.
IMQ imiquimod cream (Aldara, 250 mg: 12.5 mg × 12 packets)
MC, manufacturer: Shanghai Sangon Biotech Co., Ltd., storage condition: room temperature.

### 4. Vehicle and compound preparation

Vehicle: Preparation of 0.5% MC: 2 g of MC was precisely weighed out into a 500 mL reagent bottle with a screw cap, and a stirring bar was added. The bottle was placed on a magnetic stirrer, a small amount of hot water was added, and the magnetic stirrer was started. When MC was dissolved, water was added in 3 portions to make 400 mL. The rotational speed was adjusted to 1200 rpm and held for 15 min.

Positive drug: preparation of dexamethasone (3 mg/kg): 3 mg of the compound was precisely weighed out into a 15 mL centrifuge tube, 0.25 mL of DMSO was added, and 4.75 mL of 0.5% MC was added. The solution was aliquoted at 1 mL/tube and cryopreserved at -20 °C. One aliquot was taken out, left to thaw and well mixed before each use. The concentration was 0.6 mg/mL.

Preparation of GEN1-284 (30 mg/kg): 30 mg of the compound was precisely weighed out into a 15 mL centrifuge tube, 0.25 mL of DMSO was added, and 4.75 mL of 0.5% MC was added. The solution was aliquoted at 1 mL/tube and cryopreserved at -20 °C. One aliquot was taken out and left to thaw before each use. The concentration was 6 mg/mL.

### 5. Laboratory animals

### 5.1. Use of animals

19 Balb/c mice, female, 7-8 weeks old, weighing 18-20 g, purchased from Shanghai Slac Laboratory Animal Co., Ltd.

### 5.2. Housing of animals

The animals used in the following experiments were all Balb/c mice. After arrival at the facility of GenEros Biopharma Ltd., they were housed in an animal room under strictly controlled environmental conditions. The temperature of the room was maintained at 20-24 °C and the humidity was maintained at 30-70%. The temperature and humidity of the animal room were monitored in real time using a hygrothermograph and recorded twice daily (once in the morning and once in the afternoon). The lighting of the animal room was controlled by an electronic timer switch system: on for 12 h and off for 12 h every day (switched on at 6:00 AM and off at 18:00 PM). The animals were given *ad libitum* access to food and water.

### 6. Experimental method

### 6.1. Experimental design

### Modeling

The test animals were anesthetized with isoflurane. 2 cm² of hair was shaved off the backs of the mice, and the stubble was removed with a depilatory cream. IMQ application was performed for a total of 5 days, from day 0 to day 4. Scoring and IMQ application were performed every morning. Administration was performed in the afternoon

During the experiment, the living states of the animals were observed, and abnormal states were recorded. After the experiment ended, endpoint treatment and sample collection were performed.

### 6.2. Grouping and administration

A total of 19 animals were selected by screening in this experiment, and the animals other than those in the normal control group were divided into 7 groups of 8. The grouping is shown in Table 30:

**Table 30, Grouping and treatment of animals**

| No. | Group | Dose (mg/kg) | Number | Animal | Route of administration | Time of administration |
|---|---|---|---|---|---|---|
| 1 | Normal control | -- | n=1 | Balb/c- | PO | 5d |
| | | | | | QD | |
| 2 | Model control | -- | n=6 | Balb/c | PO | 5d |
| | | | | | QD | |
| 3 | Dexamethasone DEX | 3mpk | n=6 | Balb/c | PO | 5d |
| | | | | | QD | |
| 4 | 284-30mpk | 30mpk | n=6 | Balb/c | PO | 5d |
| | | | | | QD | |

### 6.3. Endpoint indicators

### Experimental indicators:

Desquamation (D): means epidermal cells flake off. The severity scores of skin lesions were as follows:
0 = none; this sign cannot be confirmed by careful observation
1 = mild; this sign can be confirmed but careful observation is required
2 = moderate; this sign is more visible and can be confirmed immediately
3 = severe; this sign is marked.
4 = very severe; this sign is very marked.

Induration I: 0 - skin lesions are at the same level as normal skin;
1 - skin lesions are at slightly higher levels than normal skin surface;
2 - lesions moderately stick out and have round or slope-shaped borders;
3 - skin lesions are thick and markedly stick out;
4 - skin lesions are very thick and very markedly stick out.

### Erythema E:

0 - no visible erythema;
1 - light red;
2 - red;
3 - dark red;
4 - the red is very dark

### 6.4. Statistics

Data collection and statistical graphing were performed using Excel and GraphPad Prism software 5.0. Data are expressed as Mean ± S.E.M. One-way ANOVA Dennett's test and t-test were used. P < 0.05 indicates a statistically significant difference between groups.

### 7. Results

### 7.1. Effect of test drugs on psoriasis mouse skin

Long-term application of IMQ will cause severe swelling of the skin of mice. As shown in Table 31, after oral administration of DEX, the swelling of the skin was relieved. The mice had been orally administered GEN1-284s since the beginning of modeling, and the swelling of the skin caused by IMQ induction was ameliorated compared to the CTR group, indicating that GEN1-284s can partially ameliorate the psoriatic swelling caused by IMQ induction in mice.

**Table 31**

| | The effect of compounds on skin thickening in psoriasis mice | | | | | |
|---|---|---|---|---|---|---|
| DAY | CTR | | | | | |
| 0 | 0 | 0 | 0 | 0 | 0 | 0 |
| 1 | 0.5 | 0.5 | 1 | 0 | 1 | 1 |
| 2 | 1.5 | 1 | 1.5 | 1 | 1.5 | 1.5 |
| 3 | 1.5 | 1 | 2 | 1.5 | 1 | 1.5 |
| 4 | 1.5 | 1 | 3 | 1 | 1 | 2 |
| 5 | 1.5 | 1 | 3 | 1 | 1 | 2 |

| DAY | DEX | | | | | |
|---|---|---|---|---|---|---|
| 0 | 0 | 0 | 0 | 0 | 0 | 0 |
| 1 | 0 | 0 | 0.5 | 0 | 0 | 0 |
| 2 | 0 | 0 | 0 | 0 | 0.5 | 0 |
| 3 | 0 | 0.5 | 0 | 0.5 | 0.5 | 0.5 |
| 4 | 0.5 | 0 | 0.5 | 0.5 | 0.5 | 0.5 |
| 5 | 0.5 | 0 | 0.5 | 0.5 | 0.5 | 0.5 |

| DAY | 284-30 | | | | | |
|---|---|---|---|---|---|---|
| 0 | 0 | 0 | 0 | 0 | 0 | 0 |
| 1 | 0.5 | 0.5 | 0 | 1 | 0.5 | 0.5 |
| 2 | 0.5 | 0.5 | 0.5 | 1 | 1 | 0.5 |
| 3 | 1.5 | 1 | 0.5 | 1 | 1.5 | 1 |
| 4 | 0 | 1 | 1 | 1 | 1 | 0.5 |
| 5 | 0 | 1 | 1 | 1 | 1 | 0.5 |

As shown in Table 32, the redness of the skin of the mice caused by IMQ application could not be eliminated in a short time, showing no significant difference.

**Table 32**

| | The effect of compounds on skin redness in psoriasis mice | | | | | |
|---|---|---|---|---|---|---|
| DAY | CTR | | | | | |
| 0 | 0 | 0 | 0 | 0 | 0 | 0 |
| 1 | 0.5 | 0.833333333 | 0.166666667 | 0.333333333 | 1.166666667 | 0 |
| 2 | 1.166666667 | 1 | 0.333333333 | 0.666666667 | 0.666666667 | 0.833333333 |
| 3 | 0.666666667 | 1.166666667 | 1 | 1.333333333 | 1 | 1.5 |
| 4 | 1.5 | 1.166666667 | 2.166666667 | 0.833333333 | 1.166666667 | 1.166666667 |
| 5 | 1.333333333 | 1 | 1.5 | 1 | 1.333333333 | 1 |

| DAY | DEX | | | | | |
|---|---|---|---|---|---|---|
| 0 | 0 | 0 | 0 | 0 | 0 | 0 |
| 1 | 0.666666667 | 0.166666667 | 0.333333333 | 0.5 | 0.5 | 0.666666667 |
| 2 | 0.5 | 0.666666667 | 0.5 | 0.166666667 | 0.166666667 | 0.666666667 |
| 3 | 1.166666667 | 1 | 0.5 | 0.5 | 0.333333333 | 0.666666667 |
| 4 | 0.666666667 | 0.5 | 1.166666667 | 0.5 | 1.666666667 | 1.166666667 |
| 5 | 1.166666667 | 1 | 0.833333333 | 0.666666667 | 1.5 | 1 |

| DAY | 284-30 | | | | | |
|---|---|---|---|---|---|---|
| 0 | 0 | 0 | 0 | 0 | 0 | 0 |
| 1 | 1 | 0.666666667 | 0.833333333 | 0.5 | 1.166666667 | 0 |
| 2 | 0.833333333 | 1 | 0.5 | 0.5 | 0.333333333 | 0.166666667 |
| 3 | 1 | 0.833333333 | 1 | 0.833333333 | 0.666666667 | 0.5 |
| 4 | 1.333333333 | 1 | 0.666666667 | 1 | 1.166666667 | 0.833333333 |
| 5 | 1.5 | 0.666666667 | 1 | 0.666666667 | 0.666666667 | 0.5 |

As shown in Table 33, as a large quantity of scales were produced on the mice in the later stage, this experiment evaluated the quantity of scales only rather than the size of scales. The scale score may not be instructive to this experiment.

**Table 33**

| | The effect of compounds on skin scaling in psoriasis mice | | | | | |
|---|---|---|---|---|---|---|
| DAY | CTR | | | | | |
| 0 | 0 | 0 | 0 | 0 | 0 | 0 |
| 1 | 0.5 | 0.5 | 0.3333333 | 0.3333333 | 0.3333333 | 0.1666667 |
| 2 | 0.8333333 | 0.8333333 | 0.8333333 | 0.6666667 | 1 | 0.6666667 |
| 3 | 1.1666667 | 0.6666667 | 1.8333333 | 0.8333333 | 0.5 | 1.6666667 |
| 4 | 1.6666667 | 1.3333333 | 3 | 0.8333333 | 1.5 | 2.3333333 |
| 5 | 2 | 1.3333333 | 3 | 0.6666667 | 2.3333333 | 2.6666667 |

| DAY | DEX | | | | | |
|---|---|---|---|---|---|---|
| 0 | 0 | 0 | 0 | 0 | 0 | 0 |
| 1 | 0.5 | 0.3333333 | 0.3333333 | 0.8333333 | 0.8333333 | 0.1666667 |
| 2 | 0.8333333 | 0.3333333 | 0 | 0.6666667 | 1 | 0 |
| 3 | 1.3333333 | 1.5 | 0.5 | 1 | 1.1666667 | 0.5 |
| 4 | 1.1666667 | 1 | 1.8333333 | 1.3333333 | 1.5 | 1 |
| 5 | 1.6666667 | 1.5 | 2.3333333 | 1.6666667 | 1.5 | 1.3333333 |

| DAY | 284-30 | | | | | |
|---|---|---|---|---|---|---|
| 0 | 0 | 0 | 0 | 0 | 0 | 0 |
| 1 | 0.1666667 | 0 | 0.1666667 | 0.8333333 | 0.3333333 | 0.1666667 |
| 2 | 0.1666667 | 0.5 | 0.3333333 | 0.8333333 | 1 | 0.8333333 |
| 3 | 0.8333333 | 0.5 | 1.8333333 | 0.6666667 | 1.1666667 | 0.6666667 |
| 4 | 1.3333333 | 1.6666667 | 1.1666667 | 1 | 1.5 | 1.5 |
| 5 | 1.5 | 1 | 2.3333333 | 1.3333333 | 1.8333333 | 2.5 |

As shown in Table 34, the overall therapeutic effect of GEN1-284s is similar to that of DEX; GEN1-284s has a certain therapeutic effect on IMQ-induced psoriasis.

**Table 34**

| | The overall effect of compounds on the skin of psoriasis mice | | | | | |
|---|---|---|---|---|---|---|
| DAY | CTR | | | | | |
| 0 | 0 | 0 | 0 | 0 | 0 | 0 |
| 1 | 1.5 | 1.8333333 | 1.5 | 0.6666667 | 2.5 | 1.1666667 |
| 2 | 3.5 | 2.8333333 | 2.6666667 | 2.3333333 | 3.1666667 | 3 |
| 3 | 4.5 | 2.3333333 | 5 | 3.3333333 | 2.8333333 | 4.1666667 |
| 4 | 4.6666667 | 3.5 | 8.1666667 | 2.6666667 | 3.6666667 | 5.5 |
| 5 | 4.8333333 | 3.3333333 | 7.5 | 2.6666667 | 4.6666667 | 5.6666667 |

| DAY | DEX | | | | | |
|---|---|---|---|---|---|---|
| 0 | 0 | 0 | 0 | 0 | 0 | 0 |
| 1 | 1.1666667 | 0.5 | 1.1666667 | 1.3333333 | 1.3333333 | 0.8333333 |
| 2 | 1.3333333 | 1 | 0.5 | 0.8333333 | 1.6666667 | 0.6666667 |
| 3 | 2.5 | 3 | 1 | 2 | 2 | 1.6666667 |
| 4 | 2.3333333 | 1.5 | 3.5 | 2.3333333 | 3.6666667 | 2.6666667 |
| 5 | 3.3333333 | 2.5 | 3.6666667 | 2.8333333 | 3.5 | 2.8333333 |

| DAY | 284-30 | | | | | |
|---|---|---|---|---|---|---|
| 0 | 0 | 0 | 0 | 0 | 0 | 0 |
| 1 | 1.6666667 | 1.1666667 | 1 | 2.3333333 | 2 | 0.6666667 |
| 2 | 1.5 | 2 | 1.3333333 | 2.3333333 | 2.3333333 | 1.5 |
| 3 | 3.3333333 | 2.3333333 | 3.3333333 | 2.5 | 3.3333333 | 2.1666667 |
| 4 | 2.6666667 | 3.6666667 | 2.8333333 | 3 | 3.6666667 | 2.8333333 |
| 5 | 3 | 2.6666667 | 4.3333333 | 3 | 3.5 | 3.5 |

### 8. Conclusion

In conclusion: GEN1-284s can reduce skin swelling to some extent, and its effect is similar to that of the positive drug DEX.

### Effect Example 9: Effect of GENl-284s on LPS-Induced TNFa Production

### Abstract

Inflammation is associated with the pathogenesis of many diseases, including aging, cancer and cardiovascular system dysfunction. Acute inflammation caused by lipopolysaccharide (LPS) in mice can lead to sepsis, systemic uncontrolled inflammatory reactions and, in severe cases, even death. A mouse model of sepsis was induced using lipopolysaccharide, and mice were pre-treated with GEN1-284s. With dexamethasone as a positive reference, the protective effect of GEN1-284s on mice and possible mechanisms of action were investigated.

**Objective:** This experiment evaluated the therapeutic effect of GEN1-284s in inhibiting the LPS-induced production of TNF-α in mice.

Materials and method: A total of 18 mice were used in this experiment. The mice were divided into a MOCK group (n = 6), a dexamethasone group (n = 6), and a GEN1-284s 15 mpk group (n = 6). LPS (1 mpk) was intraperitoneally injected 0.5 h after administration. The mice were sacrificed 2 h after the LPS injection, and blood samples were taken. The blood samples were left in EDTA-K2 anticoagulant tubes at room temperature for 20 min and centrifuged at 8000 rpm for 10 min. The plasma was separated and cryopreserved at -80 °C before analysis.

**Results:** In mice stimulated by LPS, the inhibition rate of GEN1-284s-15 mpk against TNF-α reached 36%, which indicates a significant difference.

**Conclusion:** GEN1-284s has an inhibitory effect on the LPS-induced production of TNF-α in mice.

### 1. Objective

This experiment evaluated the therapeutic effect of GEN1-284s in inhibiting the LPS-induced production of TNF-α in mice.

### 2. Experimental standards

This project was implemented in GenEros Biopharma (Hangzhou) Ltd. All the animal experiments involved in the implementation complied with the standard procedures for the use of laboratory animals and the guide for the care and use of laboratory animals provided by IACUC of GenEros, and the animal welfare requirements of the Office of Laboratory Animal Welfare. This experiment is a non-GLP experiment.

### 3. Experimental materials

### 3.1. Instruments

Analytical balance, model: SQP, manufacturer: Beijing Sartorius Scientific Instruments Co., Ltd.; Electronic balance, model: MP5002, manufacturer: Changzhou Tianzhiping Instruments and Equipment Co., Ltd.;

Mini vortex mixer, model: XW-80A, manufacturer: Shanghai Huxi Analysis Instrument Factory Co., Ltd. Centrifuge, model: 5424 R, manufacturer: Eppendorf.

### 3.2. Test drugs and reagents

### Test compound:

GEN1-284s, storage condition: -20 °C (high-concentration stock solution, powder); working solutions can be freshly prepared and temporarily stored at 2-8 °C for 1 day.

Dexamethasone, manufacturer: Sigma, Lot: WXBC8102V, storage condition: 4 °C.

### Reagents:

DMSO, manufacturer: Solarbio, Lot: 1012D033, storage condition: room temperature.

PEG300, manufacturer: Shanghai Yuanye Biotech Co., Ltd., storage condition: room temperature.

1×PBS, manufacturer: Hyclone, Lot: AE29449011, storage condition: 4 °C.

EDTA-K2, manufacturer: Shanghai Sangon, A600075-0100, storage condition: 4 °C.

LPS, manufacturer: sigma L2880, storage condition: 4 °C.

Mouse TNF-α ELISA Kit: manufacturer: Absin, Lot: #AA24, storage condition: 4 °C.

### 4. Vehicle and compound preparation

Positive drug:
Dexamethasone: dissolved with DMSO and PEG300 and then diluted with PBS to the desired concentration;
concentration for administration: 3 mg/kg.

### 5. Laboratory animals

### 5.1. Use of animals

18 female Balb/c mice, weighing 20-22 g, purchased from Shanghai Slac Laboratory Animal Co., Ltd.

### 5.2. Housing of animals

The animals used in the following experiments were all Balb/c mice. After arrival at the facility of GenEros Biopharma Ltd., they were housed in an animal room under strictly controlled environmental conditions. The temperature of the room was maintained at 20-24 °C and the humidity was maintained at 30-70%. The lighting of the animal room was controlled by an electronic timer switch system: on for 12 h and off for 12 h every day (switched on at 6:00 AM and off at 18:00 PM). The animals were given *ad libitum* access to food and water.

### 6. Experimental method

### 6.1. Experimental design

### Modeling

LPS (1 mpk) was intraperitoneally injected 0.5 h after administration. The mice were sacrificed 2 h after the LPS injection, and blood samples were taken. The blood samples were left in EDTA-K2 anticoagulant tubes at room temperature for 20 min and centrifuged at 8000 rpm for 10 min. The plasma was separated and cryopreserved at -80 °C before analysis.

### 6.2. Grouping and administration

| Group | Number of animals |
|---|---|
| MOCK | 6 |
| Dexamethasone (3mpk) | 6 |
| GEN1-284S (15mpk) | 6 |

The test compound was intragastrically administered to each animal in a volume of 100 µL/20 g.

| Group | Number of animals | Route of administration | Frequency | Starting time | End time |
|---|---|---|---|---|---|
| MOCK | 6 | PO | qD | 13:20 | 15:50 |
| Dexamethasone (3mpk) | 6 | PO | qD | 13:20 | 15:50 |
| GEN1-284S (15mpk) | 6 | PO | qD | 13:30 | 16:00 |

### 6.3. Endpoint indicators

The mice were tested for TNF-α level with ELISA kits, and the inhibitory effect of GEN1-284s on the TNF-α level was evaluated.

### 6.4. Statistics

Data collection and statistical graphing were performed using Excel and GraphPad Prism software 5.0. Data are expressed as Mean ± S.E.M. One-way ANOVA Dennett's test and t-test were used. P < 0.05 indicates a statistically significant difference between groups.

### 7. Results

### 7.1. Determination of TNF-α concentration in mice

According to the instructions for use, a standard curve formula (y = 0.0038x + 0.1206, R² = 0.9956) was obtained and can be used to calculate detection values.

The results show that the positive drug dexamethasone can significantly inhibit the LPS-induced production of TNF-α in mice, with an inhibition rate up to 81% (p < 0.0001, vs CTR, Two-tailed); the test drug GEN1-284s also has a significant inhibitory effect, with an inhibition rate up to 36% (p < 0.01, vs CTR, Two-tailed), which indicates a significant difference.

### 8. Conclusion

In conclusion: In mice stimulated by LPS, the inhibition rate of GEN1-284s-15 mpk against TNF-α reached 36%, which indicates a significant difference. GEN1-284s has an inhibitory effect on the LPS-induced production of TNF-α in mice.

Although specific embodiments of the present invention have been described above, it will be appreciated by those skilled in the art that these are merely illustrative examples and that many changes or modifications can be made to these embodiments without departing from the principles and spirit of the present invention. The protection scope of the present invention is therefore defined by the appended claims.
1. A fused ring-containing compound represented by formula **II,** a pharmaceutically acceptable salt thereof, a solvate thereof or a solvate of the pharmaceutically acceptable salt thereof, wherein: 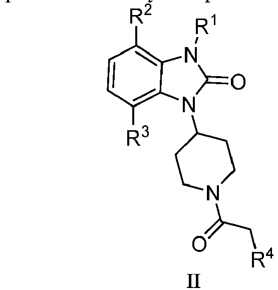
R¹ is hydrogen or 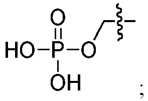
R² is hydrogen or chlorine;
R³ is hydrogen, cyano, nitro, acetyl, trifluoromethanesulfonyl, or C₁-C₃ alkyl substituted with one or more halogens;
R⁴ is 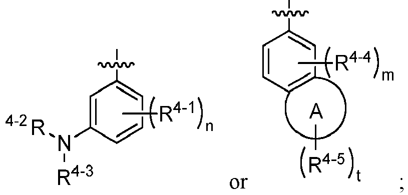
n is 0, 1, 2, 3 or 4;
R⁴⁻¹ is independently cyano, a halogen, C₁-C₃ alkyl, or C₁-C₃ alkyl substituted with one or more halogens; R⁴⁻² and R⁴⁻³ are independently C₁-C₃ alkyl;
m is 0, 1, 2, 3 or 4;
R⁴⁻⁴ is independently cyano, hydroxy, a halogen, C₁-C₃ alkyl, C₁-C₃ alkoxy, or C₁-C₃ alkyl substituted with one or more halogens;
ring A is 5- or 6-membered heteroaryl having 1, 2 or 3 heteroatoms selected from one or more of N, O and S; t is 0, 1, 2, 3 or 4;
R⁴⁻⁵ is independently cyano, hydroxy, a halogen, C₁-C₃ alkyl, C₁-C₃ alkoxy, or C₁-C₃ alkyl substituted with one or more halogens;
however, the fused ring-containing compound represented by formula **II** is not the following compounds: 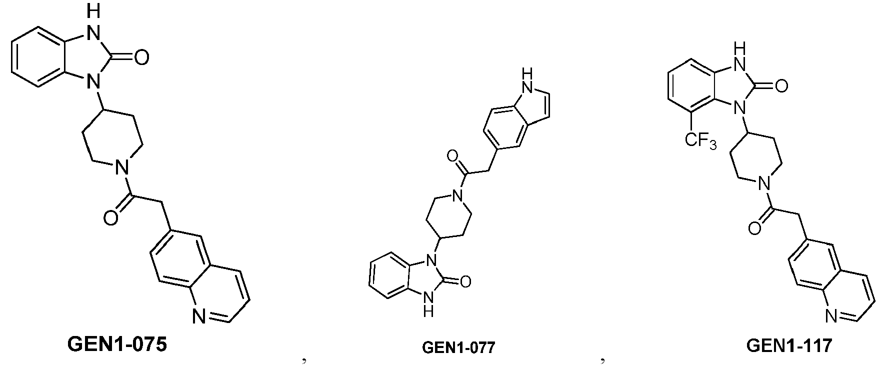 and

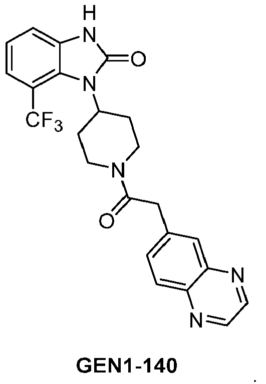

2. The fused ring-containing compound represented by formula **II,** the pharmaceutically acceptable salt thereof, the solvate thereof or the solvate of the pharmaceutically acceptable salt thereof according to claim 1, wherein R² is hydrogen;
and/or, R' is 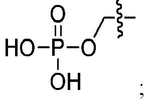
and/or, R³ is C₁-C₃ alkyl substituted with one or more halogens;
and/or, n is 0;
and/or, R⁴ is 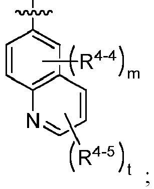
and/or, m is 0 or 1;
and/or, R⁴⁻⁴ is independently a halogen or C₁-C₃ alkyl;
and/or, t is 0 or 1;
and/or, R⁴⁻⁵ is independently cyano, a halogen, C₁-C₃ alkyl, C₁-C₃ alkoxy, or C₁-C₃ alkyl substituted with one or more halogens;
and/or, when the R¹ is hydrogen, m + t is greater than or equal to 1.

3. The fused ring-containing compound represented by formula **II,** the pharmaceutically acceptable salt thereof, the solvate thereof or the solvate of the pharmaceutically acceptable salt thereof according to claim 2,
wherein R⁴⁻⁵ is independently a halogen;
and/or, when the R¹ is hydrogen, m + t = 1.

4. The fused ring-containing compound represented by formula **II,** the pharmaceutically acceptable salt thereof, the solvate thereof or the solvate of the pharmaceutically acceptable salt thereof according to claim 1, being defined as described in any one of the following schemes: scheme 1, wherein R¹ is hydrogen or 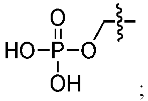 R² is hydrogen; R³ is C₁-C₃ alkyl substituted with one or more halogens; R⁴ is 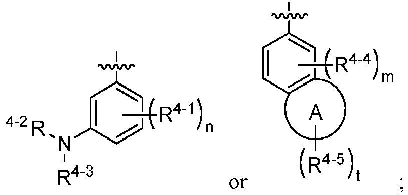 n is 0; R⁴⁻² and R⁴⁻¹ are independently C₁-C₃ alkyl; m is 0 or 1; R⁴⁻⁴ is a halogen or C₁-C₃ alkyl; ring A is 5- or 6-membered heteroaryl having 1, 2 or 3 heteroatoms selected from one or more of N, O and S; t is 0 or 1; R⁴⁻⁵ is cyano, a halogen, C₁-C₃ alkyl, C₁-C₃ alkoxy, or C₁-C₃ alkyl substituted with one or more halogens; however, the fused ring-containing compound represented by formula II is not the following compounds: 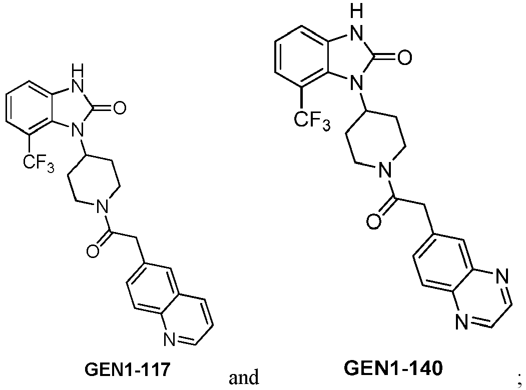 scheme 2, wherein R¹ is hydrogen or 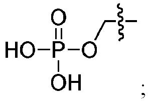 R² is hydrogen; R³ is C₁-C₃ alkyl substituted with one or more halogens; R⁴ is 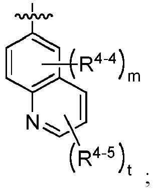 m is 0 or 1; R⁴⁻⁴ is a halogen or C₁-C₃ alkyl; t is 0 or 1; R⁴⁻⁵ is a halogen; however, the fused ring-containing compound represented by formula **II** is not 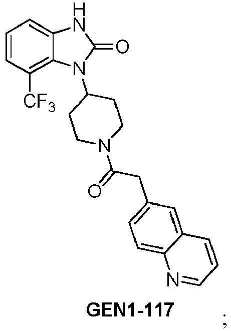 scheme 3, wherein R¹ is 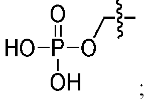 R² is hydrogen; R³ is C₁-C₃ alkyl substituted with one or more halogens; R⁴ is 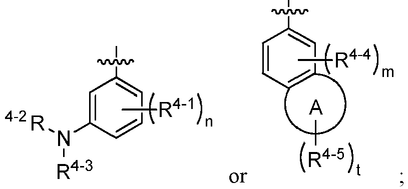 n is 0; R⁴⁻² and R⁴⁻¹ are independently C₁-C₃ alkyl; m is 0 or 1; R⁴⁻⁴ is a halogen or C₁-C₃ alkyl; ring A is 5- or 6-membered heteroaryl having 1, 2 or 3 heteroatoms selected from one or more of N, O and S; t is 0 or 1; R⁴⁻⁵ is cyano, a halogen, C₁-C₃ alkyl, C₁-C₃ alkoxy, or C₁-C₃ alkyl substituted with one or more halogens; and scheme 4, wherein R¹ is 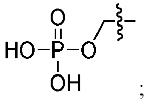 R² is hydrogen; R³ is C₁-C₃ alkyl substituted with one or more halogens; R⁴ is 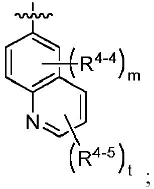 m is 0 or 1; R⁴⁻⁴ is a halogen or C₁-C₃ alkyl; t is 0 or 1; R⁴⁻⁵ is a halogen.
5. The fused ring-containing compound represented by formula **II**, the pharmaceutically acceptable salt thereof, the solvate thereof or the solvate of the pharmaceutically acceptable salt thereof according to any one of claims 1-4, wherein when the R³ is C₁-C₃ alkyl substituted with one or more halogens, the "more" means 2 or 3;
and/or, when the R³ is C₁-C₃ alkyl substituted with one or more halogens, the halogens are fluorine or chlorine; and/or, when the R³ is C₁-C₃ alkyl substituted with one or more halogens, the C₁-C₃ alkyl is methyl, ethyl, n-propyl or isopropyl;
and/or, when the R⁴⁻² is C₁-C₃ alkyl, the C₁-C₃ alkyl is methyl, ethyl, n-propyl or isopropyl;
and/or, when the R⁴⁻³ is C₁-C₃ alkyl, the C₁-C₃ alkyl is methyl, ethyl, n-propyl or isopropyl;
and/or, when the R⁴⁻¹ is a halogen, the halogen is fluorine or chlorine;
and/or, when the R⁴⁻¹ is C₁-C₃ alkyl, the C₁-C₃ alkyl is methyl, ethyl, n-propyl or isopropyl;
and/or, when the R⁴⁻¹ is C₁-C₃ alkyl substituted with one or more halogens, the "more" means 2 or 3;
and/or, when the R⁴⁻¹ is C₁-C₃ alkyl substituted with one or more halogens, the halogens are fluorine or chlorine;
and/or, when the R⁴⁻¹ is C₁-C₃ alkyl substituted with one or more halogens, the C₁-C₃ alkyl is methyl, ethyl, n-propyl or isopropyl;
and/or, when the R⁴⁻⁴ is a halogen, the halogen is fluorine or chlorine;
and/or, when the R⁴⁻⁴ is C₁-C₃ alkyl, the C₁-C₃ alkyl is methyl, ethyl, n-propyl or isopropyl;
and/or, when the R⁴⁻⁴ is C₁-C₃ alkyl substituted with one or more halogens, the "more" means 2 or 3;
and/or, when the R⁴⁻⁴ is C₁-C₃ alkyl substituted with one or more halogens, the halogens are fluorine or chlorine;
and/or, when the R⁴⁻⁴ is C₁-C₃ alkyl substituted with one or more halogens, the C₁-C₃ alkyl is methyl, ethyl, n-propyl or isopropyl;
and/or, when the ring A is 5- or 6-membered heteroaryl having 1, 2 or 3 heteroatoms selected from N, O and S, the 5- or 6-membered heteroaryl having 1, 2 or 3 heteroatoms selected from N, O and S is a thiophene ring, a pyrrole ring, a pyridine ring or a pyrazine ring;
and/or, when the R⁴⁻⁵ is a halogen, the halogen is fluorine or chlorine;
and/or, when the R⁴⁻⁵ is C₁-C₃ alkyl, the C₁-C₃ alkyl is methyl, ethyl, n-propyl or isopropyl;
and/or, when the R⁴⁻⁵ is C₁-C₃ alkoxy, the C₁-C₃ alkoxy is methoxy, ethoxy, n-propoxy or isopropoxy;
and/or, when the R⁴⁻⁵ is C₁-C₃ alkyl substituted with one or more halogens, the "more" means 2 or 3;
and/or, when the R⁴⁻⁵ is C₁-C₃ alkyl substituted with one or more halogens, the halogens are fluorine or chlorine;
and/or, when the R⁴⁻⁵ is C₁-C₃ alkyl substituted with one or more halogens, the C₁-C₃ alkyl is methyl, ethyl, n-propyl or isopropyl;
and/or, when the R¹ is 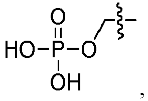 the pharmaceutically acceptable salt is a sodium salt.

6. The fused ring-containing compound represented by formula **II,** the pharmaceutically acceptable salt thereof, the solvate thereof or the solvate of the pharmaceutically acceptable salt thereof according to claim 5,
wherein when the R³ is C₁-C₃ alkyl substituted with one or more halogens, the halogens are fluorine;
and/or, when the R³ is C₁-C₃ alkyl substituted with one or more halogens, the C₁-C₃ alkyl is methyl;
and/or, when the R⁴⁻² is C₁-C₃ alkyl, the C₁-C₃ alkyl is methyl;
and/or, when the R⁴⁻³ is C₁-C₃ alkyl, the C₁-C₃ alkyl is methyl;
and/or, when the R⁴⁻¹ is a halogen, the halogen is chlorine;
and/or, when the R⁴⁻¹ is C₁-C₃ alkyl, the C₁-C₃ alkyl is methyl;
and/or, when the R⁴⁻¹ is C₁-C₃ alkyl substituted with one or more halogens, the halogens are fluorine;
and/or, when the R⁴⁻¹ is C₁-C₃ alkyl substituted with one or more halogens, the C₁-C₃ alkyl is methyl;
and/or, when the R⁴⁻⁴ is a halogen, the halogen is chlorine;
and/or, when the R⁴⁻⁴ is C₁-C₃ alkyl, the C₁-C₃ alkyl is methyl;
and/or, when the R⁴⁻⁴ is C₁-C₃ alkyl substituted with one or more halogens, the halogens are fluorine;
and/or, when the R⁴⁻⁴ is C₁-C₃ alkyl substituted with one or more halogens, the C₁-C₃ alkyl is methyl;
and/or, when the R⁴ is 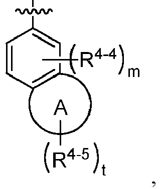 the 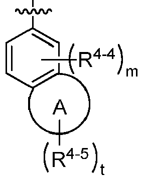 is 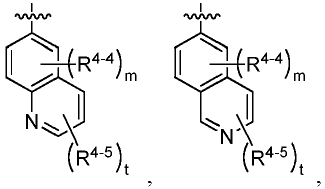 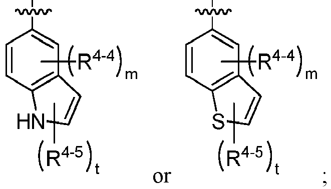
and/or, when the R⁴⁻⁵ is a halogen, the halogen is chlorine;
and/or, when the R⁴⁻⁵ is C₁-C₃ alkyl, the C₁-C₃ alkyl is methyl;
and/or, when the R⁴⁻⁵ is C₁-C₃ alkoxy, the C₁-C₃ alkoxy is methoxy;
and/or, when the R⁴⁻⁵ is C₁-C₃ alkyl substituted with one or more halogens, the halogens are fluorine;
and/or, when the R⁴⁻⁵ is C₁-C₃ alkyl substituted with one or more halogens, the C₁-C₃ alkyl is methyl;
and/or, when the R¹ is 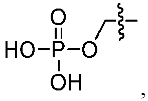 the pharmaceutically acceptable salt is a sodium salt: one or two hydrogens of 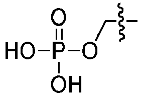 are replaced with sodium ions.

7. The fused ring-containing compound represented by formula **II,** the pharmaceutically acceptable salt thereof, the solvate thereof or the solvate of the pharmaceutically acceptable salt thereof according to claim 6, wherein when the R³ is C₁-C₃ alkyl substituted with multiple halogens, the "C₁-C₃ alkyl substituted with multiple halogens" is trifluoromethyl;
and/or, when the R⁴ is 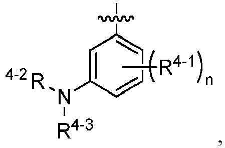 the 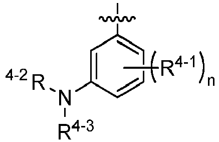 is 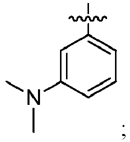
and/or, when the R⁴⁻¹ is C₁-C₃ alkyl substituted with multiple halogens, the "C₁-C₃ alkyl substituted with multiple halogens" is trifluoromethyl;
and/or, when the R⁴⁻⁴ is C₁-C₃ alkyl substituted with multiple halogens, the "C₁-C₃ alkyl substituted with multiple halogens" is trifluoromethyl;
and/or, when the R⁴⁻⁵ is C₁-C₃ alkyl substituted with multiple halogens, the "C₁-C₃ alkyl substituted with multiple halogens" is trifluoromethyl.

8. The fused ring-containing compound represented by formula **II**, the pharmaceutically acceptable salt thereof, the solvate thereof or the solvate of the pharmaceutically acceptable salt thereof according to claim 1, wherein the fused ring-containing compound represented by formula **II** is any one of the following compounds:

| **Compound No.** | **Structural formula** |
|---|---|
| GEN1-284 | 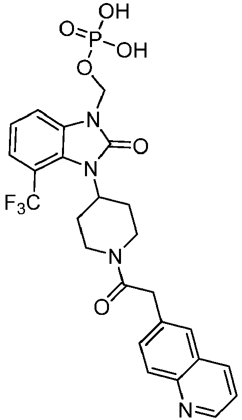 |
| GEN1-284 salt | 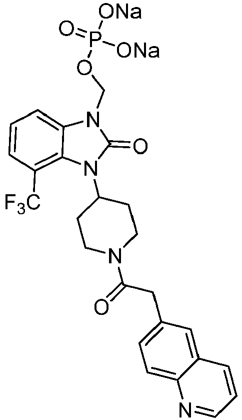 |
| GEN1-295 | 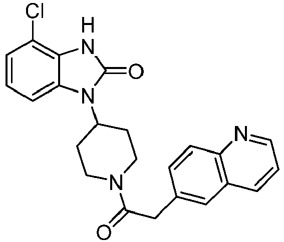 |
| GEN1-296 | 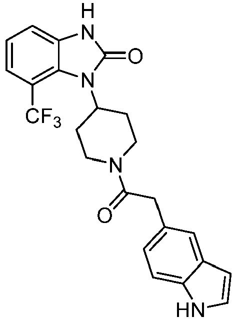 |
| GEN1-297 | 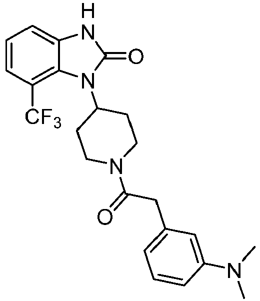 |
| GEN1-298 | 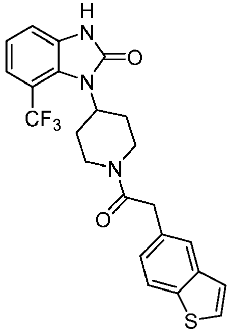 |
| GEN1-299 | 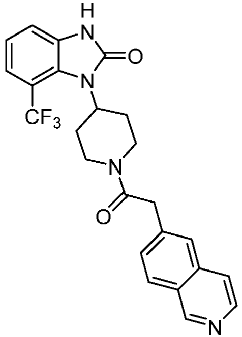 |
| GEN1-300 | 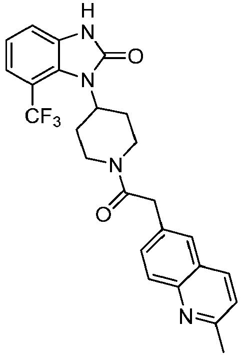 |
| GEN1-301 | 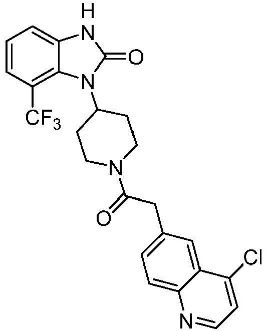 |
| GEN1-302 | 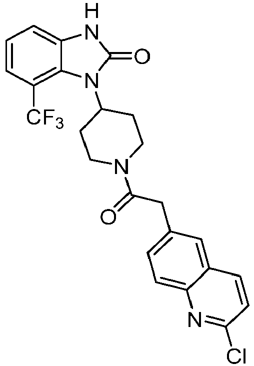 |
| GEN1-303 | 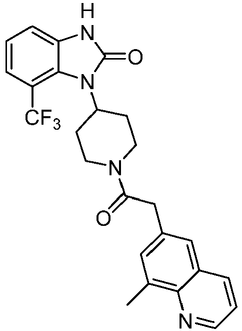 |
| GEN1-304 | 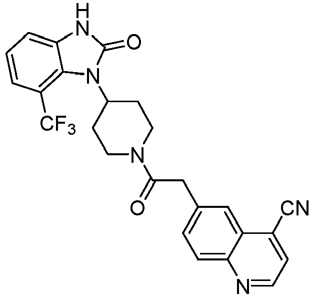 |
| GEN1-305 | 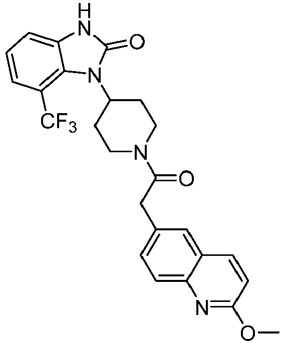 |
| GEN1-306 | 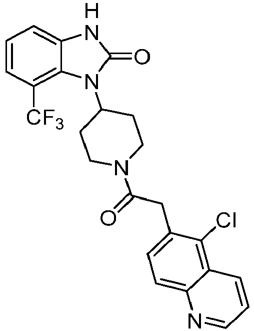 |
| GEN1-307 | 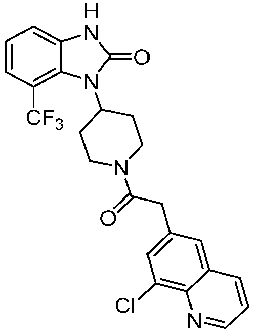 |
| GEN1-308 | 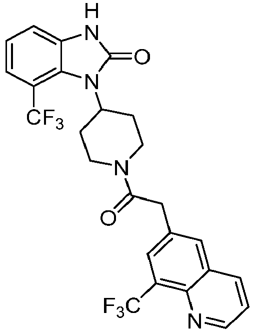 |
| GEN1-309 | 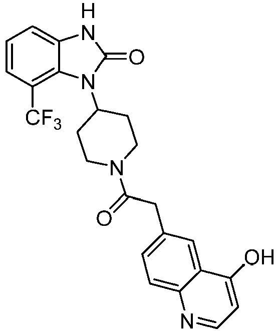 |
| GEN1-310 | 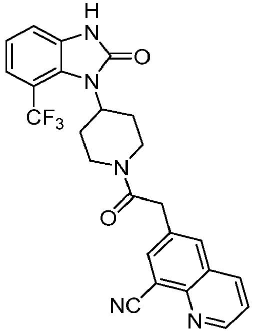 |
| GEN1-311 | 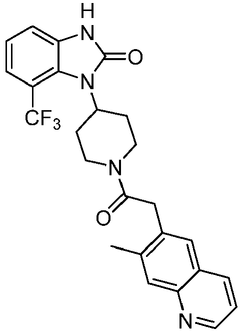 |
| GEN1-312 | 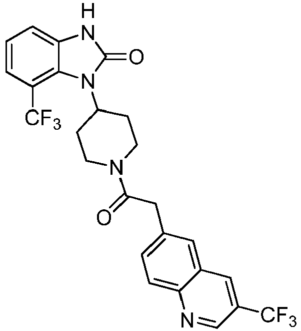 |
| GEN1-313 | 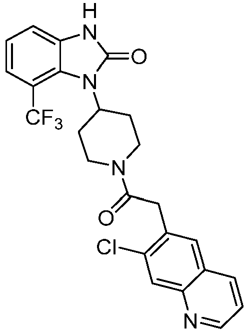 |

and/or, the pharmaceutically acceptable salt of the fused ring-containing compound represented by formula II is 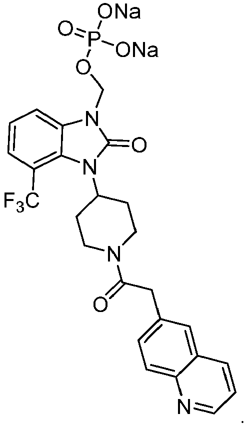
9. A pharmaceutical composition, comprising a substance **Y** and a pharmaceutical adjuvant material, wherein the substance **Y** is the fused ring-containing compound represented by formula **II,** the pharmaceutically acceptable salt thereof, the solvate thereof or the solvate of the pharmaceutically acceptable salt thereof according to any one of claims 1-8.
10. Use of a substance **Y** in the preparation of a STATS inhibitor or a TNF-α generation inhibitor, wherein
the substance **Y** is the fused ring-containing compound represented by formula **II,** the pharmaceutically acceptable salt thereof, the solvate thereof or the solvate of the pharmaceutically acceptable salt thereof according to any one of claims 1-8;
the STATS inhibitor is for use *in vitro;* the TNF-α generation inhibitor is for use *in vitro.*

11. Use of a substance **Y** in the preparation of a medicament, wherein
the substance **Y** is the fused ring-containing compound represented by formula **II**, the pharmaceutically acceptable salt thereof, the solvate thereof or the solvate of the pharmaceutically acceptable salt thereof according to any one of claims 1-8;
the medicament may be:
   (1) a medicament for treating and/or preventing a STAT5-associated disease;
      or,
   (2) a medicament for treating and/or preventing any one of the following diseases: arthritis, atherosclerosis or psoriasis.

12. The use according to claim 11, wherein the use satisfies one or more of the following conditions:
(1) the STAT5-associated disease is arthritis, atherosclerosis or psoriasis;
(2) the arthritis is rheumatoid arthritis, adjuvant arthritis or collagen-induced arthritis; and
(3) the psoriasis is imiquimod-induced psoriasis.

13. A method for treating and/or preventing a STAT5-associated disease, arthritis, atherosclerosis or psoriasis, comprising administering to a patient a therapeutically effective amount of a substance Y, wherein
the substance **Y** is the fused ring-containing compound represented by formula **II,** the pharmaceutically acceptable salt thereof, the solvate thereof or the solvate of the pharmaceutically acceptable salt thereof according to any one of claims 1-8.
14. The method according to claim 13, wherein the method satisfies one or more of the following conditions:
(1) the STAT5-associated disease is arthritis, atherosclerosis or psoriasis;
(2) the arthritis is rheumatoid arthritis, adjuvant arthritis or collagen-induced arthritis; and
(3) the psoriasis is imiquimod-induced psoriasis.

## Claims

1. A fused ring-containing compound represented by formula **II,** a pharmaceutically acceptable salt thereof, a solvate thereof or a solvate of the pharmaceutically acceptable salt thereof, wherein: 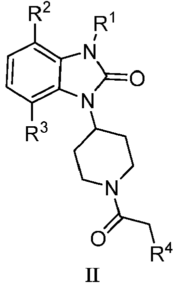
R¹ is hydrogen or 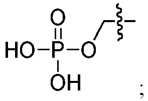
R² is hydrogen or chlorine;
R³ is hydrogen, cyano, nitro, acetyl, trifluoromethanesulfonyl, or C₁-C₃ alkyl substituted with one or more halogens;
R⁴ is 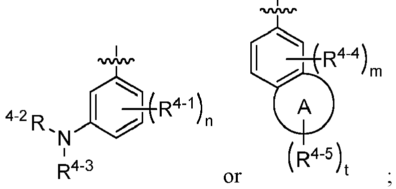
n is 0, 1, 2, 3 or 4;
R⁴⁻¹ is independently cyano, a halogen, C₁-C₃ alkyl, or C₁-C₃ alkyl substituted with one or more halogens;
R⁴⁻² and R⁴⁻³ are independently C₁-C₃ alkyl;
m is 0, 1, 2, 3 or 4;
R⁴⁻⁴ is independently cyano, hydroxy, a halogen, C₁-C₃ alkyl, C₁-C₃ alkoxy, or C₁-C₃ alkyl substituted with one or more halogens;
ring A is 5- or 6-membered heteroaryl having 1, 2 or 3 heteroatoms selected from one or more of N, O and S;
t is 0, 1, 2, 3 or 4;
R⁴⁻⁵ is independently cyano, hydroxy, a halogen, C₁-C₃ alkyl, C₁-C₃ alkoxy, or C₁-C₃ alkyl substituted with one or more halogens;
however, the fused ring-containing compound represented by formula **II** is not the following compounds:

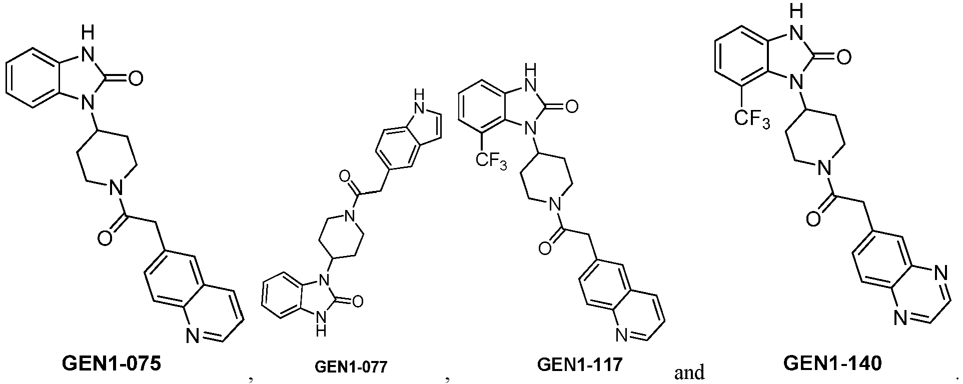

2. The fused ring-containing compound represented by formula **II,** the pharmaceutically acceptable salt thereof, the solvate thereof or the solvate of the pharmaceutically acceptable salt thereof according to claim 1, wherein R² is hydrogen;
and/or, R¹ is 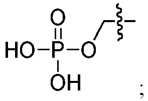
and/or, R³ is C₁-C₃ alkyl substituted with one or more halogens;
and/or, n is 0;
and/or, R⁴ is 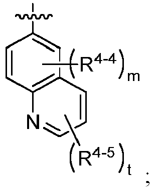
and/or, m is 0 or 1;
and/or, R⁴⁻⁴ is independently a halogen or C₁-C₃ alkyl;
and/or, t is 0 or 1;
and/or, R⁴⁻⁵ is independently cyano, a halogen, C₁-C₃ alkyl, C₁-C₃ alkoxy, or C₁-C₃ alkyl substituted with one or more halogens;
and/or, when the R¹ is hydrogen, m + t is greater than or equal to 1.

3. The fused ring-containing compound represented by formula **II,** the pharmaceutically acceptable salt thereof, the solvate thereof or the solvate of the pharmaceutically acceptable salt thereof according to claim 2,
wherein R⁴⁻⁵ is independently a halogen;
and/or, when the R¹ is hydrogen, m + t = 1.

4. The fused ring-containing compound represented by formula **II,** the pharmaceutically acceptable salt thereof, the solvate thereof or the solvate of the pharmaceutically acceptable salt thereof according to claim 1, being defined as described in any one of the following schemes:
scheme 1, wherein R¹ is hydrogen or 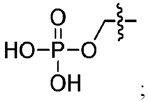 R² is hydrogen; R³ is C₁-C₃ alkyl substituted with one or more halogens; R⁴ is 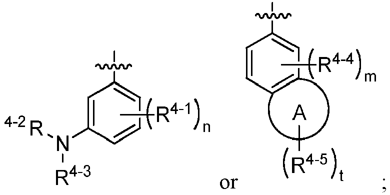 n is 0; R⁴⁻² and R⁴⁻³ are independently C₁-C₃ alkyl; m is 0 or 1; R⁴⁻⁴ is a halogen or C₁-C₃ alkyl; ring A is 5- or 6-membered heteroaryl having 1, 2 or 3 heteroatoms selected from one or more of N, O and S; t is 0 or 1; R⁴⁻⁵ is cyano, a halogen, C₁-C₃ alkyl, C₁-C₃ alkoxy, or C₁-C₃ alkyl substituted with one or more halogens; however, the fused ring-containing compound represented by formula **II** is not the following compounds: 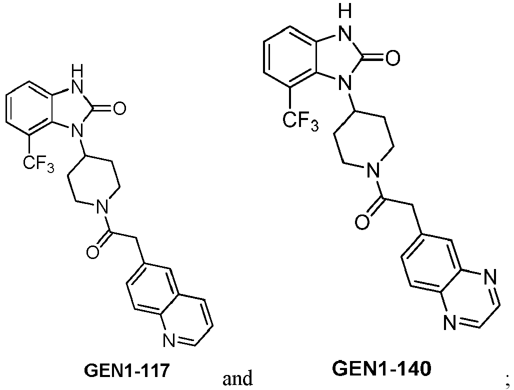 scheme 2, wherein R¹ is hydrogen or 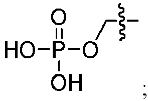 R² is hydrogen; R³ is C₁-C₃ alkyl substituted with one or more halogens; R⁴ is 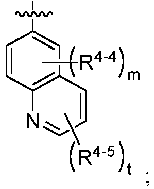 m is 0 or 1; R⁴⁻⁴ is a halogen or C₁-C₃ alkyl; t is 0 or 1; R⁴⁻⁵ is a halogen; however, the fused ring-containing compound represented by formula **II** is not 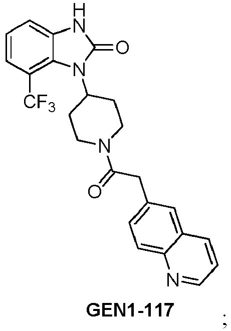 scheme 3, wherein R¹ is 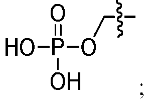 R² is hydrogen; R³ is C₁-C₃ alkyl substituted with one or more halogens; R⁴ is 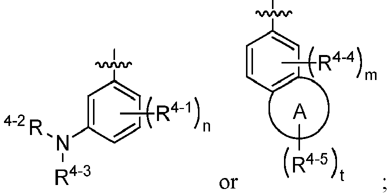 n is 0; R⁴⁻² and R⁴⁻³ are independently C₁-C₃ alkyl;
m is 0 or 1; R⁴⁻⁴ is a halogen or C₁-C₃ alkyl; ring A is 5- or 6-membered heteroaryl having 1, 2 or 3 heteroatoms selected from one or more of N, O and S; t is 0 or 1; R⁴⁻⁵ is cyano, a halogen, C₁-C₃ alkyl, C₁-C₃ alkoxy, or C₁-C₃ alkyl substituted with one or more halogens; and
scheme 4, wherein R¹ is 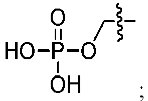 R² is hydrogen; R³ is C₁-C₃ alkyl substituted with one or more halogens; R⁴ is 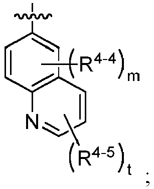 m is 0 or 1; R⁴⁻⁴ is a halogen or C₁-C₃ alkyl; t is 0 or 1; R⁴⁻⁵ is a halogen.

5. The fused ring-containing compound represented by formula II, the pharmaceutically acceptable salt thereof, the solvate thereof or the solvate of the pharmaceutically acceptable salt thereof according to any one of claims 1-4, wherein when the R³ is C₁-C₃ alkyl substituted with one or more halogens, the "more" means 2 or 3;
and/or, when the R³ is C₁-C₃ alkyl substituted with one or more halogens, the halogens are fluorine or chlorine;
and/or, when the R³ is C₁-C₃ alkyl substituted with one or more halogens, the C₁-C₃ alkyl is methyl, ethyl, n-propyl or isopropyl;
and/or, when the R⁴⁻² is C₁-C₃ alkyl, the C₁-C₃ alkyl is methyl, ethyl, n-propyl or isopropyl;
and/or, when the R⁴⁻³ is C₁-C₃ alkyl, the C₁-C₃ alkyl is methyl, ethyl, n-propyl or isopropyl;
and/or, when the R⁴⁻¹ is a halogen, the halogen is fluorine or chlorine;
and/or, when the R⁴⁻¹ is C₁-C₃ alkyl, the C₁-C₃ alkyl is methyl, ethyl, n-propyl or isopropyl;
and/or, when the R⁴⁻¹ is C₁-C₃ alkyl substituted with one or more halogens, the "more" means 2 or 3;
and/or, when the R⁴⁻¹ is C₁-C₃ alkyl substituted with one or more halogens, the halogens are fluorine or chlorine;
and/or, when the R⁴⁻¹ is C₁-C₃ alkyl substituted with one or more halogens, the C₁-C₃ alkyl is methyl, ethyl, n-propyl or isopropyl;
and/or, when the R⁴⁻⁴ is a halogen, the halogen is fluorine or chlorine;
and/or, when the R⁴⁻⁴ is C₁-C₃ alkyl, the C₁-C₃ alkyl is methyl, ethyl, n-propyl or isopropyl;
and/or, when the R⁴⁻⁴ is C₁-C₃ alkyl substituted with one or more halogens, the "more" means 2 or 3;
and/or, when the R⁴⁻⁴ is C₁-C₃ alkyl substituted with one or more halogens, the halogens are fluorine or chlorine;
and/or, when the R⁴⁻⁴ is C₁-C₃ alkyl substituted with one or more halogens, the C₁-C₃ alkyl is methyl, ethyl, n-propyl or isopropyl;
and/or, when the ring A is 5- or 6-membered heteroaryl having 1, 2 or 3 heteroatoms selected from N, O and S, the 5- or 6-membered heteroaryl having 1, 2 or 3 heteroatoms selected from N, O and S is a thiophene ring, a pyrrole ring, a pyridine ring or a pyrazine ring;
and/or, when the R⁴⁻⁵ is a halogen, the halogen is fluorine or chlorine;
and/or, when the R⁴⁻⁵ is C₁-C₃ alkyl, the C₁-C₃ alkyl is methyl, ethyl, n-propyl or isopropyl;
and/or, when the R⁴⁻⁵ is C₁-C₃ alkoxy, the C₁-C₃ alkoxy is methoxy, ethoxy, n-propoxy or isopropoxy;
and/or, when the R⁴⁻⁵ is C₁-C₃ alkyl substituted with one or more halogens, the "more" means 2 or 3;
and/or, when the R⁴⁻⁵ is C₁-C₃ alkyl substituted with one or more halogens, the halogens are fluorine or chlorine;
and/or, when the R⁴⁻⁵ is C₁-C₃ alkyl substituted with one or more halogens, the C₁-C₃ alkyl is methyl, ethyl, n-propyl or isopropyl;
and/or, when the R¹ is 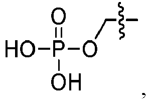 the pharmaceutically acceptable salt is a sodium salt.

6. The fused ring-containing compound represented by formula II, the pharmaceutically acceptable salt thereof, the solvate thereof or the solvate of the pharmaceutically acceptable salt thereof according to claim 5,
wherein when the R³ is C₁-C₃ alkyl substituted with one or more halogens, the halogens are fluorine;
and/or, when the R³ is C₁-C₃ alkyl substituted with one or more halogens, the C₁-C₃ alkyl is methyl;
and/or, when the R⁴⁻² is C₁-C₃ alkyl, the C₁-C₃ alkyl is methyl;
and/or, when the R⁴⁻³ is C₁-C₃ alkyl, the C₁-C₃ alkyl is methyl;
and/or, when the R⁴⁻¹ is a halogen, the halogen is chlorine;
and/or, when the R⁴⁻¹ is C₁-C₃ alkyl, the C₁-C₃ alkyl is methyl;
and/or, when the R⁴⁻¹ is C₁-C₃ alkyl substituted with one or more halogens, the halogens are fluorine;
and/or, when the R⁴⁻¹ is C₁-C₃ alkyl substituted with one or more halogens, the C₁-C₃ alkyl is methyl;
and/or, when the R⁴⁻⁴ is a halogen, the halogen is chlorine;
and/or, when the R⁴⁻⁴ is C₁-C₃ alkyl, the C₁-C₃ alkyl is methyl;
and/or, when the R⁴⁻⁴ is C₁-C₃ alkyl substituted with one or more halogens, the halogens are fluorine;
and/or, when the R⁴⁻⁴ is C₁-C₃ alkyl substituted with one or more halogens, the C₁-C₃ alkyl is methyl;
and/or, when the R⁴ is 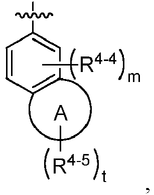 the 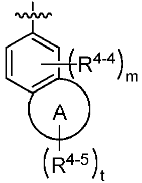 is 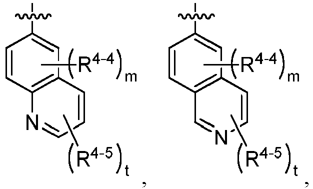 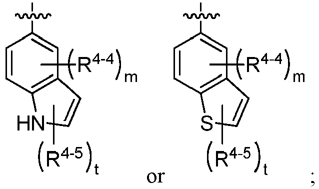
and/or, when the R⁴⁻⁵ is a halogen, the halogen is chlorine;
and/or, when the R⁴⁻⁵ is C₁-C₃ alkyl, the C₁-C₃ alkyl is methyl;
and/or, when the R⁴⁻⁵ is C₁-C₃ alkoxy, the C₁-C₃ alkoxy is methoxy;
and/or, when the R⁴⁻⁵ is C₁-C₃ alkyl substituted with one or more halogens, the halogens are fluorine;
and/or, when the R⁴⁻⁵ is C₁-C₃ alkyl substituted with one or more halogens, the C₁-C₃ alkyl is methyl;
and/or, when the R¹ is 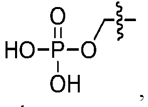 the pharmaceutically acceptable salt is a sodium salt: one or two hydrogens of 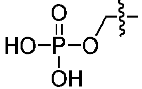 are replaced with sodium ions.

7. The fused ring-containing compound represented by formula II, the pharmaceutically acceptable salt thereof, the solvate thereof or the solvate of the pharmaceutically acceptable salt thereof according to claim 6, wherein when the R³ is C₁-C₃ alkyl substituted with multiple halogens, the "C₁-C₃ alkyl substituted with multiple halogens" is trifluoromethyl;
and/or, when the R⁴ is 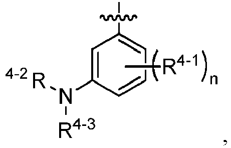 the 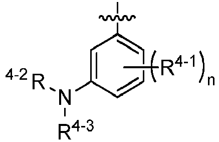 is 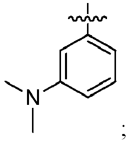
and/or, when the R⁴⁻¹ is C₁-C₃ alkyl substituted with multiple halogens, the "C₁-C₃ alkyl substituted with multiple halogens" is trifluoromethyl;
and/or, when the R⁴⁻⁴ is C₁-C₃ alkyl substituted with multiple halogens, the "C₁-C₃ alkyl substituted with multiple halogens" is trifluoromethyl;
and/or, when the R⁴⁻⁵ is C₁-C₃ alkyl substituted with multiple halogens, the "C₁-C₃ alkyl substituted with multiple halogens" is trifluoromethyl.

8. The fused ring-containing compound represented by formula II, the pharmaceutically acceptable salt thereof, the solvate thereof or the solvate of the pharmaceutically acceptable salt thereof according to claim 1, wherein the fused ring-containing compound represented by formula II is any one of the following compounds:
| **Compound No.** | **Structural formula** |
|---|---|
| GEN1-284 | 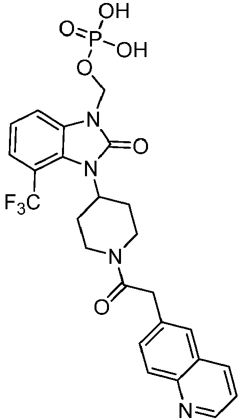 |
| GEN1-284 salt | 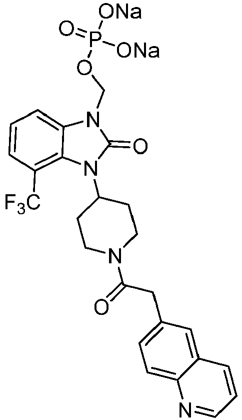 |
| GEN1-295 | 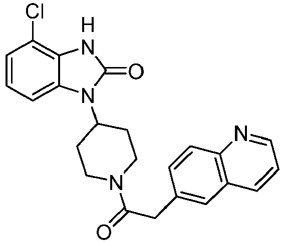 |
| GEN1-296 | 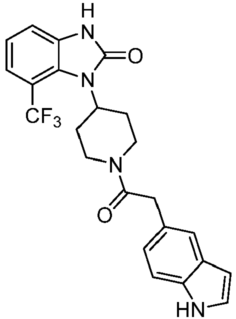 |
| GEN1-297 | 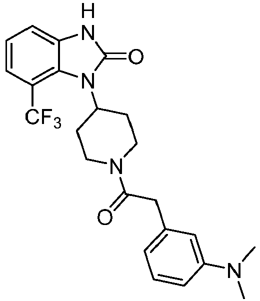 |
| GEN1-298 | 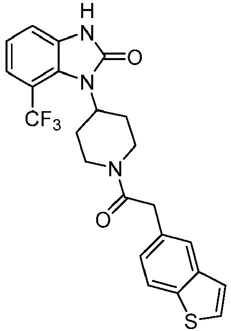 |
| GEN1-299 | 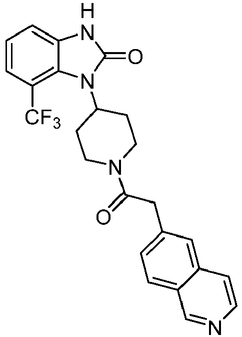 |
| GEN1-300 | 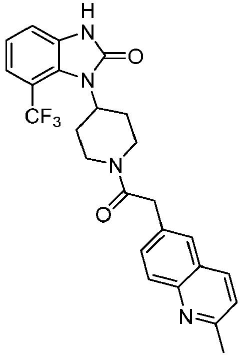 |
| GEN1-301 | 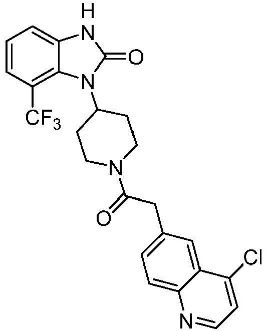 |
| GEN1-302 | 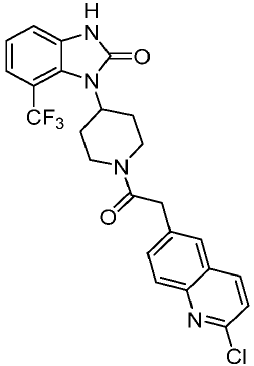 |
| GEN1-303 | 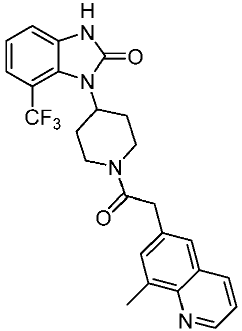 |
| GEN1-304 | 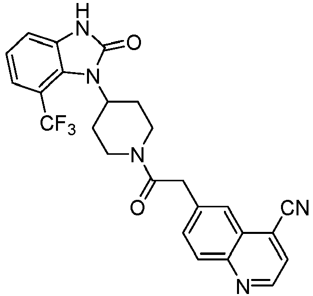 |
| GEN1-305 | 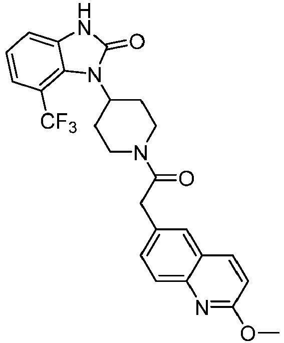 |
| GEN1-306 | 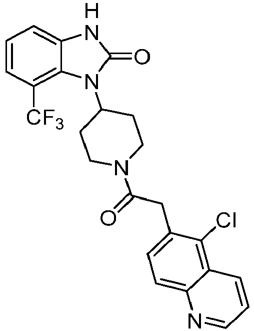 |
| GEN1-307 | 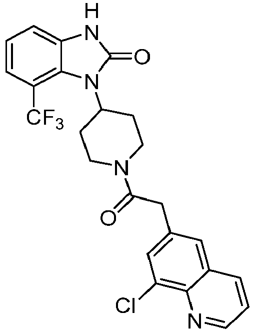 |
| GEN1-308 | 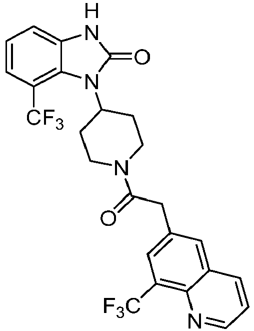 |
| GEN1-309 | 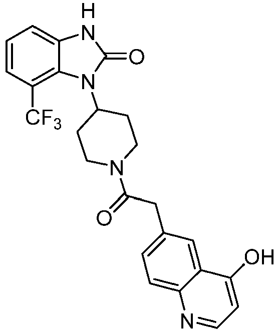 |
| GEN1-310 | 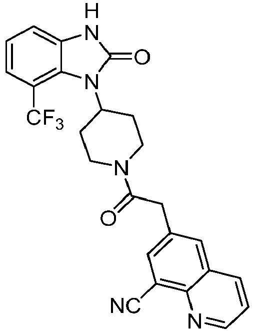 |
| GEN1-311 | 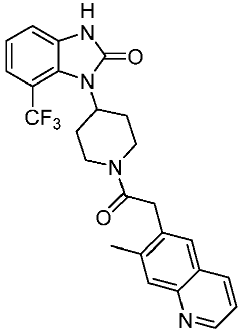 |
| GEN1-312 | 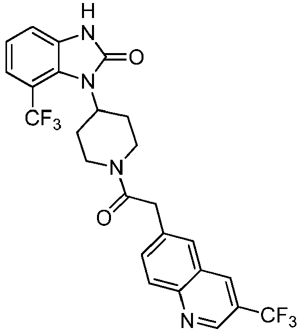 |
| GEN1-313 | 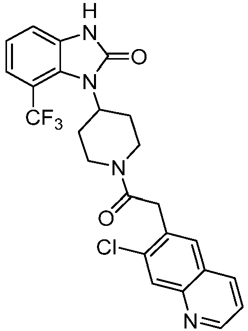 |
and/or, the pharmaceutically acceptable salt of the fused ring-containing compound represented by formula II is

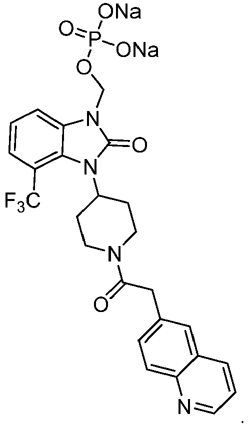

9. A pharmaceutical composition, comprising a substance **Y** and a pharmaceutical adjuvant material, wherein the substance **Y** is the fused ring-containing compound represented by formula **II,** the pharmaceutically acceptable salt thereof, the solvate thereof or the solvate of the pharmaceutically acceptable salt thereof according to any one of claims 1-8.

10. Use of a substance **Y** in the preparation of a STATS inhibitor or a TNF-α generation inhibitor, wherein the substance **Y** is the fused ring-containing compound represented by formula **II,** the pharmaceutically acceptable salt thereof, the solvate thereof or the solvate of the pharmaceutically acceptable salt thereof according to any one of claims 1-8;
the STATS inhibitor is for use *in vitro;* the TNF-α generation inhibitor is for use *in vitro.*

11. Use of a substance **Y** in the preparation of a medicament, wherein
the substance **Y** is the fused ring-containing compound represented by formula **II,** the pharmaceutically acceptable salt thereof, the solvate thereof or the solvate of the pharmaceutically acceptable salt thereof according to any one of claims 1-8;
the medicament may be:
(1) a medicament for treating and/or preventing a STATS-associated disease;
or,
(2) a medicament for treating and/or preventing any one of the following diseases: arthritis, atherosclerosis or psoriasis.

12. The use according to claim 11, wherein the use satisfies one or more of the following conditions:
(1) the STAT5-associated disease is arthritis, atherosclerosis or psoriasis;
(2) the arthritis is rheumatoid arthritis, adjuvant arthritis or collagen-induced arthritis; and
(3) the psoriasis is imiquimod-induced psoriasis.

13. A method for treating and/or preventing a STAT5-associated disease, arthritis, atherosclerosis or psoriasis, comprising administering to a patient a therapeutically effective amount of a substance **Y,** wherein
the substance **Y** is the fused ring-containing compound represented by formula **II,** the pharmaceutically acceptable salt thereof, the solvate thereof or the solvate of the pharmaceutically acceptable salt thereof according to any one of claims 1-8.

14. The method according to claim 13, wherein the method satisfies one or more of the following conditions:
(1) the STAT5-associated disease is arthritis, atherosclerosis or psoriasis;
(2) the arthritis is rheumatoid arthritis, adjuvant arthritis or collagen-induced arthritis; and
(3) the psoriasis is imiquimod-induced psoriasis.
